# EUROPEAN PATENT APPLICATION

(11) **EP 2 385 066 A1**
(43) Date of publication of application: **09.11.2011**
(21) Application number: 11170225.4
(22) Date of filing: 16.03.2006
(51) Int. Cl.: C07K 14/74, A61K 39/00, A61K 39/39

(54) **Recombinant MHC Molecules Useful for Manipulation of Antigen-Specific T Cells**

(30) Priority: 18.03.2005 US 663048 P; 31.08.2005 US 713230 P; 10.03.2006 US 373047
(62) Divisional of application: 06748442.8
(71) Applicant: Oregon Health & Science University, Portland, OR 97201-4753 (US)
(72) Inventor: Vandenbark, Arthur A., Portland, OR Oregon 97220-8063 (US); Offner, Halina, Portland, OR Oregon 97220-8063 (US); Burrows, Gregory G., Portland, OR Oregon 97221-3506 (US)
(74) Representative: Atkinson, Jennifer

(57) **Abstract**

Two-domain MHC polypeptides are useful for modulating activities of antigen-specific T-cells, including for modulating pathogenic potential and effects of antigen-specific T-cells. Exemplary MHC class II-based recombinant T-cell ligands (RTLs) of the invention include covalently linked ss1 and a1 domains, and MHC class I-based molecules that comprise covalently linked a1 and a2 domains. These polypeptides may also include covalently linked antigenic determinants, toxic moieties, and/or detectable labels.; The disclosed polypeptides can be used to target antigen-specific T-cells, and are useful, among others things, to detect and purify antigen-specific T-cells, to induce or activate T-cells, to modulate T-cell activity, including by regulatory switching of T-cell cytokine and adhesion molecule expression, to treat conditions mediated by antigen- specific T-cells, to treat or prevent autoimmune or neurodegenerative diseases, to protect axons, and to prevent or reverse demyelination.

## Description

### STATEMENT REGARDING GOVERNMENT SPONSORED RESEARCH

Aspects of this work were supported by grants from the National Institutes of Health (A143960, ES105541., NS41965, 5R42NS046877, and 1R01NS047661), the National Multiple Sclerosis Society (RG3012A and RG3468), and the Department of Veterans Affair. The United States government has certain rights in the subject matter.

### CROSS REFERENCES TO RELATED APPLICATIONS

This application is related to and claims all priority rights from United States Provisional Application No. 60/663,048, filed March 18, 2005, and United States Provisional Application No. 60/713,230, filed August 31,2005, and United States Non-Provisional Application entitled "Recombinant MHC Molecules Useful four Manipulation Of Antigen-Specific T Cells" filed March 10, 2006 under Attorney Docket No. OHSU-1-0403, each of which priority applications is incorporated herein in its entirety by reference.

### TECHNICAL FIELD

The present invention relates to recombinant polypeptides comprising major histocompatibility complex (MHC) molecular domains that mediate antigen binding and T-cell receptor (TCR) recognition, and to related compositions and methods incorporating and employing these recombinant polypeptides.

### BACKGROUND OF THE INVENTION

The initiation of an immune response against a specific antigen in mammals is brought about by the presentation of that antigen to T-cells by a major histocompatibility (MHC) complex. MHC complexes are located on the surface of antigen presenting cells (APCs); the 3-dimensional structure of MHCs includes a groove or cleft into which the presented antigen fits. When an appropriate receptor on a T-cell interacts with the MHC/antigen complex on an APC in the presence of necessary co-stimulatory signals, the T-cell is stimulated, triggering various aspects of the well characterized cascade of immune system activation events, including induction of

There are two basic classes of MHC molecules in mammals, MHC class I and MHC class II. Both classes are large protein complexes formed by association of two separate proteins. Each class includes transmembrane domains that anchor the complex into the cell membrane. MHC class I molecules are formed from two not-covalently associated proteins, the α chain and β2-microglobulin. The α chain comprises three distinct domains, α1, α2 and α3. The three-dimensional structure of the α1 and α2 domains forms the groove into which antigen fit for presentation to T-cells. The α3 domain is an Ig-fold like domain that contains a transmembrane sequence that anchors the α chain into the cell membrane of the APC. MHC class I complexes, when associated with antigen (and in the presence of appropriate co-stimulatory signals) stimulate CD8 cytotoxic T-cells, which function to kill any cell which they specifically recognize.

The two proteins which associate non-covalently to form MHC class II molecules are termed the α and β chains. The α chain comprises α1 and α2 domains, and the β chain comprises β1 and β2 domains. The cleft into which the antigen fits is formed by the interaction of the α1 and β1 domains. The α2 and β2 domains are transmembrane Ig-fold like domains that anchor the α and β chains into the cell membrane of the APC. MHC class If complexes, when associated with antigen (and in the presence of appropriate co-stimulatory signals) stimulate CD4 T-cells. The primary functions of CD4 T-cells are to initiate the inflammatory response, to regulate other cells in the immune system, and to provide help to B cells for antibody synthesis.

The genes encoding the various proteins that constitute the MHC complexes have been extensively studied in humans and other mammals. In humans, MHC molecules (with the exception of class I β2-microglobulin) are encoded by the HLA region, which is located on chromosome 6 and constitute over 100 genes. There are 3 class I MHC α chain protein loci, termed HLA-A, -B and -C. There are also 3 pairs of class II MHC α and β chain loci, termed HLA-DR (A and B), HLA-DP (A and B), and HLA-DQ (A and B). In rats, the class I α gene is termed RT1.A, while the class II genes are termed RT1.B α and RT1.B β. More detailed background information on the structure, function and genetics of MHC complexes can be found in Immunobiology: The Immune System in Health and Disease by Janeway and Travers, Current Biology Ltd./Garland Publishing, Inc. (1997) (ISBN 0-8153-2818-4), and in Bodmer et al. (1994) "Nomenclature for factors of the HLA system" Tissue Antigens vol. 44, pages 1-18.

The key role that MHC complexes play in triggering immune recognition has led to the development of methods by which these complexes are used to modulate the immune response. For example, activated T-cells which recognize "self" antigens (autoantigens) are known to play a key role in autoimmune diseases and neurodegenerative diseases (such as rheumatoid arthritis and multiple sclerosis). Building on the observation that isolated MHC class II molecules (loaded with the appropriate antigen) can substitute for APCs carrying the MHC class II complex and can bind to antigen-specific T-cells, a number of researchers have proposed that isolated MHC/antigcn complexes may be used to treat autoimmune disorders. Thus U.S. Patent Nos. 5,194,425 (Sharma et al.), and 5,284,935 (Clark et al.), disclose the use of isolated MHC class II complexes loaded with a specified autoantigen and conjugated to a toxin to eliminate T-cells that are specifically immunorcactive with autoantigens. In another context, it has been shown that the interaction of isolated MHC II/antigen complexes with T-cells, in the absence of co-stimulatory factors, induces a state of non-responsiveness known as anergy. (Quill et al., J. Immunol., 138:3704-3712(1987)). Following this observation, Sharma et al. (U.S. Patent Nos. 5,468,481 and 5,130,297) and Clark et al. (U.S. Patent No. 5,260,422) have suggested that such isolated MHC II/antigen complexes may be administered therapeutically to anergize T-cell lines which specifically respond to particular autoantigenic peptides.

Methods for using isolated MHC complexes in the detection, quantification and purification of T-cells which recognize particular antigens have been studied for use in diagnostic and therapeutic applications. By way of example, early detection of T-cells specific for a particular autoantigen would facilitate the early selection of appropriate treatment regimes. The ability to purify antigen-specific T-cells would also be of great value in adoptive immunotherapy. Adoptive immunotherapy involves the removal of T-cells from a cancer patient, expansion of the T-cells *in vitro* and then reintroduction of the cells to the patient (see U.S. Patent No. 4,690,915 to Rosenberg et al.; Rosenberg et al. New Engl. J. Med. 319:1676-1680 (1988)). Isolation and expansion of cancer specific T-cells with inflammatory properties would increase the specificity and effectiveness of such an approach.

To date, however, attempts to detect, quantify or purify antigen specific T-using isolated MHC/antigen complexes have not met with widespread success because, among other reasons, binding between the T-cells and such isolated complexes is transient and hence the T-cell/MHC/antigen complex is unstable. In an attempt to address these problems, Altman et al. (Science 274, 94-96 (1996) and U.S. Patent No. 5,635,363) proposed the use of large, covalently linked multimeric structures of MHC/antigen complexes to stabilize this interaction by simultaneously binding to multiple T-cell receptors on a target T-cell. However, these complexes are large making them difficult to produce and use.

Although the concept of using isolated MHC/antigen complexes in therapeutic and diagnostic applications holds great promise, current methods are not optimal. For example, while the complexes can be isolated from lymphocytes by detergent extraction, such procedures are inefficient and yield only small amounts of protein. Additionally, even though the cloning of the genes encoding the various MHC complex subunits has facilitated the production of large quantities of the individual subunits through expression in prokaryotic cells, the assembly of the individual subunits into MHC complexes having the appropriate conformational structure has proven difficult.

There is therefore an unmet need in the art for methods and compositions for isolating useable MHC/antigen complexes.

It is an object of the present invention to isolate MHC/antigen complexes.

It is another object of the present invention to provide recombinant polypeptides comprising MHC molecular domains that mediate antigen binding and T-cell receptor recognition.

It is further object of the present invention to provide compositions and methods for the detection, quantification, and purification of antigen-specific T-cells.

It is yet another object of the present invention to provide methods and compositions for modulating T-cell activity.

It is an additional object of the present invention to provide compositions and methods for treating T-cell mediated diseases.

It is a further object of the present invention to provide compositions and methods for treating autoimmune diseases.

It is yet another object of the present invention to provide compositions and methods for treating neurodegenerative diseases.

### SUMMARY OF EXEMPLARY EMBODIMENTS

This invention is founded on the discovery that mammalian MHC function, including but not limited to, human MHC function, can be mimicked through the use of recombinant polypeptides that include only those domains of MHC molecules that define the antigen binding cleft. These molecules are useful in the detection, quantification and purification of antigen-specific T-cells. The molecules provided herein may also be used in clinical and laboratory applications to detect, quantify and purify antigen-specific T-cells, induce anergy in T-cells, or to induce T suppressor cells, as well as to stimulate T-cells, and to treat diseases mediated by antigen-specific T-cells, including, but not limited to, autoimmune and neurodegenerative diseases.

It is shown herein that antigen-specific T-cell binding can be accomplished with a monomeric molecule comprising, in the case of human class II MHC molecules, only the α1 and β1 domains in covalent linkage (and in some examples in association with an antigenic determinant). For convenience, such MHC class II polypeptides are hereinafter referred to as "β1α1". Equivalent molecules derived from human MHC class I molecules are also provided herein. Such molecules comprise the α1 and α2 domains of class I molecules in covalent linkage and in association with an antigenic determinant. Such MHC class I polypeptides are referred to as "α1α2". These two domain molecules may be readily produced by recombinant expression in prokaryotic or eukaryotic cells, and readily purified in large quantities. Moreover, these molecules may easily be loaded with any desired peptide antigen, making production of a repertoire of MHC molecules with different T-ccll specificities a simple task.

Additionally, it is shown that despite lacking the Ig fold domains and transmembrane portions that are part of intact MHC molecules, these two domain MHC molecules refold in a manner that is structurally analogous to "whole" MHC molecules, and bind peptide antigens to form stable MHC/antigen complexes. Moreover, these two domain MHC/cpitope complexes bind T-cells in an epitope-specific manner, and inhibit cpitope-specific T-cell proliferation *in vitro.* In addition, administration of human β1α1 molecules loaded with an antigenic epitope, including, but not limited to, for example an epitope of myelin basic protein (MBP), induces a variety of T-cell transduction processes and modulates effector functions, including the cytokine and proliferation response. Thus, the two domain MHC molecules display powerful and epitope-specific effects on T-cell activation resulting in secretion of anti-inflammatory cytokines. As a result the disclosed MHC molecules are useful in a wide range of both *in viv*o and *in vitro* applications.

Various formulations of human two domain molecules are provided by the invention. In their most basic form, human two domain MHC class II molecules comprise β1 and α1 domains of a mammalian MHC class II molecule wherein the amino terminus of the α1 domain is covalently linked to the carboxy terminus of the β1 domain and wherein the polypeptide does not include the α2 or β2 domains. The human two domain MHC class I molecules comprise α1 and α2 domains of a mammalian class I molecule, wherein the amino terminus of the α2 domain is covalently linked to the carboxy terminus of the α1 domain, and wherein the polypeptide does not include an MHC class I α3 domain. For most applications, these molecules are associated, by covalent or non-covalent interaction, with an antigenic determinant, such as a peptide antigen. In certain embodiments, the peptide antigen is covalently linked to the amino terminus of the β1 domain of the class II molecules, or the α1 domain of the class I molecules. The two domain molecules may also comprise a detectable marker, such as a fluorescent label or a toxic moiety, such as ricin A, or an antigen, such as myelin basic protein (MBP), proteolipid protein (PLP), myelin oligodedrocyte glycoprotein, insulin, glutamate decarboxylase, type II Collagen, thyroglobulin, thyrodoxin, S-antigen, ach (acetylcholine) receptor, HepB antigen, pertussis toxin, myosin B, Ross River Virus, recombinant murine TPO (rmTPO), lipopolysaccharide (LPS), or antiglomerular basement membrane (anti-GMB).

Also provided are nucleic acid molecules that encode the human two domain MHC molecules, as well as expression vectors that may be conveniently used to express these molecules. In particular embodiments, the nucleic acid molecules include sequences that encode the antigenic peptide as well as the human two domain MHC molecule. For example, one such nucleic acid molecule may be represented by the formula Pr-P-B-A, wherein Pr is a promoter sequence operably linked to P (a sequence encoding the peptide antigen), B is the class I α1 or the class II β1 domain, and A is the class I α2 domain or the class II α1 domain. In these nucleic acid molecules, P, B and A comprise a single open reading frame, such that the peptide and the two human MHC domains are expressed as a single polypeptide chain. In one embodiment, B and A arc connected by a linker.

The two domain molecules may also be used *in vivo* to target specified antigen-specific T-cells. By way of example, a β1α1 molecule loaded with a portion of myelin basic protein (MBP) and administered to patients suffering from multiple sclerosis may be used to induce anergy in MBP-specific T-cells, or to induce suppressor T-cells, thus alleviating the disease symptoms. Alternatively, such molecules may be conjugated with a toxic moiety to more directly kill the disease-causing T-cells.

*In vitro*, the human two domain MHC molecules may be used to detect and quantify T-cells, and regulate T-cell function. Thus, such molecules loaded with a selected antigen may be used to detect, monitor and quantify a population of T-cells that are specific for that antigen. The ability to do this is beneficial in a number of clinical settings, such as monitoring the number of tumor antigen-specific T-cells in blood removed from a cancer patient, or the number of self-antigen specific T-cells in blood removed from a patient suffering from an autoimmune disease and/or neurodegenerative disease. In these contexts, the disclosed molecules are powerful tools for monitoring the progress of a particular therapy. In addition to monitoring and quantifying antigen-specific T-cells, the disclosed molecules may also be used to purify such cells for adoptive immunotherapy. In one specific, non-limiting example, the disclosed human MHC molecules loaded with a tumor antigen may be used to purify tumor-antigen specific T-cells from a cancer patient. These cells may then be expanded *in vitro* before being returned to the patient as part of a cancer treatment. When conjugated with a toxic moiety, the two domain molecules may be used to kill T-cells having a particular antigen specificity. Alternatively, the molecules may also be used to induce anergy in such T-cells, or to induce suppressor T-cells.

The methods and compositions of the present invention may additionally be used in the treatment of mammalian subjects including, but not limited to, humans and other mammalian subjects suffering from T-cell mediated diseases, including but not limited to auto-immune diseases, graft rejection, graft versus host disease, an unwanted delayed-type hypersensitivity reaction, or a T-cell mediated pulmonary disease. Such auto-immune diseases include, but arc not limited to, insulin dependent diabetes mellitus (IDDM), systemic lupus erythematosus (SLE), rheumatoid arthritis, coeliac disease, multiple sclerosis, neuritis, polymyositis, psoriasis, vitiligo, Sjogren's syndrome, rheumatoid arthritis, autoimmune pancreatitis, inflammatory bowel diseases, Crohn's disease, ulcerative colitis, active chronic hepatitis, glomerulonephritis, scleroderma, sarcoidosis, autoimmune thyroid diseases, Hashimoto's thyroiditis, Graves disease, myasthenia gravis, asthma, Addison's disease, autoimmune uveoretinitis, pemphigus vulgaris, primary biliary cirrhosis, pernicious anemia, sympathetic opthalmia, uveitus, autoimmune hemolytic anemia, pulmonary fibrosis or idiopathic pulmonary fibrosis. The methods and compositions of the present invention may further be used in the treatment of mammalian subjects suffering from demyelination or axonal injury or loss such as in human neurodegenerative diseases, including, but not limited to, multiple sclerosis (MS), Parkinson's disease, Alzheimer's disease, progressive multifocal leukoencephalopathy (PML), disseminated necrotizing leukoencephalopathy (DNL), acute disseminated encephalomyelitis, Schilder disease, central pontine myelinolysis (CPM), radiation necrosis, Binswanger disease (SAE), adrenoleukodystrophy, adrenomyeloneuropathy, Leber's hereditary optic atrophy, and HTLV-associated myelopathy. These and other subjects are effectively treated by administering to the subject an effective amount of the human two domain molecules effective to treat, ameliorate, prevent or arrest the progression of the T-cell mediated disease.

The compositions and methods of the of the present invention may also be used in the prevention of T-cell mediated diseases or relapses of T-cell mediated diseases including auto-immune and neurodegenerative diseases as well as other conditions that cause demyelination or axonal injury or loss in mammalian subjects, including humans. The compositions and methods of the present invention may further be used to prevent or decrease infiltration of activated inflammatory cells in to the central nervous system of mammalian subjects, including humans. The compositions and methods of the present invention may additionally be used as vaccines to induce antigen-specific regulatory cells specific for antigens particular to those tissues involved in autoimmune or neurodegenerative disorders, such as, for example myelin basic protein in multiple sclerosis. The compositions and methods of the present, invention may also be used to restore myelin and prevent or halt myelin damage.

The various formulations and compositions of the present invention may be administered with one or more additional active agents, that are combinatory formulated or coordinately administered with the purified MHC polypeptides for the treatment of T-cell mediated diseases. Such additional therapeutic agents include, but are not limited to, immunoglobulins (e.g., a CTLA4Ig, such as BMS-188667; see, e.g., Srinivas et al., J, Pharm. Sci. 85(1): 1-4, (1996), incorporated herein by reference); copolymer 1, copolymer 1-relaled peptides, and T-cells treated with copolymer 1 or copolymer 1-related peptides (see, e.g., United States Patent No. 6,844,314, incorporated herein by reference); blocking monoclonal antibodies; transforming growth factor-β; anti-TNF α antibodies; glatiramer acetate; recombinant β interferons; steroidal agents; anti-inflammatory agents; immunosuppresive agents; alkylating agents; anti-metabolites; antibiotics; corticosteroids; proteosome inhibitors; and diketopiperazines.

The foregoing and other objects, features, aspects and advantages of the present invention will become more apparent from the following sections.

### BRIEF DESCRIPTION OF THE DRAWINGS

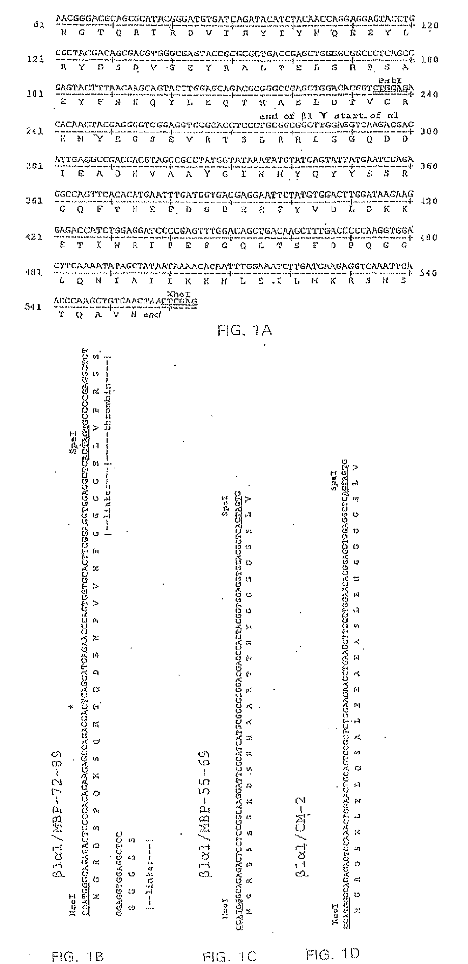

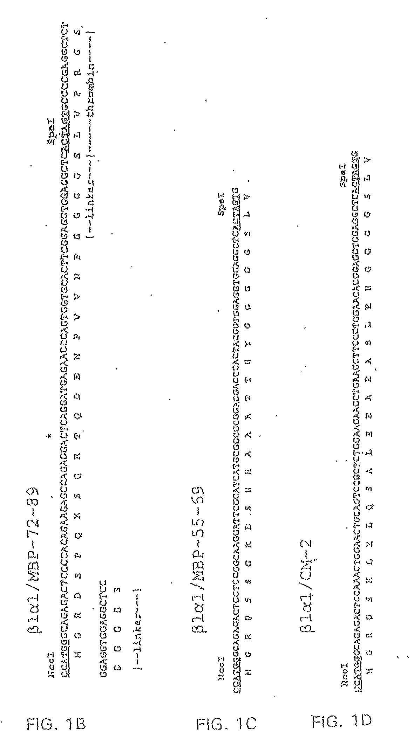

Fig. 1A shows the sequences of the prototypical ß1∝1 cassette without an antigen coding region. Unique NcoI, PstI, and XhoI restriction sites are in **bold**. The end of the ß1 domain and start of the α1 domain are indicated. Fig. 1B shows the sequence of an in-frame antigenic peptide/linker insertion sequence that can be incorporated into the expression cassette at the insertion site shown in Fig. 1A. This sequence includes the rat MBP-72-89 antigen, a flexible linker with an embedded thrombin cleavage site, and a unique SpcI restriction site that can be used for facile exchange of the antigen coding region. Example 2 below discusses the use of the equivalent peptide from Guinea pig, which has a serine in place of the threonine residue in the MBP-72-89 sequence. Figs. 1C and 1D show exemplary Nco1/SpeI fragments that can be inserted into the expression cassette in place of the MBP-72-89 antigen coding region. Fig. 1C includes the MBP-55-69 antigen, Fig. 1D includes the CM-2 antigen.

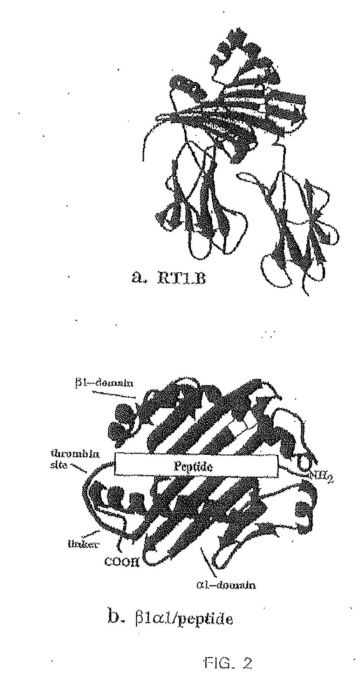

Figs. 2A and B illustrate the structure-based design of the ß1α1 molecule. Fig. 2A shows the rat class II RT1.B loaded with the encephalitogenic MBP-69-89 peptide (non-covalent association). Fig. 2B shows the single-chain ß1α1 molecule loaded with MBP-69-89,

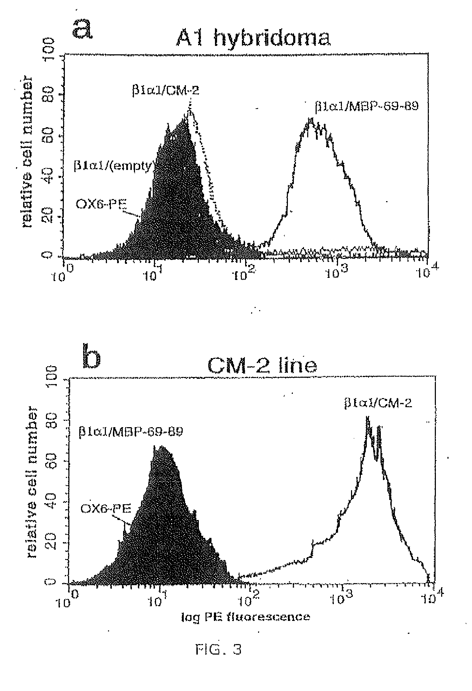

Figs. 3A and 3B show direct detection of antigen-specific ß1α1/polypeptide molecules binding rat T-cells. The A1 T-cell hybridoma (BV8S2 TCR+) and the CM-2 cell line (BV8S2 TCR-) were incubated for 17 hours at 4 C with various ß1α1 constructs, washed, stained for 15 min. with OX6-PE (α-RT1.B) or a PE-isotype control and then analyzed by FACS. Background expression of I-A on the CM-2 line was blocked with unlabeled OX-6. Fig, 3A is a histogram showing staining of the A1 hybridoma. Fig. 3B is a histogram showing staining of the CM-2 cell line.

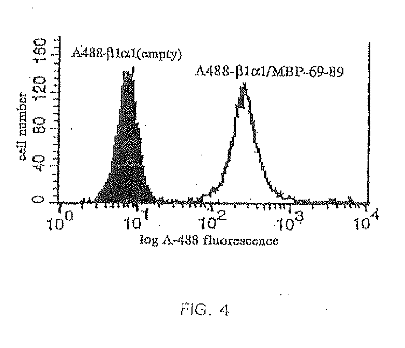

Fig. 4 is a graph illustrating binding of A488 conjugated ß1α1/polypeptide molecules to rat BV8S2 TCR. ß1α1 molecules were conjugated with Alexa-488 dye, loaded with MBP-69-89, incubated with the A1 T.-cell hybridomas (BV8S2 TCR+) for 3 hours at 4°C and then analyzed by FACS. A488- β1α1(empty) and A488- β1α1/MBP-69-89, as indicated.

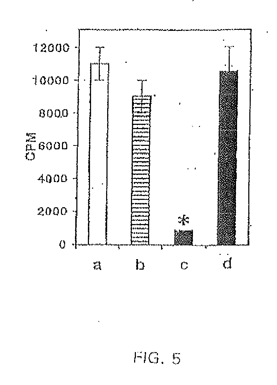

Fig. 5 is a bar graph illustrating that the β1α1/MBP-69-89 complex blocks antigen specific proliferation in an IL-2 reversible manner. Short-term T-cell lines selected with MBP-69-89 peptide from lymph node cells from rats immunized 12 days earlier with Gp-MBP/CFA were pre-treated for 24 hours with β1α1 constructs, washed, and then used in proliferation assays in which the cells were cultured with and without 20 Units/ml IL-2. Cells were incubated for three days, the last 18 hr in the presence of [³H] thymidine (0.5 µCi/10µl/well). Values indicated are the mean CPM ± SEM. Background was 210 CPM. Column a. Control proliferation assay without IL-2. Column b. 20 µM ß1α1/MBP-55-69 pretreatment. Column c. 10 nM ß1α1/MBP-69-89 pretreatment. Column d. 10 nM ß1α1/MBP-69-89 plus IL-2 during the proliferation assay. A single representative experiment is shown; the experiment was done twice. *indicates significant (p<0.001) inhibition with β1α1/MBP-69-89 versus control cultures.

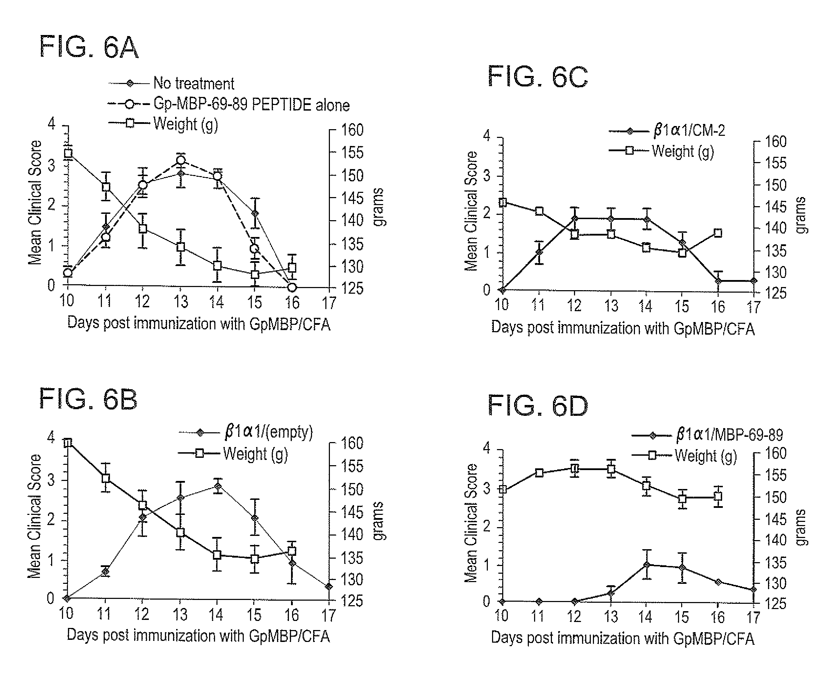

Figs. 6A-D are graphs showing clinical protection from experimental autoimmune encephalomyelitis with the ß1α1/MBP-69-89 complex. Groups of Lewis rats (n = 6) were injected with 25 µg of Gp-MBP/CFA to actively induce clinical EAE. On days 3, 7, 9, 11, and 14 after disease induction rats were given ß1α1/peptide complex, peptide alone, or were left untreated, as indicated. (6A) No treatment, or 2 µg MBP-69-89 peptide alone, as indicated. (6B) 300 µg of ß1α1/(empty) complex in saline. (6C) 300 µg of ß1α1/CM-2 complex in saline. (6D) 30 µg of ß1α1/MBP-69-89 complex in saline. Daily body weight (grams, right-hand y-axis) is plotted for the 300 µg ß1α1/peptide complex treatments. A single representative experiment is shown; the experiment was done three times. Values indicate mean clinical score ± SEM on each day of clinical disease. 30 µg of complex is equivalent to 2 µg of free peptide.

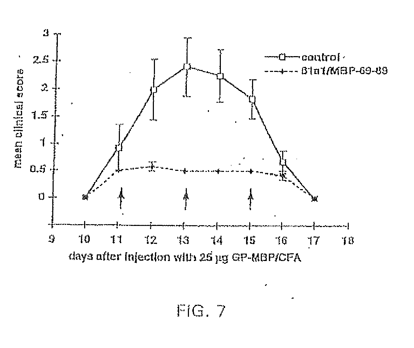

Fig. 7 is a graph illustrating treatment of established EAE with ß1α1/MBP-69-89 complex. Groups of Lewis rats (n=6) were injected with 25 µg of Gp-MBP/CFA to actively induce clinical EAE. On the day of onset of clinical signs (day 11), day 13, and day 15, rats were given 300 µg of ß1α1/MBP-69-89 complex (indicated by arrows) or were left untreated. A single representative experiment is shown; the experiment was done twice. Values indicate mean clinical scope ± SEM on each day of clinical disease.

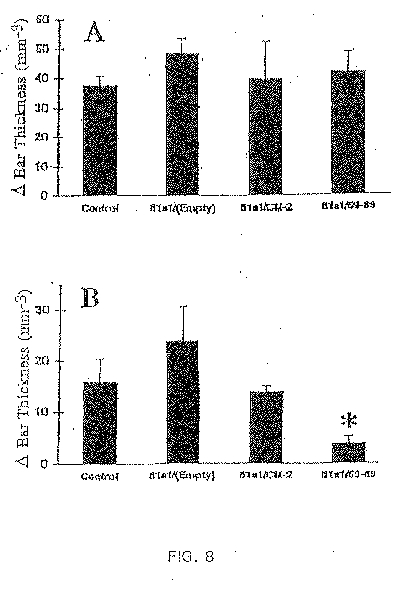

Figs. 8A and 8B are graphs showing that the ß1α1/MBP-69-89 complex specifically inhibits the DTH response to MBP 69-89. (8A) Change in ear thickness 24 hrs after challenge with PPD. (8B) Change in ear thickness 24 hrs after challenge with MBP-69-89. Values indicate mean score ± SEM *Indicates significant difference between control and treated (p = 0.01). A single representative experiment is shown; the experiment was done twice.

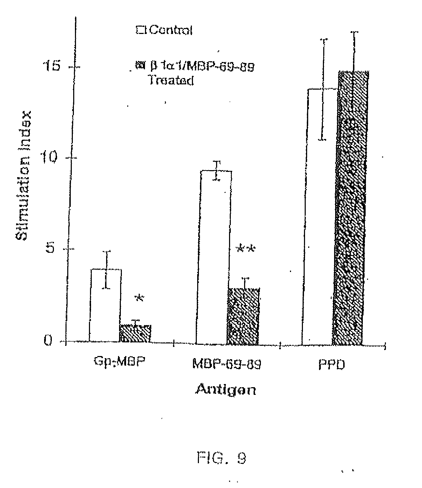

Fig. 9 is a graph showing that T-cell responses to MBP-69-89 were inhibited in Lewis rats treated with 300 µg ß1α1/MBP-69-89 complex. Lymph node cells were collected from control and treated rats after recovery of controls from EAE (day 17), and stimulated with optimal concentrations of Gp-MBP, Gp-MBP-69-89 peptide, or PPD. *Indicates significant difference between control and treated (*p < 0.05; **p < 0.001). Note inhibition with Gp MBP and MBP-69-89 peptide but not to PPD in treated rats.

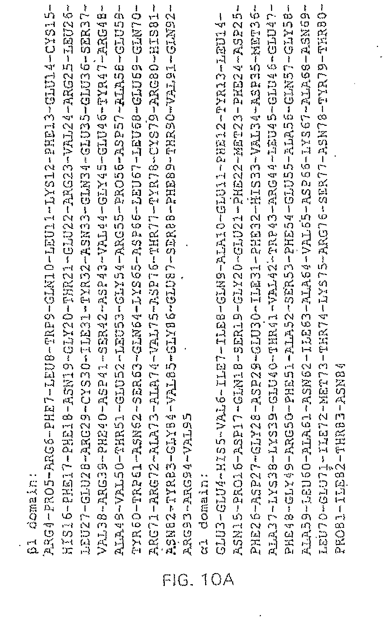

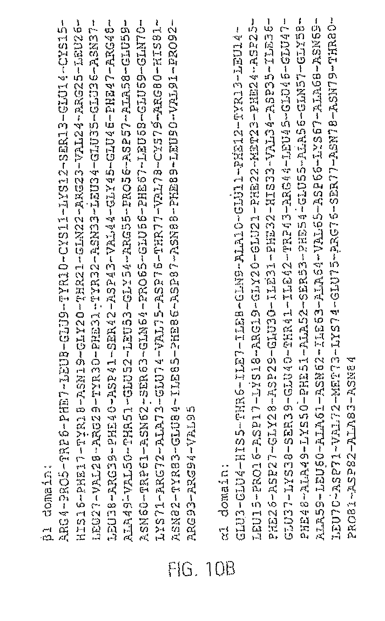

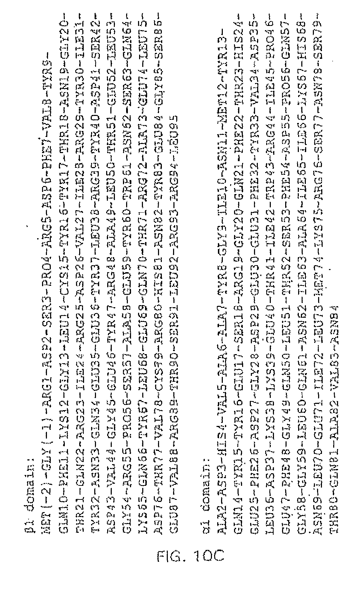

Figs. 10A, 10B, and 10C show the amino acid sequences of exemplary human (DRA and DRB1 0101) (10A), mouse (I-E^{k}) (10B) and rat (RT1.B) (10C) ß1 and α1 domains (the initiating methione and glycine sequences in the rat sequence were included in a construct for translation initiation reasons).

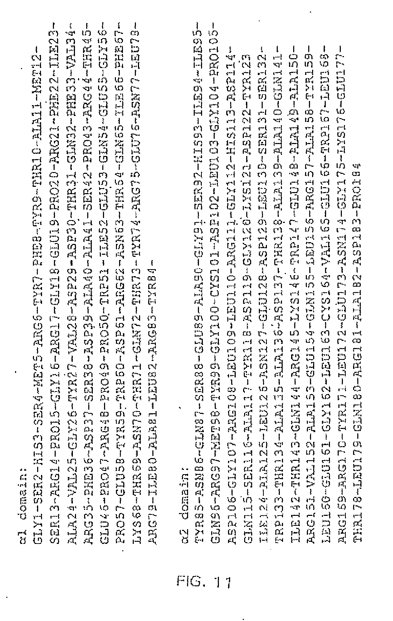

Fig. 11 shows the amino acid sequences of exemplary α1 and α2 domains derived from human MHC class I B*5301.

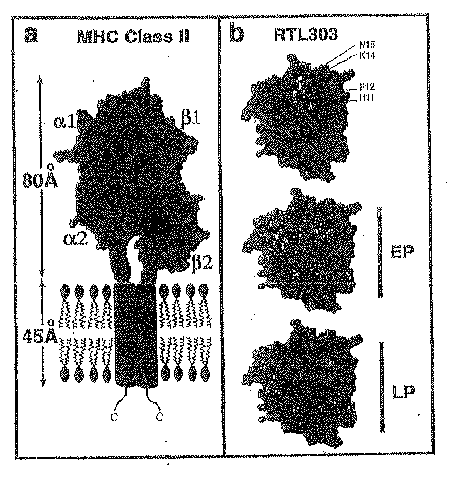

Fig. 12 shows schematic models of human HLA-DR2-derived recombinant T-cell receptor ligands (RTLs). Fig. 12(A) is a schematic scale model of an MHC class II molecule on the surface of an APC. The polypeptide backbone extra-cellular domain is based on the known crystallographic coordinates of HLA-DR2 (PDB accession code 1BX2). The transmembrane domains are shown schematically as 0.5 nm cylinders, roughly the diameter of a poly-glycine alpha-helix. The α1, α2, β1 and β2 domains are labeled, as well as the carboxyl termini of the MHC class II heterodimers. Fig. 12(B) is a schematic of the RTL303 molecule containing covalently linked β1 and α1 domains from HLA-DR2 and covalently coupled MBP85-99 peptide. The view of the RTLs is symmetry-related to the MHC class II molecule in panel (a) by rotation around the long-axis of bound peptide by ∼45° (y-axis) and ∼45° (Z-axis). Top, the same shading scheme as in panel (a), with primary T-cell receptor (TCR) contact residues H11, F12, K14 and N15 labeled. Middle, shaded according to electrostatic potential (EP). The shading ramp for EP ranges from dark (most positive) to light (most negative). Bottom, shaded according to lipophilic potential (LP). The shading ramp for LP ranges from dark (most lipophilic area of the molecule) to light (most hydrophilic area).

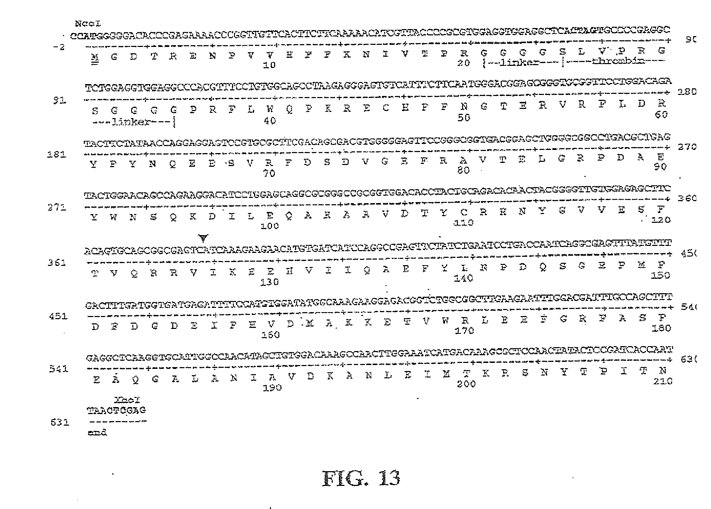

Fig. 13 is the nuclcotide and protein sequence of human HLA-DR2-derived RTL303. RTL303 was derived from sequences encoding the β-1 and α-1 domains of HLA-DR2 (human DRB1 *1501/DRA*0101) and sequence encoding the human MBP85-99 peptide. Unique NcoI, SpeI and XhoI restriction sites are in **bold**. The end of the β-1 domain and start of the α-1 domain are indicated by an arrow. RTL303 contains an in-frame peptide/linker insertion encoding the human MBP85-99 peptide (**bold**), a flexible linker with an embedded thrombin cleavage site, and a unique SpsI restriction site which can be used for rapidly exchanging the encoded amino-terminal peptide. RTL301 is identical to RTL303 except for a single point mutation resulting in an F1 50L substitution. Two additional proteins used in this study, RTL300 and RTL302, are "empty" versions of RTL301 and RTL303, respectively. These molecules lack the peptide/linker insertion (residues 16-115). Codon usage for glycines 32 and 51 have been changed from the native sequence for increased levels of protein expression in *E. coli*.

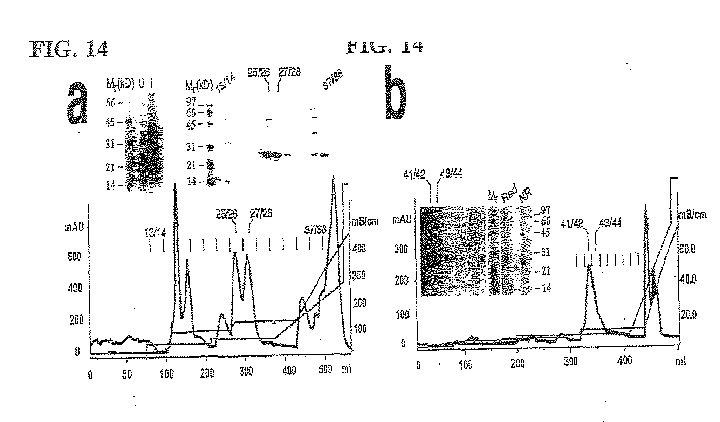

Fig. 14 shows the purification of human HLA-DR2-derived RTL303. Fig. 14(A) is the ion exchange FPLC of RTL303. Insert left: Mr, molecular weight standards; U, uninduced cells; I, induced cells, showing high-level expression of RTL303. Insert Right: Fractions 25-28 contain partially purified RTL303. Fig. 14(B) is size-exclusion chromatography of RTL303. Insert: fractions 41-44, containing purified RTL303; Mr, molecular weight standards; Red, reduced RTL303; NR, non-reduced RTL303.

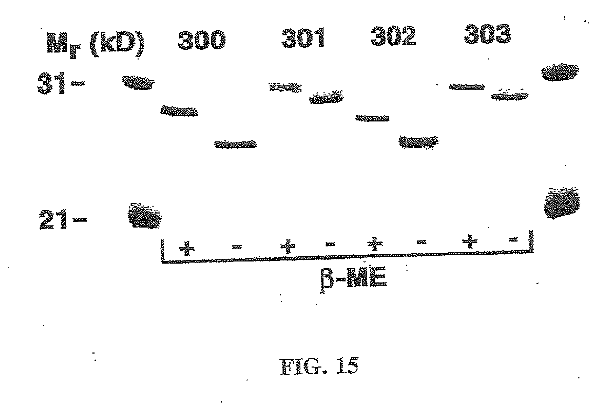

Fig. 15 is a digital image of a Western blot demonstrating purified and refolded DR2-derived RTLs have a native disulfide bond. Samples of RTLs were boiled for 5 minutes in Laemmli sample buffer with or without the reducing agent ß-mercaptoethanol (ß-ME), and then analyzed by SDS-PAGE (12%). Non-reduced RTLs (-lane) have a smaller apparent molecular weight than reduced RTLs (+ lane), indicating the presence of a disulfide bond. First and last lanes show the molecular weight standards carbonic anhydrase (31 kD) and soybean trypsin inhibitor (21.5kD). RTLs (+/-ß-ME), as indicated.

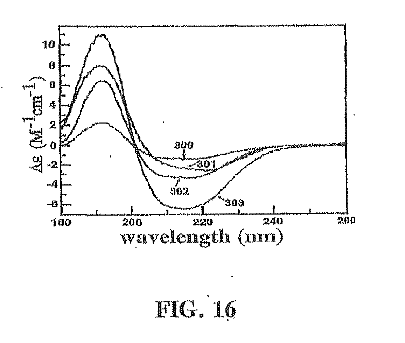

Fig. 16 is a digital image of circular dichroism showing that DR2-derived RTLs have highly ordered structures. CD measurements were performed at 20°C on a Jasco J-500 instrument using 0.1 mm cells from 260 to 180 nm. Concentration values for each protein solution were determined by amino acid analysis. Buffer, 50 mM potassium phosphate, 50 mM sodium fluoride, pH 7.8. Analysis of the secondary structure was performed using the variable selection method.

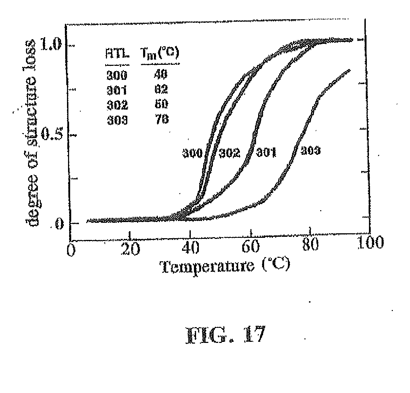

Fig. 17 is a graph of experiments that demonstrate the high degree of cooperativity and stability of DR2-derived RTLs subjected to thermal denaturation. CD spectra were monitored at 222 nm as a function of temperature. The heating rate was 10°C/hr. The graph charts the percent of unfolding as a function of temperature. 1.0 corresponds to the completely unfolded structure.

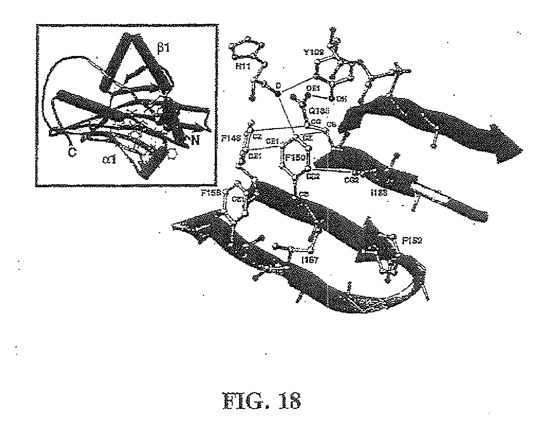

Fig. 18 is a schematic diagram of interactions of atoms within 4Å of residue F150. Distances were calculated using coordinates from 1BX2. Inset: the location of residue F150 within the RTL303 molecule.

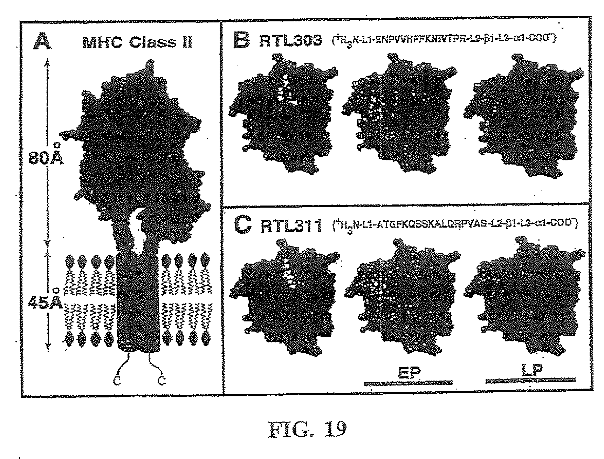

Figs. 19A, 19B, and 19C illustrate the structure-based design of the human HLA-DR2-derived RTLs. (A) is a schematic model of an MHC class II molecule on the surface of an APC. The polypeptide backbone extracellular domain is based on the crystallographic coordinates of HLA-DR1 (PDB accession code 1AQD). The transmembrane domains are shown schematically as 0.5 nm cylinders, roughly the diameter of a poly-glycine alpha-helix. The carboxyl termini of the MHC class II heterodimers are labeled. (B) is a diagram of the HLA-DR2 β1α1-derived RTL303 molecule containing covalently coupled MBP85-99 peptide. (C) is a diagram of the HLA-DR2 β1α1-derived RTL311 molecule containing covalently coupled C-ABL peptide. The view of the RTLs is symmetry-related to the MHC class II molecule in panel (a) by rotation around the long-axis of bound peptide by ~45° (y-axis) and ~45° (Z-axis). Left, the same shading scheme as in panel (A), with primary TCR contact residues labeled. Middle, shaded according to electrostatic potential (EP). The shading ramp for EP ranges from dark (most positive) to light (most negative). Right, shaded according to lipophilic potential (LP). The shading ramp for LP ranges from dark (highest lipophilic area of the molecule) to light (highest hydrophilic area). The program Sybyl (Tripos Associates, St. Louis, MO) was used to generate graphic images using an 02 workstation (Silicon Graphics, Mountain View, CA) and coordinates deposited in the Brookhaven Protein Data Bank (Brookhaven National Laboratories, Upton, NY). Structure-based homology modeling of RTLs was based on the known crystallographic coordinates of HLA-DR2 complexes with MBP peptide (DRA*0101, DRB1*1501; see, e.g., Smith et al., J. Exp. Med. 188:1511, (1998)), Amino acid residues in the HLA-DR2 MBP peptide complex (PDB accession number 1BX2) were substituted with the CABL side chains, with the peptide backbone of HLA-DR2 modeled as a rigid body during structural refinement using local energy minimization.

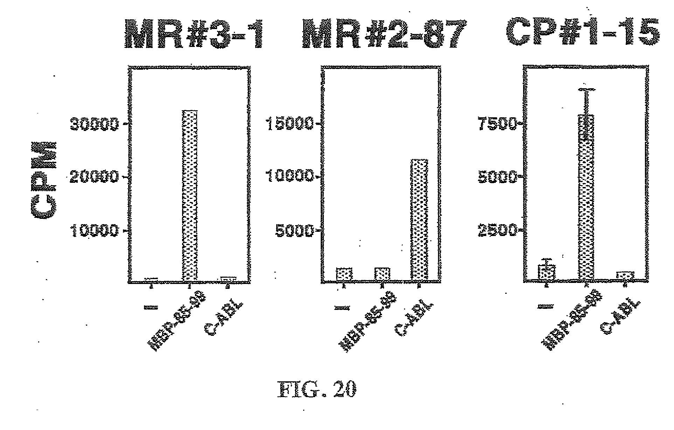

Fig. 20 is a series of bar graphs charting the response of T-cell clones. DR2 restricted T-cell clones MR#3-1, specific for MBP-85-99 peptide, and MR#2-87, specific for CABL-b3a2 peptide, and a DR7 restricted T-cell clone CP#1-15 specific for MBP-85-99 peptide were cultured at 50,000 cells/well with medium, alone or irradiated (2500 rad) frozen autologous PBMC (150,000/well) plus peptide-Ag (MBP-85-99 or CABL, 10 µg/ml) in triplicate wells for 72 hr, with 3 H-thymidine incorporation for the last 18 hr. Each experiment shown is representative of at least two independent experiments. Bars represent CPM ±SEM.

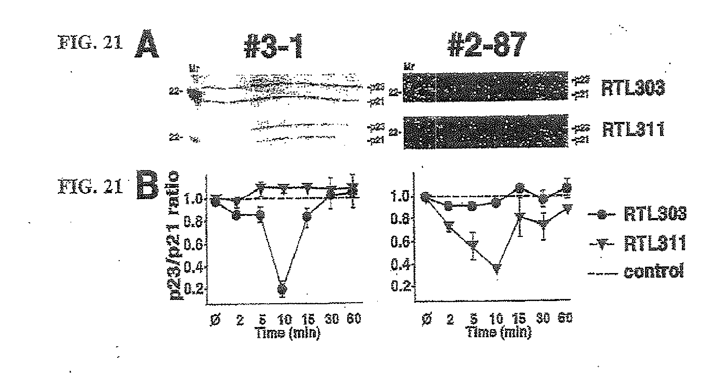

Fig. 21 is a graph illustrating that zeta chain phosphorylation induced by RTL treatment is Ag-specific. DR2 restricted T-cell clones MR#3-1 specific for MBP-85-99 peptide or MR#2-87 specific for CABL-b3a2 peptide, were incubated at 37°C with medium alone (control), or with 20 µM RTL303 or RTL311. Western blot analysis of phosphorylated ζ (zeta) shows a pair of phospho-protein species of 21 and 23 kD, termed p21 and p23, respectively. Quantification of the bands showed a distinct change in the p21/p23 ratio that peaked at 10 minutes. Each experiment shown is representative of at least three independent experiments. Points represent mean ± SEM.

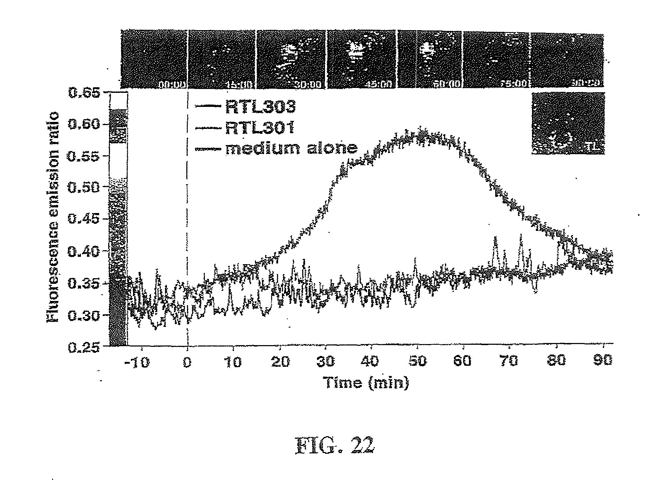

Fig. 22 shows the fluorescence emission ratio of T-cells stimulated with RTLs. RTLs induce a sustained high calcium signal in T-cells. Calcium levels in the DR2 restricted T-cell clone MR#3-1 specific for the MBP-85-99 peptide were monitored by single cell analysis. RTL303 treatment induced a sustained high calcium signal, whereas treatment with RTL301 (identical to RTL303 except a single point mutation, F150L) did not induce an increase in calcium signal over the same time period. The data is representative of two separate experiments with at least 14 individual cells monitored in each experiment.

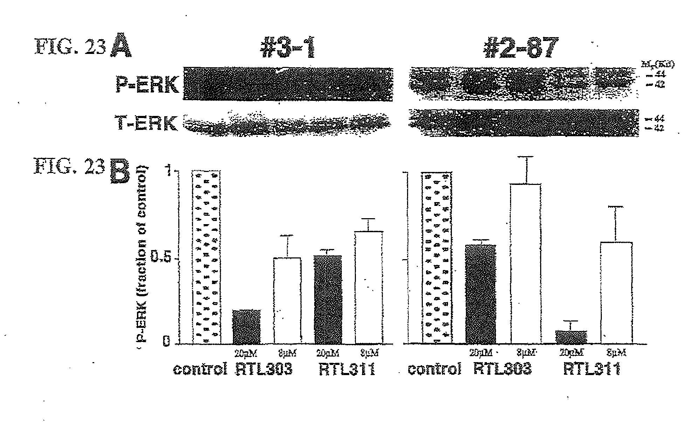

Fig. 23 is a set of bar graphs demonstrating that ERK activity is decreased in RTL treated T-cells. DR2 restricted T-cell clone MR#3-1 specific for the MBP-85-99 peptide or MR#2-87 specific for CABL b3a2 peptide were incubated for 15 min. at 37°C with no addition (control), and with 20 or 8 µM RTL303 or RTL311. At the end of the 15-min. incubation period, cells were assayed for activated, phosphorylated ERK (P-ERK) and total ERK (T-ERK). Quantification of activated P-ERK is presented as the fraction of the total in control (untreated) cells. Each experiment shown is representative of at least three independent experiments. Bars represent mean ± SEM.

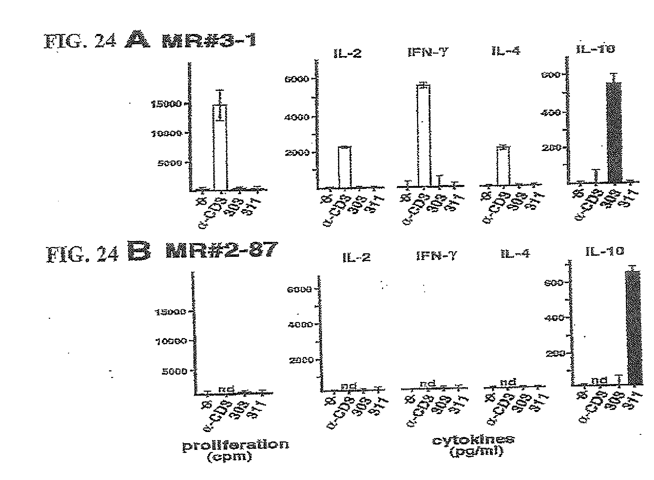

Fig. 24 is a series of graphs showing that direct antigen-specific modulation of IL-10 cytokine production in T-cell clones was induced by RTL treatment. DR2 restricted T-cell clones MR#3-1 and MR#2-87 were cultured in medium alone (-control), anti-CD3 mAb, 20 µM RTL303 or RTL311 for 72 hours. Proliferation was assessed by ³H-thymidine uptake. Cytokines (µg/ml) profiles were monitored by immunoassay (ELISA) of supernatants. Each experiment shown was representative of at least three independent experiments. Bars represent mean ± SEM. Clone MR#3-1 showed initial proliferation to anti-CD3, but not to RTLs.

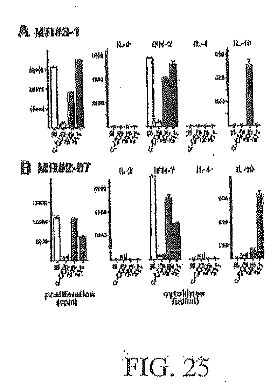

Fig. 25 is a set of graphs indicating that IL-10 cytokine production induced by RTL pre-treatment was maintained after stimulation with APC/peptide. T-cells had a reduced ability to proliferate and produce cytokines after anti-CD3 or RTL treatment, and the RTL effect was antigen and MHC specific. IL-10 was induced only by specific RTLs, and II-10 production was maintained even after restimulation with APC/antigen. T-cell clones were cultured at 50,000 cells/well with medium, anti-CD3, or 20 µM RTLs in triplicate for 48 hours, and washed once with RPMI. After the wash, irradiated (2500 rad) frozen autologous PBMC (150,000/well) plus peptide-Ag (MBP-85-99 at 10 µg/ml) were added and the cells incubated for 72 hr with ³H-thymidine added for the last 18 hr. Each experiment shown is representative of at least two independent experiments. Bars represent mean ± SEM. For cytokine assays, clones were cultured with 10 µg/ml anti-CD3 or 20 µM RTL303 or RTL311 for 48 hours, followed by washing with RPMI and restimulation with irradiated autologous PBMC (2500 rad, T:APC=1 :4) plus peptide-Ag (10 ∝g/ml) for 72 hours. Cytokines (pg/ml) profiles were monitored by immunoassay (ELISA) of supernatants. Each experiment shown is representative of at least three independent experiments. Bars represent mean ± SEM.

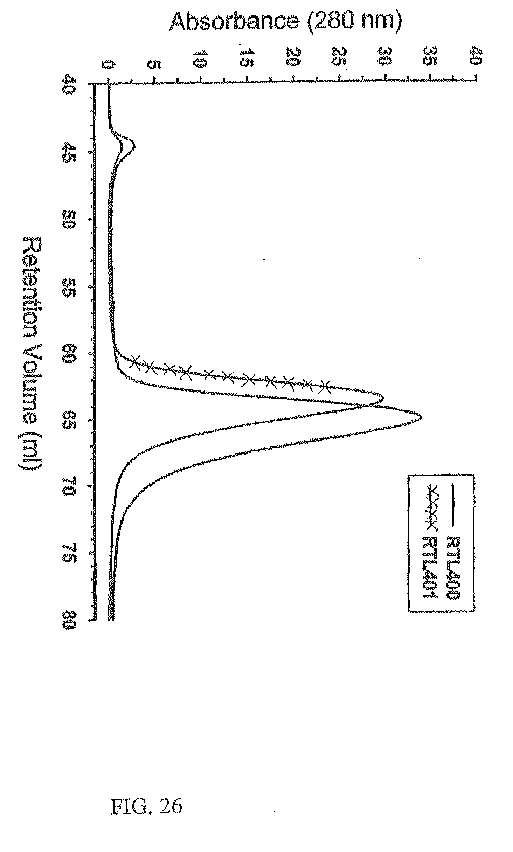

Fig. 26 presents size exclusion chromatography data for modified RTLs. Purified and refolded modified RTL400 and 401 were analyzed by size exclusion chromatography. A Superdex 75 (16/60) size exclusion column was calibrated with a set of known m.w. proteins, and *Y* = -0.029X + 6.351 (*r* = 0.995) was calculated from the slope of the standard curve, and subsequently used to estimated the size of modified RTL400 and 401.

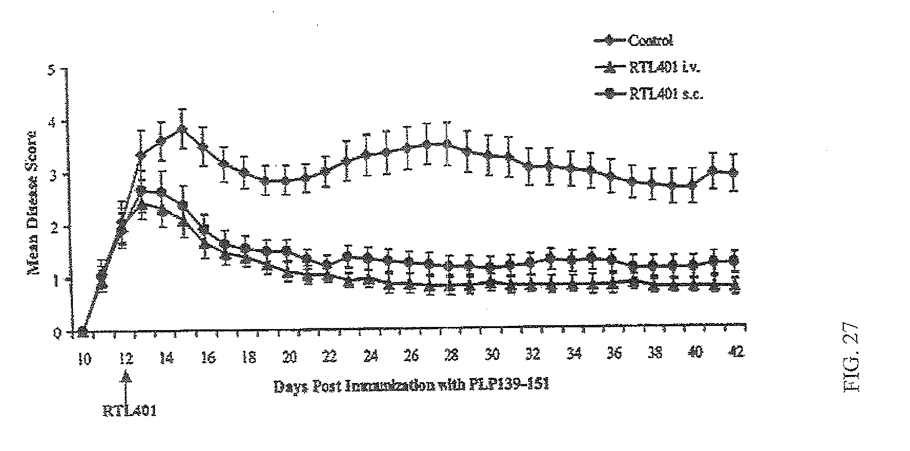

Fig. 27 illustrates how intravenous or s.c. administration of RTL401 improves EAE in SJL mice. SJL females were immunized with PLP 139-151 (ser). At disease onset (day 12), mice were treated daily with vehicle, 0.8 mg of RTL401 i.v., or 0.8 mg of RTL401 s.c. for 8 days. Mice were scored as outlined in the examples below. Data presented are the mean of two experiments for each group, with 12-14 mice per group.

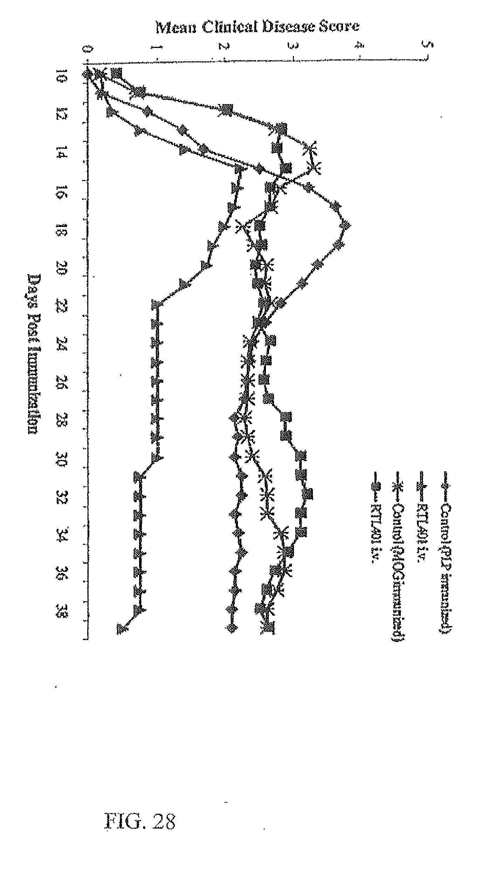

Fig. 28 illustrates how RTL treatment is specific for the cognate combination of MHC and neuroantigen peptide. B6XSJL mice (I-A^{s}/I-E^{b} MHC class II molecules) were immunized with PLP 139-151 or MOG 35-55. At disease onset, groups of mice were created with vehicle or 0.8 mg of RTL401 i.v. daily for 8 days and disease course was monitored. MOG 35-55-immunized mice did not respond to RTL treatment whereas PLP 139-151-immunized mice showed significant improvement in EAE following i.v. treatment with RTL401. Data presented are the mean of two experiments with a total of 11-16 mice per group.

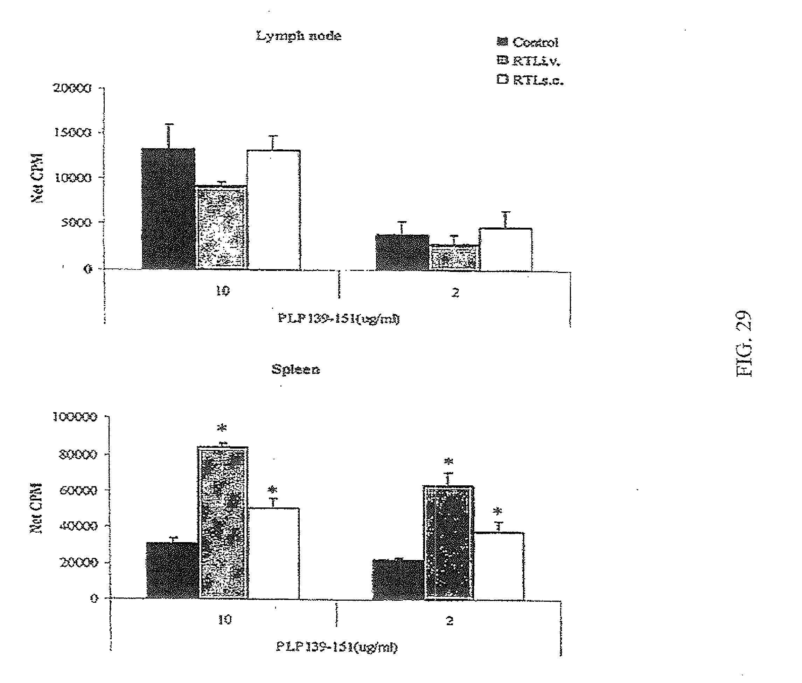

Fig. 29 illustrates T-cell proliferative response patterns. Lymph nodes and spleens were isolated from vehicle, RTL401 i.v. and RTL401 s.c. treated mice on day 42 post-immunization. The cells from three representative mice were pooled and T-cell responses were measured by proliferation to the immunizing Ag, PLP 139-151, after 72-h incubation in stimulation medium, the last 18 h in presence of [³H] thymidine. Data are presented as net cpm relative to media alone controls. Significant differences between control and experimental groups were determined using Student's *t* test (*, *p* < 0,05).

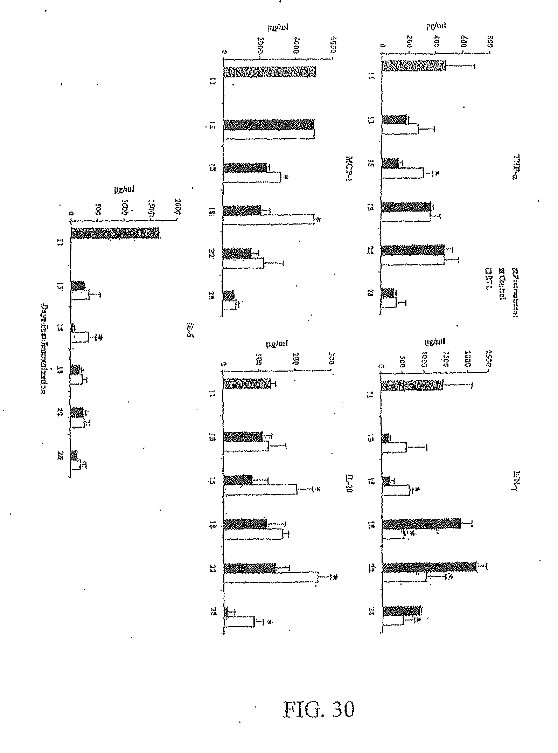

Fig. 30 illustrates T-cell cytokine response patterns. SJL mice were sacrificed at different time points following treatment with RTL401. Spleens were harvested and set up *in vitro* with 10 µg of PLP 139-151 peptide. Supernatants were harvested after 48 h and assayed for cytokine production by cytometric bead array as described below. Significant differences between control and experimental groups were determined using Student's *t* test (*, *p* < 0.05). Data are presented as the mean and SD of two mice at each time point per group.

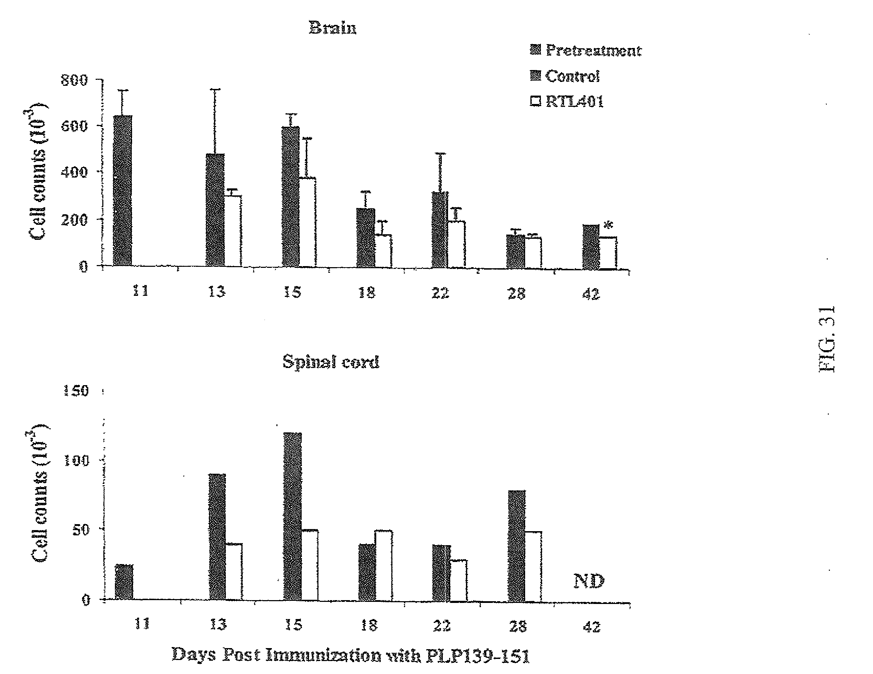

Fig. 31 shows additional CNS effects of RTL treatment. Mononuclear cells were isolated from brains and spinal cords harvested from mice at different time points following RTL401 treatment. Cells were counted by trypan blue exclusion method. Results presented are counts from two to three pooled brains or spinal cords.

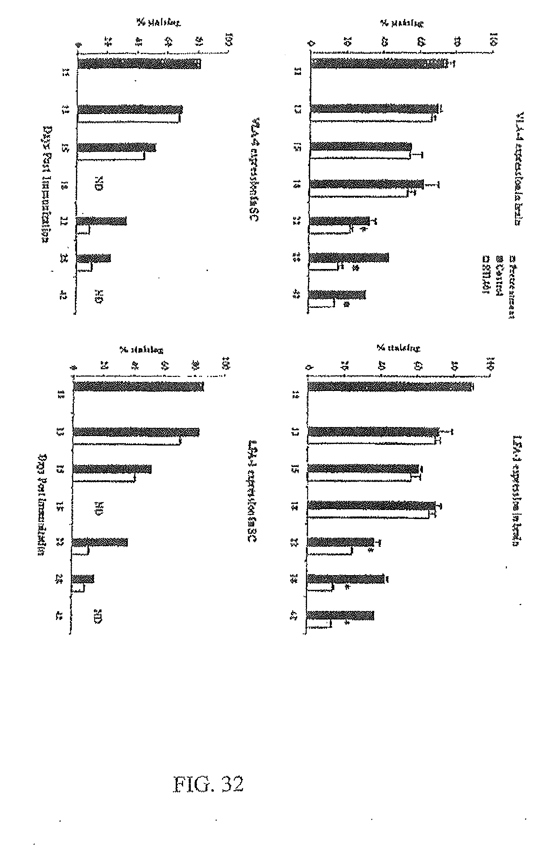

Fig. 32 illustrates that RTL treatment significantly decreases adhesion molecule expression on T-cells in the CNS. MNC's were isolated from brains any spinal cords harvested from two representative mice at different time points following RTL401 treatment. Cells were then stained with anti-mouse CD3 and anti-mouse VLA-4. or anti-mouse LFA-1 to identify the expression of these adhesion molecules on T-cells infiltrating the CNS. Data presented are percentage of total gated cells that were dual positive for CD3 and VLA-4 or LFA-1. Significance between control and experimental groups were determined using Student's *t* test (*, *p* < 0.05).

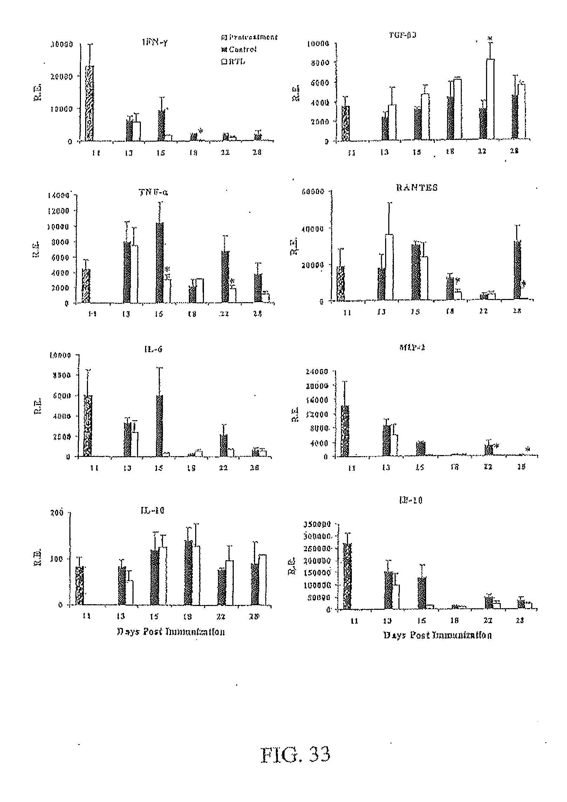

Fig. 33 illustrates the effects of RTL treatment on cytokine and chemokine gene expression as determined by real-time PCR. mRNA was isolated from whole frozen spinal cords harvested from two control and two RTL treated mice at different time points. DNA was synthesized and real-time PCR was performed using primers specific for IFN-τ, TNF-α, IL-6, IL-10, TGF-β3, RANTES, MIP-2, and IP-10. Expression of each gene was calculated relative to the expression of housekeeping gene, *L*32. Significance between control and experimental groups was determined using Student's *t* test (*, *p* < 0.05).

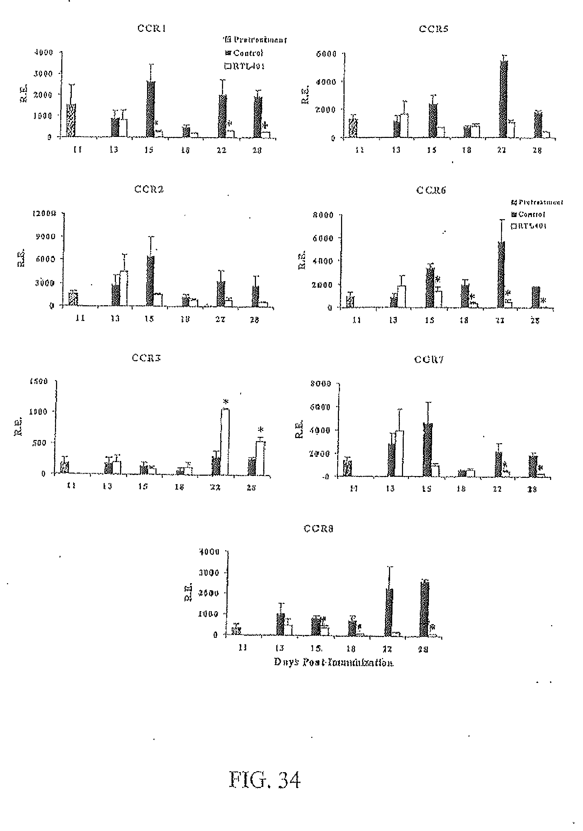

Fig. 34 provides real-time PCR quantification of relative expression of chemokine receptor genes from spinal cords of and RTL-treated mice. mRNA was isolated from whole frozen spinal cords harvested from two control and two RTL-treated mice at different time points. cDNA was synthesized and real-time PCR was performed using primers specific for CCR1, CCR2, CCR3, CCR5, CCR6, CCR7, and CCR8. Expression of each gene was calculated relative to the expression of housekeeping gene, L32. Significance between control and experimental groups was determined using Student's *t* test (*, *p* < 0.05).

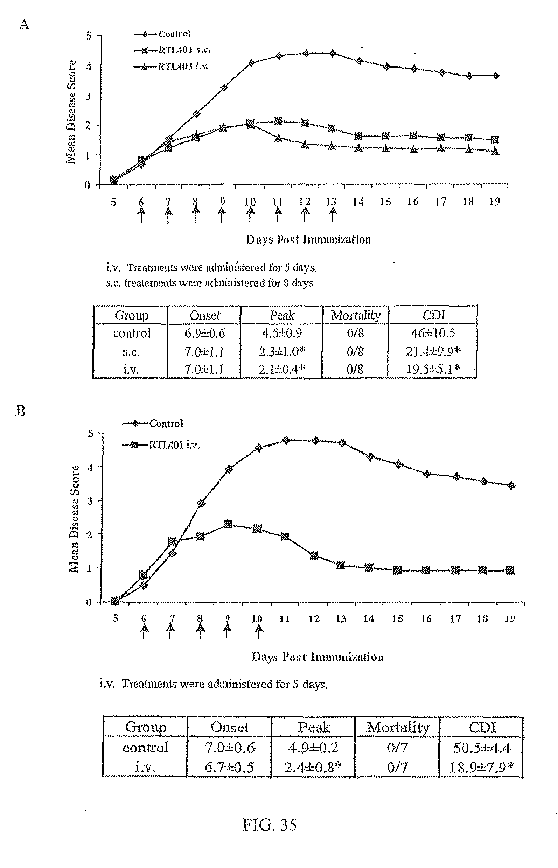

Figs. 35A and 35B illustrate the effects of RTL401 treatment on passively induced EAE in SJL mice. At disease onset (around day 6), mice were treated with vehicle (35A) or 100µg RTL401 i.v. for 5 days or s.c. for 8 days, or 100µg RTL401 i.v. (3513) for 5 days. Mice were scored as outlined in Example 15. Significant differences between control and experimental groups were determined using the Mann-Whitney test (*, p<0.05).

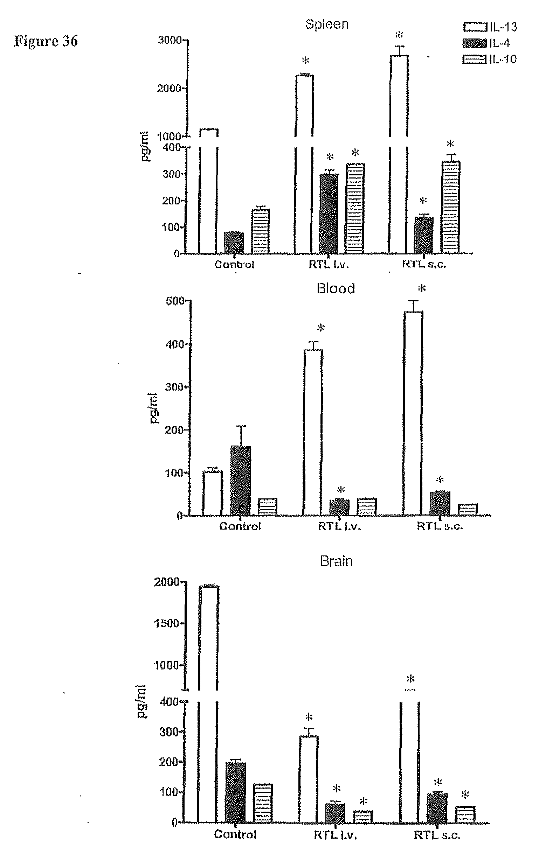

Fig. 36 illustrates the effects of RTL treatment on Th1 cytokine expression in spleen, blood and brain.

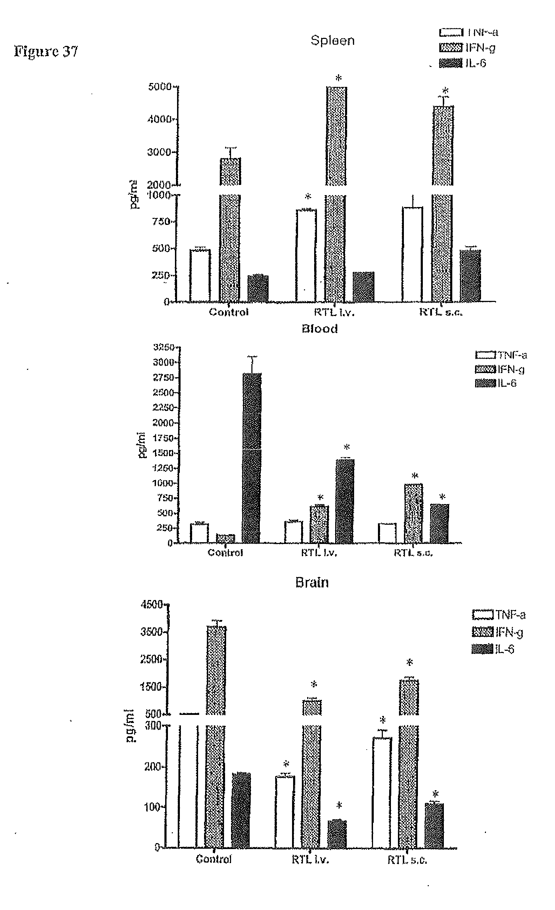

Fig. 37 illustrates the effects of RTL treatment on Th2 cytokine expression in spleen, blood and brain.

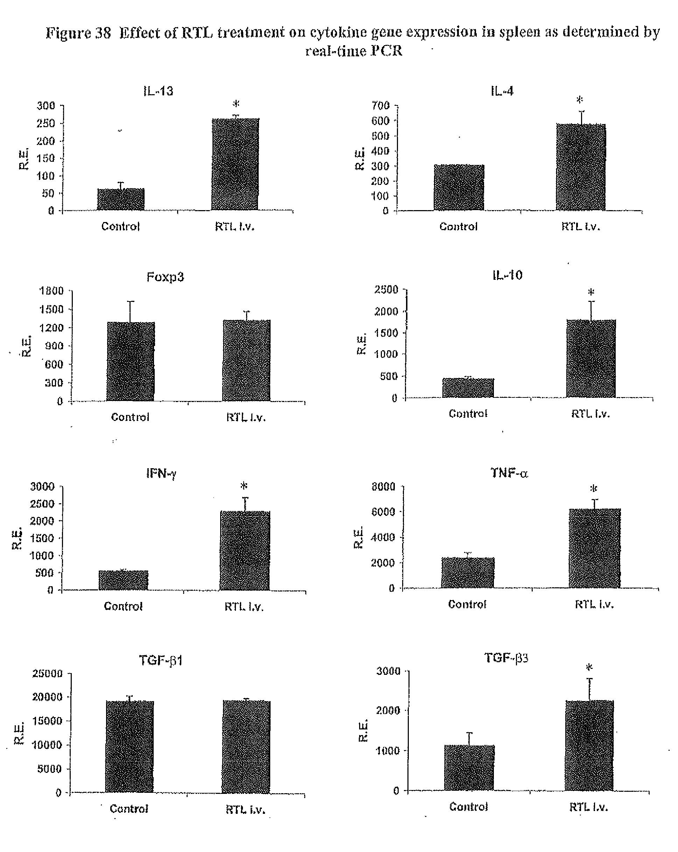

Fig. 38 illustrates the effects of RTL treatment on cytokine gene expression in spleen as determined by real-time PCR.

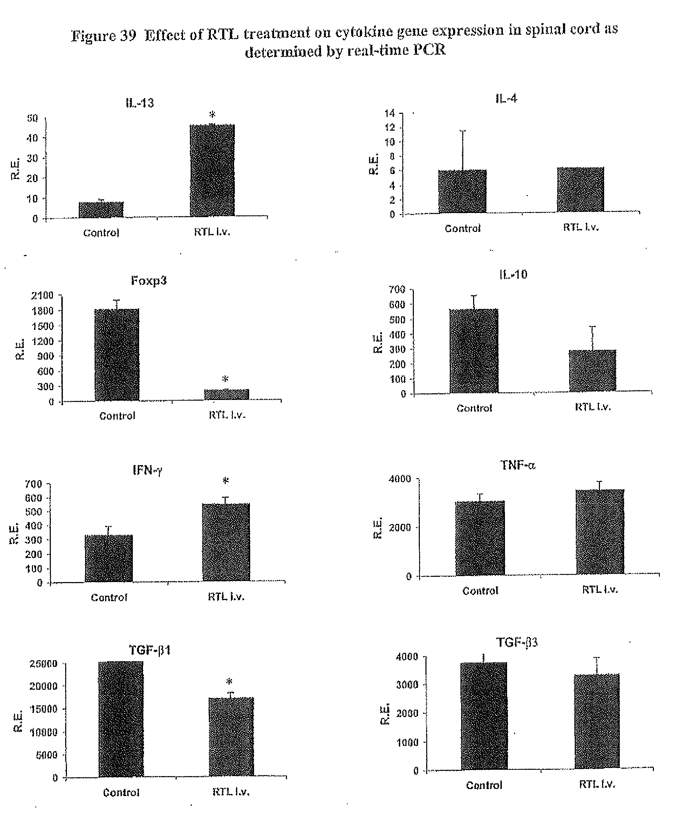

Fig. 39 illustrates the effects of RTL treatment on cytokine gene expression in spinal cord as determined by real-time PCR

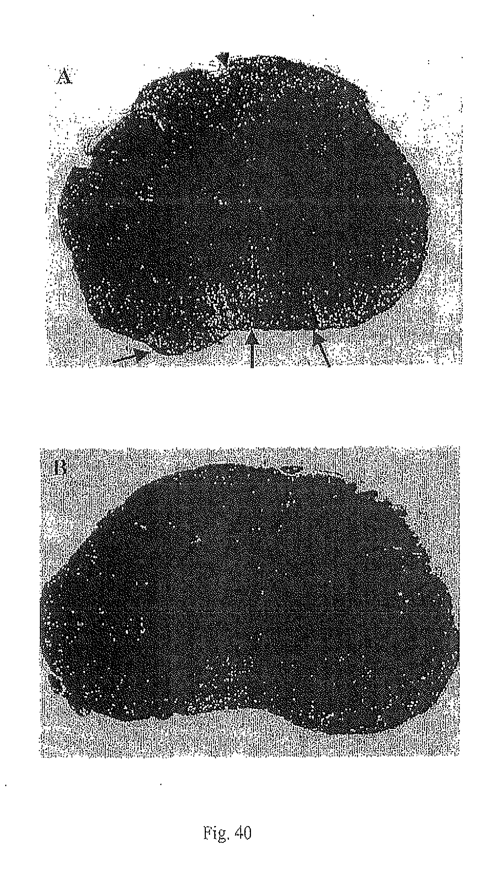

Figs. 40A and 40B are micrographs of fixed, paraffin-embedded spinal cord sections stained with hematoxylin-eosin from control (vehicle-treated) (A) or RTL-treated (B) SJL mice 19 days after passive induction of EAE. Note the mild to moderate inflammation in the cervical section of the vehicle-treated spinal cord (A) vs. little to no detectable cellular mononuclear infiltration in the RTL-treated spinal cord (B). Magnification was 12.5X. Arrows indicate the sites of inflammation in the vehicle-treated spinal cords. Data presented are representative of a total of 20 cervical sections examined from 2 mice from each group with average EAE scores of 3.5 (controls) vs. 1.0 (RTL401 treated).

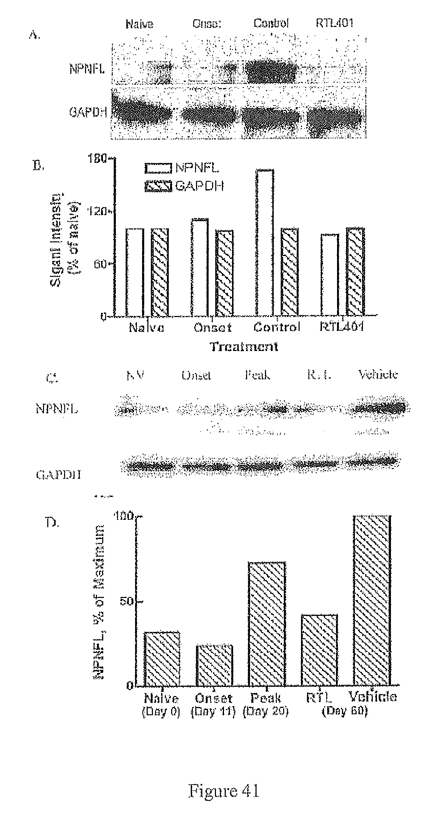

Figs. 41A, 41B, 41C and 41D demonstrate that RTL401 treatment ameliorates axonal loss in SJL mice with EAE, as indicated by the reduced amount of non-phosphorylated neurofilaments (NPNFL), a marker for axonal injury, in the CNS of SJL mice with passively induced (41A) and actively induced (41C) EAE. Figs. 41A and 41C show representative immunoblot analysis of the whole lumbar spinal cord homogenate from mice with different treatments or euthanized at different time points. Each band in Fig. 41A represents two mice per group and each band in Fig. 41C represents samples from 4 mice in each group. Figs. 41B and 41D provide a densitometric analysis of the proceeding blot. GADPH + Glyceraldehyd 3-phosphate dehydrogenase.

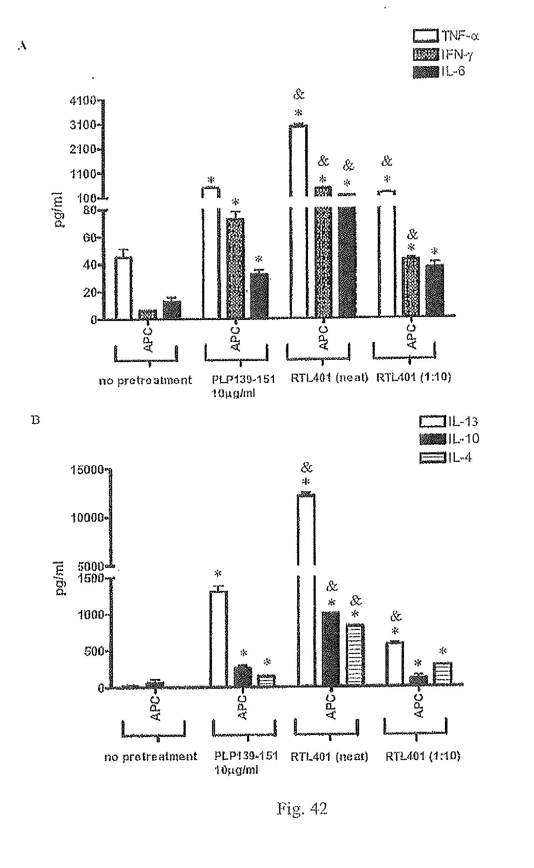

Figs. 42A and 42B are graphs demonstrating that RTL401 induces increased, expression of IL-13 and other cytokines *in vitro* in T-cells specific for PLP-139-151 peptide incubated for 24h with 100µg/ml RTL401 (neat), 10µg/ml RTL401 (1:10), 10µg/ml PLP-139-151 peptide, or medium prior to washing and incubation for 48 hours with APC but without added PLP peptide. (*) indicates significant difference (p<0,05) compared to medium pre-treated T-cells. (&) indicates significant difference (p<0.05) compared to PLP-139-151 peptide pre-treated T-cells. The data are pooled from three separate experiments.

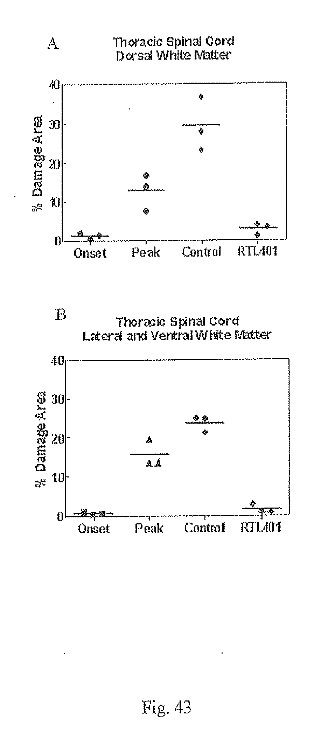

Figs. 43A and 43B arc charts of morphometric analysis of myelin damage in the dorsal (43A) and ventral/lateral white matter (43B) of the thoracic spinal cords in vehicle or RTL401 treated EAE mice receiving five consecutive RTL 401 i.v. treatments starting on day 20 and three consecutive s.c. treatments starting on day 32 and sacrificed on day 60. Onset of EAE appeared on day 11 and peak of EAE was on day 20. Each point represents an individual mouse.

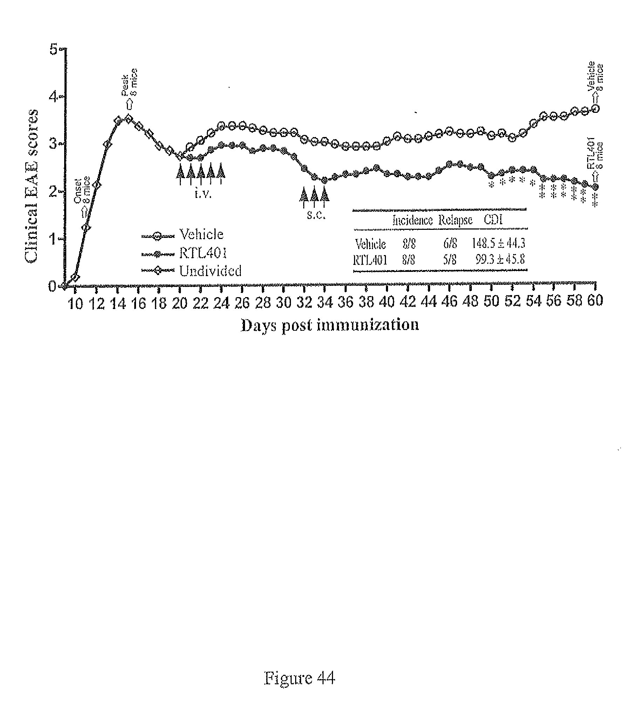

Fig. 44 is a chart demonstrating that administration of RTL401 after the peak of the disease improves the clinical evaluation of EAE in SJL mice.

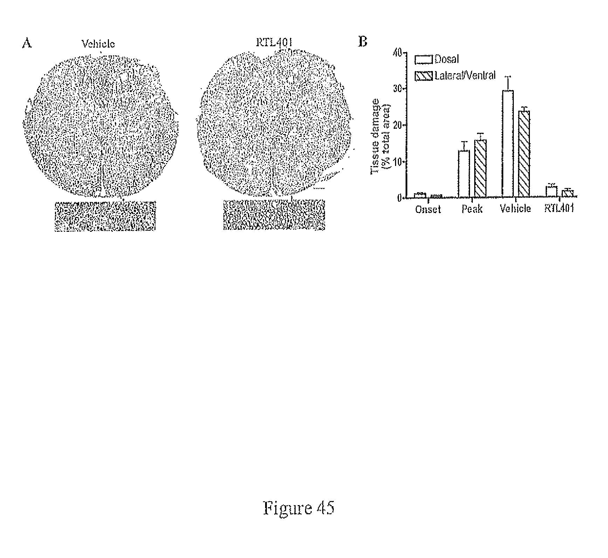

Fig. 45A provides photos representative thoracic spinal cord sections from EAE mice treated with vehicle (left image) or RTL401 (right image) sixty days after immunization. Tissue sections were stained with toludine blue (shown in black and white) and the damaged areas are manually circumscribed with red lines. Scale bars are 25 mM (low power view) or 100 mM (high power views).

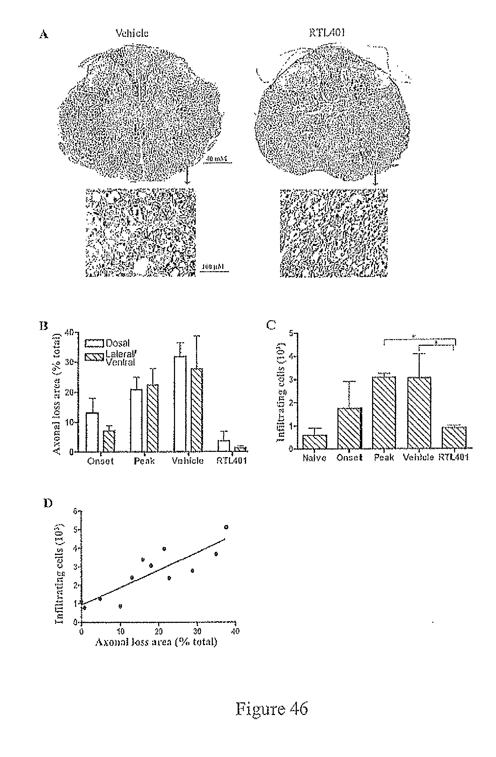

Fig. 46A provides photos of axon staining of thoracic spinal cord sections from EAE mice treated with vehicle (left image) or RTL401 (right image) 60 days after immunization. Normal axons stained brown with antibody cocktails of neurofilaments and the nucleus of infiltrating immune cells stained blue with hematoxylin. Figs. 46 B-D provide graphical data pertaining to RTL effects on cellular infiltration, axonal injury, neuroinflammation and other histopathological indicia observed in spinal cords of mice with and without RTL treatment, as indicated and further described in the Examples below.

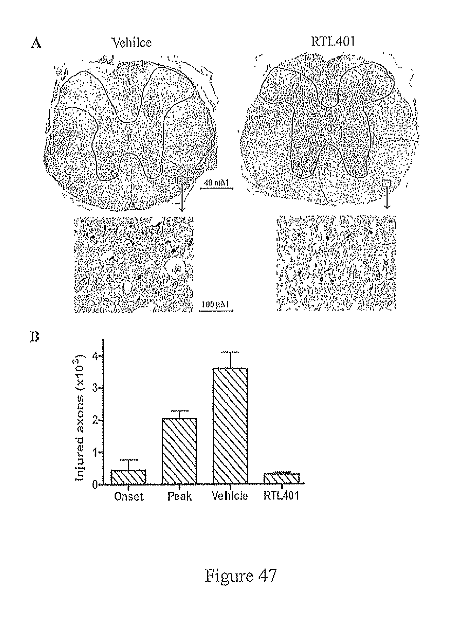

Fig. 47A provides photos of representative injured axon staining with NPNFL antibody and hematoxylin on the infiltrating immune cells of thoracic spinal cords from EAE mice treated with vehicle (left image) or RTL401 (right image) 60 days after immunization. Fig. 47B provide graphical data pertaining to RTL effects on cellular axonal injury and other histopathological indicia observed in spinal cords of mice with and without RTL treatment, as indicated and further described in the Examples below.

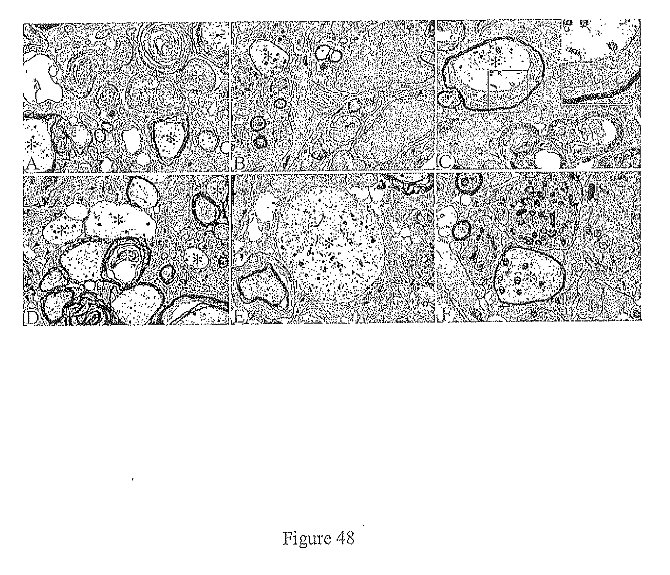

Figs. 48 A-F are representative electron micrographs showing lesion areas in spinal cords from EAE mice at the peak of the disease on day 20.

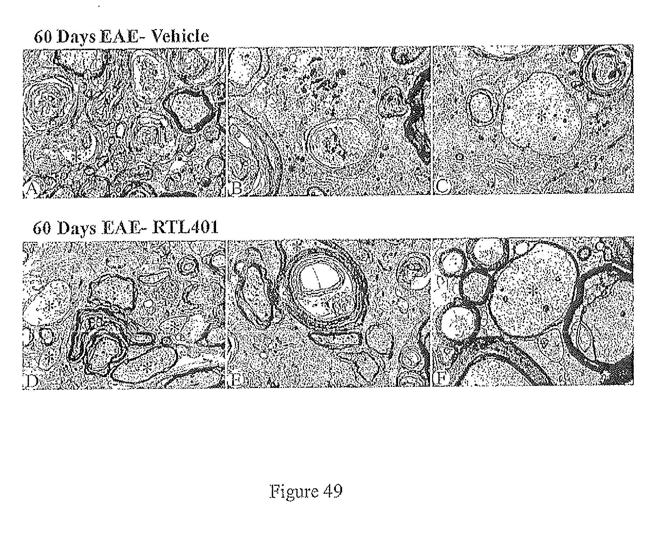

Fig. 49 contains representative micrographs showing lesion areas in spinal cords from mice with EAE evaluated on day 60, forty days after initiation of treatment with vehicle (Fig. 49 A-C) or RTL401 (Fig. 49 D-E).

### DETAILED DESCRIPTION OF EXEMPLARY EMBODIMENTS

In order to facilitate review of the various embodiments of the invention, the following definitions of terms and explanations of abbreviations are provided:

**β1α1 polypeptide**: A recombinant polypeptide comprising the α1 and β1 domains of a MHC class II molecule in covalent linkage. To ensure appropriate conformation, the orientation of the polypeptide is such that the carboxy terminus of the β1 domain is covalently linked to the amino terminus of the cd domain. In one embodiment, the polypeptide is a human β1α1 polypeptide, and includes the α1 and β1 domains for a human MHC class II molecule. One specific, non-limiting example of a human β1α1 polypeptide is a molecule wherein the carboxy terminus of the β1 domain is covalently linked to the amino terminus of the α1 domain of an HLA-DR molecule. An additional, specific non-limiting example of a human β1α1 polypeptide is a molecule wherein the carboxy terminus of the β1 domain is covalently linked to the amino terminus of the α1 domain of an a HLA-DR(either A or B), a HLA-DP(A and B), or a HLA-DQ(A and B) molecule. In one embodiment, the ß1α1 polypeptide does not include a ß2 domain. In another embodiment, the ß1α1 polypeptide does not include an α2. In yet another embodiment, the ß1α1 polypeptide does not include either an α2 or a ß2 domain.

β**1**α**1 gene:** A recombinant nucleic acid sequence including a promoter region operably linked to a nucleic acid sequence encoding a β1α1 polypeptide. In one embodiment the β1α1 polypeptide is a human β1α1 polypeptide.

α**1**α**2 polypeptide:** A polypeptide comprising the α1 and α2 domains of a MHC class I molecule in covalent linkage. The orientation of the polypeptide is such that the carboxy terminus of the α1 domain is covalently linked to the amino terminus of the α2 domain. An α1α2 polypeptide comprises less than the whole class I α chain, and usually omits most or all of the α3 domain of the α chain. Specific non-limiting examples of an α1α2 polypeptide are polypeptides wherein the carboxy terminus of the α1 domain is covalently linked to the amino terminus of the α2 domain of an HLA-A, -B or - C molecule. In one embodiment, the α3 domain is omitted from an α1α2 polypeptide, thus the α1α2 polypeptide does not include an α3 domain.

α**1**α**2 gene:** A recombinant nucleic acid sequence including a promoter region operably linked to a nucleic acid sequence encoding an α1α2 polypeptide.

**Antigen (Ag):** A compound, composition, or substance that can stimulate the production of antibodies or a T-cell response in an animal, including compositions that are injected or absorbed into an animal. An antigen reacts with the products of specific humoral or cellular immunity, including those induced by heterologous immunogens. The term "antigen" includes all related antigenic epitopes and antigenic determinants.

**Antigen Presenting Cell:** Any cell that can process and present antigenic peptides in association with class II MHC molecules and deliver a co-stimulatory signal necessary for T-cell activation. Typical antigen presenting cells include macrophages, dendritic cells, B cells, thymic epithelial cells and vascular endothelial cells.

**Autoimmune disorder:** A disorder in which the immune system produces an immune response (e.g. a B cell or a T-cell response) against an endogenous antigen, with consequent injury to tissues. Such diseases include, but are not limited to, graft rejection, graft versus host disease, an unwanted delayed-type hypersensitivity reaction, T-cell mediated pulmonary disease, insulin dependent diabetes mellitus (IDDM), systemic lupus erythematosus (SLE), rheumatoid arthritis, coeliac disease, multiple sclerosis, neuritis, polymyositis, psoriasis, vitiligo, Sjogren's syndrome, rheumatoid arthritis, autoimmune pancreatitis, inflammatory bowel diseases, Crohn's disease, ulcerative colitis, glomerulonephritis, scleroderma, sarcoidosis, autoimmune thyroid diseases, Hashimoto's thyroiditis, Graves disease, myasthenia gravis, asthma, Addison's disease, autoimmune uveoretinitis, pemphigus vulgans, primary biliary cirrhosis, pernicious anemia, pulmonary fibrosis or idiopathic pulmonary fibrosis.

**CD8+ T-cell mediated immunity:** An immune response implemented by presentation of antigens to CD8+ T-cells.

**cDNA (complementary DNA):** A piece of DNA lacking internal, noncoding segments (introns) and regulatory sequences that determine transcription. DNA is synthesized in the laboratory by reverse transcription from messenger RNA extracted from cells.

**Cytokine:** Proteins made by cells that affect the behavior of other cells, such as lymphocytes. In one embodiment, a cytokine is a chemokine, a molecule that affects cellular trafficking.

**Domain:** A domain of a polypeptide or protein is a discrete part of an amino acid sequence that can be equated with a particular function. For example, the α and P polypeptides that constitute a MHC class II molecule are each recognized as having two domains, α1, α2 and β1, β2, respectively, Similarly, the α chain of MHC class I molecules is recognized as having three domains, α1, α2 and α3. The various domains in each of these molecules are typically joined by linking amino acid sequences. In one embodiment of the present invention, the entire domain sequence is included in a recombinant molecule by extending the sequence to include all or part of the linker or the adjacent domain. For example, when selecting the α1 domain of HLA-DR A, the selected sequence will generally extend from amino acid residue number 1 of the α chain, through the entire α1 domain and will include all or part of the linker sequence located at about amino acid residues 76-90 (at the carboxy terminus of the α1 domain, between the α1 and α2 domains). The precise number of amino acids in the various MHC molecule domains varies depending on the species of mammal, as well as between classes of genes within a species. The critical aspect for selection of a sequence for use in a recombinant molecule is the maintenance of the domain function rather than a precise structural definition based on the number of amino acids, One of skill in the art will appreciate that domain function may be maintained even if somewhat less than the entire amino acid sequence of the selected domain is utilized. For example, a number of amino acids at either the amino or carboxy terminii of the α1 domain may be omitted without affecting domain function. Typically however, the number of amino acids omitted from either terminus of the domain sequence will be no greater than 10, and more typically no greater than 5 amino acids. The functional activity of a particular selected domain may be assessed in the context of the two-domain MHC polypeptides provided by this invention (i.e., the class II β1α1 or class I α1α2 polypeptides) using the antigen-specific T-cell proliferation assay as described in detail below. For example, to test a particular β1 domain, the domain will be linked to a functional α1 domain so as to produce a β1α1 molecule and then tested in the described assay. A biologically active β1α1 or α1α2 polypeptide will inhibit antigen-specific T-cell proliferation by at least about 50%, thus indicating that the component domains are functional. Typically, such polypeptides will inhibit T-cell proliferation in this assay system by at least 75% and sometimes by greater than about 90%.

**Demyelination:** Loss of myelin, a substance in the white matter that insulates nerve endings. Myelin helps the nerves receive and interpret messages from the brain at maximum speed. When nerve endings lose this substance they can not function properly, leading to patches of scarring or sclerosis.

**Epitope:** An antigenic determinant. These are particular chemical groups or peptide sequences on a molecule that are antigenic, i.e. that elicit a specific immune response. An antibody binds a particular antigenic epitope.

**Functionally Equivalent:** Sequence alterations, in either an antigen epitope or a β1α1, or an α1α2 peptide, that yield the same results as described herein. Such sequence alterations can include, but are not limited to, conservative substitutions, deletions, mutations, frame shifts, and insertions.

**IL-10:** A cytokine that is a homodimeric protein with subunits having a length of 160 amino acids. Human IL-10 has a 73 percent amino acid homology with murine IL-10. The human IL-10 gene contains four exons and maps to chromosome 1 (for review see de Waal-Malefyt R et al., Curr. Opin. Immunology 4: 314-20, 1992; Howard and O'Garra, Immunology Today 13: 198-200, 1992; Howard et al., J. Clin. Immunol. 12: 239-47, 1992).

IL-10 is produced by murine T-cells (Th2 cells but not Th1 cells) Following their stimulation by lectins. In humans, IL-10 is produced by activated CD 8+ peripheral blood T-cells, by Th0, Th1-, and Th2-like CD4+ T-cell clones after both antigen-specific and polyclonal activation, by B-cell lymphomas, and by LPS-activated monocytes and mast cells. B-cell lines derived from patients with acquired immunodeficiency syndrome and Burkitt's lymphoma constitutively secrete large quantities of IL10.

IL-10 has a variety of biological functions. For example, IL-10 inhibits the synthesis of a number of cytokines such as IFN-γ, IL-2 and TNF-α, in Th1 subpopulations of T-cells but not of Th2 cells. This activity is antagonized by IL-4. The inhibitory effect on IFN-γ production is indirect and appears to be the result of a suppression of IL-12 synthesis by accessory cells. In the human system, IL-10 is produced by, and down-regulates the function of, Th1 and Th2 cells. IL-10 is also known to inhibit the synthesis of IL-1, IL-6, and TNF-α by promoting, among other things, the degradation of cytokine mRNA. Expression of IL-10 can also lead to an inhibition of antigen presentation. In human monocytes, IFN-γ and IL-10 antagonize each other's production and function. In addition, IL-10 has been shown also to be a physiologic antagonist of IL-12. IL-10 also inhibits mitogen- or anti-CD3-induced proliferation of T-cells in the presence of accessory cells and reduces the production of IFN-γ and IL-2. IL-10 appears to be responsible for most or all of the ability of Th2 supernatants to inhibit cytokine synthesis by Th1 cells.

IL-10 can be detected with a sensitive ELISA assay. In addition, the murine mast cell line D36 can be used to bioassay human IL-10. Flow cytometry methods have also been used to detect 1L-10 (See Abrams et al. Immunol. Reviews 127: 5-24, 1992; Fiorentine et al., J. Immunol. 147: 3815-22, 1991; Kreft et al, J. Immunol. Methods 156: 125-8, 1992; Mosmann et al., J. Immunol. 145: 2938-45, 1990), sec also the Examples section below.

**Immune response:** A response of a cell of the immune system, such as a B cell, or a T-cell, to a stimulus. In one embodiment, the response is specific for a particular antigen (an "antigen-specific response"). In another embodiment, an immune response is a T-cell response, such as a Th1, Th2, or Th3 response. In yet another embodiment, an immune response is a response of a suppressor T-cell. In an additional embodiment, an immune response is a response of a dendritic cell.

**Isolated:** An "isolated" nucleic acid has been substantially separated or purified away from other nucleic acid sequences in the cell of the organism in which the nucleic acid naturally occurs, i.e., other chromosomal and extrachromosomal DNA and RNA. The term "isolated" thus encompasses nucleic acids purified by standard nucleic acid purification methods. The term also embraces nucleic acids prepared by recombinant expression in a host cell as well as chemically synthesized nucleic acids.

**Linker sequence:** A linker sequence is an amino acid sequence that covalently links two polypeptide domains. Linker sequences may be included in the recombinant MHC polypeptides of the present invention to provide rotational freedom to the linked polypeptide domains and thereby to promote proper domain folding and inter- and infra-domain bonding. By way of example, in a recombinant polypeptide comprising Ag-β1-α1 (where Ag= antigen) linker sequences may be provided between both the Ag and β1 domains and between β1 and α1 domains. Linker sequences, which are generally between 2 and 25 amino acids in length, arc well known in the art and include, but are not limited to, the glycine(4)-serine spacer described by Chaudhary *et al.* (1989). Other linker sequences are described in the Examples section below.

Recombinant MHC class I α1α2 polypeptides according to the present invention include a covalent linkage joining the carboxy terminus of the α1 domain to the amino terminus of the α2 domain. The α1 and α2 domains of native MHC class I α chains are typically covalently linked in this orientation by an amino acid linker sequence. This native linker sequence may be maintained in the recombinant constructs; alternatively, a recombinant linker sequence may be introduced between the α1 and α2 domains (either in place of or in addition to the native linker sequence).

**Mammal:** This term includes both human and non-human mammals. Similarly, the term "patient" or "subject" includes both human and veterinary subjects.

**Neurodegenerative disease:** A disorder which causes deterioration of essential cell and/or tissue components of the nervous system. Such diseases include, but are not limited to, multiple sclerosis (MS), Parkinson's disease, Alzheimer's disease, progressive multifocal leukoencephalopathy (PML), disseminated necrotizing leukoencephalpathy (DNL), acute disseminated encephalomyelitis, Schilder disease, central pontine myelinolysis (CPM), radiation necrosis, Binswanger disease (SAE), adrenoleukodystrophy, adrenomyeloneuropathy, Leber's hereditary optic atrophy, and HTLV-associate myelopathy.

**Operably linked:** A first nucleic acid sequence is operably linked with a second nucleic acid sequence when the first nucleic acid sequence is placed in a functional relationship with the second nucleic acid sequence. For instance, a promoter is operably linked to a coding sequence if the promoter effects the transcription or expression of the coding sequence. Generally, operably linked DNA sequences are contiguous and, where necessary to join two protein coding regions, the open reading frames are aligned.

**ORF (open reading frame):** A series of nucleotide triplets (codons) coding for amino acids without any termination codons. These sequences are usually translatable into a polypeptide.

**Pharmaceutical agent or drug:** A chemical compound or composition capable of inducing a desired therapeutic or prophylactic effect when properly administered to a subject.

**Pharmaceutically acceptable carriers:** The pharmaceutically acceptable carriers useful with the polypeptides and nucleic acids described herein are conventional. Remington's Pharmaceutical Sciences, by E. W. Martin, Mack Publishing Co., Easton, PA, 15th Edition (1975), describes compositions and formulations suitable for pharmaceutical delivery of the fusion proteins herein disclosed.

In general, the nature of the carrier will depend on the particular mode of administration being employed. For instance, parenteral formulations usually comprise injectable fluids that include pharmaceutically and physiologically acceptable fluids such as water, physiological saline, balanced salt solutions, aqueous dextrose, glycerol or the like as a vehicle. For solid compositions (*e.g.*, powder, pill, tablet, or capsule forms), conventional non-toxic solid carriers can include, for example, pharmaceutical grades of mannitol, lactose, starch, or magnesium stearate. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

**Preventing or treating a disease:** "Preventing" a disease refers to inhibiting the full development of a disease, for example in a person who is known to have a predisposition to a disease such as an autoimmune disorder or neurodegenerative disorder. An example of a person with a known predisposition is someone with a history of diabetes in the family, or someone who has a genetic marker for a disease, or someone who has been exposed to factors that predispose the subject to a condition, such as lupus or rheumatoid arthritis. "Preventing" a disease may also halt progression of the disease or stop relapses of a disease in someone who is exhibiting symptoms or who is currently in remission, with or without a known predisposition. "Treatment" refers to a therapeutic intervention that ameliorates a sign or symptom of a disease or pathological condition after it has begun to develop. Effectiveness of the treatment can be evaluated through a decrease in signs or symptoms of the disease or arresting or reversal of the progression of the disease, prevention of the recurrence of symptoms or prolonged periods of remission.

**Probes and primers:** Nucleic acid probes and primers may readily be prepared based on the nucleic acids provided by this invention. A probe comprises an isolated nucleic acid attached to a detectable label or reporter molecule. Typical labels include radioactive isotopes, ligands, chemiluminescent agents, and enzymes. Methods for labeling and guidance in the choice of labels appropriate for various purposes are discussed, e.g., in Sambrook *et al.* (1989) and Ausubel *et al.* (1987).

Primers are short nucleic acids, preferably DNA oligonucleotides 15 nucleotides or more in length. Primers may be annealed to a complementary target DNA strand by nucleic acid hybridization to form a hybrid between the primer and the target DNA strand, and then extended along the target DNA strand by a DNA polymerise enzyme. Primer pairs can be used for amplification of a nucleic acid sequence, e.g., by the polymerase chain reaction (PCR) or other nucleic-acid amplification methods known in the art.

Methods for preparing and using probes and primers are described, for example, in Sambrook *et al.* (1989), Ausubel *et al.* (1987), and Innis *et al.*, (1990). PCR primer pairs can be derived from a known sequence, for example, by using computer programs intended for that purpose such as Primer (Version 0.5, Ⓒ 1991, Whitehead Institute for Biomedical Research, Cambridge, MA).

**Purified:** The term purified does not require absolute purity; rather, it is intended as a relative term. Thus, for example, a purified recombinant MHC protein preparation is one in which the recombinant MHC protein is more pure than the protein in its originating environment within a cell. A preparation of a recombinant MHC protein is typically purified such that the recombinant MHC protein represents at least 50% of the total protein content of the preparation. However, more highly purified preparations may be required for certain applications. For example, for such applications, preparations in which the MHC protein comprises at least 75% or at least 90% of the total protein content may be employed.

**Recombinant:** A recombinant nucleic acid or polypeptide is one that has a sequence that is not naturally occurring or has a sequence that is made by an artificial combination of two or more otherwise separated segments of sequence. This artificial combination is often accomplished by chemical synthesis or, more commonly, by the artificial manipulation of isolated segments of nucleic acids, e.g., by genetic engineering techniques.

**Sequence identity:** The similarity between amino acid sequences is expressed in terms of the similarity between the sequences, otherwise referred to as sequence identity. Sequence identity is frequently measured in terms of percentage identity (or similarity or homology); the higher the percentage, the more similar the two sequences are. Variants of MHC domain polypeptides will possess a relatively high degree of sequence identity when aligned using standard methods. (An "MHC domain polypeptide" refers to a β1 or an α1 domain of an MHC class II polypeptide or an α1 or an α2 domain of an MHC class I polypeptide).

Methods of alignment of sequences for comparison are well known in the art. Altschul *et al.* (1994) presents a detailed consideration of sequence alignment methods and homology calculations. The NCBI Basic Local Alignment Search Tool (BLAST) (Altschul *et al.*, 1990) is available from several sources, including the National Center for Biotechnology Information (NCBI, Bethesda, MD) and on the Internet, for use in connection with the sequence analysis programs blastp, blastn, blastx, tblastn and tblastx. It can be accessed at the NCBI website. A description of how to determine sequence identity using this program is available at the NCBI website, as are the default parameters.

Variants of MHC domain polypeptides are typically characterized by possession of at least 50% sequence identity counted over the full length alignment with the amino acid sequence of a native MHC domain polypeptide using the NCBI Blast 2.0, gapped blastp set to default parameters. Proteins with even greater similarity to the reference sequences will show increasing percentage identities when assessed by this method, such as at least 60%, at least 65%, at least 70%, at least 75%, at least 80%, at least 90% or at least 95% amino acid sequence identity. When less than the entire sequence is being compared for sequence identity, variants will typically possess at least 75% sequence identity over short windows of 10-20 amino acids, and may possess sequence identities of at least 85% or at least 90% or 95% depending on their similarity to the reference sequence. Methods for determining sequence identity over such short windows are described at the NCBI website. Variants of MHC domain polypeptides also retain the biological activity of the native polypeptide. For the purposes of this invention, that activity is conveniently assessed by incorporating the variant domain in the appropriate β1α1 or α1α2 polypeptide and determining the ability of the resulting polypeptide to inhibit antigen specific T-cell proliferation *in vitro*, or to induce T suppressor cells or the expression of IL-10 as described in detail below.

**Therapeutically effective dose:** A dose sufficient to prevent advancement, or to cause regression of the disease, or which is capable of relieving symptoms caused by the disease, including, but not limited to, pain, swelling, numbness, spasticity, vertigo, dizziness, vision problems, motor control problems, balance or coordination problems, bowl dysfunction, and incontinence.

**Tolerance:** Diminished or absent capacity to make a specific immune response to an antigen. Tolerance is often produced as a result of contact with an antigen in the presence of a two domain MHC molecule, as described herein. In one embodiment, a B cell response is reduced or does not occur. In another embodiment, a T-cell response is reduced or does not occur. Alternatively, both a T-cell and a B cell response can be reduced or not occur.

**Transduced and Transformed:** A virus or vector "transduces" a cell when it transfers nucleic acid into the cell. A cell is "transformed" by a nucleic acid transduced into the cell when the DNA becomes stably replicated by the cell, either by incorporation of the nucleic acid into the cellular genome, or by episomal replication. As used herein, the term transformation encompasses all techniques by which a nucleic acid molecule might be introduced into such a cell, including transfection with viral vectors, transformation with plasmid vectors, and introduction of naked DNA by electroporation, lipofection, and particle gun acceleration.

**Vector:** A nucleic acid molecule as introduced into a host cell, thereby producing a transformed host cell. A vector may include nucleic acid sequences that permit it to replicate in the host cell, such as an origin of replication. A vector may also include one or more selectable marker genes and other genetic elements known in the art. The term "vector" includes viral vectors, such as adenoviruses, adeno-associated viruses, vaccinia, and retroviruses vectors.

Additional definitions of terms commonly used in molecular genetics can be found in Benjamin. Lewin, Genes V published by Oxford University Press, 1994 (ISBN 0-19-854287-9); Kendrew et al (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).

The following sections provide detailed guidance on the design, expression and uses of the recombinant MHC molecules of the invention. Unless otherwise stated, standard molecular biology, biochemistry and immunology methods are used in the present invention unless otherwise described. Such standard methods are described in Sambrook *et al.* (1989), Ausubel *et al*, (1987), Innis *et al.* (1990) and Harlow and Lane (1988). The following U.S. patents which relate to conventional formulations of MHC molecules and their uses are incorporated herein by reference to provide additional background and technical information relevant to the present invention: 5,130,297; 5,194,425; 5,260,422; 5,284,935; 5,468,481; 5,595,881; 5,635,363; 5,734,023.

### Design Of Recombinant MHC Class II β1α1 Molecules

The amino acid sequences of mammalian MHC class II α and β chain proteins, as well as nucleic acids encoding these proteins, are well known in the art and available from numerous sources including GenBank. Exemplary sequences are provided in Auffray *et al*. (1984) (human HLA DQ α); Larhammar *et al*. (1983) (human HLA DQ β); Das *et al.* (1983) (human HLA DR α); Tonnelle *et al.* (1985) (human HLA DR β); Lawrance *et al.* (1985) (human HLA DP α); Kelly *et al*. (1985) (human HLA DP β); Syha *et al.* (1989) (rat RT1.B α); Syha-Jedelhauser *et al*. (1991) (rat RT1.B β); Benoist *et al*. (1983) (mouse I-A α); Estess *et al*. (1986) (mouse I-A β), all of which arc incorporated by reference herein in their entirety. In one embodiment of the present invention, the MHC class II protein is a human MHC class II protein.

The recombinant MHC class II molecules of the present invention comprise the β1 domain of the MHC class 11 β chain covalently linked to the α1 domain of the MHC class II α chain. The α1 and β1 domains are well defined in mammalian MHC class II proteins. Typically, the α1 domain is regarded as comprising about residues 1-90 of the mature chain. The native peptide linker region between the α1 and α2 domains of the MHC class II protein spans from about amino acid 76 to about amino acid 93 of the α chain, depending on the particular α chain under consideration. Thus, an α1 domain may include about amino acid residues 1-90 of the α chain, but one of skill in the art will recognize that the C-terminal cut-off of this domain is not necessarily precisely defined, and, for example, might occur at any point between amino acid residues 70 100 of the α chain. The composition of the α1 domain may also vary outside of these parameters depending on the mammalian species and the particular α chain in question. One of skill in the art will appreciate that the precise numerical parameters of the amino acid sequence are much less important than the maintenance of domain function.

Similarly, the β1 domain is typically regarded as comprising about residues 1-90 of the mature β chain. The linker region between the β1 and the β2 domains of the MHC class II protein spans from about amino acid 85 to about amino acid 100 of the β chain, depending on the particular α chain under consideration. Thus, the β1 protein may include about amino acid residues 1-100, but one of skill in the art will again recognize that the C-terminal cut-off of this domain is not necessarily precisely defined, and, for example, might occur at any point between amino acid residues 75 - 105 of the β chain. The composition of the β1 domain may also vary outside of these parameters depending on the mammalian species and the particular the β chain in question. Again, one of skill in the art will appreciate that the precise numerical parameters of the amino acid sequence are much less important than the maintenance of domain function.

Exemplary β1α1 molecules from human, rat and mouse are depicted in Fig. 1. In one embodiment, the β1α1 molecules do not include a β2 domain. In another embodiment, the β1α1 molecules do not include an α2 domain. In yet a further embodiment, the β1α1 molecules do not include either an α2 or a β2 domain.

Nucleic acid molecules encoding these domains may be produced by standard means, such as amplification by polymerase chain reaction (PCR). Standard approaches for designing primers for amplifying open reading frames encoding these domains may be employed. Libraries suitable for the amplification of these domains include, for example, cDNA libraries prepared from the mammalian species in question. Such libraries are available commercially, or may be prepared by standard methods. Thus, for example, constructs encoding the β1 and α1 polypeptides may be produced by PCR using four primers: primers B1 and B2 corresponding to the 5' and 3' ends of the β1 coding region, and primers A1 and A2 corresponding to the 5' and 3' ends of the α1 coding region. Following PCR amplification of the β1 and α1 domain coding regions, these amplified nucleic acid molecules may each be cloned into standard cloning vectors, or the molecules may be ligated together and then cloned into a suitable vector, To facilitate convenient cloning of the two coding regions, restriction endonuclease recognition sites may be designed into the PCR primers. For example, primers B2 and A1 may each include a suitable site such that the amplified fragments may be readily ligated together following amplification and digestion with the selected restriction enzyme. In addition, primers B1 and A2 may each include restriction sites to facilitate cloning into the polylinker site of the selected vector. Ligation of the two domain coding regions is performed such that the coding regions are operably linked, i.e., to maintain the open reading frame. Where the amplified coding regions are separately cloned, the fragments may be subsequently released from the cloning vector and gel purified, preparatory to ligation.

In certain embodiments, a peptide linker is provided between the β1 and α1 domains. Typically, this linker is between 2 and 25 amino acids in length, and serves to provide flexibility between the domains such that each domain is free to fold into its native conformation. The linker sequence may conveniently be provided by designing the PCR primers to encode the linker sequence. Thus, in the example described above, the linker sequence may be encoded by one of the B2 or A1 primers, or a combination of each of these primers.

### Design Of Recombinant MHC Class I α α1α2 Molecules

The amino acid sequences of mammalian MHC class I α chain proteins, as well as nucleic acids encoding these proteins, are well known in the art and available from numerous sources including GenBank. Exemplary sequences are provided in Browning *et al*. (1995) (human HLA-A); Kato *et al*, (1993) (human HLA-B); Steinle *et al*. (1992) (human HLA-C); Walter *et al.* (1995) (rat Ia); Walter *et al.* (1994) (rat Ib); Kress *et al.* (1983) (mouse H-2-K); Schepart *et al*. (1986) (mouse H-2-D); and Moore *et al*. (1982) (mouse H-2-I), which are incorporated by reference herein. In one embodiment, the MHC class I protein is a human MHC class I protein.

The recombinant MHC class I molecules of the present invention comprise the α1 domain of the MHC class I α chain covalently linked to the α2 domain of the MHC class I chain. These two domains are well delined in mammalian MHC class I proteins. Typically, the α1 domain is regarded as comprising about residues 1-90 of the mature chain and the α2 chain as comprising about amino acid residues 90-180, although again, the beginning and ending points are not precisely defined and will vary between different MHC class I molecules. The boundary between the α2 and α3 domains of the MHC class I α protein typically occurs in the region of amino acids 179-183 of the mature chain. The composition of the α1 and α2 domains may also vary outside of these parameters depending on the mammalian species and the particular α chain in question. One of skill in the art will appreciate that the precise numerical parameters of the amino acid sequence are much less important than the maintenance of domain function. An exemplary α1α2 molecule is shown in Fig. 2. In one embodiment, the α1α2 molecule does not include an α3 domain.

The α1α2 construct may be most conveniently constructed by amplifying the reading frame encoding the dual-domain (α1 and α2) region between amino acid number 1 and amino acids 179-183, although one of skill in the art will appreciate that some variation in these end-points is possible. Such a molecule includes the native linker region between the α1 and α2 domains, but if desired that linker region may be removed and replaced with a synthetic linker peptide. The general considerations for amplifying and cloning the MHC class I α1 and α2 domains apply as discussed above in the context of the class II β1 and α1 domains.

### Genetic Linkage of Antigenic Polypeptide to β1α1 and α1α2 Molecules

The class II β1α1 and class I α1α2 polypeptides of the invention are generally used in conjunction with an antigenic peptide. Any antigenic peptide that is conventionally associated with class I or class II MHC molecules and recognized by a T-cell can be used for this purpose. Antigenic peptides from a number of sources have been characterized in detail, including antigenic peptides from honey bee venom allergens, dust mite allergens, toxins produced by bacteria (such as tetanus toxin) and human tissue antigens involved in autoimmune diseases. Detailed discussions of such peptides are presented in U.S. Patent Nos. 5,595,881, 5,468,481 and 5,284,935 to Kendrich et al., Sharma et al., and Clark et al., respectively, each of which is incorporated herein by reference. Exemplary peptides include, but are not limited to, those identified in the pathogenesis of rheumatoid arthritis (type II collagen), myasthenia gravis (acetylcholine receptor), diabetes (insulin, glutamate decarboxylase), Hashimoto's Thyroiditis, (Thyroglobulin), Grave's Disease (Thyrodoxin), uveitis (S-antigen), inflammatory bowel disease, (Ach (Acetylcholine) receptor), coeliac disease (cyclooxygenase-2 inhibitor, dietary hen egg white lysozome), neuritis (pertussis toxin), polymyositis (myosin B, ross river virus), glomerulonephritis (anti-GBM serum), autoimmune thyroid disease (recombinant murine TPO ectodomain), Addison's disease (syngeneic adrenal extract with Klebsiella), autoimmune uveoretinitis (retinal extract), autoimmune pancreatitis (polyinosinic:polycytidylic acid), primary biliary cirrhosis (lipoplysaccharicle derived from Salmonella minnesota Re595), and multiple sclerosis (myelin basic protein).

As is well known in the art (see for example U.S. Patent No. 5,468,481 to Sharma et al.) the presentation of antigen in MHC complexes on the surface of APCs generally does not involve a whole antigenic peptide. Rather, a peptide located in the groove between the β1 and α1 domains (in the case of MHC II) or the α1 and α2 domains (in the case of MHC I) is typically a small fragment of the whole antigenic peptide. As discussed in Janeway & Travers (1997), peptides located in the peptide groove of MHC class I molecules are constrained by the size of the binding pocket and are typically 8-15 amino acids long, more typically 8-10 amino acids in length (but see Collins *et al*., 1994 for possible exceptions). In contrast, peptides located in the peptide groove of MHC class II molecules are not constrained in this way and are often much larger, typically at least 13 amino acids in length. Peptide fragments for loading into MHC molecules can be prepared by standard means, such as use of synthetic peptide synthesis machines.

The β1α1 and α1α2 molecules of the present invention may be "loaded" with peptide antigen in a number of ways, including by covalent attachment of the peptide to the MHC molecule. This may be conveniently achieved by operably linking a nucleic acid sequence encoding the selected peptide to the 5' end of the construct encoding the MHC protein such that, in the expressed peptide, the antigenic peptide domain is linked to the N-terminus of β1 in the case of β1α1 molecules and α1 in the case of α1α2 molecules. One way of obtaining this result is to incorporate a sequence encoding the antigen into the PCR primers used to the MHC coding regions. Typically, a sequence encoding a linker peptide sequence will be included between the molecules encoding the antigenic peptide and the MHC polypeptide. As discussed above, the purpose of such linker peptides is to provide flexibility and permit proper conformational folding of the peptides. For linking antigens to the MHC polypeptide, the linker should be sufficiently long to permit the antigen to fit into the peptide groove of the MHC polypeptide. Again, this linker may be conveniently incorporated into the PCR primers. However, as discussed in Example 1 below, it is not necessary that the antigenic peptide be ligated exactly at the 5' end of the MHC coding region. For example, the antigenic coding region may be inserted within the first few (typically within the first 10) codons of the 5' end of the MHC coding sequence.

This genetic system for linkage of the antigenic peptide to the MHC molecule is particularly useful where a number of MHC molecules with differing antigenic peptides are to be produced. The described system permits the construction of an expression vector in which a unique restriction site is included at the 5' end of the MHC coding region (i.e., at the 5' end of β1 in the case of β1α1-encoding constructs and at the 5' end of α1 in the case of α1α2-encoding constructs). In conjunction with such a construct, a library of antigenic peptide-encoding sequences is made, with each antigen-coding region flanked by sites for the selected restriction enzyme. The inclusion of a particular antigen into the MHC molecule is then performed simply by (a) releasing the antigen-coding region with the selected restriction enzyme, (b) cleaving the MHC construct with the same restriction enzyme, and (c) ligating the antigen coding region into the MHC construct. In this manner, a large number of MHC-polypeptide constructs can be made and expressed in a short period of time.

An exemplary design of an expression cassette allowing simple exchange of antigenic peptides in the context of a β1α1 molecule is shown in Fig. 1. Fig 1A shows the nucleic acid sequence encoding a prototype β1α1 molecule derived from rat MHC class II RT1.B, without the presence of the antigenic peptide. The position of the insertion site for the peptide and linker between the 5^{th} and 6^{th} (serine and proline) residues of the β1 domain is indicated by a τ symbol. In order to integrate the antigen coding region, a PCR primer comprising the sequence shown in Fig. 1B joined with additional bases from the Fig. 1A construct 3' of the insertion site is employed in conjunction with a PCR primer reading from the 3' end of the construct shown in Fig. 1A.) Amplification yields a product that includes the sequence shown in Fig. 1B integrated into the β1α1 construct (i.e., with the antigenic peptide and linker sequences positioned between the codons encoding the 5^{th} and 6^{th} amino acid residues of the β1α1 sequence). In the case illustrated, the antigenic peptide is the MBP-72-89 antigen.

Notably, the MBP-72-89 coding sequence is flanked by unique Nco I and Spe I restriction enzyme sites. These enzymes can be used to release the MBP-72-89 coding region and replace it with coding regions for other antigens, for example those illustrated in Figs. 1C and 1D.

The structure of the expressed β1α1 polypeptide with covalently attached antigen is illustrated in Fig. 2B; Fig. 2A shows the secondary structure of the complete RT1B molecule (including β1, β2, α1 and α2 domains).

Nucleic acid expression vectors including expression cassettes designed as explained above will be particularly useful for research purposes. Such vectors will typically include sequences encoding the dual domain MHC polypeptide (β1α1 or α1α2) with a unique restriction site provided towards the 5' terminus of the MHC coding region, such that a sequence encoding an antigenic polypeptide may be conveniently attached. Such vectors will also typically include a promoter operably linked to the 5' terminus of the MHC coding region to provide for high level expression of the sequences.

β1α1 and α1 α2 molecules may also be expressed and purified without an attached peptide (as described below), in which case they may be referred to as "empty". The empty MHC molecules may then be loaded with the selected peptide as described below in "Antigen Loading of Empty β1α1 and α1α2 Molecules".

### Expression and Purification of Recombinant β1α1 and α1α2 Molecules

In their most basic form, nucleic acids encoding the MHC polypeptides of the invention comprise first and second regions, having a structure A-B wherein, for class I molecules, region A encodes the class Iα1 domain and region B encodes the class I α2 domain. For class II molecules, A encodes the class IIα1 domain and B encodes the class IIβ1 domain. Where a linker sequence is included, the nucleic acid may be represented as B-L2-A, wherein L2 is a nucleic acid sequence encoding the linker peptide. Where an antigenic peptide is covalently linked to the polypeptide, the nucleic acid molecule encoding this complex may be represented as P-B-A. A second linker sequence may be provided between the antigenic protein and the region B polypeptide, such that the coding sequence is represented as P-L2-B-LI-A. In all instances, the various nucleic acid sequences that comprise the MHC polypeptide (i.e., L1, L2, B, A and P) are operably linked such that the elements are situated in a single reading frame.

Nucleic acid constructs expressing these MHC polypeptides may also include regulatory elements such as promoters (Pr), enhancers and 3' regulatory regions, the selection of which will be determined based upon the type of cell in which the protein is to be expressed. When a promoter sequence is operably linked to the open reading frame, the sequence may be represented as Pr-B-A, or (if an antigen-coding region is included) Pr-P-B-A, wherein Par represents the promoter sequence. The promoter sequence is operably linked to the P or B components of these sequences, and the B-A or P-B-A sequences comprise a single open reading frame. The constructs are introduced into a vector suitable for expressing the MHC polypeptide in the selected cell type.

Numerous prokaryotic and eukaryotic systems are known for the expression and purification of polypeptides. For example, heterologous polypeptides can be produced in prokaryotic cells by placing a strong, regulated promoter and an efficient ribosome binding site upstream of the polypeptide-encoding construct. Suitable promoter sequences include the β-lactamase, tryptophan (trp), 'phage T7 and lambda P_{L} promoters. Methods and plasmid vectors for producing heterologous proteins in bacteria are described in Sambrook *et al.* (1989). Suitable prokaryotic cells for expression of large amounts of ₂m fusion proteins include *Escherichia coli* and *Bacillus subtilis*. Often, proteins expressed at high levels are found in insoluble inclusion bodies; methods for extracting proteins from these aggregates are described by Sambrook *et al.* (1989, see ch. 17). Recombinant expression of MHC polypeptides in prokaryotic cells may alternatively be conveniently obtained using commercial systems designed for optimal expression and purification of fusion proteins. Such fusion proteins typically include a protein tag that facilitates purification. Examples of such systems include, but are not limited to: the pMAL protein fusion and purification system (New England Biolabs, Inc., Beverly, MA); the GST gene fusion system (Amersham Pharmacia Biotech, Inc., Piscataway, NJ); and the pTrcHis expression vector system (Invitrogen, Carlsbad, CA). For example, the pMAL expression system utilizes a vector that adds a maltose binding protein to the expressed protein. The fusion protein is expressed in *E. coli* and the fusion protein is purified from a crude cell extract using an amylose column. If necessary, the maltose binding protein domain can be cleaved from the fusion protein by treatment with a suitable protease, such as Factor Xa. The maltose binding fragment can then be removed from the preparation by passage over a second amylose column.

The MHC polypeptides can also be expressed in eukaryotic expression systems, including *Pichia pastoris*, *Drosophila*, Baculovirus and Sindbis expression systems produced by Invitrogen (Carlsbad, CA). Eukaryotic cells such as Chinese Hamster ovary (CHO), monkey kidney (COS), HeLa, *Spodoptera frugiperda*, and *Saccharomyces* may also be used to express the MHC polypeptides. Regulatory regions suitable for use in these cells include, for mammalian cells, viral promoters such as those from CMV, adenovirus and SV40, and for yeast cells, the promoter for 3-phosphoglycerate kinase and alcohol dehydrogenase.

The transfer of DNA into eukaryotic, in particular human or other mammalian cells is now a conventional technique. The vectors are introduced into the recipient cells as pure DNA (transfection) by, for example, precipitation with calcium phosphate or strontium phosphate, electroporation, lipofection, DEAE dextran, microinjection, protoplast fusion, or microprojectile guns. Alternatively, the nucleic acid molecules can be introduced by infection with virus vectors. Systems are developed that use, for example, retroviruses, adenoviruses, or Herpes virus.

An MHC polypeptide produced in mammalian cells may be extracted following release of the protein into the supernatant and may be purified using an immunoaffinity column prepared using anti-MHC antibodies. Alternatively, the MHC polypeptide may be expressed as a chimeric protein with, for example, b-globin. Antibody to b-globin is thereafter used to purify the chimeric protein. Corresponding protease cleavage sites engineered between the b-globin gene and the nucleic acid sequence encoding the MHC polypeptide are then used to separate the two polypeptide fragments from one another after translation. One useful expression vector for generating b-globin chimeric proteins is pSG5 (Stratagene, La Jolla, CA).

Expression of the MHC polypeptides in prokaryotic cells will result in polypeptides that are not glycosylated. Glycosylation of the polypeptides at naturally occurring glycosylation target sites may be achieved by expression of the polypeptides in suitable eukaryotic expression systems, such as mammalian cells.

Purification of the expressed protein is generally performed in a basic solution (typically around pH 10) containing 6M urea. Folding of the purified protein is then achieved by dialysis against a buffered solution at neutral pH (typically phosphate buffered saline (PBS) at around pH 7.4).

### Antigen Loading of Empty β1α1 and α1α2 Molecules

Where the β1α1 and α1α2 molecules are expressed and purified in an empty form (i.e., without attached antigenic peptide), the antigenic peptide maybe loaded into the molecules using standard methods. Methods for loading antigenic peptides into MHC molecules is described in, for example, U.S. Patent No. 5,468,481 to Sharma et al. herein incorporated by reference in its entirety. Such methods include simple co-incubation of the purified MHC molecule with a purified preparation of the antigen.

By way of example, empty ß1α1 molecules (1mg/ml; 40uM) maybe loaded by incubation with a 10-fold molar excess of peptide (1mg/ml; 400uM) at room temperature, for 24 hours. Thereafter, excess unbound peptide may be removed by dialysis against PBS at 4°C for 24 hours. As is known in the art, peptide binding to ß1α1 can be quantified by silica gel thin layer chromatography (TLC) using radiolabeled peptide. Based on such quantification, the loading may be altered (e.g., by changing the molar excess of peptide or the time of incubation) to obtain the desired result.

### Other Considerations

### (a) Sequence variants

While the foregoing discussion uses naturally occurring MHC class I and class II molecules and the various domains of these molecules as examples; one of skill in the art will appreciate that variants of these molecules and domains may be made and utilized in the same manner as described. Thus, reference herein to a domain of an MHC polypeptide or molecule (e.g., an MHC class II β1 domain) includes both naturally occurring forms of the referenced molecule, as well as molecules that are based on the amino acid sequence of the naturally occurring form, but which include one or more amino acid sequence variations. Such variant polypeptides may also be defined in the degree of amino acid sequence identity that they share with the naturally occurring molecule. Typically, MHC domain variants will share at least 80% sequence identity with the sequence of the naturally occurring MHC domain. More highly conserved variants will share at least 90% or at least 95% sequence identity with the naturally occurring sequence. Variants of MHC domain polypeptide also retain the biological activity of the naturally occurring polypeptide. For the purposes of this invention, that activity is conveniently assessed by incorporating the variant domain in the appropriate β1α1 or α1α2 polypeptide and determining the ability of the resulting polypeptide to inhibit antigen specific T-cell proliferation *in vitro*, as described in detail below.

Variant MHC domain polypeptides include proteins that differ in amino acid sequence from the naturally occurring MHC polypeptide sequence but which retain the specified biological activity. Such proteins may be produced by manipulating the nuclcotide sequence of the molecule encoding the domain, for example by site-directed mutagenesis or the polymerase chain reaction. The simplest modifications involve the substitution of one or more amino acids for amino acids having similar biochemical properties, i.e. a "conservative substitution." Conservative substitution tables providing functionally similar amino acids are well known in the art. The following six groups each contain amino acids that are conservative substitutions for one another and are likely to have minimal impact on the activity of the resultant protein.

### Alanine (A), Serine (S), Threonine (T);

2) Aspartic acid (D), Glutamic acid (E);
3) Asparagine (N), Glutamine (Q);
4) Arginine (R), Lysine (K);
5) Isoloucine (I), Leucine (L), Methionine (M), Valine (V); and
6) Phenylalanine (F), Tyrosine (Y), Tryptophan (W). (see, e.g., Creighton, Proteins (W. H. Freeman & Co., New York, N.Y. 1984)).

More substantial changes in biological function or other features may be obtained by selecting substitutions that are less conservative than those shown above, i.e., selecting residues that differ more significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. The substitutions which in general are expected to produce the greatest changes in protein properties will be those in which (a) a hydrophilic residue, e.g., seryl or threonyl, is substituted for (or by) a hydrophobic residue, e.g., leucyl, isoleucyl, phenylalanyl, valyl or alanyl; (b) a cystyl or prolyl is substituted for (or by) any other residue; (c) a residue having an electropositive side chain, e.g., lysyl, arginyl, or histadyl, is substituted for (or by) an electronegative residue, e.g., glutamyl or aspartyl; or (d) a residue having a bulky side chain, e.g., phenylalanyl, is substituted for (or by) one not having a side chain, e.g., glycyl. The effects of these amino acid substitutions or deletions or additions may be assessed through the use of the described T-cell proliferation assay.

At the nucleic acid level, one of skill in the art will appreciate that the naturally occurring nucleic acid sequences that encode class I and II MHC domains may be employed in the expression, vectors, but that the invention is not limited to such sequences. Any sequence that encodes a functional MHC domain may be employed, and the nucleic acid sequence may be adapted to conform with the codon usage bias of the organism in which the sequence is to be expressed.

### (b) Incorporation of Detectable Markers

For certain *in vivo* and *in vitro* applications, the MHC molecules of the present invention may be conjugated with a detectable label. A wide range of detectable labels are known, including radionuclides (e.g., gamma-emitting sources such as indium-111), paramagnetic isotopes, fluorescent markers (e.g., fluorescein), enzymes (such as alkaline phosphatase), cofactors, chemiluminescent compounds and bioluminescent compounds. The binding of such labels to the MHC polypeptides may be achieved using standard methods. U.S. Patent No. 5,734,023 (incorporated herein by reference) contains an extensive discussion of the labeling of MHC polypeptide derivatives using such labels. Where the detectable marker is to be covalently linked to the MHC molecule in a directed manner (i.e., rather than being randomly attached) it will generally be linked to the C terminus of the molecule so as to minimize interference with a peptide antigen linked at the N terminus.

### (c) Conjugation of Toxic Moieties

For certain uses of the disclosed MHC polypeptides, particularly *in vivo* therapeutic applications aimed at depleting certain T-cell populations, the polypeptides may be conjugated with a toxic moiety. Numerous toxic moieties suitable for disrupting T-cell function are known, including, but not limited to, protein toxins, chemotherapeutic agents, antibodies to a cytotoxic T-cell surface molecule, lipases, and radioisotopes emitting "hard" e.g., beta radiation. Examples of such toxins and methods of conjugating toxins to MHC molecules are described in U.S. Patent No. 5,284,935 (incorporated herein by reference). Protein toxins include ricin, diphtheria and, Pseudomonas toxin. Chemotherapeutic agents include doxorubicin, daunorubicin, methotrexate, cytotoxin, and antisense RNA. Radioisotopes such as yttrium-90, phosphorus-32, lead-212, iodine-131, or palladium-1 09 may also be used. Where the toxic moiety is to be covalently linked to the MHC molecule in a directed manner (i.e., rather than being randomly attached) it will generally be linked to the C terminus of the molecule so as to minimize interference with a peptide antigen linked at the N terminus.

In other aspects of the invention, modified recombinant T-cell receptor ligands (RTL) are designed and constructed which comprise a major histocompatibility complex (MHC) component that incorporates one or more redesigned surface structural features which have been recombinantly introduced into an otherwise native MHC polypeptide sequence. Typically, modified RTLs of the invention are rationally designed and constructed to introduce one or more amino acid changes at a solvent-exposed target site located within, or defining, a self-binding interface found in the native MHC polypeptides.

The self-binding interface that is altered in the modified RTL typically comprises one or more amino acid residue(s) that mediate(s) self-aggregation of a native MHC polypeptide, or of an "unmodified" RTL incorporating the native MHC polypeptide. Although the self-binding interface is correlated with the primary structure of the native MHC polypeptide, this interface may only appear as an aggregation" promoting surface feature when the native polypeptide is isolated from the intact MHC complex and incorporated in the context of an "unmodified" RTL.

Thus, in certain embodiments, the self-binding interface may only function as a solvent-exposed residue or motif of an unmodified RTL after the native polypeptide is isolated from one or more structural element(s) found in an intact MHC protein, In the case of exemplary MHC class II RTLs described herein (e.g., comprising linked β1 and α1 domains), the native β1α1. structure only exhibits certain solvent-exposed, self-binding residues or motifs after removal of Ig-fold like, β2 and α2 domains found in the intact MHC II complex. These same residues or motifs that mediate aggregation of unmodified β1α1 RTLs, are presumptively "buried" in a solvent-inaccessible conformation or otherwise "masked" (i.e., prevented from mediating self-association) in the native or progenitor MHC II complex (likely through association with the Ig-fold like, β2 and α2 domains).

Certain modified RTLs of the invention include a multi-domain structure comprising selected MHC class I or MHC class II domains, or portions of multiple MHC domains that are necessary to form a minimal Ag recognition/T-cell receptor (TCR) interface (i.e., which is capable of mediating Ag binding and TCR recognition). In certain embodiments, the modified RTL comprises a "minimal TCR interface", meaning a minimal subset of MHC class I or MHC class II domain residues necessary and sufficient to mediate functional peptide binding and TCR-recognition. TCR recognition requires that the modified RTL be capable of interacting with the TCR in an Ag-specific manner to elicit one or more TCR-mediated T-cell responses, as described herein.

In the case of modified RTLs derived from human class II MHC molecules, the RTLs will most often comprise α1 and β1 MHC polypeptide domains of an MHC class II protein, or portions thereof sufficient to provide a minimal TCR interface. These domains or subportions thereof may be covalently linked to form a single chain (sc) MHC class II polypeptide. Such RTL polypeptides are hereinafter referred to as "α1β1" sc MHC class II polypeptides. Equivalent sc MHC constructs can be modeled from human MHC class I proteins, for example to form RTLs comprising α1 and α2 domains (or portions thereof sufficient to provide a minimal TCR interface) of a class I MHC protein, wherein the RTL is optionally "empty" or associated with an Ag comprising a CD8+ T-cell epitope.

RTL constructs comprising sc MHC components have been shown to be widely useful for such applications as preventing and treating Ag-induced autoimmune disease responses in mammalian model subjects predictive of autoimmune disease therapeutic activity in humans (Burrows et al., J. Immunol. 161:5987, 1998; Burrows et al., J. Immunol. 164:6366, 2000). In other aspects, these types of RTLs have been demonstrated to inhibit T-cell activation and induce anti-inflammatory cytokine (e.g., IL-10) secretion in human DR2-restricted T-cell clones specific for MBP-85-95 or BCR-ABL b3a2 peptide (CABL) (Burrows et al., J. Immunol. 167:4386, 2001; Chang et al., J. Biol. Chem. 276:24170, 2001).

Additional RTL constructs have been designed and tested by inventors in the instant application, which include a MOG-35-55/DR2 construct (VG312) shown to potently inhibit autoimmune responses and lead to immunological tolerance to the encephalitogenic MOG-35-55 peptide and reverse clinical and histological signs of EAE (Vandenbark et al., J. Immunol. 171:127-33, 2003). Numerous additional RTL constructs that are useful for modulating T-cell immune responses and can be employed within the invention are available for use within the methods and compositions of the invention (see, e.g., United States Patent No. 5,270,772, issued August 7, 2001; United States Provisional Patent Application No. 60/200,942, filed May 1, 2000; United States Patent Application No. 101936,467, filed by Burrows et al. on September 7, 2004; United States Patent No. 6,270,772, issued August 7, 2001; United States Patent Application No. 09/847,172, filed May 1, 2001; and United States Patent No. 6,81.5,171, issued November 9, 2004, each incorporated herein by reference).

To evaluate the biological function and mechanisms of action of modified RTLs of the invention, antigen-specific T-cells bearing cognate TCRs have been used as target T-cells for various assays (*see*, *e.g.,* Burrows et al., J. Immunol. 167:4386, 2001). More recently, inventors in the current application have provided novel T-cell hybridomas that are uniquely adapted for use in screens and assays to identify and characterize RTL structure and function (see, e.g., United States Provisional Patent Application No. 60/586,433, filed July 7, 2004; and Chou et al., J. Neurosci. Res. 77: 670-680, 2004). To practice these aspects of the invention, T-cell hybrids are constructed and selected that display an Ag-specific, TCR-mediated proliferative response following contact of the hybrid with a cognate Ag and APCs. This proliferative response of T hybrids can in turn be detectably inhibited or stimulated by contacting the hybrids with a modified RTL of interest, which yields a modified, Ag-specific, TCR-mediated proliferation response of the hybrid. The modified proliferation response of the hybrid cell accurately and reproducibly indicates a presence, quantity, and/or activity level of the modified RTL in contact with the T-cell hybrid.

Within certain embodiments of the invention, an isolated, modified recombinant RTL which has a reduced potential for aggregation in solution comprises an "MHC component" in the form of a single chain (sc) polypeptide that includes multiple, covalently-linked MHC domain elements. These domain elements are typically selected from a) α1 and β1 domains of an MHC class II polypeptide, or portions thereof comprising an Ag-binding pocket/T-cell receptor (TCR) interface; or b) α1 and α2 domains of an MHC class I polypeptide, or portions thereof comprising an Ag-binding pocket/TCR interface. The MHC component of the RTL is modified by one or more amino acid substitution(s), addition(s), deletion(s), or rearrangement(s) at a target site corresponding to a "self-binding interface" identified in a native MHC polypeptide component of an unmodified RTL. The modified RTL exhibits a markedly reduced propensity for aggregation in solution compared to aggregation exhibited by an unmodified, control RTL having the same fundamental MHC component structure, but incorporating the native MHC polypeptide defining the self-binding interface.

As used herein, "native MHC polypeptide" refers to intact, naturally-occurring MHC polypeptides, as well as to engineered or synthetic fragments, domains, conjugates, or other derivatives of MHC polypeptides that have an identical or highly conserved amino acid sequence compared to an aligned sequence in the naturally-occurring MHC polypeptide (e.g., marked by 85%, 90%, 95% or greater amino acid identity over an aligned stretch of corresponding residues. The "native MHC polypeptide" having the self-associating interface will often be an MHC polypeptide domain incorporated within an unmodified RTL, and the self-associating interface may only be present in such a context, as opposed to when the native MHC polypeptide is present in a fully intact, native MHC protein (e.g., in a heterodimeric MHC class II protein complex).

Thus, in the case of MHC class II RTLs, removal of the β2 and α2 domains to create a smaller, more useful (e.g., β1α1) domain structure for the RTL (comprising a minimal TCR interface) results in "unmasking" (i.e., rendering solvent-exposed) certain self-binding residues or motifs that comprise target sites for RTL modification according to the invention. These unmasked residues or motifs can be readily altered, for example by site-directed mutagenesis, to reduce or eliminate aggregation and render the RTL as a more highly monodisperse reagent in aqueous solution.

To evaluate the extent of monodispersal of these modified RTLs, an unmodified or "control" RTL may be employed which has the same basic polypeptide construction as the modified RTL, but features the native MHC polypeptide sequence (having one or more amino acid residues or motifs comprising the self-binding interface and defining a solvent-exposed target site for the modification when the native polypeptide is incorporated in the RTL).

The modified RTLs of the invention yield an increased percentage of monodisperse molecules in solution compared to a corresponding, unmodified RTL (i.e., comprising the native MHC polypeptide and bearing the unmodified, self-binding interface). In certain embodiments, the percentage of unmodified RTL present as a monodisperse species in aqueous solution may be as low as 1%, more typically 5-10% or less of total RTL protein, with the balance of the unmodified RTL being found in the form of higher-order aggregates. In contrast, modified of the present invention will yield at least 10%-20% monodisperse species in solution. In other embodiments, the percentage of monomeric species in solution will range from 25%-40%, often 50%-75%, up to 85%, 90%, 95% or greater of the total RTL present, with a commensurate reduction in the percentage of aggregate RTL species compared to quantities observed for the corresponding, unmodificl RTLs under comparable conditions.

The self-binding interface that is altered in the MHC polypeptide to form the modified RTL may comprise single or multiple amino acid residues, or a defined region, domain, or motif of the MHC polypeptide, which is characterized by an ability to mediate self-binding or self-association of the MHC polypeptide and/or RTL. As used herein, "self-binding" and "self-association" refers to any intermolecular binding or association that promotes aggregation of the MHC polypeptide or RTL in a physiologically-compatible solution, such as water, saline, or serum.

As noted above, MHC class II molecules comprise non-covalently associated, α- and ß-polypeptide chains. The α-chain comprises two distinct domains termed α1 and α2. The β-chain also comprises two domains, β1 and β2. The peptide binding pocket of MHC class II molecules is formed by interaction of the α1 and β1 domains. Peptides from processed antigen bind to MHC molecules in the membrane distal pocket formed by the ß1 and α1 domains (Brown *et al*., 1993; Stern *et al*., 1994). Structural analysis of human MHC class II/peptide complexes (Brown et al., Nature 364:33-39, 1993; Stern et al., Nature 368:215, 1994) demonstrate that side chains of bound peptide interact with "pockets" comprised of polymorphic residues within the class II binding groove. The bound peptides have class II allele-specific motifs, characterized by strong preferences for specific amino acids at positions that anchor the peptide to the binding pocket and a wide tolerance for a variety of different amino acids at other positions (Stern et al., Nature 368:215, 1994; Rammensee et al., Immunogenetics 41: 178, 1995). Based on these properties, natural populations of MHC class II molecules are highly heterogeneous. A given allele of class II molecules on the surface of a cell has the ability to bind and present over 2000 different peptides. In addition, bound peptides dissociate from class II molecules with very slow rate constants. Thus, it has been difficult to generate or obtain homogeneous populations of class II molecules bound to specific antigenic peptides.

The α2 and β2 domains of HHC class II molecules comprise distinct, transmembrane Ig-fold like domains that anchor the α- and β-chains into the membrane of the APC. In addition, the α2 domain is reported to contribute to ordered oligomerization during T-cell activation (König et al., J. Exp. Med. 182:778-787,1995 ), while the ß2 domain is reported to contain a CD4 binding site that co-ligates CD4 when the MHC-antigen complex interacts with the TCR αβ heterodimer (Fleury et al., Cell 66:1037-1049, 1991; Cammarota et al., Nature 356:799-801, 1992 ; König et al., Nature 356:796-798, 1992; Hang et al., J. Immunol. 158:216-225, 1997).

RTLs modeled after MHC class II molecules for use within the invention typically comprise small (e.g., approximately 200 amino acid residues) molecules comprising all or portions of the α1 and ß1 domains of human and non-human MHC class II molecules, which are typically genetically linked into a single polypeptide chain (with and without covalently coupled antigenic peptide). Exemplary MHC class II-derived "ß1α1" molecules retain the biochemical properties required for peptide binding and TCR engagement (including TCR binding and/or partial or complete TCR. activation). This provides for ready production of large amounts of the engineered RTL for structural characterization and immunotherapeutic applications. The MHC component of MHC class II RTLs comprise a minimal, Ag-binding/T-cell recognition interface, which may comprise all or portions of the MHC class II α1 and β1 domains of a selected MHC class II molecule. These RTLs are designed using the structural backbone of MHC class II molecules as a template. Structural characterization of RTLs using circular dichroism indicates that these molecules retain an antiparallel β-sheet platform and anti-parallel α-helices observed in the corresponding, native (i.e., wild-type sequence) MHC class II heterodimer. These RTLs also exhibit a cooperative two-state thermal folding-unfolding transition. When the RTL is covalently linked with Ag peptide they often show increased stability to thermal unfolding relative to empty RTL molecules.

In exemplary embodiments of the invention, RTL design is rationally based on crystallographic coordinates of human HLA-DR, HLA-DQ, and/or HLA-DP proteins, or of a non-human (e.g., murine or rat) MHC class II protein. In this context, exemplary RTLs have been designed based on crystallographic data for HLA DR1 (PDB accession code 1AQD), which design parameters have been further clarified, for example, by sequence alignment with other MHC class II molecules from rat, human and mouse species. The program Sybyl (Tripos Associates, St Louis, MO) is an exemplary design tool that can be used to generate graphic images using, for example, an O2 workstation (Silicon Graphics, Mountain View, CA) and coordinates obtained for HLA-DR, HLA-DQ, and/or HLA-DP molecules. Extensive crystallographic characterizations are provided for these and other MHC class II proteins deposited in the Brookhaven Protein Data Bank (Brookhaven National Laboratories, Upton, NY).

Detailed description of HLA-DR crystal structures for use in designing and constructing modified RTLs of the invention is provided, for example, in Ghosh et al., Nature 378:457, 1995; Stern et al., Nature 368:215, 1994; Murthy et al., Structure 5:1385, 1997; Bolin et al., J.Med.Chem. 43:2135, 2000; Li et al., J. Mol. Biol. 304:177, 2000; Hennecke et al., Embo J. 19:5611, 2000; Li et al., Immunity 14:93, 2001; Lang et al., Nat. Immunol. 3:940, 2002; Sundberg et al., J. Mol. Biol. 319:449, 2002; Zavala-Ruiz et al., J. Biol, Chem. 278:44904, 2003; Sundberg et al., Structure 11:1151,2003. Detailed description of HLA-DQ crystal structures is provided, for example, in Sundberg et al., Nat. Struct. Biol, 6:123, 1999; Li et al., Nat. Immunol. 2:501, 2001; and Siebold et al., Proc. Nat. Acad. Sci. USA 101:1999, 2004. Detailed description of a murine MHC I-A^{U} molecule is provided, for example, in He et al., Immunity 17:83, 2002. Detailed description of a murine MHC class II I-Ad molecule is provided, for example, in Scott et al., Immunity 8:319, 1998. Detailed description of a murine MHC class II I-Ak molecule is provided, for example, in Reinherz et al., Science 286:1913, 1999, and Miley et al., J. Immunol 166:3345, 2001. Detailed description of a murine MHC allele I-A(G7) is provided, for example, in Corper et al., Science 288:501, 2000. Detailed description of a murine MHC class II H2-M molecule is provided, for example, in Fremont et al., Immunity 9:385, 1998. Detailed description of a murine MHC class II H2-Ieβ molecule is provided, for example, in Krosgaard et al., Mol. Cell 12:1367, 2003; Detailed description of a murine class II Mhc I-Ab molecule is provided, for example, in Zhu et al., J. Mol. Biol. 326:1157, 2003. HLA-DP Lawrance et al., Nucleic Acids Res. 1985 Oct 25; 13(20): 7515-7528

Structure-based homology modeling is based on refined crystallographic coordinates of one or more MHC class I or class II molecule(s), for example, a human DR molecule and a murine I-E^{k} molecule. In one exemplary study by Burrows and colleagues (Protein Enginering 12:771-778, 1999), the primary sequences of rat, human and mouse MHC class II were aligned, from which it was determined that 76 of 256 α-chain amino acids were identical (30%), and 93 of the 265 β-chain amino acids were identical (35%). Of particular interest, the primary sequence location of disulfide-bonding cysteines was conserved in all three species, and the backbone traces of the solved structures showed strong homology when superimposed, implying an evolutionarily conserved structural motif, with side-chain substitutions designed to allow differential antigenic-peptide binding in the peptide-binding groove.

Further analysis of MHC class I and class II molecules for constructing modified RTLs of the invention focuses on the "exposed" (i.e., solvent accessible) surface of the β-sheet platform/anti-parallel α-helix that comprise the domain(s) involved in peptide binding and T-cell recognition. In the case of MHC class II molecules, the α1 and β1 domains exhibit an extensive hydrogen-bonding network and a tightly packed and "buried" (i.e., solvent inaccessible) hydrophobic core. This tertiary structure is similar to molecular features that confer structural integrity and thermodynamic stability to the α-helix/β-sheet scaffold characteristic of scorpion toxins, which therefore present yet additional structural, indicia for guiding rational design of modified. RTLs herein (see, e.g., Zhao et al., J. Mol. Biol. 227:239, 1992; Housset, J. Mol. Biol. 238:88-91, 1994; Zinn-Justin et al., Biochemistry 35:8535-8543, 1996).

From these and other comparative data sources, crystals of native MHC class II molecules have been found to contain a number of water molecules between a membrane proximal surface of the β-sheet platform and a membrane distal surfaces of the α2 and β2 Ig-fold domains. Calculations regarding the surface area of interaction between domains can be quantified by creating a molecular surface, for example for the β1α1 and α2β2 Ig-fold domains of an MHC II molecule, using an algorithm such as that described by Connolly (Biopolymers 25:1229-1247, 1986) and using crystallographic coordinates (e.g., as provided for various MHC class II molecules in the Brookhaven Protein Data Base.

For an exemplary, human DR1 MHC class II molecule (PDB accession numbers 1SEB, 1AQD), surface areas of the β1α1 and α2β2-Ig-fold domains were calculated independently, defined by accessibility to a probe of radius 0.14 nm, about the size of a water molecule (Burrows et al., Proteins Engineering 12:771-778, 1999). The surface area of the MHC class II αβ-heterodimer was 156 nm², while that of the β1α1 construct was 81 nm² and the α2β2-Ig-fold domains was 90 nm². Approximately 15 nm² (18.5%) of the β1α1 surface was found to be buried by the interface with the Ig-fold domains in the MHC class II αβ-heterodimer. Side-chain interactions between the β1α1-peptide binding and Ig-fold domains (α2 and β2) were analyzed and shown to be dominated by polar interactions with hydrophobic interactions potentially serving as a "lubricant" in a highly flexible "ball and socket" type inter face.

These and related modeling studies suggest that the antigen binding domain of MHC class II molecules remain stable in the absence of the α2 and β2 Ig-fold domains, and this production has been born out for production of numerous, exemplary RTLs comprising an MHC class II "α1β1" architecture. Related findings were described by Burrows et al. (J. Immunol. 161:5987-5996, 1998) for an "empty" β1α1 RTL, and four α1β1 RTL constructs with covalently coupled rat and guinea pig antigenic peptides: β1 1-Rt-MBP-72-89, β1 1-Gp-MBP-72-89, β1 1-Gp-MBP-55-69 and β1 1-Rt-CM-2. For each of these constructs, the presence of native disulfide bonds between cysteines (β15 and β79) was demonstrated by gel shift assay with or without the reducing agent β-mercaptoethanol (β-ME). In the absence of β-ME, disulfide bonds are retained and the RTL proteins typically move through acrylamide gels faster due to their more compact structure. These data, along with immunological findings using MHC class II-specific monoclonal antibodies to label conserved epitopes on the RTLs generally affirm the conformational integrity of RTL molecules compared to their native MHC II counterparts (Burrows *et al*., 1998, supra; Chang et al., J. Biol. Chem. 276:24170-14176, 2001; Vandenbark et al., J. Immunol. 171:127-133,2003). Similarly, circular dichroism (CD) studies of MHC class II-derived RTLs reveal that β1α1 molecules have highly ordered secondary structures. Typically, RTLs of this general construction shared the β-sheet platform/anti-parallel α-helix secondary structure common to all class II antigen binding domains. In this context, β1α1 molecules have been found to contain, for example, approximately 30% α-helix, 15% β-strand, 26% β-turn and 29% random coil structures. RTLs covalently bound to Ag peptide (e.g., MBP-72-89, and CM-2) show similar, although not identical, secondary structural features. Thermal denaturation studies reveal a high degree of cooperativity and stability of RTL molecules, and the biological integrity of these molecules has been demonstrated in numerous contexts, including by the ability of selected RTLs to detect and inhibit rat encephalitogenic T-cells and treat experimental autoimmune encephalomyelitis.

According to these and related findings provided herein (or described in the cited references which are collectively incorporated herein for all disclosure purposes), RTL constructs of the invention, with or without an associated antigenic peptide, retain structural and conformational integrity consistent with that of refolded native MHC molecules. This general finding is exemplified by results for soluble single-chain RTL molecules derived from the antigen-binding/TCR interface comprised of all or portions of the MHC class II β1 and α1 domains. In more detailed embodiments, these exemplary MHC class II RTLs lack the α2 domain and β2 domain of the corresponding, native MHC class II protein, and also typically exclude the transmembrane and intra-cytoplasmic sequences found in the native MHC II protein. The reduced size and complexity of these RTL constructs, exemplified by the "β1α1" MHC II RTL constructs, provide for ready and predictable expression and purification of the RTL molecules from bacterial inclusion bodies in high yield (e.g., up to 15-30 mg/l cell culture or greater yield).

In native MHC class II molecules, the Ag peptide binding/T-cell recognition domain is formed by well-defined portions of the α1 and β1 domains of the α and β polypeptides which fold together to form a tertiary structure, most simply described as a β-sheet platform upon which two anti-parallel helical segments interact to form an antigen-binding groove. A similar structure is formed by a single exon encoding the α1 and α2 domains of MHC class I molecules, with the exception that the peptide-binding groove of MHC class II is open-ended, allowing the engineering of single-oxon constructs that encode the peptide binding/T-cell recognition domain and an antigenic peptide ligand.

As exemplified herein for MHC class II proteins, modeling studies highlighted important features regarding the interface between the β1α1 and α2β2-Ig-fold domains that have proven critical for designing modified, monodisperse RTLs of the invention. The α1 and β1 domains show an extensive hydrogen-bonding network and a tightly packed and "buried" (i.e., solvent inaccessible) hydrophobic core. The β1α1 portion of MHC class II proteins may have the ability to move as a single entity independent from the α2β2-Ig-fold 'platform'. Besides evidence of a high degree of mobility in the side-chains that make up the linker regions between these two domains, crystals of MHC class II I-Ek contained a number of water molecules within this interface (Jardetzky et al., Nature 368:711-715, 1994; Fremont et al., Science 272:1001-1004, 1996; Murthy et al., Structure 5:1385,1997). The interface between the β1α1 and α2β2-Ig-fold domains appears to be dominated by polar interactions, with hydrophobic residues likely serving as a 'lubricant' in a highly flexible 'ball and socket' type interface. Flexibility at this interface may be required for freedom of movement within the α1 and β1 domains for binding/exchange of peptide antigen. Alternatively or in combination, this interaction surface may play a role in communicating information about the MHC class II-peptide molecular interaction with TCRs back to the APC.

Following these rational design guidelines and parameters, the instant inventors have successfully engineered modified, monodisperse derivatives of single-chain human RTLs comprising peptide binding/TCR recognition portions of human MHC class II molecules (e.g., as exemplified by a HLA-DR2b (DRA*0101/DRB1*1501). Unmodified RTLs constructed from the α1 and β1 domains of this exemplary MHC class II molecule retained biological activity, but formed undesired, higher order aggregates in solution.

To resolve the problem of aggregation in this exemplary, unmodified RTL, site-directed mutagenesis was directed towards replacement of hydrophobic residues with polar (*e.g*., serine) or charged (*e.g*., aspartic acid) residues to modify the β-sheet platform of the DR2-derived RTLs. According to this rational design procedure, novel RTL variants were obtained that were determined to be predominantly monomeric in solution. Size exclusion chromatography and dynamic light scattering demonstrated that the novel modified RTL were monomeric in solution, and structural characterization using circular dichroism demonstrated a highly ordered secondary structure of the RTLs.

Peptide binding to these "empty," modified RTLs was quantified using biotinylated peptides, and functional studies showed that the modified RTLs containing covalently tethered peptides were able to inhibit antigen-specific T-cell proliferation *in vitro*, as well as suppress experimental autoimmune encephalomyelitis *in vivo*. These studies demonstrated that RTLs encoding the Ag-binding/TCR recognition domain of MHC class II molecules are innately very robust structures. Despite modification of the RTLs as described herein, comprising site-directed mutations that modified the β-sheet platform of the RTL, these molecules retained potent biological activity separate from the Ig-fold domains of the progenitor class II structure, and exhibited a novel and surprising reduction in aggregation in aqueous solutions. Modified RTLs having these and other redesigned surface features and monodisperal characteristics retained the ability to bind Ag-peptides, inhibit T-cell proliferation in an Ag-specific manner, and treat, *inter alia*, autoimmune disease *in vivo*.

Additional modifications apart from the foregoing surface feature modifications can be introduced into modified RTLs of the invention, including particularly minor modifications in amino acid sequence(s) of the MHC component of the RTL that are likely to yield little or no change in activity of the derivative or "variant" RTL molecule. Preferred variants of non-aggregating MHC domain polypeptides comprising a modified RTLs are typically characterized by possession of at least 50% sequence identity counted over the full length alignment with the amino acid sequence of a particular non-aggregating MHC domain polypeptide using the NCBI Blast 2.0, gapped blastp set to default parameters. Proteins with even greater similarity to the reference sequences will show increasing percentage identities when assessed by this method, such as at least 60%, at least 65%, at least 70%, at least 75%, at least 80%, at least 90% or at least 93% sequence identity. When less than the entire sequence is being compared for sequence identity, variants will typically possess at least 75% sequence identity over short windows of 10-20 amino acids, and may possess sequence identities of at least 85% or at least 90% or 95% depending on their similarity to the reference sequence. Methods for determining sequence identity over such short windows are known in the art as described above. Variants of modified RTLs comprising non-aggregating MHC domain polypeptides also retain the biological activity of the non-variant, modified RTL. For the purposes of this invention, that activity may be conveniently assessed by incorporating the variation in the appropriate MHC component of a modified RTL (e.g., a β1α1 MHC component) and determining the ability of the resulting RTL/Ag complex to inhibit Ag-specific T-cell proliferation *in vitro*, as described herein.

### (d) Pharmaceutical Formulations

Suitable routes of administration of purified MHC polypeptides of the present invention include, but are not limited to, oral, buccal, nasal, aerosol, topical, transdermal, mucosal, injectable, slow release, controlled release, iontophoresis, sonophoresis, and other conventional delivery routes, devices and methods. Injectable delivery methods include, but are not limited to, intravenous, intramuscular, intraperitoneal, intraspinal, intrathecal, intracerebroventricular, intraarterial, and subcutaneous injection.

Amounts and regimens for the administration of the selected MHC polypeptides will be determined by the attending clinician. Effective doses for therapeutic application will vary depending on the nature and severity of the condition to be treated, the particular MHC polypeptide selected, the age and condition of the patient and other clinical factors. Typically, the dose range will be from about 0.1 µg/kg body weight to about 100mg/kg body weight. Other suitable ranges include doses of from about 100 µg/kg to 1mg/kg body weight. In certain embodiments, the effective dosage will be selected within narrower ranges of, for example, 1-75 µg/kg, 10-50 µg/kg, 15-30 µg/kg, or 20-30 µg/kg. These and other effective unit dosage amounts may be administered in a single dose, or in the form of multiple daily, weekly or monthly doses, for example in a dosing regimen comprising from 1 to 5, or 2-3, doses administered per day, per week, or per month. The dosing schedule may vary depending on a number of clinical factors, such as the subject's sensitivity to the protein. Examples of dosing schedules are 3 µg/kg administered twice a week, three times a week or daily; a dose of 7 µg/kg twice a week, three times a week or daily; a dose of 10 µg/kg twice a week, three times a week or daily; or a dose of 30 µg/kg twice a week, three times a week or daily.

The amount, timing and mode of delivery of compositions of the invention comprising an effective amount of purified MHC polypeptides will be routinely adjusted on an individual basis, depending on such factors as weight, age, gender, and condition of the individual, the severity of the T-cell mediated disease, whether the administration is prophylactic or therapeutic, and on the basis of other factors known to effect drug delivery, absorption, pharmacokinetics, including half-life, and efficacy. Thus, following administration of the purified MHC polypeptides composition according to the formulations and methods of the invention, test subjects will exhibit a 10%, 20%, 30%, 50% or greater reduction, up to a 75-90%, or 95% or greater, reduction, in one or more symptoms associated with a targeted T-cell mediated disease, as compared to placebo-treated or other suitable control subjects.

Within additional aspects of the invention, combinatorial formulations and coordinate administration methods are provided which employ an effective amount of purified MHC polypeptide, and one or more additional active agent(s) that is/are combinatorially formulated or coordinately administered with the purified MHC polypeptide-yielding an effective formulation or method to modulate, alleviate, treat or prevent a T-cell mediated disease in a mammalian subject. Exemplary combinatorial formulations and coordinate treatment methods in this context employ a purified MHC polypeptide in combination with one or more additional or adjunctive therapeutic agents. The secondary or adjunctive methods and compositions useful in the treatment of T-cell mediated diseases include, but are not limited to, combinatorial administration with immunoglobulins (e.g., a CTLA4Ig, such as BMS-188667; see, e.g., Srinivas et al., J. Pharm. Sci. 85(1):1-4, (1996), incorporated herein by reference); copolymer 1, copolymer 1-related peptides, and T-cells treated with copolymer 1 or copolymer 1-related peptides (see, e.g., United States Patent No. 6,844,314, incorporated herein by reference); blocking monoclonal antibodies, transforming growth factor-β, anti-TNF α antibodies; steroidal agents; anti-inflammatory agents; immunosuppressive agents; alkylating agents; anti-metabolites; antibiotics; corticosteroids; proteosome inhibitors; and diketopiperazines. To practice the coordinate administration methods of the invention, a MHC polypeptide is administered, simultaneously or sequentially, in a coordinate treatment protocol with one or more of the secondary or adjunctive therapeutic agents contemplated herein, for example a secondary immune modulatory agent. The coordinate administration may be done in either order, and there may be a time period while only one or both (or all) active therapeutic agents, individually and/or collectively, exert their biological activities. A distinguishing aspect of all such coordinate treatment methods is that the purified MHC polypeptide composition may elicit a favorable clinical response, which may or may not be in conjunction with a secondary clinical response provided by the secondary therapeutic agent. Often, the coordinate administration of a purified MHC polypeptide with a secondary therapeutic agent as contemplated herein will yield an enhanced therapeutic response beyond the therapeutic response elicited by either or both the purified MHC polypeptide and/or secondary therapeutic agent alone.

The pharmaceutical compositions of the present invention may be administered by any means that achieve their intended purpose. The purified MHC polypeptides of the present invention are generally combined with a pharmaceutically acceptable carrier appropriate for the particular mode of administration being employed. Dosage forms of the purified MHC polypeptide of the present invention include excipients recognized in the art of pharmaceutical compounding as being suitable for the preparation of dosage units as discussed above. Such excipients include, without intended limitation, binders, fillers, lubricants, emulsifiers, suspending agents, sweeteners, flavorings, preservatives, buffers, wetting agents, disintegrants, effervescent agents and other conventional excipients and additives.

The compositions of the invention for treating T-cell mediated diseases and associated conditions and complications can thus include any one or combination of the following: a pharmaceutically acceptable carrier or excipient; other medicinal agent(s); pharmaceutical agent(s); adjuvants; buffers; preservatives; diluents; and various other pharmaceutical additives and agents known to those skilled in the art. These additional formulation additives and agents will often be biologically inactive and can be administered to patients without causing deleterious side effects or interactions with the active agent.

If desired, the purified MHC polypeptide of the invention can be administered in a controlled release form by use of a slow release carrier, such as a hydrophilic, slow release polymer. Exemplary controlled release agents in this context include, but are not limited to, hydroxypropyl methyl cellulose, having a viscosity in the range of about 100 cps to about 100,000 cps or other biocompatible matrices such as cholesterol.

Purified MHC polypeptides of the invention will often be formulated and administered in an oral dosage form, optionally in combination with a carrier or other additive(s). Suitable carriers common to pharmaceutical formulation technology include, but are not limited to, microcrystalline cellulose, lactose, sucrose, fructose, glucose, dextrose, or other sugars, di-basic calcium phosphate, calcium sulfate, cellulose, methylcellulose, cellulose derivatives, kaolin, mannitol, lactitol, maltitol, xylitol, sorbitol, or other sugar alcohols, dry starch, dextrin, maltodextrin or other polysaccharides, inositol, or mixtures thereof. Exemplary unit oral dosage forms for use in this invention include tablets, which may be prepared by any conventional method of preparing pharmaceutical oral unit dosage forms can be utilized in preparing oral unit dosage forms. Oral unit dosage forms, such as tablets, may contain one or more conventional additional formulation ingredients, including, but not limited to, release modifying agents, glidants, compression aides, disintegrants, lubricants, binders, flavors, flavor enhancers, sweeteners and/or preservatives. Suitable lubricants include stearic acid, magnesium stearate, talc, calcium stearate, hydrogenated vegetable oils, sodium benzoate, leucine carbowax, magnesium lauryl sulfate, colloidal silicon dioxide and glyceryl monostearate. Suitable glidants include colloidal silica, fumed silicon dioxide, silica, talc, fumed silica, gypsum and glyceryl monostearate. Substances which may be used for coating include hydroxypropyl cellulose, titanium oxide, talc, sweeteners and colorants.

Additional purified MHC polypeptides of the invention can be prepared and administered in any of a variety of inhalation or nasal delivery forms known in the art. Devices capable of depositing aerosolized purified MHC formulations in the sinus cavity or pulmonary alveoli of a patient include metered dose inhalers, nebulizers, dry powder generators, sprayers, and the like. Methods and compositions suitable for pulmonary delivery of drugs for systemic effect are well known in the art. Additional possible methods of delivery include deep lung delivery by inhalation (Edwards *et al*., 1997; Service, 1997). Suitable formulations, wherein the carrier is a liquid, for administration, as for example, a nasal spray or as nasal drops, may include aqueous or oily solutions of purified MHC polypeptides and any additional active or inactive ingredient(s).

Further compositions and methods of the invention are provided for topical administration of purified MHC polypeptides for the treatment of T-cell mediated diseases. Topical compositions may comprise purified MHC polypeptides and any other active or inactive component(s) incorporated in a dermatological or mucosal acceptable carrier, including in the form of aerosol sprays, powders, dermal patches, sticks, granules, creams, pastes, gels, lotions, syrups, ointments, impregnated sponges, cotton applicators, or as a solution or suspension in an aqueous liquid, non-aqueous liquid, oil-in-water emulsion, or water-in-oil liquid emulsion. These topical compositions may comprise purified MHC polypeptides dissolved or dispersed in a portion of water or other solvent or liquid to be incorporated in the topical composition or delivery device. It can be readily appreciated that the transdermal route of administration may be enhanced by the use of a dermal penetration enhancer known to those skilled in the art. Formulations suitable for such dosage forms incorporate excipients commonly utilized therein, particularly means, e.g. structure or matrix, for sustaining the absorption of the drug over an extended period of time, for example, 24 hours. Transdermal delivery may also be enhanced through techniques such as sonophoresis (Mitragotri *et al*., 1996).

Yet additional purified MHC polypeptide formulations are provided for parenteral administration, e.g. intravenously, intramuscularly, subcutaneously or intraperitoneally, including aqueous and non-aqueous sterile injection solutions which may optionally contain anti-oxidants, buffers, bacteriostats and/or solutes which render the formulation isotonic with the blood of the mammalian subject; and aqueous and non-aqueous sterile suspensions which may include suspending agents and/or thickening agents. The formulations may be presented in unit-dose or multi-dose containers. Purified MHC polypeptide formulations may also include polymers for extended release following parenteral administration. The parenteral preparations may be solutions, dispersions or emulsions suitable for such administration, The subject agents may also be formulated into polymers for extended release following parenteral administration. Pharmaceutically acceptable formulations and ingredients will typically be sterile or readily sterilizable, biologically inert, and easily administered. Such polymeric materials are well known to those of ordinary skill in the pharmaceutical compounding arts. Parenteral preparations typically contain buffering agents and preservatives, and injectable fluids that are pharmaceutically and physiologically acceptable such as water, physiological saline, balanced salt solutions, aqueous dextrose, glycerol or the like Extemporaneous injection solutions, emulsions and suspensions may be prepared from sterile powders, granules and tablets of the kind previously described. Preferred unit dosage formulations are those containing a daily dose or unit, daily sub-dose, as described herein above, or an appropriate fraction thereof, of the active ingredient(s).

In more detailed embodiments, purified MHC polypeptides may be encapsulated for delivery in microcapsules, microparticles, or microspheres, prepared, for example, by coacervation techniques or by interfacial polymerization, for example, hydroxymethylcellulose or gelatin-microcapsules and poly(methylmethacylate) microcapsules, respectively, in colloidal drug delivery systems (for example, liposomes, albumin microspheres, microemulsions, nano-particles and nanocapsules), through the use of viral vectors or in macroemulsions. These methods could be used to deliver the purified MHC polypeptides to cells in the nucleic acid form for subsequent translation by the host cell.

### Exemplary Applications of Recombinant β1α1 and α1α2 Molecules

The class II β1α1 and class I α1α2 polypeptides of the present invention are useful for a wide range of *in vitro* and *in vivo* applications. Indeed, as a result of the biological activities of these polypeptides, they may be used in numerous applications in place of either intact purified MHC molecules, or antigen presenting cells that express MHC molecules.

*In vitro* applications of the disclosed polypeptides include the detection, quantification and purification of antigen-specific T-cells. Methods for using various forms of MHC-derived complexes for these purposes are well known and are described in, for example, U.S. Patent Nos. 5,635,363 and 5,595,881, each of which is incorporated by reference herein in its entirety. For such applications, the disclosed polypeptides may be free in solution or may be attached to a solid support such as the surface of a plastic dish, a microtiter plate, a membrane, or beads. Typically, such surfaces are plastic, nylon or nitrocellulose. Polypeptides in free solution are useful for applications such as fluorescence activated cell sorting (FACS). For detection and quantification of antigen-specific T-cells, the polypeptides are preferably labeled with a detectable marker, such as a fluorescent marker.

The T-cells to be detected, quantified or otherwise manipulated are generally present in a biological sample removed from a patient. The biological sample is typically blood or lymph, but may also be tissue samples such as lymph nodes, tumors, joints etc. It will be appreciated that the precise details of the method used to manipulate the T-cells in the sample will depend on the type of manipulation to be performed and the physical form of both the biological sample and the MHC molecules. However, in general terms, the β1α1/peptide complex or α1α2/peptide complex is added to the biological sample, and the mixture is incubated for sufficient time (e.g., from about 5 minutes up to several hours) to allow binding. Detection and quantification of T-cells bound to the MHC/peptide complex may be performed by a number of methods including, where the MHC/peptide includes a fluorescent label, fluorescence microscopy and FACS. Standard immunoassays such as ELISA and RIA may also be used to quantify T-cell- MHC/peptide complexes where the MHC/peptide complexes are bound to a solid support. Quantification of antigen-specific T-cell populations will be especially useful in monitoring the course of a disease. For example, in a multiple sclerosis patient, the efficacy of a therapy administered to reduce the number of MBP-reactive T-cells may be monitored using MHC/MBP antigen complexes to quantify the number of such T-cells present in the patient. Similarly, the number of anti-tumor T-cells in a cancer patient may be quantified and tracked over the course of a therapy using MHC/tumor antigen complexes.

FACS may also be used to separate T-cell - MHC/peptide complexes from the biological sample, which may be particularly useful where a specified population of antigen-specific T-cells is to be removed from the sample, such as for enrichment purposes. Where the MHC/peptide complex is bound to magnetic beads, the binding T-cell population may be purified as described by Miltenyi *et al*. (1990). By way of example, anti-tumor T-cells in the blood of a cancer patient may be purified using these methods, expanded *in vitro* and returned to the patient as part of an adoptive immunotherapy treatment.

A specified antigen-specific T-cell population in the biological sample may be anergized by incubation of the sample with MHC/peptide complexes containing the peptide recognized by the targeted T-cells. Thus, when these complexes bind to the TCR in the absence of other co-stimulatory molecules, a state of anergy is induced in the T-cell. Such an approach is useful in situations where the targeted T-cell population recognizes a self-antigen, such as in various autoimmune diseases. Alternatively, the targeted T-cell population may be killed directly by incubation of the biological sample with an MHC/peptide complex conjugated with a toxic moiety.

T-cells may also be activated in an antigen-specific manner by the polypeptides of the invention. For example, the disclosed MHC polypeptides loaded with a specified antigen may be adhered at a high density to a solid surface, such as a plastic dish or a magnetic bead. Exposure of T-cells to the polypeptides on the solid surface can stimulate and activate T-cells in an antigen-specific manner, despite the absence of co-stimulatory molecules. This is likely attributable to sufficient numbers of TCRs on a T-cell binding to the MHC/peptide complexes that co-stimulation is unnecessary for activation.

In one embodiment, suppressor T-cells are induced. Thus, when the complexes bind to the TCR in the proper context, suppressor T-cells are induced *in vitro*. In one embodiment, effector functions are modified, and cytokine profiles are altered by incubation with a MHC/peptide complex.

*In vivo* applications of the disclosed polypeptides include the amelioration of conditions mediated by antigen-specific T-cells. Such conditions include, but are not limited to, allergies, auto-immune diseases, graft rejection, transplant rejection, graft versus host disease, an unwanted delayed-type hypersensitivity reaction, or a T-cell mediated pulmonary disease. Such auto-immune diseases include, but are not limited to, insulin dependent diabetes mellitus (IDDM), systemic lupus erythematosus (SLE), rheumatoid arthritis, coeliac disease, multiple sclerosis, neuritis, polymyositis, psoriasis, vitiligo, Sjogren's syndrome, rheumatoid arthritis, autoimmune pancreatitis, inflammatory bowel diseases, Crohn's disease, ulcerative colitis, active chronic hepatitis, glomerulonephritis, seleroderma, sarcoidosis, autoimmune thyroid diseases, Hashimoto's thyroiditis, Graves disease, myasthenia gravis, asthma, Addison's disease, autoimmune uveoretinitis, pemphigus vulgaris, primary biliary cirrhosis, pernicious anemia, sympathetic opthalmia, uveitis, autoimmune hemolytic anemia, pulmonary fibrosis, chronic beryllium disease or idiopathic pulmonary fibrosis. Other researchers have described various forms of MHC polypeptides that may be used to treat these conditions and the methods used in those systems are equally useful with the MHC polypeptides of the present invention. Exemplary methodologies are described in U.S. Patent Nos. 5,130,297, 5,284,935, 5,468,481, 5,734,023 and 5,194,425 (herein incorporated by reference). By way of example, the MHC/peptide complexes may be administered to a subject in order to induce anergy in self-reactive T-cell populations, or these T-cell populations may be treated by administration of MHC/peptide complexes conjugated with a toxic moiety. Alternatively, the MHC/peptide complexes may be administered to a subject to induce T suppressor cells or to modify a cytokine expression profile. The disclosed molecules may also be used to boost immune response in certain conditions such as cancer and infectious diseases.

*In vivo* applications of the disclosed polypeptides also include the amelioration of demyelination or neuroaxonal injury or loss. Such demyelination neuroaxonal injury may be caused by auto T-cell mediated diseases such as autoimmune diseases as well as neurodegenerative diseases including, but not limited to, multiple sclerosis (MS), Parkinson's disease, Alzheimer's disease, progressive multifocal leukoencephalopathy (PML), disseminated necrotizing leukoencephalopathy (DNL), acute disseminated encephalomyelitis, Schilder disease, central pontine myelinolysis (CPM), radiation necrosis, Binswanger disease (SAE), adrenoleukodystrophy, adrenomyeloneuropathy, Leber's hereditary optic atrophy, and HTLV-associated myelopathy.

In treating demyelination or neuroaxonal injury or loss, RTLs may be administered to a subject, including a mammalian subject, in need of treatment. Such administration may prevent degeneration of or restore myelin, as well as prevent, reduce or repair axonal damage or loss. Administration of RTLs to subjects, including human subjects, in need of treatment, may halt or stop the progression of a T-cell mediated disease such as an auto-immune disease or neurodegenerative disease. Such treatment may also be administered prophylactically to prevent relapses or initiation of a T-cell mediated disease in subjects at risk for the development of such a disease.

The compositions and methods of the present invention may also be administered to treat inflammation in subjects in need of such treatment. Inflammation may be present in the central nervous system (CNS), spinal cord, spleen, or other bodily system. The compositions and methods of the present invention may be administered to prevent or decrease infiltration of inflammatory cells into the CNS, spinal cord, spleen, or other bodily system, or to upregulate anti-inflammatory factors.

Treatments with the compositions and methods of the present invention may be administered alone or in a combinatorial formulation or coordinately with other therapeutic agents, including, but not limited to, interferon beta-1a; interferon beta-1b; glatiramer acetate; mitoxantrone; corticosteroids; muscle relaxants including but not limited to baclofen, dantrolene, tizanidine, cyclobenzaprine, clonazepam, and diaxepam; anticholinergics including but not limited to, propantheline, tolterodine, and dicyclomine; urinary tract antispasmodics such as oxybutynin; tricyclic antidepressants including but not limited to amitriptyline and imipramine; antidiuretic hormones including but not limited to, desmopressin, and DDAVP; anticonvulsants, including but not limited to, carbamazepine, phenytoin, and acetazolamide; central nervous system stimulants including pemoline; selective serotonin reuptake inhibitors (SSRIs) including, but not limited to, citalopram, fluoxetine, paroxetine, and sertraline; and non-steroidal anti-inflammatories. Such combinatorial administration may be done simultaneously or sequentially in either order, and there may be a time period while only one or both (or all) active therapeutic agents individually and/or collectively exert their biological activities.

Various additional aspects of the invention are provided herein which employ features, methods or materials that are known in the art or which are disclosed in Applicants' prior patent applications, including but not limited to: U.S. Patent Application No. 09/847,172, filed May 1, 2001; U.S. Provisional Patent Application No. 60/200,942, filed May 1, 2000; International Publication No. WO 02/087613 A1, published November 7, 2002; U.S. Patent No. 6,270,772; U.S. Provisional Patent Application No. 60/064,552, filed September 16, 1997; and U.S. Provisional Patent Application No. 60/064,555, filed October 10,1997; U.S. Provisional Patent Application Number 60/500,660, filed September 5, 2003; U.S. Patent Application No. 10/936,467, filed September 7, 2004; and U.S. Provisional Patent Application No. 60/586,433, filed July 8, 2004, each of which is incorporated herein by reference in its entirety for all purposes.

The following examples illustrate certain aspects of the invention, but are not intended to limit in any manner the scope of the invention.

### Example 1

### Cloning, Expression and In vitro Folding of β1α1 Molecules

A prototypical nucleic acid construct was produced that encoded a single polypeptide chain with the amino terminus of the MHC class II α1 domain genetically linked to the carboxyl terminus of the MHC class II β1 domain. The sequence of this prototypical construct, made from the rat RT1B - and ß-chain cDNAs is shown in Fig. 1A (SEQ ID NO:1).

RT1B α1- and ß1-domain encoding cDNAs were prepared by PCR amplification of cloned RT1B α - and ß-chain cDNA coding sequences (α6, ß118, respectively) obtained from Dr. Konrad Reske, Mainz, FRG (Syha *et al*., 1989; Syha-Jedelhauser *et al*., 1991). The primers used to generate ß1 were:

5'-AATTCCTCGAGATGGCTCTGCAGACCCC-3' (XhoI 5' primer) (SEQ ID NO:9); 5'-TCTTGAGCTCCAAGCCGCCGCAGGGAGGTG-3' (3' ligation primer) (SEQ ID NO:10). The primers used to generate α1 were:

5'-CGGCGGCTTGGAGGTCAAGACCACATTGAGG-3' (5' ligation primer) (SEQ ID NO:11); 5'-GCCTCGGTACCTTAGTTGACAGCTTGGGTTGAATTTG-3' (KpnI 3' primer) (SEQ ID NO:12). Additional primers used were:

5-CAGGGACCATGGGCAGAGACTCCCCA-3' (NcoI 5' primer) (SEQ ID NO:13); and 5'-GCCTCCTCGAGTTAGTTGACAGCTTGGGTT-3' (XhoI 3' primer) (SEQ ID NO: 14). Step one involved production of cDNAs encoding the ß1 and α1 domains. PCR was conducted with Taq polymerase (Promega, Madison, WI) through 28 cycles of denaturation at 94.5°C for 20 seconds, annealing at 55°C for 1.5 minutes and extension at 72°C for 1.5 minutes, using ß118 as template and the XhoI 5' primer and 3' ligation primer as primers and α6 cDNA as template and the 5' ligation primer and KpnI 3' primer. PCR products were isolated by agarose gel electrophoresis and purified using Gene-Clean (Bio 101, Inc., La Jolla, CA).

In step two, these products were mixed together without additional primers and heat denatured at 94.5°C for 5 minutes followed by 2 cycles of denaturation at 94.5°C for 1 minute, annealing at 60°C for 2 minutes and extension at 72°C for 5 minutes. In step three, the annealed, extended product was heat denatured at 94.5°C for 5 minutes and subjected to 26 cycles of denaturation at 94.5°C for 20 seconds, annealing at 60°C for 1 minute and extension at 72°C for 1 minute, in the presence of the XhoI 5' primer and KpnI 3' primer. The final PCR product was isolated by agarose gel electrophoresis and Gene-Cleaned. This produced a 656 base pair cDNA encoding the ß1 1 molecule. The cDNA encoding the ß1α1 molecule was moved into cloning vector pCR2.1 (Invitrogen, Carlsbad, CA) using Invitrogen's TA Cloning® kit. The cDNA in pCR2.1 was used as template and PCR was conducted through 28 cycles of denaturation at 94.5°C for 20 seconds, annealing at 55 C for 1.5 minutes and extension at 72°C for 1.5 minutes, using the NcoI 5' primer and XhoI 3' primer. The PCR products were cleaved with the relevant restriction enzymes and directionally cloned into pET21d+ (Novagen, Madison, WI; Studier *et al*., 1990). The constructs were confirmed by DNA sequencing. The ß1α1 molecule used in these studies differs from wild-type in that it contains a β-1 domain Q12R amino acid substitution.

For insertion of the peptide/linker cartridge (shown in Fig. 1A), the following approach was used. For insertion of the peptide/linker cartridge (shown in Fig. 1A), the following approach was used. The 210 bp peptide/linker cartridge was amplified using the XhoI 5' primer and a primer of sequence:

5'-GAAATCCCGCGGGGAGCCTCCACCTCCAGAGCCTCGGGGC ACTAGTGAGCCTCCACCTCCGAAGTGCACCACTGGGTTCTCATCCTGAGTCC TCTGGCTCTTCTGTGGGGAGTCTCTGCCCTCAGTCC-3' (3'-MBP-72-89/linker ligation primer) (SEQ ID NO:15) and the original full-length ß118 cDNA as a template. A 559 bp cDNA with a 5' overhang for annealing to the peptide/linker cartridge cDNA was generated using a primer: 5'-GCTCCCCGCGGGATTTCGTGTACCAGTTCAA-3' (5' peptide/linker ligation primer) (SEQ ID NO: 16); and the Kpn I 3' primer and the 656 bp ß1α1 cDNA as the amplification template. Annealing and extension of the two cDNAs resulted in the 750 bp full-length ß1a1/MBP-72-89 construct. Modifications at the 5' and 3' ends of the ß1α1 and ß1α1/MBP-72-89 cDNAs were made for subcloning into pET21d+ (Novagen, Madison, WI; Studier *et al.*, 1990) using the NcoI 5' primer and the XhoI 3' primer. The primers used to generate the MBP-55-69/ linker cartridge were

5'-TATTACCATGGGCAGAGACTCCTCCGGCAAGGATTCGCATC ATGCGGCGCGGACGACCCACTACGGTGGAGGTGGAGGCTCACTAGTGCCCC -3' (5'MBP-55-69 primer) (SEQ ID NO:17) and

5'-GGGGCACTAGTGAGCCTCCACCTCCACCGTAGTGGGTCGTC CGCGCCGCATGATGCGAATCCTTGCCGGAGGAGTCTCTGCCCATGGTAATA-3' (3'MBP-55- 69 primer) (SEQ ID NO:18). These were gel purified, annealed and then cut with NcoI and XhoI for ligation into ß1α1/MBP-72-89 digested with NcoI and XhoI, to produce a plasmid encoding the ß1α1/MBP- 55-69 covalent construct. The primers used to generate the Guinea pig MBP-72-89/linker cartridge were

5'-TATTACCATGGGCAGAGACTCCCCACAGAAGAGCCAGAGG TCTCAGGATGAGAACCCAGTGGTGCACTTCGGAGGTGGAGGCTCACACTAGTG CCCC-3' (5' Gp-MBP-72-89 primer) (SEQ ID NO:28) and

5'GGGGCACTAGTGAGCCTCCACCTCCGAAGTGCACCACTGGG TTCTCATCCTGAGACCTCTGGCTCTCTGTGGGGAGTCTCTGCCCATGGTAA T-3' (3' Gp-MBP-72-89 primer) (SEQ ID NO:29). These were gel purified, annealed and then cut with Ncol and XhoI for ligation into ß1α1/MBP-72-89 digested with NcoI and Xhol, to produce a plasmid encoding the ß1α1/Gp-MBP-72-89 covalent construct. The primers used to generate the CM-2/linker cartridge were

5'-TATTACCATGGGCAGAGACTCCAAACTGGAACTGCAGTCCG CTCTGGAAGAAGCTGAAGCTTCCCTGGAACACGGAGGTGGAGGCTCACTAG TGCCCC-3' (5' CM-2 primer) (SEQ ID NO:19) and

5'-GGGGCACTAGTGAGCCTCCACCTCCGTGTTCCAGGGAAGCT TCAGCTTCTTCCAGAGCGGACTGCAGTTCCAGTTTGGAGTCTCTGCCCATGG TAATA-3' (3' CM-2 primer) (SHQ ID NO:20). These were gel purified, annealed and then cut with NcoI and XhoI for ligation into ß1α1/MBP-72-89 digested with NcoI and XhoI, to produce a plasmid encoding the ß1α1/CM-2 covalent construct,

Protein expression was tested in a number of different *E. coli* strains, including a thioredoxin reductase mutant which allows disulfide bond formation in the cytoplasm (Derman *et al.*, 1993). With such a small molecule, it became apparent that the greatest yield of material could be readily obtained from inclusion bodies, refolding the protein after solubilization and purification in buffers containing 6M urea. Accordingly, *E. coli* strain BL21(DB3) cells were transformed with the pET21d+ construct containing the ß1α1-encoding sequence. Bacteria were grown in one liter cultures to mid-logarithmic phase (OD₆₀₀ = 0.6-0.8) in Luria-Bertani (LB) broth containing carbenicillin (50 µg/ml) at 37°C. Recombinant protein production was induced by addition of 0.5 mM isopropyl ß-D-thiogalactoside (IPTG). After incubation for 3 hours, the cells were centrifuged and stored at -80°C before processing. All subsequent manipulations of the cells were at 4°C. The cell pellets were resuspended in ice-cold PBS, pH 7.4, and sonicated for 4 x 20 seconds with the cell suspension cooled in a salt/ice/water bath. The cell suspension was then centrifuged, the supernatant fraction was poured off, the cell pellet resuspended and washed three times in PBS and then resuspended in 20 mM ethanolamine/G M urea, pH 10, for four hours. After centrifugation, the supernatant containing the solubilized recombinant protein of interest was collected and stored at 4°C until purification. Recombinant ß1α1 construct was purified and concentrated by FPLC ion-exchange chromatography using Source 30Q anion-exchange media (Pharmacia Biotech, Piscataway, no) in an XK26/20 column (Pharmacia Biotech), using a step gradient with 20 mM ethanolamine/6M urea/1M NaCl, pH 10. The homogeneous peak of the appropriate size was collected, dialyzed extensively against PBS at 4°C, pH 7.4, and concentrated by centrifugal ultrafiltration with Centricon-10 membranes (Amicon, Beverly, MA). The dialysis step, which removed the urea from the protein preparation and reduced the final pH, resulted in spontaneous re-folding of the expressed protein. For purification to homogeneity, a finish step used size exclusion chromatography on Superdex 75 media (Pharmacia Biotech) in an HR16/50 column (Pharmacia Biotech). The final yield of purified protein varied between 15 and 30 mg/L of bacterial culture.

Conformational integrity of the molecules was demonstrated by the presence of a disulfide bond between cysteines ß15 and ß79 as detected on gel shift assay, and the authenticity of the purified protein was verified using the OX-6 monoclonal antibody specific for RT1B by Western Blotting. Circular dichroism (CD) reveals that the ß1α1 molecules have highly ordered secondary structures. The empty ß1a1 molecule contains approximately 30% alpha-helix, 15% beta-strand, 26% beta-turn, and 29% random coil structures. Comparison with the secondary structures of class II molecules determined by x-ray crystallography provides strong evidence that the ß1a1 molecules share the beta-sheet platform/anti-parallel alpha-helix secondary structure common to all class II antigen binding domains. Furthermore, thermal denaturation revealed a high degree of cooperativity and stability of the molecules.

### Example 2

### ß1α1Mololecules Bind T Lymphocytes in an Epitope-Specific Manner

The ß1α1 molecule produced as described above was tested for efficacy (T-cell binding specificity) using the Experimental Autoimmune Encephalomyelitis systems. EAE is a paralytic, inflammatory, and sometimes demyelinating disease mediated by CD4+ T-cells specific for central nervous system myelin components including myelin basic protein (MBP). EAE shares similar immunological abnormalities with the human demyelinating disease MS (Paterson, 1981) and has been a useful model for testing preclinical therapies. (Weiner *et al.*, 1993; Vandenbark *et al.*, 1989; Howell e*t al.*, 1989; Oksenberg *et al.*, 1993; Yednock *et al.*, 1992; Jameson *et al.*, 1994; Vandenbark *et al.*, 1994). In Lewis rats, the dominant encephalitogenic MBP epitope resides in the 72-89 peptide (Bourdette *et al.*, 1991). Onset of clinical signs of EAE occurs on day 10-11, and the disease lasts four to eight days with the majority of invading T lymphocytes localized in the CNS during this period.

Test and control peptide for loading into the purified ß1α1 molecules were synthesized as follows: Gp-MBP-69-89 peptide (GSLPQKSQRSQDENPVVHF) (SEQ ID NO:25), rat-MBP-69-89 peptide (GSLPQKSQRTQDENPVVHF) (SEQ ID NO:30), Gp-MBP-55-69 peptide (SGKDSHHAARTTHYG) (SEQ ID NO:26), and cardiac myosin peptide CM-2 (KLELQSALEEAEASLEH) (SEQ ID NO:27) (Wegmann *et al.*, 1994) were prepared by solid-phase techniques (Hashim *et al.*, 1986). The Gp-MBP peptides are numbered according to the bovine MBP sequence (Vandenbark *et al.*, 1994; Martenson, 1984). Peptides were loaded onto ß1α1 at a 1:10 protein:peptide molar ratio, by mixing at room temperature for 24 hours, after which all subsequent manipulations were performed at 4°C. Free peptide was then removed by dialysis or centrifugal ultrafiltration with Centricon-10 membranes, serially diluting and concentrating the solution until free peptide concentration was less than 2 µM.

T-cell lines and the A1 hybridoma were prepared as follows: short-term T-lymphocyte lines were selected with MBP-69-89 peptide from lymph node cells of naive rats or from rats immunized 12 days earlier with Gp-MBP/CFA as described by Vandenbark *et al.*, 1985. The rat Vß8.2+ T-cell hybridoma C14/BW12-12A1 (A1) used in this study has been described previously (Burrows *et al.*, 1996). Briefly, the A1 hybridoma was created by fusing an encephalitogenie LEW(RT1¹) T-cell clone specific for Gp-BP-72-89 (White *et al.*, 1989; Gold et al, 1991) with a TCR (α/ß) negative thymoma, BW5147 (Golding *et al.*, 1985). Wells positive for cell growth were tested for IL-2 production after stimulation with antigen in the presence of APCs (irradiated Lewis rat thymocytes) and then subcloned at limiting dilution. The A1 hybridoma secretes IL-2 when stimulated in the presence of APCs with whole Gp-BP or Gp-BP-69-89 peptide, which contains the minimum epitope, MBP-72.-89.

Two-color immunofluorescent analysis was performed on a FACScan instrument (Becton Dickinson, Mountain View, CA) using CellQuest^{™} software. Quadrants were defined using non-relevant isotype matched control antibodies. ß1α1 molecules with and without loaded peptide were incubated with the A1 hybridoma (10 µM ß1α1/peptide) for 17 hours, 4°C, washed three times, stained with fluorochrome (FITC or PE) conjugated antibodies specific for rat class II (OX6-PE), and TCR Vß8.2 (PharMingen, San Diego, CA) for 15 minutes at room temperature, and analyzed by flow cytometry. The CM-2 cell line was blocked for one hour with unconjugated OX6, washed and then treated as the A1 hybridoma. Staining media was PBS, 2% fetal bovine serum, 0.01% azide.

Epitope-specific binding was evaluated by loading the ß1α1 molecule with various peptides and incubating ß1α1/peptide complexes with the A1 hybridoma that recognizes the MBP-72-89 peptide (Burrows *et al.*, 1997), or with a cardiac myosin CM-2-specific cell line. As is shown in Fig. 3A, the ß1α1 construct loaded with MBP-69-89 peptide (ß1α1/MBP-69-89) specifically bound to the A1 hybridoma, with a mean fluorescence intensity (MFI) of 0.8 x 10³ Units, whereas the filed construct loaded with CM-2 peptide (ß1α1/CM-2) did not stain the hybridoma. Conversely, ß1α1/CM-2 specifically bound to the CM-2 line, with a MFI of 1.8 x 10³ Units, whereas the ß1α1/MBP-69-89 complex did not stain the CM-2 line (Fig. 3B). The ß1α1 construct without exogenously loaded peptide does not bind to either the A1 hybridoma (Fig. 3A) or the CM-2 line. Thus, bound epitope directed the specific binding of the ß1α1/peptide complex.

### Example 3

### ß1α1 Molecules Conjugated With A Fluorescent Label

To avoid using a secondary antibody for visualizing the interaction of ß1α1/peptide molecules with TCR (such as OX-6, used above), a ß1α1 molecule directly conjugated with a chromophore was produced. The Alexa-488^{™} dye (A488; Molecular Probes, Eugene, OR) has a spectra similar to fluorescein, but produces protein conjugates that are brighter and more photo-stable than fluorescein conjugates. As is shown in Fig. 4, when loaded with MBP-69-89, A488-conjugated ß1α1 (molar ratio dye/protein = 1) bound to the A1 hybridomas (MCI = 300 Units), whereas empty ß1α1 did not.

### Example 4

### ß1α1 Molecules Inhibit Epitope-Specific T-cell Proliferation In vitro

T-cell proliferation assays were performed in 96-well plates as described previously (Vandenbark *et al.*, 1985). Briefly, 4 X 10⁵ cells in 200 µl/well (for organ stimulation assays) or 2 X 10⁴ T-cells and 1 X 10⁶ irradiated APCs (for short-term T-cell lines) were incubated in RPMI and 1% rat serum in triplicate wells with stimulation medium only, Con A, or antigen with or without supplemental IL-2 (20 Units/ml) at 37°C in 7% CO₂. The cultures were incubated for three days, for the last 18 hr in the presence of [³H]thymidine (0.5 uCi/10 µl/well). The cells were harvested onto glass fiber filters and [³H]thymidine uptake was assessed by liquid scintillation. In some experiments, the T-cells were pretreated for 24 hours with ß1α1 constructs (with and without loaded peptides), washed, and then used in proliferation assays with and witliout IL-2, as above. Mean counts per minute ± SD were calculated from triplicate wells and differences between groups determined by Student's t-test,

A range of concentrations (10 nM to 20 µM) of peptide-loadcd ß1α1 complexes were pre-incubated with an MBP-69-89 specific T-cell line prior to stimulation with the MBP-69-89 peptide, + APC (antigen-presenting cell). As is shown in Fig. 5, pre-treatment of MBP-69-89 specific T-cells with 10 nM ß1α1/MBP-69-89 complex significantly inhibited proliferation (>90%), whereas pre-incubation with 20 µM ß1α1/MBP-55-69 complex produced a nominal (27%) but insignificant inhibition. Of mechanistic importance, the response inhibited by the ß1α1/MBP-69-89 complex could be fully restored by including 20 Units/ml of IL-2 during stimulation of the T-cell line (Fig. 5) suggesting that the T-cells had been rendered anergic by exposure to the ß1α1/MBP-69-89 complex.

### Example 5

### Antigen-Loaded ß1α1 Molecules Suppress Induction and Treat Existing Signs of EAE

Female Lewis rats (Harlan Sprague-Dawley, Inc., Indianapolis, Indiana), 8-12 weeks of age, were used for clinical experiments in this study. The rats were housed under germ-free conditions at the Veterans Affairs Medical Center Animal Care Facility, Portland, Oregon, according to institutional guidelines. Active EAE was induced in the rats by subcutaneous injection of 25 µg guinea, pig myelin basic protein (GP-MBP) or 200 µg GP-MBP-69-89 peptide in Freund's complete adjuvant supplemented with 100 or 400 µg *Mycobacterium tuberculosis* strain H37Ra (Difco, Detroit, MI), respectively. The clinical disease course induced by the two emulsions was essentially identical, with the same days of onset, duration, maximum severity, and cumulative disease index. The rats were assessed daily for changes in clinical signs according to the following clinical rating scale: 0, no signs; 1, limp tail; 2, hind leg weakness, ataxia; 3, paraplegia; and 4, paraplegia with forelimb weakness, moribund condition. A cumulative disease score was obtained by summing the daily disability scores over the course of EAE for each affected rat, and a mean cumulative disease index (CDI) was calculated for each experimental group.

On days 3, 7, 9, 11 and 14 disease induction, the rats were given ß1α1 peptide complex, peptide alone, or were left untreated as indicated. As can be seen in Figure 6 and Table 1, intravenous injection (i,v.) of 300 µg of the ß1α1/MBP-69-89 complex in saline suppressed the induction of clinical and histological signs of EAE.

**Table 1. Characterization of infiltrating spinal cord cells at the peak of EAE in control and β1α1/MBP-69-89 protected rats.**

| Spinal cord | Total* | OX40+ | Vß8.2+ | Vß8.2+/OX40+ |
|---|---|---|---|---|
| Protected | 200 | 38 | 10 | 5 |
| Control | 7300 | 1750 | 980 | 667 |

| | | | | |
|---|---|---|---|---|
| *Number of cells/spinal cord x 10⁻³ | | | | |

Spinal cord mononuclear cells were isolated by a discontinuous percol gradient technique and counted as previously described (Bourdette *et al.,* 1991). The cells were stained with fluorochrome (FITC or PE) conjugated antibodies specific for rat CD4, CD8, CD11b, CD45ra, TCR Vß8.2 and CD134 (PharMingen, Sam Diego, CA) for 15 min. at mom temperature and analyzed by flow cytometry. The number of positive staining cells per spinal cord was calculated by multiplying the percent staining by the total number of cells per spinal cord. Control and ß1α1/MBP-69-89 protected rats were sacrificed at peak and recovery of clinical disease. Spinal cords were dissected and fixed in 10% buffered formalin. The spinal cords were paraffin-embedded and sections were stained with luxol fast blue-periodic acid schiff-hematoxylin for light microscopy.

Injection of as little as 30 µg of the ß1α1/MBP-69-89 complex following the same time course was also effective, completely suppressing EAE in 4 of 6 rats, with only mild signs in the other 2 animals. All of the control animals that were untreated, that received 2 µg MBP-69-89 peptide alone (the dose of free peptide contained in 30 µg of the complex), or that received 300 µg of the empty ß1α1 construct developed a comparable degree of paralytic EAE (Table 2). Interestingly, injection of 300 µg of a control ß1α1/CM-2 peptide complex produce a mild (about 30%) suppression of EAE (Fig. 6 and Table 2). In parallel with the course of disease, animals showed a dramatic loss in body weight (Fig. 6), whereas animals treated with the ß1α1/MBP-69-89 complex showed no significant loss of body weight throughout the course of the experiment.

**Table 2. Effect of β1α1/peptide complexes on EAE in Lewis rats.**

| Treatment of EAE^{a} | Incidence | Day of Onset | Duration (days) | Maximum Disease Score | Cumulative Disease Index |
|---|---|---|---|---|---|
| Untreaed^{b} | 11/11 | 12 ± 1^{c} | 5 ± 1 | 2.9 ± 0.3 | 10.0 ± 2.2 |
| 2 µg MBP-69-89 | 6/6 | 12 ± 1 | 6 ± 1 | 3.3 ± 0.3 | 11.2 ± 1.9 |
| ß1α1/(empty) 300 µg | 5/5 | 12 ± 1 | 6 ± 1 | 2.9 ± 0.6 | 9.7 ± 2.1 |
| ß1α1/CM-2 300µ | 5/5 | 12 ± 1 | 6 ± 2 | 1.9 ± 0.8 | 7.2 ± 2.6* |
| ß1α1/MBP-69-89 300 µg | 0/6* | --- | --- | 0 ± 0** | 0 ± 0** |
| ß1α1/MBP-69-89 30 µg | 2/6 | 14 ± 0 | 4 ± 0 | 0.2 ± 0.1** | 0.7 ± 0.3** |

| | | | | | |
|---|---|---|---|---|---|
| ^{a} EAE was induced with either Gp-BP/CFA or MBP-69-89/CFA. ^{b} Combined controls from two experiments. ^{c} Values represent the mean ± S.D. * P 0.05 **P 0.01 1 | | | | | |

To evaluate the effect of the construct on established disease, Lexis rats were treated with 300 µg of the ß1α1/MBP-69-89 complex on the first day of disease onset, with follow-up injections 48 and 96 hours later. EAE in the control rats progressed to complete hind limb paralysis, whereas no progression of the disease occurred in any of the treated animals (Fig. 7). The mild course of EAE (mean cumulative index, MCl = 3 ± 0.13) in the treated group was significantly less than the severe course of EAE in the control group (MCI = 11.2 ± 2.7, p = 0.013), although the duration of disease (6 days) was the same in both groups.

Consistent with the complete lack of inflammatory lesions in spinal cord histological sections, suppression of EAE with the ß1α1/MBP-69-89 complex essentially eliminated the infiltration of activated inflammatory cells into the CNS. Mononuclear cells were isolated from the spinal cords of control and protected animals at peak and recovery of clinical disease and examined by FACS analysis. The total number of mononuclcar cells isolated from spinal cords of control animals at peak of clinical disease (day 14) was 40-fold higher than from protected animals evaluated at the same time point (Table 1). Moreover, protected animals had 72% fewer activated (OX40+), Vß8.2+ T-cells in the spinal cord when compared to control animals (Table 1). CD4+ and CD8+ T-cells, macrophages and B cell numbers were also significantly reduced in protected animals. The number of mononuclear cells isolated after recovery from EAE was reduced 4.5-fold in protected animals (0.64 x 10⁵ cells/spinal cord) compared to control animals (2.9 x 10⁵ cells/spinal cord). Protected animals also had 10-fold fewer activated (OX40+), Vß8.2+ T-colis in the spinal cord than control animals after recovery from disease.

Treatment with ß1α1/MBP-69-89 complex specifically inhibited the delayed-type hypersensitivity (DTH) response to MBP-69-89. As shown in Fig. 8A, changes in ear thickness 24 hours after challenge with PPD were unaffected by in animals treated with ß1 1 or ß1α1 loaded with peptides. However, as is shown in Fig. 8B, while animals treated with ß1α1 alone or complexed with CM-2 had no effect on the DTH response, animals treated with the ß1α1/MBP-69-89 complex showed a dramatic inhibition of the DTH response to MBP-69-89.

Treatment of EAE with the ß1α1/MBP-69-89 complex also produced an inhibition of lymph node (LN) T-cell responses. As is shown in Fig. 9, LN cells from rats treated with the suppression protocol (Fig. 6) were inhibited 2-4 fold in response to MBP or the MBP-69-89 peptide compared to control rats. This inhibition was antigen specific, since LN T-cell responses to PPD (stimulated by the CFA injection) were the same in treated and control groups. T-cell responses tested in rats treated after disease onset (Fig. 7) were also inhibited, in an IL-2 reversible manner. LN cell responses to MBP and MBP-69-89 peptide were optimal (S.I = 4-5X) at low antigen (Ag) concentrations (4 µg/ml), and could be enhanced 2-flold with additional IL-2. In contrast, responses were inhibited in treated rats, with optimal LN cell responses (±3X) requiring higher Ag concentrations (20-50 µg/ml). However, in the presence of IL-2, responses could be restored to a level comparable to control rats (S.I. = 6-11X) without boosting Ag concentrations.

In the present polypeptide, comprising the MHC class II ß1 and α1 domains are described. These molecules lack the β2 domain, the ß2 domain known to bind to CD4, and transmembrane and intra-cytoplasmic sequences. The reduced size and complexity of the ß1α1 construct permits expression and purification of the molecules from bacterial inclusion bodies in high yield. The ß1α1 molecules are shown to refold in a manner that allows binding of allele-specific peptide epitopes and to have excellent solubility in aqueous buffers. When complexed with peptide antigen, direct detection of the ß1α1/peptide complexes to T-cells can be visualized by FACS, with the specificity of binding determined by the peptide antigen. The ß1α1/69-89 complex exerted powerful and selective inhibitory effects on T-cell activation *in vitro* and *in vivo.* Because of its simplicity, biochemical stability, biological properties, and structural similarity with human class II homologs, the ß1α1 construct represents a template for producing a novel class of TCR ligands.

Direct binding studies using the A1 hybridoma specific for MBP-72-89 showed distinct staining with ß1α1/MBP-69-89, with a 10-fold increase in MFI over background, and was not stained with ß1α1/CM-2 nor "empty" Blab. In a reciprocal manner, binding studies using a CM-2 specific cell line showed strong staining with ß1α1/CM-2 and no staining with ß1α1/MBP-69-89. Thus, bound epitope, directed specific interaction of the ß1α1/peptide complexes. Identification of antigen-specific T-cells has been possible in a few systems (McHeyzer *et al*., 1995; MacDonald *et al*., 1993; Walker *et al*., 1995; Reiner *et al*., 1993), using labeled anti-idiotypic T-ceM receptor antibodies as specific markers, but the general approach of staining specific T-cells with their ligand has failed because soluble peptide-MHC complexes have an inherently fast dissociation rate from the T-cell antigen receptor (Corr *et al*., 1995; Matsui *et al*., 1994; Syulkev *et al*., 1994). Multimeric peptide-MHC complexes containing four-domain soluble MHC molecules have been used to stain antigen-specific T lymphocytes (Altman *et al*., 1996), with the ability to bind more than one T-cell receptor (TCR) on a single T-cell presumably giving the multimeric molecules a correspondingly slower dissociation rate. Staining with ß1α1/peptide complexes, while specific, did take an incubation period of approximately 10 hours to saturate. The extraordinarily bright staining pattern of the A1 hybridoma with the ß1α1/MBP-69-89 complex, and the CM-2 line with ß1α1/CM-2, coupled with the length of time it takes to achieve binding saturation, suggests that this molecule might have a very slow off-rate once bound to the TCR. These complexes and modified versions of them would be unusually well suited to directly label antigen-specific T-cells for purposes of quantification and recovery.

The ß1α1/peptide complex was highly specific in its ability to bind to and inhibit the function of T-cells. *In vitro* proliferation of MBP-specific T-cells was inhibited >90% with the ß1α1/MBP-69-89 complex, and *in vivo* there was a nearly complete inhibition of clinical and histological EAE.

The most profound biological activity demonstrated for ß1α1/MBP-69-89 was its ability to almost totally ablate the encephalitogenic capacity of MBP-69-89 specific T-cells *in vivo*. Injection of this complex after initiation of EAE nearly completely suppressed clinical and histological signs of EAE, apparently by directly inhibiting the systemic activation of MBP-69-89 specific T-cells, and preventing recruitment of inflammatory cells into the CNS. Moreover, injection of ß1α1/MBP-69-89 after onset of clinical signs arrested disease progression, demonstrating the therapeutic potential of this molecular construct. Interestingly, the effect of the complex on already activated T-cells was not only to inhibit stimulation, but also to reduce sensitivity to antigen, with optimal activation after treatment requiring a 10-fold increase in antigen concentration.

From a drug engineering and design perspective this prototypic molecule represents a major breakthrough. The demonstrated biological efficacy of the ß1α1/MBP-69-89 complex in EAE raises the possibility of using this construct as a template for engineering human homologs for treatment of autoimmune diseases, such as multiple sclerosis, that likely involves inflammatory T-cells directed at CNS proteins. One candidate molecule would be HLA-DR2/MBP-84-102, which includes both the disease-associated class II allele and a known immunodominant epitope that has been reported to be recognized more frequently in MS patients than controls. However, because of the complexity of T-cell response to multiple CNS proteins and their component epitopes, it is likely that a more general therapy may require a mixture of several MHC/Ag complexes. The precision of inhibition induced by the novel ß1α1/MBP-69-89 complex reported herein represents an important first step in the development of potent and selective human therapeutic reagents. With this new class of reagent, it may be possible to directly quantify the frequency and prevalence of T-cells specific for suspected target autoantigens, and then to selectively eliminate them in affected patients. Through this process of detection and therapy, it may then be possible for the first time to firmly establish the pathogenic contribution of each suspected T-cell specificity.

### Example 6

### Design, Engineering and Production of Human Recombinant T-cell Receptor Ligands Derived from HLA-DR2 Experimental Procedures

### Homology Modeling

Sequence alignment ofMHC class II molecules from human, rat and mouse species provided a starting point for these studies (Burrows, *et al.,* 1999). Graphic images were generated with the program Sybyl (Tripos Associates, St. Louis, MO) and an 02 workstation (IRIX 6.5, Silicon Graphics, Mountain View, CA) using coordinates deposited in the Brookhaven Protein Data Bank (Brookhaven National Laboratories, Upton, NY). Structure-based homology modeling was based on the refined crystallographic coordinates of human DR2 (Smith *et al*., 1998; Li *et al.,* 2000) as well as DR1 (Brown *et al*., 1996; Murthy *et al*., 1997), murine I-E k molecules (Fremont *et al.,* 1996), and scorpion toxins (Zhao *et al.,* 1992; Housset *et al.,* 1994; Zinn-Justin *et al.,* 1996). Amino acid residues in human DR2 (PDB accession numbers 1BX2) were used. Because a number of residues were missing/not located in the crystallographic data (Smith *et al.,* 1998), the correct side chains were inserted and the peptide backbone was modeled as a rigid body during structural refinement using local energy minimization.

### Recombinant TCR ligands (RTLs)

For production of the human RTLs, mRNA was isolated (Oligotex Direct mRNA Mini Kit; Qiagen, Inc., Valencia, CA) from L466.1 cells grown in RPMI media. First strand cDNA synthesis was carried out using Superscript II Rnase H-reverse transcriptase (Gibco BRL, Grand Island, NY).

Using the first strand reaction as template source, the desired regions of the DRB*1501 and DRA*0101 DNA sequences were amplified by PCR using Taq DNA polymerase (Gibco BRL, Grand Island, NY), with an annealing temperature of 55°C. The primers used to generate ß1 were 5'-ATTACCATGGGGGACACCCGACCACGTTT-3' (huNcoI→, SEQ ID NO:28) and 5'-GGATGATCACATGTTCTTCTTTGATGACTCGCCGCTGCACTGTGA-3' (hu ß1α1 Lig←, SEQ ID NO:29). The primers used to generate α1 were 5'-TCACAGTGCAGCGGCGAGTCATCAAAGAAGAACATGTGATCATCC-3' (hu ß1α1 Lig→ SEQ ID NO:30) and 5'-TGGTGCTCGAGTTAATTGGTGATCGGAGTATAGTTGG-3' (huXhoI← SEQ ID NO:31).

The amplification reactions were gel purified, and the desired bands isolated (QIAquick Gel Extraction Kit; Qiagen, Inc., Valencia, CA). The overhanging tails at the 5'-end of each primer added overlapping segments and restriction sites (Ncol and XhoI) at the ends of each PCR amplification product. The two chains were linked in a two step PCR reaction. In the first step, 5 µl of each purified amplification product were added to a 50 µl primer free PCR reaction, and cycled five times at an annealing temperature of 55°C. A 50 µl reaction mix containing the huNcoI → and huXhoI ← primers was then added directly to the initial reaction, and cycled 25 times at an annealing temperature of 50°C. Taq DNA Polymerase (Promega, Madison, WI) was used in each step. The final 100 µl reaction was gel purified, and the desired hu ß1α1 amplification product isolated.

The hu ß1α1 insert was ligated with the PCR 2.1 plasmid vector (TA Cloning kit, Invitrogen, Carlsbad, CA), and transformed into an INVa'F bacterial cloning host. PCR colony screening was used to select a single positive colony, from which plasmid DNA was isolated (QlAprep Spin Mini Kit, Qiagen, Inc., Valencia CA). Plasmid was cut with NcoI and Xhol restriction enzymes (New England BioLabs Inc., Beverly, MA), gel purified, and the hu ß1α1 DNA fragment isolated. The hu ß1α1 DNA insert was ligated with NcoI/XhoI digested pBT-21d(+) plasmid expression vector (Novagen, Inc., Madison, WI), and transformed into BL21(DE3) expression host (Novagen, Inc., Madison, WI). Bacterial colonies were selected based on PCR colony and protein expression screening.

Plasmid DNA was isolated from positive colonies (QIAquick Gel Extraction Kit, Qiagen Inc., Valencia, CA) and sequenced with the T7 5'-TAATACGACTCACTATAGGG-3' (SEQ ID NO:32) and T7 terminator ← 5'-GCTAGTTAGCTCAGCGG-3' (SEQ ID NO:33) primers. After sequence verification a single clone was selected for expression of the hu ß1α1 peptide (RTL300).

A 30 amino acid huMBP-85-99/peptide linker cartridge was genetically inserted into the "empty" hu ß1α1 (RTL300) coding sequence between Arg5 and Pro6 of the β1 chain. The 90 bp DNA sequence encoding peptide-Ag and linker was inserted at position 16 of the RTL300 DNA construct in a three step PCR reaction, using Taq DNA Polymerase (Promega, Madison, WI).

In the first step, pET-21d(+)/RTL300 plasmid was used as template in two separate PCR reactions. In the first reaction, the region from the start of the T7 priming site of the pET-21d(+) plasmid to the point of insertion within the hu ß1α1 (RTL300) sequence was amplified with the following primers; 5'-GCTAGTTATTGCTCAGCGG-3'(T7→, SEQ ID NO:33), and 5'-AGGCTGCCACAGGAAACGTGGGCCTCCACCTCCAGAGCCTCGGGGCACTAGT GAGCCTCCACCTCCACGCGGGGTAACGATGTTTTTGAAGAAGTGAACAACCG GG TTTTCTCGGGTGTCCCCCATGGTAAT-3' (huMBP-85-99Lig←, SEQ ID NO.34).

In the second reaction, the region from the point of insertion within the hu ß1α1 (RTL300) sequence to the end of the T7-terminator priming site was amplified with the following primers: 5'-CCACGTTTCCTGTGGCAGCC-3' (huMBP-85-99Lig→SEQ ID NO:35), and 5'-GCTAGTTATTGCTCAGCGG-3' (T7terminator ← SEQ ID NO:33).

Each reaction was gel purified, and the desired bands isolated.

In the second step, 5 µl of each purified amplification product was added to a primer free PCR reaction mix, and cycled for 5 times at an annealing temperature of 50°C. In the third step, a 50 µl PCR, 'amplification mix' containing the 5'-TAATACOACTCACTATAGGG-3' (T7 →SEQ ID NO:32) and 5'-GCTAGTTATTGCTCAGCGG-3' (T7terminator ← SEQ ID NO:33) primers was then added directly to the 'anneal-extend' reaction, and the entire volume cycled 25 times using a 55°C annealing temperature. The non-complimentary 5' tail of the huMBP-85-99lig ← primer included DNA encoding the entire peptide/linker cartridge, and the region down-stream from the point of insertion.

The resulting amplification product hybridized easily with the PCR. product produced in the second reaction, via the complimentary 3' and 5' ends of each respectively. DNA polymerase then extended from the 3'-end of each primer, creating the full length hu ß1α1/huMBP-85-99 (RTL301) construct, which acted as template in the 'amplification' step. The reaction was purified using agarose gel electrophoresis, and the desired hu ß1α1/huMBP-85-99 (RTL301) band isolated. The PCR product was then cut with NcoI and XhoI restriction enzymes, gel purified, ligated with a similarly cut pET-21d(+) plasmid expression vector, and transformed into a BL21(DE3) *E. coli* expression host. Transformants were screened for protein expression and the presence of the desired insert with a PCR colony screen. Plasmid DNA was isolated from several positive clones and sequenced. A single positive clone was selected for expression of the hu ß1α 1/huMBP-85-99 peptide (RTL301).

Repeated sequence analysis of pET-21d(+)/RTL300 and pET-21d(+)/RTL301 plasmid DNA constructs revealed the same thymine to cytosine single base pair deviation at position 358 and position 458 (RTL300 and RTL301 numbering, respectively), than had been reported previously for HLA-DRA*0101 (Genebank accession #M60333), which resulted in an F150L mutation in the RTL300 and RTL301 molecules (RTL301 numbering).

Site directed mutagenesis was used to revert the sequence to the Genebank #M60333 sequence. Two PCR reactions were performed using the pET-21d(A-)/RTL300 and pET-21 d(+)/RTL301 plasmids as template. For RTL300 the primers: 5'-TAATACGACTCACTATAGGG-3' (T7 →SEQ ID NO:32), and 5'-TCAAAGTCAAACATAAACTCGC-3' (huBA-F150L ← SEQ ID NO:36) were used.

For RTL301 the primers: 5'-GCGAGTTTATGTTTGACTTTGA-3' (huBA-F150L →SEO ID NO:37), and 5'-GCTAGTTATTGCTCAGCGG-3' (T7terminator ←, SEQ ID NO:33) were used.

The two resulting amplification products were gel purified, and isolated (QIAquick gel extraction kit, Qiagen, Valencia, CA), annealed, and amplified as described earlier, based on the complimentary 3' and 5' ends of each of the PCR products. The final amplification reactions were gel purified, and the desired PCR products isolated. The NcoI and XhoI restriction sites flanking each were then used to subclone the RTL DNA constructs into fresh pET-21d(+) plasmid for transformation into BL21(DE3) competent cells and plasmid sequence verification. Positive clones were chosen for expression of the "empty" HLA-DR.2 ß1α1-derived RTL302 molecule and the MBP-85-99- peptide coupled RTL303 molecule (Fig. 2).

### Expression and in vitro folding of the RTL constructs

E. coli strain BL21 (DE3) cells were transformed with the pET21 d+/RTL vectors. Bacteria were grown in one liter cultures to mid-logarithmic phase (OD 600 # 0.6-0.8) in Luria-Bertani (LB) broth containing carbenicillin (50 µg/ml) at 37°C. Recombinant protein production was induced by addition of 0.5 mM isopropyl ß-D-thiogalactoside (IPTG). After incubation for 3 hours, the cells were collected by centrifugation and stored at -80°C before processing, All subsequent manipulations of the cells were at 4°C. The cell pellets were resuspended in ice-cold PBS, pH 7.4, and sonicated for 4 x 20 seconds with the cell suspension cooled in a salt/ice/water bath. The cell suspension was then centrifuged, the supernatant fraction was poured off, the cell pellet resuspended and washed three times in PBS and then resuspended in 20 mM ethanolamine/6 M urea, pH 10, for four hours. After centrifugation, the supernatant containing the solubilized recombinant protein of interest was collected and stored at 4°C until purification.

The recombinant proteins of interest were purified and concentrated by FPLC ion-exchange chromatography using Source 30Q anion-exchange media (Pharmacia Biotech, Piscataway, NJ) in an XK26/20 column (Pharmacia Biotech), using a step gradient with 20 mM ethanolamine/6M urea/1M NaCl, pH 10. The proteins were dialyzed against 20 mM ethanol amine, pH 10.0, which removed the urea and allowed refolding of the recombinant protein. This step was critical. Basic buffers were required for all of the RTL molecular constructs to fold correctly, after which they could be dialyzed into PBS at 4°C and concentrated by centrifugal ultra-filtration with Centricon-10 membranes (Amicon, Beverly, MA). For purification to homogeneity, a finish step was included using size exclusion chromatography on Superdex 75 media (Pharmacia Biotech) in an HR16/50 column (Pharmacia Biotech). The final yield of purified protein varied between 15 and 30 mg/L of bacterial culture.

### Circular dichroism and thermal transition measurements

CD spectra were recorded on a JASCO J-500A spectropolarimeter with an IF-500 digital interface and thermostatically controlled quartz cells (Hellma, Mulheim, Germany) of 2,1, 0.5, 0.1 and 0.05 mm path length depending on peptide concentration. Data are presented as mean residue weight ellipticities. Calibration was regularly performed with (+)-10-camphorsulfonic acid (Sigma) to molar ellipticities of 7780 and-16,160 deg. cm²/dmol at 290.5 and 192.5 nm, respectively (Chen *et al.*, 1977). In general, spectra were the average of four to five scans from 260 to 180 µm recorded at a scanning rate of 5 nm/min. with a four second time constant. Data were collected at 0.1 µm Spectra were averaged and smoothed using the built-in algorithms of the Jasco program and buffer basclines were subtracted. Secondary structure was estimated with the program CONTIN (Provencher *et al.*, 1981). Thermal transition curves were recorded at a fixed wavelength of 222 nm. Temperature gradients from 5 to 90 or 95°C were generated with a programmer controlled circulating water bath (Lauda PM350 and RCS20D). Heating and cooling rates were between 12 and 18°C/h. Temperature was monitored in the cell with a thermistor and digital thermometer (Omega Engineering), recorded and digitized on an XY plotter (HP7090A, Hewlett Packard), and stored on disk. The transition curves were normalized to the fraction of the peptide folded (F) using the standard equation: F = ([U] - [U]u)/ ([U]n - [U]u), where [U]n and [U]u represent the ellipticity values for the fully folded and fully unfolded species, respectively, and [U] is the observed ellipticity at 222 nm.

### Example 7

### Homology modeling

Previous protein engineering studies have described recombinant T-cell receptor ligands (RTLs) derived from the α-1 and β-1 domains of rat MHC class II RT1.B (Burrows *et al.*, 1999). Homology modeling studies of the heteroclimeric MHC class II protein HLA-DR2, and specifically, the α-1 and β-1 segments of the molecule that comprise the antigen binding domain, were conducted based on the crystal structures of human DR (Smith *et al.*, 1998; Li *et al.*, 2000; Brown *et al.*, 1993; Murthy *et al.*, 1997). In the modeling studies described herein, three facets of the source proteins organization and structure were focused on: (1) The interface between the membrane-proximal surface of the β-sheet platform and the membrane distal surfaces of the α-2 and β-2 Ig-fold domains, (2) the internal hydrogen bonding of the α-1 and β-1 domains that comprise the peptide binding/TCR recognition domain, and (3), the surface of the RTLs that was expected to interact with the TCR.

Side-chain densities for regions that correspond to primary sequence between the β-1 and β-2 domains of human DR and murine I-E^{κ} showed evidence of disorder in the crystal structures (Smith *et al.*, 1998; Li *et al.*, 2000; Brown *et al.*, 1993; *et al.*, 1997; Fremont *et al.*, 1996), supporting the notion that these serve as linker regions between the two domains with residue side-chains having a high degree of freedom of movement in solution. High resolution crystals of MHC class II DR1 and DR2 (Smith *et al.*, 1998; Li *et al.*, 2000; Brown *et al.*, 1993; Murthy *et al.*, 1997) contained a large number of water molecules between the membrane proximal surface of the ß-sheet platform and the membrane distal surfaces of the α2 and ß2 Ig-fold domains. The surface area of interaction between domains was quantified by creating a molecular surface for the ß1α1 and α2ß2 Ig-fold domains with an algorithm developed by Michael Connolly (Connolly, 1986) using the crystallographic coordinates for human DR2 available from the Brookhaven Protein Data Base (1BX2). In this algorithm the molecular surfaces are represented by "critical points" describing holes and knobs. Holes (maxima of a shape function) are matched with knobs (minima). The surface areas of the α1ß1 and α2ß2-Ig-folddomains were calculated independently, defined by accessibility to a probe of radius 0.14 nm, about the size of a water molecule. The surface area of the MHC class IIαß-heterodimer was 160 nm², while that of the RTL construct was 80 11m 2 and the α2ß2-Ig-fold domains was 90 nm². Approximately 15 nm² (19%) of the RTL surface was buried by the interface with the Ig-fold domains in the MHC class II αß-heterodimer.

Human, rat and murine MHC class II alpha chains share 30% identity and the beta chains share 35% identity. The backbone traces of the structures solved using X-ray crystallography showed strong homology when superimposed, implying an evolutionarily conserved structural motif. The variability between the molecules is primarily within the residues that delineate the peptide-binding groove, with side-chain substitutions designed to allow differential antigenic-peptide binding. The α1 and ß1 domains of HLA-DR showed an extensive hydrogen-bonding network and a tightly packed and buried hydrophobic core. This tertiary structure appears similar to the molecular interactions that provide structural integrity and thermodynamic stability to the alpha-helix/beta-sheet scaffold characteristic of scorpion toxins (Zhao *et al.*, 1992; Housset *et al.*, 1994; Zinn-Justin *et al.*, 1996). The ß1-domain of MHC class II molecules contains a disulfide bond that covalently couples the carboxyl-terminal end to the first strand of the anti-parallel ß-sheet platform contributed by the ß1-domain. This structure is conserved among MHC class II molecules from rat, human and mouse, and is conserved within the α2 domain of MHC class 1. It appears to serve a critical function, acting as a "linchpin" that allows primary sequence diversity in the molecule while maintaining its tertiary structure. Additionally, a "network" of conserved aromatic side chains (Burrows, et al, 1999) appear to stabilize the RTLs. The studies described herein demonstrate that the antigen binding domain remains stable in the absence of the α2 and ß2 fold domains.

### Example 8

### Expression and production of RTLs

Novel genes were constructed by splicing sequence encoding the amino terminus of HLA-DR2 α-1 domain to sequence encoding the carboxyl terminus of the β-1 domain. The nomenclature RTL ("recombinant TCR ligand") was used for proteins with this design (see US Patent No. 6,270,772). In the studies described herein, experiments are presented that used the "empty" RTL with the native sequence (RTL302), a covalent construct that contained the human MBP-85-99 antigenic peptide (RTL303), and versions of these molecules (RTL300, "empty"; RTL301, containing MBP-85-99) that had a single phenylalanine to leucine alteration (F150L, RTL303 numbering) that eliminated biological activity (See Fig. 13). Earlier work had demonstrated that the greatest yield of material could be readily obtained from bacterial inclusion bodies, refolding the protein after solubilization and purification in buffers containing 6M urea (Burrows *et al*., 1999). Purification of the RTLs was straightforward and included ion exchange chromatography followed by size exclusion chromatography (Fig. 14).

After purification, the protein was dialyzed against 20 mM ethanolamine, pH 10.0, which removed the urea and allowed refolding of the recombinant protein. This step was critical. Basic buffers were required for all of the RTL molecular constructs to fold correctly, after which they could be dialyzed into PBS at 4°C for *in vivo* studies. The final yields of "empty" and antigenic peptide-coupled RTLs was approximately 15-30 mg/liter culture.

### Example 9

### Biochemical Characterization and Structural Analysis of Human RTLs

Oxidation of cysteines 46 and 110 (RTL303 amino acid numbering, corresponding to DR2 beta chain residues 15 and 79) to reconstitute the native disulfide bond was demonstrated by a gel shift assay (Fig. 15), in which identical samples with or without the reducing agent β-mercaptoeth-anol (β-ME) were boiled 5 minutes prior to SDS-PAGE. In the absence of disulfide bonds are retained and proteins typically demonstrate a higher mobility during electrophoresis through acrylamide gels due to their more compact structure. Representative examples of this analysis are shown for the "empty" RTL300 and RTL302, and the MBP-coupled RTL301 and RTL303 molecules (Fig. 15). All of the RTL molecules produced showed this pattern, indicating presence of the native conserved disulfide bond. These data represent a confirmation of the conformational integrity of the molecules.

Circular dichroism (CD) demonstrated the highly ordered secondary structures of RTL 302 and RTL303 (Fig. 16; Table 3). RTL303 contained approximately 38% alpha-helix, 33% beta-strand, and 29% random coil structures. Comparison with the secondary structures of class II molecules determined by x-ray crystallography (Smith *et al*., 1998; Li *et al.,* 2000; Brown *et al*., 1993; Murthy *et al*., 1997; Fremont *et al*., 1996) provided strong evidence that RTL303 shared the beta-sheet platform/anti-parallel alpha-helix secondary structure common to all class II antigen binding domains (Table 3, Fig. 16).

**Table 3. Secondary structure analysis of RTLs and MHC class II β-1/α-1 domains.**

| Molecule | description | α-helix | β-sheet^{c} | other | total | Reference |
|---|---|---|---|---|---|---|
| RTL201 | RT1.B β1α1/Gp-MBP72-89 | 0.28 | 0.39 | 0.33 | 1.0 | Burrows *et. al.,* 1999 |
| RTL300 | DR2 β1α1(F150L)a | - | - | - | ND^{B} | Chang *et al.,* 2001 |
| RTL301 | DR2 β1α1/hu-MBP85-99 | 0.20 | 0.35 | 0.46 | 1.0 | Chang *et al.,* 2001 |
| RTL302 | DR2 β1α1(empty) | 0.26 | 0.31 | 0.43 | 1.0 | Chang *et al.,* 2001 |
| **RTL303** | DR2 β1α1/hu-MBP85-99 | **0.38** | **0.33** | **0.29** | **1.0** | Chang *et al.,* 2001 |
| **IBX2** | DR2 (DRA*0101, | **0.32** | **0.37** | **0.31** | **1.0** | Smith *et al.,* 1998 |
| IAQD | DR1 (DRA*0101, DRB1 | 0.32 | 0.37 | 0.31 | 1.0 | Murthy *et al.,* 1997 |
| IIAK | murine I-A^{k} | 0.34 | 0.37 | 0.29 | 1.0 | Fremont *et al.,* 1996 |
| IIEA | murine I-E^{k} | 0.27 | 0.31 | 0.42 | 1.0 | Fremont *et al.,* 1996 |

| | | | | | | |
|---|---|---|---|---|---|---|
| a F150L based on RTL303 numbering (See Fig. 2). B RTL300 CD data could not be fit using the variable selection method. c β-sheet includes parallel and anti-parallel β-sheet and β-turn structures. | | | | | | |

Structure loss upon thermal denaturation indicated that the RTLs used in this study are cooperatively folded (Fig. 17). The temperature (Tₘ) at which half of the structure is lost for RTL303 is approximately 78°C, which is similar to that determined for the rat RT1.B MHC class II-derived RTL201 (Burrows *et al.,* 1999). RTL302, which does not contain the covalently coupled Ag-peptide, showed a 32% decease in alpha-helical content compared to RTL303 (Table 3). This decrease in helix content was accompanied by a decrease in thermal stability of 36% (28°C) compared to RTL 303, demonstrating the stabilization of the RTL molecule, and by inference, the antigen-presentation platform of MHC class II molecules, that accompanies peptide binding. Again, this trend is similar to what has been observed using rat RTL molecules (Burrows *et al.,* 1999), although the stabilization contributed by the covalently coupled peptide is approximately 3-fold greater for the human RTLs compared to rat RTLs.

The F150L modified RTL301 molecule showed a 48% decrease in alpha-helical content (Table 3) and a 21% (16°C) decrease in thermal stability compared to RTL303. RTL300, which had the F150L modification and lacked the covalently-coupled Ag-peptide, showed cooperativity during structure loss in thermal denaturation studies, but was extremely unstable (T m = 48°C) relative to RTL302 and RTL303, and the secondary structure could not be determined from the CD data (Figs. 16, 17; Table 3). An explanation for the thermal stability data comes from molecular modeling studies using the coordinates from DR2a and DR2b MHC class II crystal structures (PDB accession code 1FV1 and 1BX2; Smith *et al.*, 1998; Li *et al*., 2000). These studies demonstrated that F150 is a central residue within the hydrophobic core of the RTL structure (Fig. 18), part of a conserved network of aromatic side chains that appears to stabilize the secondary structure motif that is completely conserved in human class II molecules and is highly conserved between rat, mouse and human MHC class 11.

**Table 4. Interactions of residues within 4Å of F150^{a}**

| atom 1 ID | atom 2 ID | distance (Å) |
|---|---|---|
| I133.CG2 (A:I7)^{b} | **F150.CD2** (A:F24) | 3.75 |
| I133.CG2 | **F150-CE2** | 3.75 |
| Q135.CB (A:Q9) | **F150.CE1** | 3.65 |
| Q135.CG | F148.CZ (A:F22) | 4.06 |
| Q135.OE1 | Y109.OH (B:Y78) | 2.49 |
| F148.CE1 | **F150.CE1** | 4.07 |
| **F150.CB** | F158.CE1 (A:F32) | 3.64 |
| **F150.CZ** | H11.O(C:H90) | 3.77 |
| Y109.CE1 | H11.O | 3.12 |

| | | |
|---|---|---|
| ^{a}F150 (RTL303 numbering) is F24 of the beta chain of DR2. The distances were calculated using coordinates from 1BX2 (Smith *et al*., 1998). ^{b}The residue are numbered as shown in Fig. 7, with the 1BX2 residue number in parenthesis. For example, F1 50.CE2 is equivalent to B:F24.CE2; atom CE2 of residue F24 on chain B of the heterodimeric 1BX2 crystal structure. Chain C is the bound antigenic peptide. | | |

The motif couples three anti-parallel beta-sheet strands to a central unstructured stretch of polypeptide between two alpha-helical segments of the α-1 domain. The structural motif is located within the α-1 domain and "caps" the α-1 domain side at the end of the peptide binding groove where the amino-terminus of the bound Ag-peptide emerges.

Thus, soluble single-chain RTL molecules have been constructed from the antigen-binding β1 and α1 domains of human MHC class II molecule DR2. The RTLs lack the α2 domain, the β2 domain known to bind to CD4, and the transmembrane and intra-cytoplasmic sequences. The reduced size of the RTLs gave us the ability to express and purify the molecules from bacterial inclusion bodies in high yield (15-30 mg/L cell culture). The RTLs refolded upon dialysis into PBS and had excellent solubility in aqueous buffers.

The data presented herein demonstrate clearly that the human DR2-derived RTL302 and RTL303 retain structural and conformational integrity consistent with crystallographic data regarding the native MHC class II structure. MHC class II molecules form a stable heterodimer that binds and presents antigenic peptides to the appropriate T-cell receptor (Fig. 12). While there is substantial structural and theoretical evidence to support this model (Brown *et al*., 1993; Murthy *et al*., 1997; Fremont *et al*., 1996; Ploegh *et al*., 1993; Schafer *et al*., 1995), the precise role that contextual information provided by the MHC class II molecule plays in antigen presentation, T-cell recognition and T-cell activation remains to be elucidated. The approach described herein used rational protein engineering to combine structural information from X-ray crystallographic data with recombinant DNA technology to design and produce single chain TCR ligands based on the natural MHC class II peptide binding/T-cell recognition domain. In the native molecule this domain is derived from portions of the alpha and beta polypeptide chains which fold together to form a tertiary structure, most simply described as a beta-sheet platform upon which two anti-parallel helical segments interact to form an antigen-binding groove. A similar structure is formed by a single exon encoding the α-1 and α-2 domains of MHC class I molecules, with the exception that the peptide-binding groove of MHC class II is open-ended, allowing the engineering of single-exon constructs that incorporate the peptide binding/T-cell recognition domain and an antigenic peptide ligand (Kozono *et al*., 1994).

From a drug engineering and design perspective, this prototypic molecule represents a major breakthrough. Development of the human RTL molecules described herein separates the peptide binding (1β1 domains from the platform 2β2 Ig-fold domains) allowing studies of their biochemical and biological properties independently, both from each other and from the vast network of information exchange that occurs at the cell surface interface between APC and T-cell during MHC/peptide engagement with the T-cell receptor. Development of human RTL molecules described herein allows careful evaluation of the specific role played by a natural TCR. ligand independent from the platform (2β2 Ig-fold domains of MHC class II).

When incubated with peptide specific Th1 cell clones in the absence of APC or costimulatory molecules, RTL303 initiated a subset of quantifiable signal transduction processes through the TCR. These included rapid ζ chain phosphorylation, calcium mobilization, and reduced ERK kinase activity, as well as IL-10 production. Addition of RTL303 alone did not induce proliferation. T-cell clones pretreated with cognate RTLs prior to restimulation with APC and peptide had a diminished capacity to proliferate and secrete IL-2, and secreted less IFN-γ (Importantly, IL-10 production persisted (see below)). These data elucidate for the first time the early signaling events induced by direct engagement of the external TCR interface, in the absence of signals supplied by co-activation molecules,

Modeling studies have highlighted a number of interesting features regarding the interface between the β1α1 and α2β2-Ig-fold domains. The α1 and β1 domains showed an extensive hydrogen-bonding network and a tightly packed and buried hydrophobic core. The RTL molecules, composed of the α1 and β1 domains may have the ability to move as a single entity independent from the α2β2-Ig-fold "platform." Flexibility at this interface may be required for freedom of movement within the α1 and β1 domains for binding/exchange of peptide antigen. Alternatively or in combination, this interaction surface may play a potential, role in communicating information about the MHC class II/peptide molecules interaction with TCRs back to the APC.

Critical analysis of the primary sequence of amino acid residues within two helical turns (7.2 residues) of the conserved cysteine 110 (RTL303 numbering) as well as analysis of the β-sheet platform around the conserved cysteine 46 (RTL303 numbering) reveal a number of interesting features of the molecule, the most significant being very high diversity along the peptide-binding groove face of the helix and β-sheet platform. Interestingly, the surface exposed face of the helix composed of residues L99, E100, R103, A104, D107, R111, and Y114 (Fig. 1) is conserved in all rat, human and mouse class II and may serve an as yet undefined function.

Cooperative processes are extremely common in biochemical systems. The reversible transformation between an alpha-helix and a random coil conformation is easily quantified by circular dicroism. Once a helix is started, additional turns form rapidly until the helix is complete. Likewise, once it begins to unfold it tends to unfold completely. A normalized plot of absorption of circularly polarized light at 222 nm versus temperature (melting curve) was used to define a critical melting temperature (Tₘ) for each RTL molecule. The melting temperature was defined as the midpoint of the decrease in structure loss calculated from the loss of absorption of polarized light at 222 nm. Because of their size and biochemical stability, RTLs will serve as a platform technology for development of protein drugs with engineered specificity for particular target cells and tissues.

### Example 10

### TCR Signaling

Development of a minimal TCR ligand allows study of TCR signaling in primary T-cells and T-cell clones in the absence of costimulatory interactions that complicate dissection of the information cascade initiated by MHC/peptide binding to the TCR α and β chains. A minimum "T-cell receptor ligand" conceptually consists of the surface of an MHC molecule that interacts with the TCR and the 3 to 5 amino acid residues within a peptide bound in the groove of the MHC molecule that are exposed to solvent, facing outward for interaction with the TCR. The biochemistry and biophysical characterization of Recombinant TCR Ligands (RTLs) derived from MHC class II are described above, such as the use of the α-1 and β-1 domains of HLA-DR2 as a single exon of approximately 200 amino acid residues with various amino-terminal extension containing antigenic peptides. These HLA-DR2-derived RTLs fold to form the peptide binding/T-cell recognition domain of the native MHC class II molecule.

Inflammatory Th1, CD4+ T-cells are activated in a multi-step process that is initiates by co-ligation of the TCR and CD4 with MHC/peptide complex present on APCs. This primary, antigen-specific signal needs to be presented in the proper context, which is provided by co-stimulation through interactions of additional T-cell surface molecules such as CD28 with their respective conjugate on APCs. Stimulation through the TCR in the absence of co-stimulation, rather than being a neutral event, can induce a range of cellular responses from full activation to anergy or cell death (Quill *et al*., 1984). As described herein Ag-specific RTLs were used induce a variety of human T-cell signal transduction processes as well as modulate effector functions, including cytokine profiles and proliferative potential.

### Recombinant TCR Ligands

Recombinant TCR Ligands were produced as described above.

### Synthetic peptides.

MBP85-99 peptide (ENPVVHFFKNIVTPR, SEQ ID NO:38) and "CABL", BCR-ABL b3a2 peptide (ATGFKQSSKALQRPVAS, SEQ ID NO:39) (ten Bosch *et al.,* 1995) were prepared on an Applied Biosystems 432A (Foster City, CA) peptide synthesizer using fmoc solid phase synthesis. The MBP peptide was numbered according to the bovine MBP sequence (Martenson, 1984). Peptides were prepared with carboxy terminal amide groups and cleaved using thianisole/1,2-ethanedithiol/dH ₂ O in trifluoroacetic acid (TFA) for 1.5 hours at room temperature with gentle shaking. Cleaved peptides were precipitated with 6 washes in 100% cold tert-butylmethyl ether, lyophilized, and stored at -70 °C under nitrogen. The purity of peptides was verified by reverse phase HPLC on an analytical Vydac C18 column.

### T-cell clones.

Peptide-specific T-cell clones were selected from peripheral blood mononuclear cells (PBMC) of a multiple sclerosis (MS) patient homozygous for HLA-DRB1*1501 and an MS patient homozygous for HLA-DRB1*07, as determined by standard serological methods and further confirmed by PCR amplification with sequence-specific primers (PCR-SSP) (Olerup *et al.,* 1992). Frequencies of T-cells specific for human MBP85-99 and CABL were determined by limiting dilution assay (LDA). PBMC were prepared by ficoll gradient centrifugation and cultured with 10 µg/ml of either MBP85-99 or CABL peptide at 50,000 PBMC/well of a 96-well U-bottomed plate plus 150,000 irradiated (2500 rad) PBMC/well as antigen-presenting cells (APCs) in 0.2 ml medium (RPMI 1640 with 1% human pooled AB serum, 2 mM L-glutamine, 1 mM sodium pyruvate, 100 unit/ml penicillin G, and 100 µg/ml streptomycin) for 5 days, followed by adding 5 ng/ml IL-2 (R & D Systems, Minneapolis, MN) twice per week. After three weeks, the culture plates were examined for cellular aggregation or "clump formation" by visual microscopy and the cells from the "best" 20-30 clump-forming wells among a total of 200 wells per each peptide Ag were expanded in 5 ng/ml IL-2 for another 1-2 weeks. These cells were evaluated for peptide specificity by the proliferation assay, in which 50,000 T-cells/well (washed 3x) were incubated in triplicate with 150,000 freshly isolated and irradiated APC/well plus either medium alone, 10 mg/ml MBP85-99 or 10 mg/ml CABL pep- tide for three days, with ³H-Tdy added for the last 18 hours. Stimulation index (S.I.) was calculated by dividing the mean CPM of peptide-added wells by the mean CPM of the medium alone control wells. T-cell isolates with the highest S.I. for a particular peptide antigen were selected and expanded in medium containing 5 ng/ml IL-2, with survival of 1-6 months, depending on the clone, without further stimulation.

### Sub-cloning and expansion of T-cell number.

Selected peptide-specific T-cell isolates were sub-cloned by limiting dilution at 0.5 T-cells/well plus 100,000 APC/well in 0.2 ml medium containing 10 ng/ml anti-CD3 (Pharmingen, San Diego, CA) for three days, followed by addition of 5 ng/ ml IL-2 twice per week for 1-3 weeks. All wells with growing T-cells were screened for peptide-specific response by the proliferation assay and the well with the highest S.I. was selected and continuously cultured in medium plus IL-2. The clonality of cells was determined by RT-PCR, with a clone defined as a T-cell population utilizing a single TCR V P gene. T-cell clones were expanded by stimulation with 10 ng/ml anti-CD3 in the presence of 5x10⁶ irradiated (4500 rad) EBV-transformed B cell lines and 25 x 10⁶ irradiated (2500 rad) autologous APC per 25 cm² flask in 10% AB pooled serum (Bio-Whittaker, MD) for 5 days, followed by washing and resuspending the cells in medium containing 5 ng/ml IL-2, with fresh IL-2 additions twice/week. Expanded T-cells were evaluated for peptide-specific proliferation and the selected, expanded T-cell clone with the highest proliferation S.I. used for experimental procedures.

### Cytokine detection by ELISA.

Cell culture supernatants were recovered at 72 hours and frozen at -80°C until use. Cytokine measurement was performed by ELISA as previously described (Bebo *et al*., 1999) using cytokine specific capture and detection antibodies for IL-2, IFN-γ, IL-4 and IL-10 (Pharmingen, San Diego, CA). Standard curves for each assay were generated using recombinant cytokines (Pharmingen), and the cytokine concentration in the cell supernatants was determined by interpolation.

### Flow cytometry.

Two color immunofluorescent analysis was performed on a FACScan instrument (Becton Dickinson, Mountain View, CA) using CellQuest TM software. Quadrants were defined using isotype matched control Abs.

### Phosphotyrosine assay.

T-cells were harvested from culture by centrifuging at 400 x g for 10 min, washed, and resuspended in fresh RPMI. Cells were treated with RTLs at 20 µM final concentration for various amounts of time at 37°C. Treatment was stopped by addition of ice-cold RPMI, and cells collected by centrifugation. The supernatant was decanted and lysis buffer (50 mM Tris pH 7.5, 150 mM NaCl, 1% NP-40, 0.5% deoxycholate, 0.1% SDS, 1 mM AEBSF [4-(2-aminoethyl) henzenesulfonylfluoricle,HCl], 0.8 µM aprotinin, 50 µM bestatin, 20 µM leupeptin, 10 µM pepstatin A, 1 mM activated sodium orthovanadate, 50 mM NaF, 0.25 mM bpV [potassium bisperoxo(1,10-phenanthroline) oxovanadate], 50 µM phenylarsine oxide) was added immediately. After mixing at 4°C for 15 min to dissolve the cells, the samples were centrifuged for 15 min. The cell lysate was collected and mixed with an equal volume of sample loading buffer, boiled for 5 min and then separated by 15% SDS-PAGE. Protein was transferred to PVDF membrane for western blot analysis. Western blot block buffer: 10mM Tris-HCl (pH 7.5), 100 mM NaCl, 0.1% Tween-20, 1% BSA. Primary antibody: anti-phosphotyrosine, clone 4G10, (Upstate Biotechnology, Lake Placid, NY). Secondary and tertiary antibody from ECF Western blot kit (Amersham, Picataway, NJ). The dried blot was scanned using a Storm 840 scanner (Molecular Dynamics, Sunnyvale, CA) and chemifluorescence quantified using ImageQuant version 5.01 (Molecular Dynamics).

### ERK Activation Assay.

T-cells were harvested and treated with RTLs as for ζ phosphotyrosine assay. Western blot analysis was performed using anti-phosph-ERK (Promega, Madison WI) at 1:5000 dilution or anti-ERK kinase (New England Biolabs, Beverly, MA) at 1: 1500 dilution and visualized using ECF Western Blotting Kit. Bands of interest were quantified as described for ζ phosphotyrosine assay.

### Ca2⁺ imaging.

Human T-cells were plated on polylysine-coated 35 mm glass bottom dishes and cultured for 12-24 hr in medium containing IL-2. Fura-2 AM (5 mM) (Molecular Probes) dissolved in the culture medium was loaded on the cells for 30 min. in CO2 incubator. After rinse of fura-2 and Additional 15 min. incubation in the culture medium, the cells were used for calcium measurement. Fluorescent images were observed by an upright microscope (Axioskop FS, Zeiss) with a water immersion objective (UmplanFL 60x/0.8, Olympus). Two wavelengths of the excitation UV light (340 nm or 380 nm) switched by a monochromator (Polychrome 2, Till Photonics) were exposed for 73 msec at 6 seconds interval. The intensity of 380 nm UV light was attenuated by a balancing filter (UG11, OMEGA Optical). The excitation UV light was reflected by a dichroic mirror (FT 395 nm, Carl Zeiss) and the fluorescent image was band-passed (BP500-530, Carl Zeiss), amplified by an image intensifier (C7039-02, Hamamatsu Photonics) and exposed to multiple format cooled CCD camera (C4880, Hamamatsu Photonics). The UV light exposure, CCD control, image sampling and acquisition were done with a digital imaging system (ARGUS HiSCA, Hamamatsu Photonics). The background fluorescence was subtracted by the imaging sys-tem. During the recording, cells were kept in a culture medium maintained at 30°C by a stage heater (DTC-200, Dia Medical). The volume and timing of drug application were regulated by a trigger-driven superfusion system (DAD-12, ALA Scientific instruments).

### Example 11

### The Effect of Human RTLs on Human T-cell Clones

Two different MHC class II DR2-derived RTLs (HLA-DR2b: DRA*0101, DRB1*1501) were used in this study (Fig. 19). RTL303 (β1α1/MBP85-99) and RTL311 (β1α1/CABL) differ only in the antigen genetically encoded at the amino terminal of the single exon RTL. The MBP85-99 peptide represents the immuno-dominant MBP determinant in DR2 patients (Martin *et al*., 1992) and the C-ABL peptide (ten Bosch *et al*., 1995) contains the appropriate motif for binding DR2. The human T-cell clones used in this study were selected from a DR2 homozygous patient and a DR7 homozygous MS patient

Structure-based homology modeling was performed using the refined crystallographic coordinates of human DR2 (Smith *et al.,* 1998) as well as DR1 (Brown *et al*., 1993; Murky *et al*., 1997), murine I-E k molecules (Fremont *et al*., 1996), and scorpion toxins (Zhao *et al*., 1992). Because a number of amino acid residues in human DR2 (PDB accession number 1BX2) were missing/not located in the crystallographic data (Smith *et al.,* 1998), the correct side chains based on the Sequence of DR2 were substituted in the sequence and the peptide backbone was modeled as a rigid body during structural refinement using local energy minimization. These relatively small (approx. 200 amino acid residues) RTLs were produced in *Escherichia coli* in large quantities and refolded from inclusion bodies, with a final yield of purified protein between 15-30 mg/L of bacterial culture (Chang *et al*., 2001). Fig. 19 is a schematic scale model of an MHC class II molecule on the surface of an APC (Fig. 19A). The HLA-DR2 β1α1-derived RTL303 molecule containing covalently coupled MBP-85-99 peptide (Fig. 19B, left) and the HLA-DR2 β1α1-derived RTL311 molecule containing covalently coupled CABL peptide (Fig. 19C, left), are shown in Fig. 19A with the primary TCR contact residues labeled. The P2 His, P3 Phe, and P5 Lys residues derived from the MBP peptide are prominent, solvent exposed residues. These residues arc known to be important for TCR recognition of the MBP peptide. The corresponding residues in the C-ABL peptide (P2 Thr, P3 Gly, P5 Lys) are also shown. Immediately striking is the percentage of surface area that is homologous across species. When shaded according to electrostatic potential (EP) (Connolly, 1983) (Fig. 19B, 19C, middle), or according to lipophilic potential (LP) (Heiden *et al.,* 1993) (Fig. 19B, 19C, right), subtleties between the molecules are resolved that likely play a specific role in allowing TCR recognition of antigen in the context of the DR2-derived RTL surface.

The design of the constructs allows for substitution of sequences encoding different antigenic peptides using restriction enzyme digestion and ligation of the constructs. Structural characterization using circular dichroism demonstrated that these molecules retained the anti-parallel beta-sheet platform and antiparallel alpha-helices observed in the native class II heterodimer, and the molecules exhibited a cooperative two-state thermal unfolding transition (Chang *et al*., 2001). The RTLs with the covalently-linked Ag-peptide showed increased stability to thermal unfolding relative to "empty" RTLs, similar to what was observed for rat RT1.B RTLs.

DR2 and DR7 homozygous donor-derived Ag-specific T-cell clones expressing a single TCR BV gene were used to evaluate the ability of Ag-specific RTLs to directly modify the behavior of T-cells. Clonality was verified by TCR BV gene expression, and each of the clones proliferated only when stimulated by specific peptide presented by autologous APC. DR2 homozygous T-cell clone MR#3-1 was specific for the MBP85-99 peptide and DR2 homozygous clone MR#2-87 was specific for the CABL peptide. The DR7 homozygous T-cell clone CP#1-15 was specific for the MBP85-99 peptide (Fig. 20).

### Example 12

### RTL Treatment Induced Early Signal Transduction Events

Phosphorylation of the ζ chain in the DR2 homozygous T-cell clones MR#3-1 and MR#2-87 was examined. MR#3-1 is specific for the MBP85-99 peptide carried by RTL303, and MR#2-87 is specific for the CABL peptide carried by RTL311. The antigenic peptides on the amino terminal end of the RTLs are the only difference between the two molecules. The TCR-ζ chain is constitutively phosphorylated in resting T-cells, and changes in levels of ζ chain phosphorylation are one of the earliest indicators of information processing through the TCR. In resting clones, ζ was phosphorylated as a pair of phospho-protein species of 21 and 23 kD, termed p21 and p23, respectively. Treatment of clone MR#3-1 with 20 µM RTL303 showed a distinct change in the p23/p21 ratio that reached a minimum at 10 minutes (Fig. 21). This same distinct change in the p23/p2l ratio was observed for clone MR#2-87 when treated with 20 µM RTL311 (Fig. 21). Only RTLs containing the peptide for which the clones were specific induced this type of ζ- phosphorylation, previously observed after T-cell activation by antagonist ligands(27,28).

Calcium levels were monitored in the DR2 homozygous T-cell clone MR#3-1 specific for the MBP85-99 peptide using single cell analysis. While there is a general agreement that calcium mobilization is a specific consequence of T-cell activation, the pattern of response and dosage required for full activation remain controversial (Wülfing *et al*., 1997). It appears that four general patterns of intra-cellular calcium mobilization occur with only the most robust correlating with full T-cell proliferation. RTL303 treatment induced a sustained high calcium signal, whereas RTL301 (identical to RTL303 except a single point mutation that altered folding properties, F150L) showed no increase in calcium signal over the same time period (Fig. 22).

RTL effects were further evaluated on levels of the extracellular regulated protein kinase ERK, a key component within the Ras signaling pathway known to be involved in the control of T-cell growth and differentiation (Li *et al*., 1996). The activated form of ERK kinase is itself phosphorylated (Schaeffer *et al*.,1999), and thus a straightforward measure of ERK activity was to compare the fraction of ERK that is phosphorylated (ERK-P) relative to the total cellular ERK present (T-ERK). Within 15 min. after treatment with RTLs, the level of ERK-P was drastically reduced in an Ag-specific fashion. 20 µM RTL303 reduced ERK-P by 80% in clone #3-1 and 20 µM RTL311 reduced ERK-P by 90% in clone #2-87 (Fig. 23).

The early signal transduction events that were altered by Ag-specific RTL treatment on the cognate T-cell clones led us to investigate the effect of RTL treatment on cell surface markers, proliferation and cytokines. Cell surface expression levels of CD25, CD69 and CD134 (OX40) were analyzed by multicolor flow cytometry at 24 and 48 hr after treatment with RTLs and compared to APC/peptide or Con A stimulated cells. CD69 (Vilanova *et al*., 1996) was already very high (~80% positive) in these clones. APC/peptide induced Ag-spceific increases in both CD25 (Kyle *et al*., 1989) and CD 134 (Weinberg *et al*., 1996) that peaked between 48 and 72 hours, while RTL treatment had no effect on these cell surface markers. RTL treatment induced only subtle increases in apoptotic changes as quantified using Annexin V staining and these were not Ag-specific, Treatment of T-cell clones with RTLs did not induce proliferation when added in solution, immobilized onto plastic microtiter plates, nor in combination with the addition of anti-CD28.

Upon activation with APC plus Ag, clone MR#3-1 (MBP85-99 specific) and MR#2-87 (CABL specific) showed classic Th1 cytokine profiles that included IL-2 production, high IFN-γ and little or no detectable IL-4 or IL-10. As is shown in Fig 24A, activation through the CD3- chain with anti-CD3 antibody induced an initial burst of strong proliferation and production of IL-2, IFN-γ, and surprisingly, IL-4, but no IL-10. In contrast, upon treatment with RTL303, clone MR#3-1 continued production of IFN-γ, but in addition dramatically increased its production of IL-10 (Fig. 24A). IL-10 appeared within 24 hours after addition of RTL303 and its production continued for more than 72 hours, to three orders of magnitude above the untreated or RTL311 treated control. In contrast, IL-2 and IL-4 levels did not show RTL induced changes (Fig. 24A). Similarly, after treatment with RTL311, Clone MR#2-87 (CABL specific) also showed a dramatic increase in production of IL-10 within 24 hours that continued tor greater than 72 hours above the untreated or RTL303 treated control (Fig. 24B). Again, IL-2 and levels did not show detectable RTL induced changes, and IFN-γ production remained relatively constant (Fig. 6B). The switch to IL-10 production was exquisitely Ag-specific, with the clones responding only to the cognate RTL carrying peptide antigen for which the clones were specific. The DR7 homozygous T-cell clone CP#1-15 specific for MBP-85-99 showed no response to DR2-derived RTLs, indicating that RTL induction of IL-10 was also MHC restricted.

To assess the effects of RTL pre-treatment on subsequent response to antigen, T-cell clones pretreated with anti-CD3 or RTLs were restimulated with APC/peptide, and cell surface markers, proliferation and cytokine production were monitored. RTL pre-treatment had no effect on the cell surface expression levels of CD25, CD69 or CD134 (OX40) induced by restimulation with APC/peptide compared to T-cells stimulated with APC/peptide that had never seen RTLs, and there were no apoptotic changes observed over a 72 hour period using Annexin V staining.

Anti-CD3 pretreated T-cells were strongly inhibited, exhibiting a 71 % decrease in proliferation and >95% inhibition of cytokine production, with continued IL-2R (CD25) expression (Table 5; Fig. 25), a pattern consistent with classical anergy (Elder *et al*., 1994).

**Table 5. Ag-specific inhibition of T-cell clones by pre-culturing with RTLs.**

| **Donor 1** | | | | | |
|---|---|---|---|---|---|
| **Clone #3-1** | | **Pre-Cultured with RTL303*** | | **Pre-Cultured with RTL311** | |
| | Untreated | 20 µM | 10 µM | 20 µM | 10 µM |
| +APC** | 439 ± 221 | 549 ± 70 | 406 ± 72 | 491 ± 50 | 531 ± 124 |
| +APC+MBP-85-99 (10 µg/ml) | 31725 ± 592 | 18608 ± 127 | 29945 ± 98 | 35172 ± 41 | 32378 ± 505 |
| **inhibition (%)** | - | **-42.3 (p<0.0.1)** | **-5.6** | **0** | **0** |
| | | | | | |
| **Clone #2-87** | | | | | |
| +APC | 1166 ± 24 | 554 ± 188 | 1229 ± 210 | 1464 ± 281 | 1556 ± 196 |
| +APC+C-ABL,-b2a3 (10 µg/ml) | 11269 ± 146 | 11005 ± 204 | 14298 ± 1669 | 5800 ± 174 | 7927 ± 575 |
| **Inhibition (%)** | - | **0** | **0** | **-57.0 (p<0.001)** | **-36.9 (p<0.01)** |
| | | | | | |
| **Donor 2 Clone #1-15** | | | | | |
| I-APC | 258 ±± 48 | 124 ± 7 | ND | 328 ± 56 | ND |
| +APC+MBP-85-99 (10)-µg/ml) | 7840 ± 1258 | 7299 ± 1074 | ND | 8095 ± 875 | ND |
| **Inhibition (%)** | - | **-5.1** | | **0** | |

| | | | | | |
|---|---|---|---|---|---|
| *Soluble RTL303 or RTL311 were co-cultured with T-cell clones at 200,000 T-cells/200 µl medium for 48 hours followed by washing twice with RPMI 1640 prior to the assay. **2 x 10⁵ irradiated (2500 rad) autologous PBMC were added at ratio 4:1 (APC:T) for 3 days with ³H-Thymidine incorporation for the last 18 hr. The p values were based on comparison to "untreated" control. | | | | | |

Clone MR#3-1 showed a 42% inhibition of proliferation when pretreated with 20 µM RTL303, and clone MR#2-87 showed a 57% inhibition of proliferation when pretreated with 20 µM RTL311 (Table 5; Fig. 25). Inhibition of proliferation was also MHC class II-specific, as clone CP#1-15 (HLA-DR7 homozygous donor; MBP85-99 specific) showed little change in proliferation after pre-treatment with RTL303 or RTL311. Clone MR#3-1 pretreated with RTL303 followed by restimulation with APC/Ag showed a 25% reduction in IL-2, a 23% reduction in IFN-γ and no significant changes in IL-4 production (Fig. 25). Similarly, clone MR#2-87 showed a 33% reduction in IL-2, a 62% reduction in IFN-γ production, and no significant change in IL-4 production. Of critical importance, however, both RTL-pretreated T-cell clones continued to produce IL-10 upon restimulation with APC/peptide (Fig. 25).

The results presented above demonstrate clearly that the rudimentary TCR ligand embodied in the RTLs delivered signals to Th1 cells and support the hypothesis of specific engagement of RTLs with the αβ-TCR signaling. Signals delivered by RTLs have very different physiological consequences than those that occur following anti-CD3 antibody treatment.

In the system described herein, anti-CD3 induced strong initial proliferation and secretion of IL-2, IFN-γ, and IL-4 (Fig. 24). Anti-CD3 pre-treated T-cells that were restimulated with APC/antigen had markedly reduced levels of proliferation and cytokine secretion, including IL-2, but retained expression of IL-2R, thus recapitulating the classical energy pathway (Fig. 25). In contrast, direct treatment with RTLs did not induce proliferation, Th1 cytokine responses, or IL-2R expression, but did strongly induce IL-10 secretion (Fig. 24). RTL pretreatment partially reduced. proliferation responses and Th1 cytokine secretion, but did not inhibit IL-2R expression upon restimulation of the T-cells with APC/antigen. Importantly, these T-cells continued. to secrete IL-10 (Fig. 25). Thus, it is apparent that the focused activation of T-cells through antibody crosslinking of the CD3-chain had vastly different consequences than activation by RTLs presumably through the exposed TCR surface. It is probable that interaction of the TCR with MHC/antigen involves more elements and a more complex set of signals than activation by crosslinking CD3-chains, and the results described herein indicate that signal transduction induced by anti-CD3 antibody may not accurately portray ligand-induced activation through the TCR. Thus, CD3 activation alone likely does not comprise a normal physiological pathway.

The signal transduction cascade downstream from the TCR is very complex. Unlike receptor tyrosine kinases, the cytoplasmic portion of the TCR lacks intrinsic catalytic activity. Instead, the induction of tyrosine phosphorylation following engagement of the TCR requires the expression of non-receptor kinases. Both the Src (Lck and Fyn) family and the Syk/ZAP-70 family oftyrosine kinases are required for normal TCR signal transduction (Elder *et al.,* 1994). The transmembrane CD4 co-receptor interacts with the MHC class II β-2 domain. This domain has been engineered out of the RTLs. The cytoplasmic domain of CD4 interacts strongly with the cytoplasmic tyrosine kinase Lck, which enables the CD4 molecule to participate in signal transduction. Lck contains an SH3 domain which is able to mediate protein-protein interactions (Ren *et al*., 1993) and which has been proposed to stabilize the formation of Lck homodimers, potentiating TCR signaling following co-ligation of the TCR and co-receptor CD4 (Eck *et al*., 1994). Previous work indicated that deletion of the Lck SH3 domain interfered with the ability of an oncogenic form of Lck to enhance IL-2 production, supporting a role for Lck in regulating cytokine gene transcription (Van Oers *et al*., 1996; Karnitz *et al*., 1992). T-cells lacking functional Lck fail to induce Zap-70 recruitment and activation, which has been implicated in down-stream signaling events involving the MAP kinases ERK1 and ERK2 (Mege *et al*., 1996).

While the complete molecular signal transduction circuitry remains undefined, RTLs induce rapid antagonistic effects on ζ-chain and ERK kinase activation. The intensity of the p21 and p23 forms of ζ increased together in a non peptide-Ag specific fashion (Fig. 21A), while the ratio of p23 to p21 varied in a peptide-Ag specific manner (Fig. 21B), due to a biased decrease in the level of the p23 moiety. The antagonistic effect on ERK phosphorylation also varied in a peptide-Ag specific manner (Fig. 21A). RTL treatment also induced marked calcium mobilization (Fig. 22). The fact that all three of these pathways were affected in an antigen specific fashion strongly implies that the RTLs are causing these effects through direct interaction with the TCR.

The results described herein demonstrate the antigen-specific induction by RTLs of IL-10 secretion. This result was unexpected, given the lack of IL-10 production by the Thl clones when stimulated by APC/antigen or by anti-CD3 antibody. Moreover, the continued secretion of IL-10 upon restimulation of the RTL pre-treated clones with APC/antigen indicates that this pathway was not substantially attenuated during reactivation. This result suggests that TCR interaction with the RTL results in default IL-10 production that persists even upon re-exposure to specific antigen. The elevated level of IL-10 induced in Th1 cells by RTLs has important regulatory implications for autoimmune diseases such as multiple sclerosis because of the known anti-inflammatory effects of this cytokine on Th1 cell and macrophage activation (Negulescu *et al*., 1996).

It is likely that the pathogenesis of MS involves autoreactive Th1 cells directed at one or more immunodominant myelin peptides, including MBP-85-99. RTLs such as RTL303 could induce IL-10 production by these T-cells, thus neutralizing their pathogenic potential. Moreover, local production of IL-10 after Ag-stimulation in the CNS could result in the inhibition of activation of bystander T-cells that may be of the same or different Ag specificity, as well as macrophages that participate in demyelination. Thus, this important new finding implies a regulatory potential that extends beyond the RTL-ligated neuroantigen specific T-cell. RTL induction of IL-10 in specific T-cell populations that recognize CNS antigens could potentially be used to regulate the immune system while preserving the T-cell repertoire, and may represent a novel strategy for therapeutic intervention of complex T-cell mediated autoimmune diseases such as MS.

### Example 13

### Vaccination Induced Bystander Suppression for the Treatment of Autoimmune Disease

The pathogenesis of a variety of human diseases including allergies, graft rejection, transplant rejection, graft versus host disease, an unwanted delayed-type hypersensitivity reaction, T-cell mediated pulmonary disease, insulin dependent diabetes mellitus (IDDM), systemic lupus erythematosus (SLE), rheumatoid arthritis, coeliac disease, multiple sclerosis, neuritis, polymyositis, psoriasis, vitiligo, Sjogren's syndrome, rheumatoid arthritis, autoimmune pancreatitis, inflammatory bowel diseases, Crohn's disease, ulcerative colitis, active chronic hepatitis, glomerulonephritis, scleroderma, sarcoidosis, autoimmune thyroid diseases, Hashimoto's thyroiditis, Graves disease, myasthenia gravis, asthma, Addison's disease, autoimmune uveoretinitis, pemphigus vulgaris, primary biliary cirrhosis, pernicious anemia, sympathetic opthalmia, uveitis, autoimmune hemolytic anemia, pulmonary fibrosis, chronic beryllium disease and idiopathic pulmonary fibrosis appear to involve antigen-specific CD4+ T-cells.

It is thought that pathogenic T-cells home to the target tissue where autoantigen is present, and, after local activation, selectively produce Th1 lymphokines. This cascade of events leads to the recruitment and activation of lymphocytes and monocytcs that ultimately destroy the target tissue. Activation of CD4+ T-cells *in vivo* is a multi-step process initiated by co-ligation of the TCR and CD4 by the MHC class II/peptide complex present on APC (signal I), as well as co-stimulation through additional T-cell surface molecules such as CD28 (signal 2). Ligation of the TCR in the absence of co-stimulatory signals has been shown to disrupt normal T-cell activation, inducing a range of responses from anergy to apoptosis. Within the context of this model of T-cell activation, a direct approach toward Ag-driven immunosuppression would be to present the complete TCR ligand, Ag in the context of MHC, in the absence of costimulatory signals that are normally provided by specialized APCs.

Bystander suppression is the effect produced by regulatory cells, in most cases T-cells, responding to antigen expressed by a particular tissue that is proximal to autoantigens. The regulatory cells then produce a microenvironment, most likely through the production of cytokines (e.g. TGF-β, IL-10 or IL-13) which suppress the response of the autoimmune cells. The ability to induce bystander T regulatory cells by vaccination has promising potential for an immune based autoimmune therapy, as the difficult task of determining disease specific autoantigens is no longer necessary. Vaccine strategies designed to induce these antigen-specific regulatory cells only need to express antigens specific to the tissue undergoing autoimmune attack. Therefore, in diseases where the autoantigen is unknown or where there may be multiple antigens (for example, multiple sclerosis (MS), type 1 diabetes, or rheumatoid arthritis) vaccination only needs to be directed to antigens particular to those tissues in conjunction with MHC. Thus, for MS, vaccination is, for example, directed to myelin basic protein (see above), for diabetes, vaccination is, for example, directed to insulin, and for rheumatoid arthritis, vaccination is, for example, directed to Type II collagen respectively.

There are several animal based autoimmune models that can be used to test the use of MHC/peptide complex for the treatment of an autoimmune disorder including, but not limited to, those in Table 6. For example, the non-obese diabetic (NOD) mouse model is an animal model system wherein animals develop diabetes with increasing age. To test the efficacy of a particular antigen/MHC complex, groups of animals at the prediabetic stage (4 weeks or younger) are vaccinated with, for example, insulin-MHC complex. The number of animals developing diabetes, and the rate that the animals develop diabetes, is then analyzed. Similarly, in the Hashimoto's mouse model system, to test the efficacy of a vaccine, groups of animals prior to the development of symptoms are vaccinated with a thyrodoxin/MHC complex. The number of animals developing the disease, and the rate that the animals develop the disease, is then analyzed.

**Table 6**

| **Examples of Human Autoimmune Disorders** | | |
|---|---|---|
| **Human Disease** | **Animal Model** | **Antigen of Use** |
| Multiple Sclerosis | Experimental autoimmune encephalitis (EAE) mouse model and Lewis rat | Myelin basic protein (MBP) proteolipid protein (PLP) and myelin oligodedrocyte glycoprotein |
| Diabetes | NOD mice | Insulin, glutamate decarboxylase |
| Arthritis and related MCTD (mixed connective tissue disease) | Chicken, Mice and Rats | Type II collagen |
| Hashimoto's Thyroiditis, Grave's Disease Uveitus | Mice, Lewis Rats, and OS chickens Mice | Thyroglobulin, Thyrodoxin S-antigen |
| Inflammatory Bowel Disease | MDr1a Knockout Mice | Ach (acetylcholine) Receptor |
| Polyarteritis | Mice | HepB Antigen |
| Myasthenia Gravis | Mice | |
| Transplantation rejection | Mice Islet cell transplantation | Insulin, glutamate decarboxylase |
| Coeliac Disease | mice expressing a transgenic T-cell receptor that recognizes hen egg-white lysozyme peptide 46-61 | Cyclooxegenase-2 inhibitor, dietary hen egg white lysozome |
| Neuritis | Experimental autoimmne neuritis(EAN) in Lewis Rats | Pertussis toxin |
| Polymyositis | Guinea Pigs, Mice | Myosin B of Rabbit shredded muscle, Ross River virus (RRV) |
| Sjogren's syndrome | NOD mice, MRL/lpr mice | |
| Crohn's disease | SAMP1/Yit mice | |
| Ulcerative colitis | Galphai2(-/-) mice | |
| Glomeralonephritis | Rats | Anti-Gbm serum |
| Autoimmune thyroid disease | Mice | recombinant murine TPO (rmTPO) ectodomain |
| Addison's disease | Mice | syngeneic adrenal extract mixed with Klebsiella O3 lipopolysaccharide (KO3 LPS) |
| Autoimmune uveoretinitis | Experimental Autoimmune Uveoretinitis (EAU) Lewis rats | Retinal extract |
| Autoimmune pancreatitis | MRL/Mp-/+(MRL/+) mice | Polyinosinic:polycylidylic acid (poly I:C) |
| Primary biliary cirrhosis | C57/BL mice | Lipopolysaccharide (LPS) derived from Salmonella minnesota Re595 |
| Autoimmune Gastritis (Pernicious anemia) | C311/He mice; BALB/c mice | gastric II/K-ATPase. lymphoid irradiation |
| Hemolytic anemia | CD47-deficient nonobese diabetic (NOD) | |

In the NOD model or in the Hashimoto's model, the antigen/MHC complex delays the progression of the disease, or provides protection from developing the disease, when compared to animals primed with a nucleic acid encoding an unrelated antigen or as compared to untreated controls. The immune cell type that provides this protection is then studied by adoptive transfer studies to untreated mice (e.g., in NOD mice the transplantation of specific populations of immune cells, such as CD4, CD8, NK or B cells, into untreated NOD animals). Thus the cell population responsible for the regulation of the inflammatory response is determined.

For the adoptive transfer experiments, groups of Balb/c are given either peptide/MHC complex or a nucleic acid encoding the peptide/MHC complex. CD4+, CD8+, B220 and NK1.1+ cells are isolated by immunomagnetic bead separation. These different cell types are then transferred to naive NOD mice by IV injection. These animals receiving the transferred cells are then observed form signs of disease onset. Animals receiving peptide/MHC complex exhibit a delayed onset or no disease progression compared to controls.

### Example 14

### Monomeric RTLs Reduce Relapse Rate and Severity of Experimental Autoimmune Encephalomyelitis through Cytokine Switching

As described herein above, oligomeric recombinantTCR ligands (RTLs) are useful for treating clinical signs of experimental autoimmune encephalomyelitis (EAE) and inducing long-term T-cell tolerance against encephalitogenic peptides. In the present example, monomeric I-A^{s}/PLP 139-151 peptide constructs (RTL401) are produced and demonstrated to be useful for alleviating autoimmune responses in SJL/J mice that develop relapsing EAE after injection of PLP 139-151 peptide in CFA. RTL401 given. i.v. or s.c., but not empty RTL400 or free PLP 139-151 peptide, prevented relapses and significantly reduced clinical severity of EAE induced by PLP 139-151 peptide in SJL/J or (C57BL/6 x SJL)F₁ mice, but did not inhibit EAE induced by PLP 178-191 or MBP 84-104 peptides in SJL/J mice, or MOG 35-55 peptide in (C57BL/6 x SJL/J)F₁ mice. RTL treatment of EAE caused stable or enhanced T-cell proliferation and secretion of IL-10 in the periphery, but reduced secretion of inflammatory cytokines and chemokines. In the central nervous system (CNS), there was a modest reduction of inflammatory cells, reduced expression of very late activation Ag-4, lymphocyte function-associated Ag-1, and inflammatory cytokines, chemokines, and chemokine receptors, but enhanced expression of Th2-related factors, IL-10, TGF-β3, and CCR3. These results indicate that monomeric RTL therapy induces a cytokine switch that curbs the encephalitogenic potential ofPLP 139-151-specific T-cells without fully preventing their entry into CNS, wherein they reduce the severity of inflammation. This mechanism differs from that observed using oligomeric RTL therapy in other EAE models. These results indicate clinical utility of this novel class of peptide/MHC class II constructs in patients with multiple sclerosis who have focused T-cell responses to known encephalitogenic myelin peptides.

As noted above, RTLs designed for modulating of T-cell activity will typically include only the minimal TCR interface, which involves the α1 and β1 MHC domains covalently linked to peptide without CD4 binding. These constructs signal directly through the TCR as a partial agonist (Wang *et al*., 2003), prevented and treated MBP-induced monophasic EAE in Lewis rats (Burrows *et al.,* 1998; Burrows *et al.,* 2000), inhibited activation but induced IL-10 secretion in human DR2-restricted T-cell clones specific for MBP 85-99 or cABL peptides (Burrows *et al*., 2001; Change *et al*., 2001), and reversed chronic clinical and histological EAE induced by MOG 35-55 peptide in DR2 transgenic mice (Vandenbark *et al*., 2003). To further evaluate the therapeutic properties of recombinant TCR ligands (RTLs), an RTL was designed and tested for use in SJL mice that develop a relapsing form of EAE after injection with PLP 139-151 peptide in CFA. This RTL, comprised of an I-A^{s}/PLP 139-151 peptide construct (RTL401), prevented relapses and reversed clinical and histological EAE through a mechanism involving cytokine switching that differs strikingly from our previous studies using rat and human RTLs in other models of EAE.

### Mice

SJL and (C57BL/6 x SJL)F₁ mice were obtained from Jackson Immunoresearch Laboratories (Bar Harbor, ME) at 6-7 wk of age. The mice were housed in the Animal Resource Facility at the Portland Veterans Affairs Medical Center (Portland, OR) in accordance with institutional guidelines.

### Antigens

Mouse PLP 139-151 (HSLGKWLGHPDKF (SEQ ID NO:40)), PLP 178-191 (NTWTTCQSIAFPSK (SEQ ID NO:41)), MOG 35-55 (MEVGWYRSPFSRVVHLYRNGK (SEQ ID NO:42)), and MBP 84-104 (VHFFKNFVTPRTPPPSQGKGR (SEQ ID NO:43)) peptides were synthesized using solid phase techniques and purified by HPLC at Beckman Institute, Stanford University (Palo Alto, CA).

### RTL construction and production

General methods for the design, cloning, and expression of RTLs have been described herein above and elsewhere (see, e.g., Burrows *et al*., 1998; Burrows et *al.,* 1999; Chang *et al*., 2001). In brief, mRNA was isolated from the splenocytes of SJL mice using an Oligotex Direct mRNA mini-kit (Qiagen, Valencia, CA). cDNA of the Ag binding/TCP recognition domain of murine I-A^{s} MHC class II β1 and α1 chains was derived from mRNA using two pairs of PCR primers. The two chains were sequentially linked by a 5-aa linker (GGQDD (SEQ ID NO:44)) in a two-step PCR with *Nco*I and *Xho*I restriction sites added to the amino terminus of the β1 chain and to the carboxyl terminus of the α1 chain, respectively, to create RTL400. The PLP 139-151 peptide with a linker (GGGGSLVPRGSGGGG (SEQ ID NO:45)) was covalently linked to the 5' end of the β1 domain of RTL400 to form RTL401. The murine I-A^{s} β1α1 insert was then ligated into pET21d(+) vector and transformed into Nova blue *Escherichia coli* host (Novagen Inc., Madison, WI) for positive colony selection and sequence verification. RTL400 and RTL401 plasmid constructs were then transformed into *E. coli* strain BL21 (DE3) expression host (Novagen Inc.). The purification of proteins has been described previously (Chang *et al.,* 2001). The final yield of protein varied between 15 and 30 mg/L bacterial culture.

### Dynamic light scattering (DLS) analysis

Light scattering experiments were conducted in a DynaPro molecular sizing instrument (Protein Solutions, Charlottesville, VA). The protein samples, in 20 mM Tris-Cl buffer at pH 8.5, were filtered through 100 nm Anodise membrane filters (Whatman, Clifton, NJ) at a concentration of 1.0 mg/ml, and 20 µl of filtered sample were loaded into a quartz cuvette and analyzed with a 488-nm laser beam. Fifty spectra were collected at 4°C to get an estimation of the diffusion coefficient and relative polydispersity of the protein in aqueous solution. Data were then analyzed with Dynamics software V.5.25.44 (Protein Solutions) and buffer baselines were subtracted. Data were expressed as the means of hydrodynamic radius of the sample using nanometer as a unit The m.w. of the RTLs was estimated with Dynamics software V.5.25.44 (Protein Solutions).

### Circular dichroism (CD) analysis

CD analyses were performed as previously described (Chang *et al*., 2001) using an Aviv Model 215 CD spectrometer (Aviv Associates, Lakewood, NJ), except that the recombinant proteins were in Tris-Cl buffer at pH 8.5. Spectra were averaged and smoothed using built-in algorithms with buffer baselines subtracted. Secondary structure was estimated using a deconvolution software package (CDNN version 2.1) and the Variable Selection method (Compton *et al.,* 1986).

### Induction of EAE and treatment with RTLs

SJL mice were inoculated s.c. in the flanks with 0.2 ml of an emulsion containing 150 µg of PLP 139-151 peptide and an equal volume of CFA containing 150 µg of heat-killed *Mycobacterium tuberculosis* H37RA (M.Tb.; Difco, Detroit, MI) as described previously (Bebo *et al*., 2001). The (C57BL/6 x SJL)F₁ mice were immunized s.c in the flanks with 0.2 ml of an emulsion containing 200 µg of MOG 35-55 peptide or 150 µg of PLP 139-151 peptide and an equal amount of CFA containing 200 µg of heat-killed M.Tb. In a separate experiment, SJL mice were immunized s.c in the flanks with 0.2 ml of an emulsion containing 150 µg of PLP 139-151 or 150 µg of PLP 178-191 peptides, or 0.1 ml of an emulsion containing 200 µg of MBP 84-104 peptide and an equal volume of CFA containing 200 µg of heat-killed *M. tuberculosis*. The mice immunized with MBP 84-104 peptide were boosted a week later with the same peptide in CFA. On the day of immunization boost and 2 days after, the mice were injected i.p. with 200 ng of pertussis toxin (Ptx; List Biological Laboratories, Campbell, CA). The mice were assessed daily for signs of EAE according to the following scale; 0, normal; 1, limp tail or mild hindlimb weakness; 2, moderate hindlimb weakness or mild ataxia; 3, moderately severe hindlimb weakness; 4, severe hindlimb weakness or mild forelimb weakness or moderate ataxia; 5, paraplegia with no more than moderate forelimb weakness; and 6, paraplegia with severe forelimb weakness or severe ataxia or moribund condition.

At disease onset, mice were treated with vehicle (20 mM Tris-HCl); 100 µg of RTL400 or RTL401 given i.v. daily for 3 or 4 days, or 8 consecutive days with antihistamine (25 mg/kg) or 100 µg of RTL400 and RTL401 given s.c. for 8 days, or 10 µg free PLP 139-151 peptide given i.v. or s.c. for 8 consecutive days. Groups of control and treated mice were evaluated statistically for differences in disease incidence, day of onset, mortality, and presence or absence of relapse (χ² test), and for differences in Peak Clinical Score and Cumulative Disease Index (sum of daily scores) (Kruskal-Wallis Test). All *in vitro* data were generated from mice treated at disease onset with vehicle or 100 µg of RTL401 i.v. for 8 days. Mice were sacrificed at different time points following treatment with RTL401 for immunological and histological analyses.

### Histopathology

The intact spinal cords were removed from mice at the peak of clinical disease and fixed in 10% formalin. The spinal cords were dissected after fixation and embedded in paraffin before sectioning. The sections were stained with luxol fast blue/periodic acid-Schiff-hematoxylin to assess demyclination and inflammatory lesions, and analyzed by light microscopy. Semiquantitative analysis of inflammation and demyclination was determined by examining at least 10 sections from each mouse.

### Proliferation assay

Draining lymph node (LN) and spleens were harvested from vehicle- and RTL-treated mice at varying time points after immunization as indicated. A single cell suspension was prepared by homogenizing the tissue through a fine mesh screen. Cells were cultured in a 96-well flat-bottom tissue culture plate at 4 x 10⁵ cells/well in stimulation medium either alone (control) or with test Ags (PLP 139-151, PLP 178-191, and MBP 841-104 peptides) at varying concentrations. Cells were incubated for 3 days at 37°C in 7% CO₂. Cells were then pulsed with 0.5 µCi of [*methyl*-³H]thymidine (PerkinElmer, Boston, MA) for the final 18 h of incubation. The cells were harvested onto glass fiber filters, and tritiated thymidine uptake was measured by a liquid scintillation counter. Means and standard deviations (SD) were calculated from triplicate wells. Net cpm was calculated by subtracting control cpm from Ag-induced cpm.

### Cytokine determination by cytometric bead array (CBA)

LN and spleen cells were cultured at 4 x 10⁶ cells/well in a 24-well flat-bottom culture plate in stimulation medium with 2 µg/ml PLP 139-151 peptide for 48 h. Supcrnatants were then harvested and stored at -80°C until tested for cytokines. The mouse inflammation CBA kit was used to detect IL-12, TNF-α, IFN-γ, MCP-1, IL-10, and IL-6 simultaneously (BD Biosciences, San Diego, CA). Briefly, 50 µl of sample was mixed with 50 µl of the mixed capture beads and 50 µl of the mouse PE detection reagent. The tubes were incubated at room temperature for 2 h in the dark, followed by a wash step. The samples were then resuspended in 300 µl of wash buffer before acquisition on the FACScan. The data were analyzed using the CBA software (BD Biosciences). Standard curves were generated for each cytokine using the mixed bead standard provided in the kit, and the concentration of cytokine in the supernatant was determined by interpolation from the appropriate standard curve.

### FACS staining for very late activation Ag (VLA-4) and lymphocyte function-associated Ag (LFA-1) expression

Mononuclcar cells from the brain were isolated on a Percoll density gradient as previously described (Bourdette *et al.*, 1991). Cells were then stained with CD3 FITC (BD PharMingen, San Diego, CA) and VLA-4-PE or LFA-1-PB (Southern Biotechnology Associates, Birmingham, AL) expression by adding 1 µl of Ab per 1 x 10⁶ cells. Cells were incubated at 4°C for 20 min, and then washed two times with staining medium (1x PBS, 3% FBS, 0.02% sodium azide) before FACTS analysis on a FACScan instrument (BD Biosciences) using CellQuest software (BD Biosciences). Dual positive T-cells were calculated as a percentage of total mononuclear cells analyzed.

### RNA isolation and RT-PCR

Total RNA was isolated from spinal cords using the RNeasy mini-kit protocol (Qiagen) and then converted to cDNA using oligo(dT), random hexamers, and Superscript RT II enzyme (Invitrogen, Grand Island, NY). Real-time PCR was performed using Quantitect SYBR Green PCR master mix (Qiagen) and primers (synthesized by Applied Biosystems, Foster City, CA). Reactions were conducted on the ABI Prism 7000 Sequence Detection System (Applied Biosystems) using the listed primer sequences (5' to 3') to detect the following genes; L32: (F: GGA AAC CCA GAG GCA TTG AC (SEQ ID NO:46); R: TCA GGA TCT GGC CCT TGA AC (SEQ ID NO:47)); IFN-γ: (F:TGC TGA TGG GAG GAG ATG TCT (SEQ ID NO:48); R: TGC TGTCTG GCC TGCTGT TA (SEQ ID NO:49)); TNF-α: (F: CAG CCG ATG GGT TGT ACC TT (SEQ ID NO:50); R: GGC AGC CTT GCC CCTTGA (SEQ ID NO:51)); IL-10: (F: GAT GCC CCA GGC AGA GAA (SEQ ID NO:52); R: CAC CCA GGG AAT TCA AAT GC (SEQ ID NO:53)); IL-6: (F: CCA CGG CCT TCC CTA CTT C (SEQ ID NO:54); R:TGG GAG TGG TAT CCT CTG TGA A (SEQ ID NO:55)); TGF-β3: (F: GGG ACA GAT CTT GAG CAA GC (SEQ ID NO:56); R: TGC AGC CTT CCT CCC TCT C (SEQ ID NO:57)); RANTES: (F: CCT CAC CAT CAT CCT CAC TGC A (SEQ ID NO:58); R: TCT TCT CTG GGT TGG CAC ACA C (SEQ ID NO:59); macrophage-inflammatory protein (MIP)-2; (F:TGG GCT GCT GTC CCT CAA (SEQ ID NO:60); R: CCC GGG TGC TGT TTG TTT T (SEQ ID NO:61)); IP-10: (F: CGA TGA CGG GCC AGT GA (SEX ID NO:62); R:CGC AGO GAT GAT TTC AAG CT (SEQ ID NO:63)); CCR1: (F: GGG CCC TAG CCA TCT TAG CT (SEQ ID NO:64); R: TCC CAC TGG GCC TTA AAA AA (SEQ ID NO:65)); CCR2: (F: GTG TAG ATA GCA ACA AGC CTC AAA G (SEQ ID NO:66); R: CCC CCA CAT AGG GAT CAT GA (SBQ ID NO:67); CCR3: (F: GGG CAC CAC CCT GTG AAA (SEQ ID NO:68); R: TGG AGG CAG GAG CCA TGA (SEQ ID NO:69)); CCR5: (F: CAA TTT TCC AGC AAG ACA ATC CT (SEQ TD NO:70); R: TCT CCT GTG GAT CGG GTA TAG AC (SEQ ID NO:71); CCR6: (F: AAG ATG CCT GGC TTC CTC TGT (SEQ ID NO:72); R: GGT CTG CCT GGA GAT GTA GCT T (SEQ ID NO:73)); CCR7: (F: CCA GGC ACG CAA CTT TGA G (SEQ ID NO:74); R: ACT ACC ACC ACA GCA ATG ATC (SEQ ID NO:75); CCR8: (F: CCA GCGATC TTC CCA TTC TTC (SEQ ID NO:76); R: GCC CTG CAC ACT CCC CTT A (SEQ ID NO:77)).

In the studies described above, RTLs were shown to reverse clinical and histological signs of disease in Lewis rats that developed monophasic EAE (Burrows *et al.*, 1998; Burrows *et al.*, 1999), as well as in Tg DR2 (DRB 1 * 1501) mice that developed chronic EAE (Vandenbark *et al.*, 2003). In the instant example, the efficacy of RTL therapy on relapsing EAE induced by PLP 139-151 peptide in SJL/J mice was further demonstrated. Treatment of EAE in SJL mice required mouse MHC class II design modifications and included the α1 and β1 domains of the I-A^{s} molecule covalently bound to the PLP 139-151 peptide (RTL401) or the RTL without bound peptide (RTL400).

### Biochemical characterization of mouse RTLs

CD analysis shows that the human RTLs have a secondary structure composition similar to the TCR recognition/peptide-binding α1β1 domain of native human MHC class II molecule as determined by x-ray crystallography (Chang et al., 2001; Smit *et al.*, 1998; Li *et al.*, 2000). CD data observed in the current investigation showed that murine RTLs shared a similar anti-parallel β-sheet platform, and α-helix secondary structure common to all murine MHC class II Ag-binding domains (Fremont *et al.*, 1998; He *et al.*, 2002; Scott *et al.*, 1998). The size exclusion chromatography data (Fig. 26) and hydrodynamic analysis using DLS indicated that the purified and refolded RTL400 and RTL401 were monodispersed molecules in Tris-Cl buffer. Fractions of each peak from the size exclusion column were collected and analyzed by CD. Secondary structure analysis using the Variable Selection method (Compton *et al.*, 1986) indicated that murine RTLs maintain high order of secondary structure similar to native murine I-A^{k} and I-Aⁿ MHC class II molecules (Fremont *et al.*, 1998; He *et al.*, 2002).

### Dose-dependent inhibition of PLP peptide-induced EAE in SJL mice

In initial preclinical studies, SJL/J mice with established signs of EAE were treated with varying numbers of daily i.v. injections of 100 µg of RTL401 containing PLP 139-151 peptide. As is shown in Fig. 27, control mice typically developed a relapsing EAE disease course, with onset of the initial episode of acute disease occurring on day 11-12 after injection of PLP 139-151 peptide/CFA and peak clinical scores developing on day 15, followed by a clinical improvement that lasted until day 20. The first relapse was evident by day 22 in essentially all the mice, reaching a second peak on days 27-28. The mice generally had subsequent remissions and may have had additional relapses or developed chronic But these variations in clinical course occurred sporadically in individual mice.

Treatment with 100 µg of RTL40 i.v. beginning on day 12 and continuing for 8 consecutive days had the greatest effect on clinical EAE (Fig. 27; Table 7), although fewer daily i.v. injections (3 or 4 consecutive days) were only nominally less effective (Table 7). Compared with vehicle-treated controls, all three regimens ameliorated clinical disease within the first 24 h, reduced the peak severity of the first clinical episode, and essentially eliminated relapses (Fig. 27; Table 7). RTL401 treatment reduced the daily clinical score to minimally detectable disease that was maintained even after cessation of treatment for nearly 4 wk, and significantly reduced the cumulative disease index (Table 7). Mice receiving eight daily i,v, doses of 100 µg of RTL401 were treated with antihistamines to prevent development of allergic responses to RTLs. Treatment of mice with the same regimen of antihistamine alone had no effect on the course of relapsing EAE (Table 7). In contrast to mice treated with RTL401, mice treated with the eight daily i.v. doses of 100 ng of empty RTL400 construct or a molar equivalent dose of free PLP 139-151 peptide (10 µg peptide/injection) with antihistamine did not experience significant clinical benefit compared with untreated control mice (Table 7).

**Table 7. Effect of RTL401 and RTL400 treatment on EAE in SJL/J mice immunized with PLP 139-151/CFA**

| | |
|---|---|
| | Incidence |
| | Onset. |
| | Peak |
| | Mortality |
| | Relapse |
| | CDI |
| Control | |
| | 13/13 |
| | 112 ± 0.6 |
| | 4.2 ± 1.4 |
| | 0/13 |
| | 9/13 |
| | 96.7 ± 33.7 |
| PLP 139-151 (10 µg) | |
| | 4/4 |
| | 11 ± 0.0 |
| | 4.7 ± 0.5 |
| | 0/4 |
| | 3/4 |
| | 87.1 ± 19.3 |
| Anti-histamine | |
| | 4/4 |
| | 11.5 ± 0.6 |
| | 5.2 ± 0.3 |
| | 0/4 |
| | 3/4 |
| | 118 ± 24.9 |
| RTL400 | |
| | 6/6 |
| | 11.2 ± 0.4 |
| | 4.8 ± 0.9 |
| | 1/6 |
| | 4/6 |
| | 116.2 ± 43.3 |
| RTI401 (3 days i.v.) | |
| | 4/4 |
| | 11.5 ± 0.6 |
| | 3.1 ± 1.1^{abcd} |
| | 0/4 |
| | 0/4 |
| | 45.3 ± 12.6^{abed} |
| RTL401 (4 days i.v.) | |
| | 4/4 |
| | 11.7 ± 0.9 |
| | 3.9 ± 0.9^{d} |
| | 0/4 |
| | 0/4 |
| | 50.5 ± 22.2^{abcd} |
| RTL401 (8 days i.v.) | |
| | 14/14 |
| | 11.2 ± 0.4 |
| | 2.9 ± 1.4^{abcd} |
| | 0/14 |
| | 1/ 14^{abcd} |
| | 35.4 ± 25.5^{abcd} |

| | |
|---|---|
| ^{a} Significant difference compared to control *p* < 0.05. ^{b} Significant difference compared to peptide, *p* < 0.05. ^{c} Significant difference compared to RTL400, *p* < 0.05. ^{d} Significant difference compared to anti-histamine, *p* < 0.05. | |

As is shown in Fig. 27 and Table 8, eight daily injections of 100 µg of RTL401 administered by the s.c. route was also effective in treating EAE, nominally reducing the relapse rate, and significantly reducing daily clinical scores and the cumulative disease index in a manner similar to i.v. injections. In contrast, comparable s.c. injections of the empty RTL400 construct or a molar equivalent dose of free PLP 139-151 peptide did not have any effect on the clinical course of EAE in SJL mice. These results demonstrate that both i.v. and s.c. administration of RTL4 01 reduced relapses of EAE and produced long-lasting clinical benefit even after cessation of RTL treatment on day 20.

**Table 8. Effect of RTL401 treatment on SJL females immunized with PLP 139-151, PLP 178-191 or MBP 84-104**

| | |
|---|---|
| | Incidence |
| | Onset |
| | Peak |
| | Mortality |
| | Relapse |
| | Mean CDI |
| control (PLP 139-1551) | |
| | 13/13 |
| | 11.2 ± 0.6 |
| | 4.2 ± 1.4 |
| | 0/13 |
| | 9/13 |
| | 96.7 ± 33.7 |
| RTL i.v. | |
| | 14/14 |
| | 11.2 ± 0.4 |
| | 2.9 ± 1.4^{a} |
| | 0/14 |
| | 1/14^{a} |
| | 35.4 ± 25.5^{a} |
| RTLs.c | |
| | 12/12 |
| | 11.2 ± 0.4 |
| | 3.1 ± 1.3 |
| | 0/12 |
| | 4/12 |
| | 45.5 ± 16.8^{a} |
| Control(PLP 178-191) | |
| | 5/5 |
| | 11.4 ± 0.6 |
| | 3.0 ± 1.5 |
| | 0/5 |
| | 2/5 |
| | 53.3 ± 16.1 |
| RTL i.v. | |
| | 6/6 |
| | 11.3 ± 0.5 |
| | 2.1 ± 1.8 |
| | 0/6 |
| | 3/6 |
| | 39.2 ± 15.7 |
| Control(MBP 84-104) | |
| | 6/6 |
| | 11.3 ± 0.5 |
| | 3.8 ± 1.7 |
| | 0/6 |
| | 4/6 |
| | 51.3 ± 23.6 |
| RTL i.v. | |
| | 6/6 |
| | 11.5 ± 0.6 |
| | 2.2 ± 1.0 |
| | 0/6 |
| | 4/6 |
| | 41.9 ± 14.0 |

| | |
|---|---|
| ^{a} Significant difference between control and treatment, groups, *p* < 0.05. Incidence: number of mice that get sick in a group. Onset: Day when first clinical signs of EAE is observed. Peak: Maximum EAE score. Relapse: Number of mice that show a decrease in EAE score by 1 point for 48 h followed by an increase in EAE score for 48 h. Mean CDI: Cumulative disease index; sum of the daily scores for the entire length of the experiment. | |

### RTL treatment effect on EAE is peptide-specific and requires cognate MHC

To evaluate peptide specificity of RTL treatment *in vivo*, RTL401 was used to treat EAE induced in SJL/J mice with two different encephalitogenic peptides, PLP 178-191 and MBP 84-104, both restricted by I-As. Eight daily i.v. injections of 100 µg of RTL401 did not significantly affect the overall severity or relapse rate of EAE induced by either peptide compared with vehicle-treated control mice (*p* > 0.2), although in each case a nominal reduction in the cumulative disease index was observed. Day 42 LN responses in PLP 178-191 and MBP 84-10/1 peptide-immunized mice with EAE were specific only for the immunizing peptide, and no responses were observed to PLP 139-151 peptide, indicating a lack of epitope spreading.

To further evaluate the requirement; for MHC and peptide specificity of RTL treatment, RTL401 was used to treat EAE induced by either PLP 139-151 peptide or MOG 35-55 peptide in (C57BL/6 x SJL) F₁ mice. These mice express both I-A^{s} and I-E^{b} MHC class II molecules that restrict PLP 139-151 (I-A^{s}) and MOG 35-55 (I-E^{b}) peptides, in both cases producing an encephalitogenic response. As is shown in Fig. 28, treatment at disease onset with eight daily i.v. injections of 100 µg of RTL401 significantly reduced the severity of EAE induced by PLP 139-151 peptide, but had no effect on EAE induced by MOG 35-55 peptide. These data demonstrate that RTL treatment of EAE is specific for the cognate combination of MHC and neuroantigen peptide.

### Effects of RTL401 treatment on peripheral T-ce// responses ex vivo

LNs and spleen cells from vehicle control and RTL401 treated (eight daily i.v. injections of 100 µg) SJL/J mice with EAE were analyzed during the course of treatment for proliferation and cytokine responses to the immunizing PLP 139 151 peptide. Immune cell responses were assessed just after disease onset but before treatment (day 11), 24 h after initiation of treatment (day 13), at the peak of the initial clinical episode (day 15), at the first remission (day 18), at the beginning of the first relapse (day 22), at the peak of the first relapse (day 28), and at the end of the first relapse (day 42). In contrast to previously published results in DR2-expressing mice (Vandenbark *et al*., 2003), there was no significant inhibitory effect of RTL treatment on proliferation responses at any time during the course of EAE. As exemplified in Fig. 29, treatment with RTL401 non-dnanymhihited proliferation responses to PLP 139-151 peptide in LN cultures, but significantly enhanced proliferation of splenocyte cultures at several time points, including on day 42 as shown in Fig. 29. In contrast, RTL401 treatment had mixed effects on cytokine secretion from PLP 139-151-stimulated splenocytes (Fig. 30). One day after initiation of RTL401 treatment (day 13), there were no significant changes in cytokine responses compared with control mice. Surprisingly, at the peak of the first episode of EAE (day 15), there was enhanced secretion of both inflammatory (TNF-α, IFN-γ, MCP-1, and IL-6) and anti-inflammatory (IL-10) factors in splenocyte cultures from RTL401-treated vs. control mice. However, during remission from the first episode of EAE (day 18), the cytokine picture changed witch strongly reduced levels of IFN-γ, still enhanced levels of MCP-1, but no significant differences in TNF-α, IL-6, or IL-10 in RTL401-treated mice. At onset of the first relapse (day 22), there was again a significant reduction in secreted IFN-τ in RTL401-treated mice, but no significant differences in the other inflammatory factors (Fig. 30). Of possible importance for systemic regulation, there was a significant increase in secreted IL-10 levels by PLP 139-151-reactive splenocytes from RTL401-treated mice at both the onset and peak of the first relapse (days 22 and 28, respectively). Both IgG1 and IgG2a Abs were detected in serum during the course of EAE, but levels showed only minor fluctuations as a result of RTL401 treatment.

### Effects of RTL401 treatment on CNS during EAE

To further evaluate the effects of RTL401 therapy on EAE, histological sections were obtained and phenotypic and functional analyses of CNS cells were conducted. Histological sections of spinal cords taken on day 46 showed reduced inflammatory lesions and decreased demyelination in RTL401-treated vs. control mice. More specifically, spinal cords from RTL-treated mouse showed dense mononuclear infiltration with only very slight or no apparent loss of myelin stain in the surrounding myelinated tissue. Spinal cords from control, non-RTL-treated mouse showed multiple regions of dense mononuclear cell infiltration with considerable, diffuse loss of myelin stain in the regions adjacent to the mononuclear infiltrate. This reduction in inflammatory activity found in RTL401-treated mice was reflected by a decrease in the number of inHammatory mononuclear cells obtained from brain and spinal cord tissue over the course of treatment; (Fig. 31). The reduction of inflammatory cells was most pronounced at onset and peak of the first clinical episode (days 13 and 15), and at onset of the first relapse (day 22), was marked by an overall decrease of CD4⁺ T-cells (from 43 to 23%) but an increase in CD11b⁺ monocytes/macrophages (from 38 to 60%) as determined by FACS analysis. Moreover, the number of T-cells expressing adhesion/homing markers VLA-4 and LFA-1 was consistently reduced in brains and spinal cords from RTL401-treated mice on days 22, 28, and 42 (brain only) after EAE induction (Fig. 32). From day 15 on, RT-PCR analysis of spinal cord tissue from RTL-401-treatcd mice also showed moderate to strong reduction in expression of mRNA for inflammatory cytokines (IFN-γ, TNF-α and IL-6) and chcmokincs (RANTES, MIP-2, and IP-10), but enhanced expression of TGF-β3 (Fig. 33), consistent with other data indicating a protective role for this cytokine (Matejuk *et al*., 2004). Expression of IL-10 was very low throughout the EAE disease course in spinal cords from RTL-treated mice, with only a slight enhancement in RTL401-treated mice during the first relapse (day 22; Fig. 33). Interestingly, expression of most chemokine receptors (CCR1, CCR2, CCR5, CCR6, CCR7, and CCR8) was moderately to strongly reduced in spinal cord tissue from RTL401-treated mice beginning at the peak of the first episode (day 15; Fig. 34). In contrast, expression of CCR3 (Th2 associated) appeared to be uniquely enhanced in spinal cord tissue collected, from RTL401-treated vs. control mice during the first relapse (days 22 and 28, Fig. 34).

### Effects of RTL401 on Thoracic Spinal Cord White Matter

In another experiment, RTL401 was used to treat EAE induced by PLP-139-151 in SL/J mice. Onset of EAE was evident on day 11 with the peak reached on day 20. The mice received five consecutive treatments of RTL401 by i.v. starting on day 20 and three consecutive treatment s.c. starting on day 32. Mice were sacrificed on day 60 by CO₂ inhalation. Spinal cords were removed by insuffocation and fixed in 10% formalin/PBS. Paraffin sections were prepared and stained with hematoxylin and eosin. Neurological lesions were graded on each of 10 cross sections per spinal cord. As can be seen in Figure 43 and Tables 9 and 10, treatment with RTL 401 significantly decreased the amount of myelin damage in the dorsal, lateral and central white matter of the thoracic section of the spinal cord.

**Table 9. Clinical scores of individual mice.**

| Mouse# | Onset | Peak | Control | RTL401 |
|---|---|---|---|---|
| 1 | 1.5 | 4.5 | 4.5 | 2 |
| 2 | 1.5 | 4.5 | 4 | 1.5 |
| 3 | 1.5 | 4.5 | 4 | 1.5 |

**Table 10. One-way ANOVA analysis of variance followed by Newman-Kuels multiple comparisons tests. *Comparison significant statistically.**

| Comparison | Dorsal | Lateral and Ventral |
|---|---|---|
| Peak vs. Onset | P < 0.05 * | P < 0.001 * |
| Vehicle vs. Onset | P < 0.001 * | P < 0.001 * |
| Vehicle vs. Peak | P < 0.01 * | P < 0.01 * |
| RL401 vs. Vehicle | P < 0.001 * | P < 0.001 * |
| RTL40 vs. Peak | P < 0,05* | P < 0.001 * |
| RTL401 vs. Onset | P > 0.05 | P > 0.05 |

The foregoing disclosure evinces successful design and demonstration of the efficacy of oligomeric RTLs specific for both human and rat T-cells that reversed clinical EAE and induced long-term T-cell tolerance. In the current example, the design characteristics and therapeutic effects of a monomeric murine RTL401 (I-A^{s}/PLP 139-151 peptide) on a relapsing model of EAR in SJL/J mice are demonstrated. Generally, RTL401 had very similar structural characteristics and therapeutic effects on EAE compared with previously designed molecules, although some important differences were noted in its effects on the activation and inflammatory properties of targeted encephalitogenic T-cells. A similar monomeric form of human DR2 RTL has been produced and tested in HLA-DR2 transgenic mice developing chronic EAE, which is also useful within various embodiments of the current invention (see, e.g., U.S. Provisional Patent Application Number 60/500,660, filed September 5, 2003; and U.S. Patent Application No. 10/936,467, filed September 7, 2004; and Huan *et al*., 2004, each of which is incorporated herein by reference in its entirety).

Secondary structure analysis from CD spectra of murine RTLs indicated that RTL400 and RTL401 maintained a high order of secondary structure similar to native murine I-A^{k} and I-A^{u} MHC class II molecules (Fremont *et al*., 1998; Hc *et al.,* 2002). The recombinant RTL is a relatively small molecule (~24 kDa) containing a native disulfide bond between cysteine 17 and 79 (RTL401 amino acid numbering, corresponding to murine I-A^{s} β-chain residues 42 and 104). This disulfide bond was retained upon refolding, demonstrated by comparing mobility during electrophoresis (SDS-PAGE) of the RTL in the presence or absence of the reducing reagent, 2-ME. Both RTL4100 and RTL401 showed a higher mobility in the absence of 2-ME, indicative of a more compact structure compared with the reduced RTLs. Together, these data represent a primary confirmation of the conformational integrity of the molecule. Unlike the human HLA-DR2 construct and rat I-A constructs that tended to aggregate during the refolding process, the mouse RTL constructs appeared to be monodispersed molecules, based on light scattering and size exclusion chromatography analyses.

Of potential clinical importance, these monodispersed molecules induced specific and significant inhibition of PLP 139-151 peptide-induced EAE, but not EAE induced by other myelin poptides when administered *in vivo*. The investigations herein demonstrate potent activity of this minimal TCR ligand to reverse clinical signs of EAE and prevent relapses for at least 26 days after completion of a single 3-, 4-, or 8-day course of daily RTL injections. Disease expression after RTL treatment; was minimal, although persistent, unlike the complete abrogation of clinical signs observed in RTL-treated DR2 Tg mice (Vandenbark *et al*., 2003). One explanation for chronic low-level EAE might be epitope spreading (Lehman *et al.,* 1992; Vanderlught, 2003). It is notable in this context that the RTL-treated mice described herein did not develop T-cell responses to other known subdominant encephalitogenic peptides, including PLP 178-191 or MBP 84-104. Although i.v. injections provided the lowest cumulative EAE scores, s. c. injections were also highly effective. This finding will facilitate future application of RTL therapy to humans, in whom the s.c route of injection is preferable due to ease of injection and reduced risk of hypersensitivity reactions. Such reactions were noted in i.v. RTL-treated SJL/J mice, but could be controlled by injection of antihistamines.

Mechanistically, the murine RTL401 appeared to possess several differences compared with our human DR2/MOG 35-55 construct that inhibited chronic EAE in DR2 transgenic mice (Vandenbark *et al*., 2003) and our rat RT-1B¹/MBP 72-89 construct that inhibited monophasic EAE in Lewis rats (Burrows *et al.,* 2000). Both previous constructs were oligomeric and induced a striking reduction of LN T-cell responses, as assessed by proliferation and secretion of inflammatory cytokines including IFN-γ and TNF-α. In contrast, the murine I-A^{s}/PLP 139-151 construct did not significantly reduce T-cell proliferation responses to PLP 139-151 peptide, but instead, enhanced splenocyte proliferation and secretion of both inflammatory (TNF-α and IFN-γ) and anti-inflammatory (IL-10) cytokines during the first 3 days of treatment (Fig. 30). In general, variations in expression of inflammatory cytokines mirrored periods of EAE relapses and remission in control SJL/J mice, with more expression noted on days 15 (peak of initial episode) and 22 (first relapse) than on day 18 (remission). However, continued treatment with RTL401 resulted in strongly decreased levels of IFN-γ, while at the same time maintaining elevated IL-10 levels (Fig. 30). These data indicate that in SJL mice, RTLs induced a cytokine switch rather than anergy or apoptosis in treated T-cells that still allowed homing to the target organ (CNS). Interestingly, treatment of human T-cell clones *in vitro* with DR2/MBP 85-99 or DR2/cABL peptide RTLs led to a similar enhancement of IL-10 secretion, raising the possibility of an RTL-induced cytokine switch mechanism in humans as well (Burrows, *et al*., 2001). Other Th2 cytokines such as IL-4 and IL-5 may also be involved.

The mechanistic differences observed in the periphery apparently resulted in differences in CNS as well. Histological sections of spinal cord tissue from RTL-treated SJL mice showed less demyelination, but only a modest reduction of inflammatory lesions. Moreover, both brain and spinal cord tissue from RTL401-treated mice had only a slight reduction in numbers of infiltrating cells, unlike in RTL312-treated DR2 mice protected from EAE that had a more drastic reduction of infiltrating CNS cells (Vandenbark *et al*., 2003). During the first relapse, the RTL-treated SJL/J mice had a significant reduction in the percent of infiltrating cells expressing VLA-4 and LFA-1, adhesion molecules that are known to be important in EAE to direct homing of leukocytes to the perivascular sites of inflammatory lesions in CNS tissue (Gordon *et al*., 1995; Theien *et al.,* 2001). Further analysis of mRNA from CNS tissue also demonstrated a striking reduction in expression of inflammatory cytokines(IFN-γ, TNF-α, and IL-6) and chemokines (RANTES, MIP-2, and IP-10), but enhanced expression of anti-inflammatory cytokines (TGF-β₃ and IL-10). IL-10 is known to inhibit IFN-γ production and clinical expression of EAE (Cua *et al*., 1999), and an association with increased expression of TGF-β₃ and EAE protection has also been reported (Matejuk *et al.*, 2004). The expression pattern for inflammatory chemokine receptors in CNS appeared to be related to the clinical disease course of EAE, with strongest expression at the peak of the initial episode and/or the beginning of the first relapse.

In addition to these findings, CCR1, CCR2, and CCR7 appeared to be expressed preferentially in control mice during the first episode clear, whereas CCR5, CCR6, CCR8 were more strongly expressed during the first relapse. Of importance, treatment with RTL401 reduced expression of all these CCRs during both clinical episodes of EAE (Fig. 34). In studies in C57BL/6 mice with EAE, enhanced expression of CCR1, CCR2, and CCR5 in CNS at the peak of EAE was observed (Matejuk *et al*., 2001). Moreover, *in vitro* treatment of encephalitogenic T-cells with IL-12 and IL-18, respectively, enhanced expression of IFN-γ/CCR5 and TNF-α/CCR4/CCR7 and potentiated transfer ofBAE (Ito *et al.,* 2003). CCR5 up-regulation by IL-12 has also been reported to enhance LFA-1-mediated adhesiveness (Mukai *et al*., 2000), and CCR7 binding to its ligand, MIP-3b, promotes proliferation of CD4⁺ T-cells and progression of autoimmunity (Ploix *et al*., 2001). Based on their pattern of expression during EAE and their strong down-regulation by RTL401, the current findings also implicate CCR6 (Schutyser, 2003) and CCR8 (Romagnani, 2002) as inflammatory CCRs that may contribute to EAE. In contrast to its inhibitory effects on inflammatory CCRs, RTL401 treatment strongly enhanced expression of CCR3 that has been associated with Th2 responses (Salusto *et al*., 1998) during the initiation and peak of the first relapse (Fig. 34). This enhancement of CCR3 in EAE-protected mice is reminiscent of the strong up-regulation of CCR3 in BV8S2 transgenic mice successfully treated with TCR BV8S2 determinants (Matejuk *et al.,* 2000). Taken together, these findings indicate that regulation of CCR expression is an important function of the RTL treatment mechanism.

Thus, the systemic effects of RTL therapy that promoted a cytokine switch in response to the encephalitogenic PLP 139-151 peptide apparently produced a non-encephalitogenic T-cell phenotype that retained some ability to infiltrate CNS tissue. However, the infiltrating cells from RTL401-treated mice clearly had reduced inflammatory capability, enhanced secretion of anti-inflammatory factors, and enhanced expression of a protective CCR. Thus, replacement of the disease-initiating encephahtogenic T-cells in CNS by RTL-altered ways associated with partial resolution of inflammatory lesions and reversal of clinical disease. However, the persistent low-level EAE might result from incomplete regulation induced by our postulated T-cell cytokine switch mechanism and the residual compact lesions found in the spinal cord sections. The cytokine switch mechanism considered here differs from an anergy mechanism reported previously in SJL/J mice by others using purified natural four domain I-A^{s} molecules loaded with PLP 139-151 peptide, or from an apparent deletional mechanism in HLA-DR2 mice treated with an aggregated form of a two-domain RTL (Vandenbark *et al*., 2003).

In conclusion, the instant example demonstrates for the first time the potent therapeutic effects of a murine minimal TCR ligand in a relapsing model of EAE in SJL mice. A single course of i.v. or s.c. RTL injections prevented relapses and induced long-term clinical benefits that appeared to be mediated by a cytokine switch mechanism involving IL-10, TGF-β3, and CCR3, leading to a moderation of CNS inflammation and demyelination. These results strongly support the clinical application of this novel class of pepticle/MHC class II constructs as treatment for T-cell-mediated autoimmune diseases such as multiple sclerosis.

### Example 15

### Cytokine Switching and Related RTL Effects on T-cell Biology in the CNS and Peripheral Sites In Experimental Autoimmune Encephalomyelitis

### Animals

Female SJL mice were obtained from The Jackson Laboratory (Bar Harbor, ME) at 7-8 weeks of age. The mice were housed at the animal facility at Portland Veterans Affairs Medical Center in accordance with institutional guidelines.

### RTL construction and production

Methods for the design, cloning and expression of RTL401 were employed as described above in Example 14. The murine I-A^{s} β1α1 insert was then ligated into pET21d(+) vector and transformed into Nova blue *E. Coli* host (Novagen, Inc., Madison, WI) for positive colony selection and sequence verification. RTL400 and RTL401 plasmid constructs were then transformed into *E. Coli* strain BL21 (DE3) expression host (Novagen, Inc., Madison, WI). The purification of proteins was conducted as described previously (Chang *et al.*, 2001). The final yield of purified protein varied between 15 to 30 mg/L of bacterial culture.

### Dynamic Light scattering (DLS) analysis

Light scattering experiments were conducted in a DynaPro molecular sizing instrument (Protein Solutions, Inc., Charlottesille, VA). The protein samples, in 20 mM Tris-Cl buffer at pH 8.5, were filtered through 100 nm Anodisc membrane filters (Whatman, Clifton, NJ) at a concentration of 1.0 mg/ml and 20 µl of filtered sample were loaded into a quartz cuvette and analyzed with a 488 nm laser beam. Fifty spectra were collected at 4° C to determine the diffusion coefficient and relative polydispersity of the protein in aqueous solution. Data were then analyzed with Dynamics software V.5.25.44 (Protein Solutions, Charlottesville, VA) and buffer baselines were subtracted. Data were expressed as means of hydrodynamic radius of sample using nm as a unit. The molecular weight of the RTLs was determined with Dynamics software V.5.25.44 (Protein Solutions, Charlottesville, VA).

### Circular dichroism (CD) analysis

CD analyses were preformed as previously described (Chang *et al.,* 2001) using an Aviv Model 215 CD spectrometer (Aviv Associates, Lakewood, NJ), except that the recombinant proteins were in Tris-Cl buffer at pH 8.5. Spectra were averaged and smoothed using built-in algorithms with buffer baselines subtracted. Secondary structure was determined using a built-in deconvolution software package (CDNN version 2.1) and the Variable Selection method (Compton *et al.,* 1986).

### Organ cell transfer RTL treatment

SJL mice were immunized with 150µg PLP 139-151 (ser) in 200µg Complete Freund's Adjuvant. Ten days post immunization, lymph nodes and spleens were harvested and cultured *in vitro* in the presence of 10ug/ml PLP 139-251 in stimulation medium containing 2% fetal bovine serum for 48h. Cells were then washed and 15 million blasting cells were injected i.p. into SJL mice. The mice were assessed daily for signs of EAE according to the following scale; 0 = normal; 1 = limp tail or mild hind limb weakness; 2 = moderate hind limb weakness or mild ataxia; 3 = moderately severe hind limb weakness; 4 = severe hind limb weakness or mild forelimb weakness or moderate ataxia; 5 = paraplegia with no more than moderate forelimb weakness; and 6 = paraplegia with severe forelimb weakness or severe ataxia or moribund condition. The cumulative disease index (CDI) is the sum of the daily EAE scores for each mouse for the entire duration of the experiment. The CDI is presented as mean ± SD for each group. At the onset of clinical signs of EAE, the mice were divided into 3 groups and treated as controls or with 100ml of RTL 401 i.v. along with antihistamine for 5days or RTL401 s.c. for 8 days. Mice were monitored for disease until they were sacrificed for ex vivo analyses.

### Histopathology

Intact spinal cords were removed from mice on day 19 of clinical disease and fixed in 10% formalin. The spinal cords were dissected after fixation and embedded in paraffin before sectioning. The sections were stained with hematoxylin/eosin (HE) to assess inflammatory lesions, and analyzed by light microscopy. Semiquantitative analysis of inflammation was determined by examining at least 10 replicates of the cervical, thoracic and lumbar sections from each mouse.

### Western blot (immunoblotting) detection of non-phosphorylated neurofilaments

The procedure was carried out as described by Pitt *et al*. (2000). PBS-perfused spinal cords were homogenized in ice-cold RIPA-1- buffer (50 mM Tris-HCl, pH 7.5, 150 mM NaCl, 1% NP-40, 0.5 % deoxycholate, 0.1% SDS, 1 mM NaCO₃), and protease inhibitors and incubated for 15 min with shaking. After centrifugation (14,000 x g at 4°C for 15 min), the supernatant was collected and the protein concentration measured and adjusted using RIP A+ buffer. Samples were denatured in sampling buffer for 10 min at 70°C, then separated by 10% SDS-PAGE and blotted onto a PVDF membrane. After transfer, the membrane was blocked four 1 hr in 3% BSA. immunodetection was accomplished by incubation overnight at 4°C with primary monoclonal antibody SMI 32 (1:5,000 dilution in 3% BSA and 0.05% Tween-20, purchased from Sternberger Monoclonals, Lutherville, MD) specific for non-phosphorylated neurofilaments. After being washed, the blots were incubated with horseradish peroxidase (HRP)-labeled goat antibody against mouse IgG (1:5,000 dilution in 3%) BSA and 0.05% Tween-20, purchased from Pierce Biotechnology, Inc., Rockford, IL) for 1 hr and then washed. Blots were developed with a SuperSignal West Pico Chemiluminescent kit (Pierce). To control the amounts of protein loaded, the membranes were stripped with the Restore Western Blot. Stripping Buffer (Pierce) and detected again with a monoclonal antibody for Glyceraldehyde 3-phosphate dehydrogcnase (GAPDH) purchased from Chemicon (Temecula, CA). After being developed, the films were scanned and quantified with Image Quant software (Amersham, Piscataway, NJ).

### Proliferation assay

PLP-specific T-cells were cultured *in vitro* in the presence of media alone, PLP139-151 (10µg/ml), RTL 401 (neat) or RTL 401(1:10) for 24h. Cells were then washed thoroughly and 20,000 T-cells were cultured along with 2X10⁵ antigen presenting cells in 96-well flat bottom plates in stimulation media either alone or with PLP139-151 at 10µg/ml or 2 µg/ml. Cells were then incubated for 2 days at 37°C in 7% CO₂. Cells were then pulsed with 0.5µCi of [methyl-³H]thymidine (Perkin Elmer, Boston, MA) for the final 18h of incubation. The cells were harvested onto glass fiber filters and tritiated thymidine uptake was measured by a liquid scintillation counter. Means and standard deviations were calculated from triplicate wells. Stimulation indices were determined by calculating the ratio of antigen-specific cpm to control cpm.

### Cytrokine determination by Cytometric Bead Array (CBA)

Brains were pooled from 3 mice from each group and processed through a fine mesh screen. The mononuclear cells were then isolated on a 40% - 80% Percoll gradient and 1x10⁶ brain cells were cultured along with 3x10⁶ irradiated splenocytes (used as filler cells) in a 24-well plate in the presence of 10µg/ml PLP-139-151 peptide for 48h. Spleen and blood mononuclear cells were cultured at 4x10⁶ cells/well in a 24 well flat bottom culture plate in stimulation medium with 2µg/ml PLP-139-151 peptide for 48h.

Supernatants from the samples were then harvested and stored at -80°C until tested for cytokines. The mouse inflammation CBA kit was used to detect IL-12p40, TNF-α, IFN-γ, MCP-1, IL-10 and IL-6 simultaneously (BD Bioscience). Briefly, 50µl of sample was mixed with 50µl of the mixed capture beads and 50µl of the mouse PE detection reagent. The tubes were incubated at room temperature for 2hrs in the dark, followed by a wash step. The samples were then resuspended in 300µl of wash buffer before acquisition on the FACScan. The data were analyzed using the CBA software (BD Biosciences). Standard curves were generated for each cytokine using the mixed bead standard provided in the kit and the concentration of cytokine in the supernatant was determined by interpolation from the appropriate standard curve.

### ELISA for detection of IL-13 and IL-4

Spleens, blood and brains from control, RTL i.v. and RTL s.c mice were harvested on day 19 post immunization and 4 X10⁶ cells were cultured in stimulation medium in the presence of 10µg/ml PLP139-151 for 48h. For the *in vitro* assays, cells were cultured in the presence of APC with or without PLP139-151 (2µg/ml). Supernatants were harvested and frozen at -80° C until further testing. 96 well plates were coated with 100µl of anti-mouse IL-13 or IL-4 capture antibody (4µg/ml) in 1X PBS or sodium bicarbonate coating buffer. Plates were incubated at 4°C overnight. Plates were then washed with wash buffer (1X PBS/0.05% Tween-20) and blocked with blocking buffer (1X PBS, 2% BSA) for 2h at room temperature, Plates were then washed and 100µl of sample or standard was added to each well. Il-13 plates were incubated at room temperature for 2h while IL-4 plates were incubated at 4°C overnight. The follollowing day, plates were washed and 100µl of biotinylated antibody (IL-13 or IL-4) was added. IL-13 plates were incubated at room temperature for 2h while IL-4 plates were incubated at room for 45 min. Plates were then washed and 100ml of 1:200 diluted HRP was added to IL-13 plates and 1:400 diluted HRP was added to the IL-4. plates. Plates were incubated at room temperature for 30 min. followed by a wash step. This was followed by addition of 100µl TMB chromogen (KPL, Gaithersburg, Maryland). The plates were allowed to develop for approx. 30 min. and reaction, stopped by adding 100µl stop solution (KPL, Gaithersburg, Maryland). The optical density was then measured at 450nm.

### RNA isolation and RT-PCR

Total DNA was isolated from spinal cords using the RNeasy mini kit protocol (Qiagen, Valencia, CA) and then converted to cDNA using oligo dT, random hexamers and Superscript RT II enzyme (Invitrogen, Grand Island, NY). Real-time PCR was performed using Quantitect SYBR Green PCR master mix (Qiagen) and primers (synthesized by ABI). Reactions were conducted on the ABI Prism 7000 Sequence Detection System (Applied Biosystems, Foster City, CA) using conventional, commercially available primers to detect the following genes: L32: (F: GGA AAC CCA GAG GCA TTG AC (SEQ ID NO: 46); R: TCA GGA TCT GGC CCT TGA AC (SEQ ID NO: 47)); IFN-γ: (F: TGC TGA TGG GAG GAG ATG TCT (SEQ ID NO: 48); R: TGC TGT CTG GCC TGC TGT TA (SEQ ID NO: 49)); TNF-α: (F: CAG CCG ATG GGT TGT ACC TT (SEQ ID NO): 50); R: GGC AGC CTT GTC CCT TGA(SEQ ID NO:51)); IL-10: (F: GAT GCC CCA GGC AGA GAA(SEQ ID NO:51); R: CAC CCA GGG AAT TCA AAT GC(SEQ ID NO:52)); TGF-β1: (F: CCG CTT CTG CTC GCA CTC (SEQ ID NO: 78); R: GGT ACC TCC CCC TGG CTT(SEQ ID NO:79));TGF-β3: (F: GGG ACA GAT CTT GAG CAA GC(SEQ ID NO:56);R: TGC AGC CTT CCT CCC TCT C(SEQ ID NO:57)); IL-13: (F: ACT GCT CAG CTA CAC AAA GCA ACT(SEQ ID NO:80); R: TGA GAT GCC CAG GGA TGG T (SEQ ID NO:81));IL-4: (F: GGA GAT GGA TGT GCC AAA CG (SEQ ID NO:82); R: CGA GCT CAC TCT CTG TGG TGT T(SEQ ID NO:83)); FoxP3: (F: GGC CCT TCT CCA GGA CAG A(SEQ ID NO:84); R: GCT GAT CAT GGC TGG GTT GT(SEQ ID NO: 85)), with the L32 housekeeping gene included as a control. Statistical difference between vehicle and treatment groups was determined by the Mann-Whitney test. Differences in cytokine levels were evaluated by Student's t test. A P value ≤ 0.0.5 was considered significant.

### Passively induced RAE is treated with RTL401

SJL mice were injected with 15 million PLP139-151 specific T-cells. At onset of clinical signs of EAE (day 6), mice were treated with vehicle or RTL401 intravenously (Fig. 35A) for 5 days or RTL401 subcutaneously for days. Both the i.v. and s.c. routes of administration were very effective at suppressing clinical signs of disease. The vehicle treated mice (n=8) showed a cumulative disease index (CDI) of 46±10.5, whereas the i.v. treated mice (n=8) had a CDI of 19.5±5.1 compared to 21.4:1:9.9 in the s.c. treated mice. The peak disease score was also significantly lower for both i.v. and s.c. treated mice (4.5±0.9 for vehicle vs. 2.3±1.0 for s.c group vs. 2.1±0.4 for the i.v. group), p<0.01), representing only a minimal progression ofEAE in the RTL401-treated group prior to sustained reduction in clinical scores. The striking therapeutic effect of RTL401 was highly reproducible in a second experiment (CDI of 50.5±4.4 for the vehicle group vs. 18.9±7.9 for the RTL i.v, treated mice, n=7 for each group, p<0,01, Fig. 35B). The peak intensity of disease was also markedly suppressed following RTL i.v. treatment (4.9±0.2 in control vs. 2.4±0.8 in RTL treated mice).

### RTL treatment reduces inflammation in CNS

Histopathological examination of spinal cords taken on day 19 from vehicle-treated mice showed inflammatory lesions with dense and focal mononuclcar infiltrates (Fig. 40A). In contrast, there was a significant reduction of these lesions in day 19 spinal cords of RTL401-treated mice (Fig. 40B). Treatment with RTL401 also resulted in a 60% reduction in recovered mononuclear cells from brain tissue (2 x 10⁶ from vehicle vs. 8 X 10⁵ from RTL i.v.).

### RTL treatment preserves axons during EAE

Relapsing and progressive EAE results in axonal loss similar to that observed in MS. To evaluate the effects of RTL therapy on axonal survival during EAE, we assessed non-phosphorylated neurofilaments (NPNFL) by Western blots in spinal cords of RTL401 and vehicle-treated mice (i.v. route) on day 19 after T-ccll transfer. At onset of EAE when treatment began (day 6), the signal intensities of staining for NPNFL and the control marker, GAPDH, were unchanged in mice with a clinical score of 2.0 relative to asymptomatic naïve mice (Figs. 41A and 41 B). However, at the completion of treatment on day 19, vehicle-treated mice with a clinical score of 4.0 had a 60% increase in staining for NPNFL compared to naïve or pre-treated mice or RTL401-treated mice with a clinical score of 1.5 that showed no evidence of axonal loss (Figs. 41A and 41B). The results from this and two repeat experiments indicated that early treatment with RTL401 preserved neurofilaments and prevented further atonal loss via progression of EAE.

### Cytokine production following RTL treatment

Spleen, blood and brain were harvested from control, RTL i.v. and RTL s.c. treated mice on day 19. Mononuclear cells were isolated and then cultured in the presence of 10µg/ml PLP-139-151 peptide for 48h, and the culture supernatants were then assayed for the level of secreted cytokines. In splenocytes from vehicle-treated mice with EAE, the predominant cytokines induced by the PLP-139-151 peptide were IFN-γ and IL-13. Treatment with RTL401 i.v. and s.c. induced significant increases in the production of both Th1 (TNF-α, IFN-γ, IL-6, Fig. 37) and Th2 cytokines (IL-13, IL-4, IL-10, Fig. 36). In blood cells from mice with EAE, the cytokine pattern induced by the PLP-139-151 peptide was strikingly different, with predominant secretion of IL-6 and low to moderate levels of the remaining Th1 and Th2 cytokines (Figs. 36 and 37). Treatment with RTL401 i.v. and s.c. resulted in a 50-75% reduction in IL-6 and IL-4, but more than a 4-fold *increase* in IFN-γ and IL-13 production (Figs. 36 and 37). TNF-α and IL-10 levels were low initially and did not change after treatment with RTL401.

In brain mononuclear cells from mice with EAE, as in spleen, IFN-γ and IL-13 were the predominant cytokines induced by the PLP-139-151 peptide (Figs. 36 and 37). In contrast, treatment with RTL401 i.v. and s.c had a strong suppressive impact on both pro- and anti-inflammatory responses in the brain (Figs. 36 and 37), possibly due to the decrease in the number of infiltrating lymphocytes. VLA-4 and LFA-1 expression was strongly decreased in the blood and brain as well, also possibly due to decreased cellular infiltration.

### RTL401 pretreatment effects on PLP-139-151 specific T-cells in vitro

To demonstrate how RTLs affect *T-cell* responses using an *in vitro* model, PLP-139-151 peptide-specific *T-cells* used in the passive transfer experiments were incubated with 100 or 10µg/ml of RTL401 for 24h prior to the addition of irradiated splenocyte APCs and further incubation for 48h to assess cytokine secretion profiles. As controls, the *T-cells* were pre-incubated with medium or 10µg/ml free PLP-139-151 peptide, which represents the molar equivalent of peptide contained in the 100µg/ml dose of the RTL401 construct. As shown in Figs. 42A and 42B, PLP-139-151 specific T-cells pre-incubatcd with medium produced negligible levels (<50pg/ml) of both inflammatory (TNF-α, IFN-γ & IL-6) and non-inflammatory (IL-13, IL-10 & IL-4) cytokines. T-cells pre-incubated with free PLP-139-151 peptide had substantial increases in secretion of all cytokines, particularly IL-13 (1,000pg/ml) and to a lesser extent, TNF-α (400pg/ml). Pre-incubation of T-cells with 100µg/ml RL401 (neat) produced a striking increase in secretion of all cytokines assayed, again with a predominant effect on IL-13 (12,000µg/ml, a 12-fold increase) and a lesser effect on TNF-α (3,000pg/ml, a 7.5-fold increase). Pre-incubation of the T-cells with 10µg/ml RTL 01 (1:10) produced cytokine responses similar to 10µg/ml free PLP peptide, even though the concentration of bound peptide in the RTL401 preparation was only ~1µg/ml. These results demonstrate that pre-incubation of PLP-specific T-cells with RTL401 prior to addition of APC but without additional peptide induces significantly greater cytokine secretion than the molar equivalent of PLP peptide, resulting in predominant secretion of the Th2 cytokine, IL-13. Given the importance of IL-13 for protection against. EAE, these data strongly implicate IL-13 as a dominant regulatory cytokine involved in reduction of disease severity and progression mediated by RTL therapy.

### mRNA expression in RTL treated splenocytes and spinal cord

The gene expression of cytokine mRNA was assessed in the spleen and spinal cords of mice with passive EAE that were treated with RTL401 on day 19. In the spleens of RTL-treated mice, there was a significant increase in secreted proteins and proinflammatory cytokines, IFN-γ and TNF-α, but also a dramatic increase in Th2 cytokines, IL-13, IL-4 and IL-10 (Fig. 38), the Tr1 cytokine (IL-10), and TGF- β3, which was previously associated with protection against EAE (Matejuk *et al.,* 2003) in splenocytes from RTL401 i.v. treated mice (Fig. 38). No significant changes were detected in the expression of T-reg marker, Foxp3, or of TGF-β1, suggesting that neither T-reg cells nor Th3 cells were involved in the RTL treatment mechanism (Fig. 38). These changes were representative of data averaged from 3 separate experiments shown in Table 11.

**Table 11: Average fold-change I S.D. in real-time PCR message levels from spleen and spinal cord evaluated in 3 separate experiments from RTL401 vs. vehicle-treated mice.**

| | IL-13 | IL-4 | Foxp3 | IL-10 | IFN-γ | TNF-α | TGF-β1 | TGF-β3 |
|---|---|---|---|---|---|---|---|---|
| RTL401 spleen | 4.1 ± 0.9 | 1.4 ± 0.6 | 0.6 ± 0.3 | 2.2 ± 1.6 | 2.7 ± 1.0 | 1.7 ± 1.2 | 0.8 ± 0.1 | 1.6 ± 0.9 |
| RTL401 spinal cord | 3.1 ± 1.4 | 0.7 ± 0.5 | 0.3 ± 0.2 | 0,4 ± 0.2 | 1.2 ± 0.4 | 1.0 ± 0.9 | 0.4 ± 0.3 | 1.8 ± 1.0 |

In the spinal cords of RTL-treated mice, mRNA expression was increased 6-fold for IL-13 expression and 2-fold for IFN-γ expression (Fig. 39 and Table 11). mRNA for FoxP3, IL-10, and TGF-β1 were decreased, but mRNA for IL-4, TNF-α, and TGF-β3 were unchanged (Fig. 39).

The foregoing results indicate that RTL therapy was as effective at inhibiting passive EAE (induced by transfer of activated PLP-139-151 specific T-cells from immunized donors to naïve recipients) as had been demonstrated for active EAE. The RTL401 treatment effects were reflected by a more pronounced (60%) reduction of infiltrating mononuclear cells into the CNS, minimal inflammatory lesions in the spinal cord, and preservation of axons that were lost in vehicle-treated mice during progression of EAE. RTL401 therapy of passive EAE enhanced production of both pro-inflammatory and anti-inflammatory cytokines by PLP-139-151 specific T-cells, a profile that strongly resembled that observed during treatment of the acute phase of actively induced EAE. These findings are significant for evincing therapeutic efficacy of RTL compositions and methods of the invention, particularly in the context of clinical embodiments that do not employ CFA adjuvant, because passive EAE has an earlier onset and does not involve use of CFA adjuvant as the actively induced EAE did. Active induction of EAE with PLP-139-151/CFA induces a strong Th1 response in spleen due to the CFA. Thus, passive transfer without CFA is more representative of a disease condition, since CFA is not used.

RTL401 treatment induced a relatively modest increase in IFN-γ, and minor or no changes in TNF-α, IL-4 and IL-10 in blood. The effect of RTL therapy demonstrated here was to enhance secretion of both Th1 and Th2 cytokines in spleen. RTL treatment of passive EAE yielded an increase in IL-13 and IL-4, as well as IL-10, as determined by ELISA and CBA.

RT-PCR data in spleen show an increase in expression of both pro- and anti-inflammatory cytokines. However, it is important to note that FoxP3 (T-reg marker) and TGF-β1 (Th3 marker) are not changed, suggesting that an important mechanism of RTL therapy is through induction of Th2 cytokines.

RT-PCR in the spinal cord shows an increase in IL-13, but a decrease in FoxP3 and TGF-β1, suggesting that IL-13-producing Th2 cells are crossing into the spinal cord, while other cell types are not. Expression of IFN-γ is also increased in spinal cords of RTL-treated mice. However, because it is possible to induce EAE in IFN-γ knockout mice, the precise role of IFN-γ remains to be clarified.

The induction of IL-13 by RTL-targeted T-cells obtained from the spleen, blood, and CNS was strong and persistent. Additionally, pre-incubation with RTL401 *in vitro* primed PLP-139-151 specific T-cells to secrete high levels (>10µg/ml) of IL-13 upon addition of APC, without further exposure to PLP-139-15I peptide. RTL401 treatment enhanced the model of EAE with parallel secretion of lesser amounts of IFN-γ, with variable production of other cytokines. These results support a mechanism in which RTL therapy induces a cytokine switch in targeted T-cells, thus reprogramming pathogenic T-cells to produce anti-inflammatory cytokines that help to reduce inflammation in the CNS. Additionally, treatment with RTL401 at onset of EAE can prevent formation of non-phosphorylatcd neurofilaments, an indicator of axonal loss in CNS that markedly increased over a two-week period in vehicle-treated mice with EAE. The protective effect of RTL401 therapy on axonal survival has also been demonstrated in the model of active induction of EAE.

The strong induction of IL-13 by RTL401 may explain a number of observations related to therapy of EAE in SJL/J mice. IL-13 is an important regulatory cytokine in EAE, as demonstrated by antibody reversal of the EAE-protective function of a PLP-139-151 reactive T-cell clone stimulated with an altered peptide ligand (Young e*t al.,* 2000). It is secreted by activated Th2 cells and is known to possess regulatory functions as well as to mediate the pathogenesis of allergic inflammation. It shares many properties with IL-4, owing to the common expression of the IL-4α subunit in their respective receptors (Hershey *et al.,* 2003). Unlike the IL-4 receptor, the IL-13 receptor is expressed on many immune and tissue cells including B cells, basophils, eosinophils, mast cells, endothelial cells, fibroblasts, monocytes, macrophages, respiratory epithelial cells, and smooth muscle cells (Hershey *et. al.,* 2003), but *not* on T-cells (Zurawski *et al*., 1994). This receptor distribution promotes class switching to IgG4 and IgE and promotes hypersensitivity, a possible side effect in SJL/J mice of multiple i.v. injections of RTL401, for which anti-histamines are routinely administered. (Huan, 2004). However, it also precludes a direct IL-13 regulatory effect on pathogenic Th1 cells in EAE. Alternatively, IL-13 has been shown to inhibit, the production or pro-inflammatory factors produced by monocytes and macrophages, including cytokines (IL-1, IL-6, IL-8, TNF-α, and IL-12 (deVries *et al.,* 1994), but *not* IFN-γ), reactive oxygen and nitrogen intermediates, and prostaglandins (Hershey *et al.,* 2003). The cytokine profile of PLP-139-151 reactive mononuclcar cells in blood after RTL401 treatment recapitulates this effect, with strongly enhanced levels of IL-13 in combination with a marked decrease in IL-6, a highly inflammatory cytokine known to be essential for induction of EAE (Samoliova *et al.,* 1998). Analysis of blood from treated murine model subjects may be particularly representative of effects in human subjects. The pattern of cytokine secretion in murine test animals was different than in spleen. The dramatic increase in IL-13, accompanied by a decrease in IL-4 in the RTL treated mice indicates that RTL effects in the blood of treated patients can be assessed using diagnostic and management methods and compositions of the invention useful for monitoring IL-13 levels.

### Example 16

### Efficacy of RTL401 in Treating Myelin and Axonal Injuries in SJL Mice With Actively-Induced EAE.

### Animals

Female SJL mice were obtained from The Jackson Laboratory (Bar Harbor, ME) at 7-8 weeks of age. The mice were housed at the animal facility at Portland Veterans Affairs Medical Center in accordance with institutional guidelines.

### RTL construction and production

Methods for the design, cloning and expression of RTL401 were employed as described above in Example 14. The murine I-A^{s} β1α1 insert was then ligated into pET21d(+) vector and transformed into Nova blue *E. Coli* host (Novagen, Inc., Madison, WI) for positive colony selection and sequence verification. RTL400 and RTL401 plasmid constructs were then transformed into *E*. *Coli* strain BL21(DE3) expression host (Novagen, Inc., Madison, WI). The purification of proteins was conducted as described previously (Chang *et al*., 2001). The final yield of purified protein varied between 15 to 30 mg/L of bacterial culture.

### Inducation of EAE and RTL treatment

Active EAE was induced in female SJL mice (8 per group) by inoculation with 150µg PLP139-151 (ser) in 200µg Complete Freund's Adjuvant. Starting on day 20 after immunization, one group of mice received 5 daily i.v. injections of 100µg of RTL401 followed by 3 consecutive daily s.c. injections of 100 µg of RTL401 starting from Day 32. The mice were assessed daily for signs of EAE after inoculation according to the following scale after immunization: 0 = normal; 1 = limp tail or mild hind limb weakness; 2 = moderate hind limb weakness or mild ataxia; 3 = moderately severe hind limb weakness; 4 = severe hind limb weakness or mild forelimb weakness or moderate ataxia; 5 = paraplegia with no more than moderate forelimb weakness; and 6 = paraplegia with severe forelimb weakness or severe ataxia or moribund condition. Vehicle treated mice were sacrificed on Day 11 (onset of EAE), Day 20 (just past peak of EAE and Day 60 (conclusion of the experiment). As can be seen in Figure 44, administration of RTL401 improved the mean clinical score of the EAE induced SJL mice.

### Histopathology

At 20 (peak) or 60 days post-immunization, mice were deeply anesthetized with isofturane, heparinized, and perfused with 4 % paraformaldehyde in 0.1.M phosphate-balanced buffer (pH 7.4) for 10 seconds followed by 100 ml of 5% glutaraldehyde in 0,1 M phosphate-balanced buffer (pH 7.4) and then stored at 4°C for 24 hours. The spinal cords were dissected from the spinal columns and 1-2mm length sections from the cervical, thoracic, and lumbar cords were sampled, For histopathology with toluidine blue stain and electron microscopy analysis, tissues were placed in 0.1M phosphate-balance buffer (pH 7.4), postfixed with 1% osmium tetroxide (in 0.1 M phosphate buffer) for 2.5 hours, dehydrated in ethanol and embedded in plastic. Semithin sections (0.5µm) were stained with toluidine blue. The images were captured with a compound microscope equipped with a digital camera at 25x magnification. Thin sections, (80-90 nm) were stained with uranyl acetate and lead citrate and examined (by BGG) using a JOEL 100CX electron microscope.

### CNS Morphometric Analysis

Tissue sections were analyzed blinded to treatment status. The percentage of the spinal cord showing damage was determined in the mid-thoracic cord. Regions in the dorsal columns and the lateral/ventral white matter tracts containing damaged fibers were circumscribed on photomontages (final magnification x100) of the entire spinal cord. Damaged areas were labeled with red lines and measured using a Summasketch III (Summagraphics, Seymour CT) digitizing tablet and BIOQUANT software (R&M Biometrics, Nashville, TN). Measurements were also made of the total area (damaged and undamaged) of the dorsal column and the lateral/ventral columns. Cumulative percent lesion areas were calculated for each region and for the combined total damage of each region. As shown in Fig.45, myelin damage in the spinal cords of RTL401-treated mice was drastically reduced compared to vehicle-treated mice euthanized on Day 60. In addition, in comparing the degree of myelin damage in RTL-treatcd mice to those in vehicle-treated mice euthanized on Day 11, Day 20 and Day 60, it was found that RTL401-treatment reversed the development of myelin damage in EAE.

Figure 48 contains representative electron micrographs showing lesion areas in spinal cords from the EAE mice at the peak of the disease (sacrificed on Day 20). Figure 48A is a low power view (magnification x4,000) of a typical lesion area showing Wallerian-like axonal degeneration (white asterisks) and active demyclination (black asterisks). Figure 48B is a higher power view (magnification x8,000) showing infiltrating cells (white asterisks) and a remyelinating axon (black asterisk). Figure 48C shows active demyelination (black asterisk) and loss of the myelin sheath visible at a magnification of x6,700 with an inset view of the boxed region magnified x14,001). Figure 48D shows active demyelination (white asterisk), medium to large sized remyelinating axons (black asterisk), and several very small axons (arrowheads) at a magnification of x5,000. Figure 48B shows a large demyelinated axon (black asterisk) at a lower power magnification of x5,000. Figure 48F shows a higher power view (magnification x14,000) of a large remyelinating axon (black asterisk) and an end bulb of a dystrophic axon (white asterisk),

On Day 60, representative electron micrographs showing lesion areas in spinal cords from control mice (Figures 49 A, B and C) and RTL401 treated mice (Figures 49 D, E, and F) show increased remyelination in the RTL401 treated mice. Figure 49A is a low power (magnification x4,000) view of a typical lesion area showing marked continued Wallerian-like axonal degeneration (white asterisks) and demyelination (black asterisk) with few infiltrating cells or regenerating axonal sprouts. Figure 49B is a higher power view (magnification x8,000) showing Wallerian-like axonal degeneration (white asterisk) and active demyelination (black asterisk). Figure 49C is a higher power view (magnification x6,7000) of a large, remyelinaiing axon as shown by the thin myelinated sheath. As can be seen in Figure 49D-F there is much more remyelination in the RTL-treated mice. Figure 49D is a low power view (magnification x4,000) of a typical lesion area showing continued Wallerian-like axonal degeneration (white asterisk), including a dystrophic axon (arrow) and demyelinated and remyelinating axons (black asterisks). However, there are also prominent remyelinating axons and several small atonal sprouts (arrowheads). Figure 49E is a low power view (magnification x 5,000) of a large fiber (black asterisk) undergoing active demyelination (white asterisk) and three very small axons/regenerating sprouts (arrowheads). Figure 49F is a higher power view (magnification x14,000) of a medium-sized, remyelinating axon as shown by the relatively thin myelinated sheath (black asterisk).

At peak disease (On Day 20), there was considerable ongoing Wallerian-like axonal degeneration and large numbers of infiltrating cells (Fig, 48). However, normal recovery processes were able to compensate for the degree of damage as revealed by the presence of remyelinating axons and very small axons, most likely representing regenerating sprouts (Fig. 48 D and F). Untreated control animals show continued worsening of the disease process by 60 days, as shown by the increase in Wallerian-like axonal degeneration, continued axonal demyelination and the lack of axonal sprouts (Fig. 49 A-C). In contrast, the RTL-treated animals demonstrated reduced pathology from peak diseases on Day 60, as demonstrated by the decrease in continued degeneration, increased numbers of remyelinating axons and the presence of an increased number of axonal sprouts from peak diseases. (Fig. 49 D-F).

The electron microscopic observations indicate that RTL treatment prevents continued inflammation, reducing the degree of damage from peak diseases and enabling remyelination and axonal regeneration to occur. Remyelination and axonal sprouting were also observed in SJL mice given FK506 (at either an imnmnosuppressant or non-immunosuppressant dose) or a nonimmunosuppressant FK506 derivative (FK1706) (Gold, *et al*. 2004), indicating that these are common features of these models regardless of the underlying process leading to recovery from damage.

### RTL treatment preserves axons during EAE

Relapsing and progressive EAE results in axonal loss similar to that observed in MS.

To determine the level of axonal injury in EAE mice, spinal cords from 4 additional mice from each group were cut into thoracic and lumbar sections 60 days after immunization. Four of the thoracic cords were fixed and subjected to histochemical staining for total axons with SMI312, an antibody for neurofilaments, and for injured axons with SM132, an antibody for non-phosphorylated neurofilament (NPNFL, a marker of axonal injury). The infiltration of immune cells was analyzed with hematoxylin staining for nuclei. As depicted in Fig. 46A, axons were stained dark brown with SM1312 and infiltrating cells were stained bright blue with hematoxylin. Without therapeutic intervention, axonal staining was markedly reduced in the presence of infiltrating immune cells, resulting in severe loss of SMI312 staining in the outer region of white matter, where most neuroinflammation occurred. Axons in the spinal cord of RTL401-treated mice, to the contrary, were well preserved. Hematoxylin blue stained immune cells were much less frequent in the spinal cords of RTL-treated mice (Fig. 46A and C). The areas of axonal loss in the dorsal and lateral/ventral spinal cords of vehicle vs. RTL401 -treated mice were 31.8 % and 27.6 % vs. 3.5 % and 1.3 %, respectively. Statistical analyses indicated that RTL401-treatment significantly reversed the trend of progressive development of both axonal injury and neuroinflammation (Fig. 46B and C, Table II). As shown in Fig. 46D, the degree of axonal damage correlated significantly with neuroinflammation, as demonstrated by Pearson's correlation analysis (r=0,8636, P (two-tailed)=0.0003). This observation suggests that RTL401 may reduce CNS damage by reducing infiltration of immune cells into the spinal cord.

The degree of ongoing damage in EAE mice was also investigated by detecting the number of injured axons with SM132 staining. Different from SMI312, the SMI32 antibody specifically stains non-phosphorylated neurofilaments (NPNFL) that are present only in injured and demyelinated axons. This staining thus demonstrates the degree of ongoing damage rather than a reduction in axonal staining. As is shown in Fig. 47, RTL401-treated mice showed much less axonal injury and secondary demyelination in the white matter of the thoracic spinal cord. Similar to reduced axonal staining, the degree of ongoing axonal injury and demyelination appeared to be associated closely with inflammation. Additionally, immunoblotting For NPNFL with SMI32 demonstrated that axonal injury in both lumbar and thoracic spinal cord tissue from EAE mice was reduced on Day 00 after RTL401-treatment compared to samples from mice at the peak of EAE or in vehicle-treated mice evaluated on Day 60 (Fig. 41).

**Table 12: One-way ANOVA analysis of variance followed by Newman-kuels multiple comparison tests which documents statistically significant axonal loss reduction in RTL401 treated mice. *Comparison statistically significant.**

| ***Comparison*** | ***Dorsal*** | ***Lateral and Ventral*** |
|---|---|---|
| RTL401 vs. Vehicle | p < 0,01* | P < 0.05* |
| RTL401 vs. Peak | P < 0.05* | P < 0.05* |
| RTL401 vs. Onset | P > 0.05 | P > 0.05 |
| Onset vs. Vehicle | P < 0.05* | P > 0.05 |
| Onset vs. Peak | P > 0.05 | P > 0.05 |
| Peak vs. Vehicle | P > 0.05 | P > 0.05 |

**Table 13: One-way ANOVA analysis of variance followed by Newman-Kuels multiple comparison tests which documents statistically significant reduction in the number of injured axons in RTL401 treated mice. *Comparison statistically significant**

| ***Comparison*** | **P value** |
|---|---|
| RTL401 vs. Vehicle | P < 0.001* |
| RTL401 vs. Peak | P < 0.05* |
| RTL401 vs. Onset | P > 0.05 |
| Onset vs. Vehicle | P < 0.01* |
| Onset vs. Peak | P < 0.05* |
| Peak vs. Vehicle | P < 0.05* |

Tables 12 and 13 show that the effect of RTL on axonal loss reduction and reduction of the number of injured axons is statistically significant. Western blot results also demonstrated that the level of NPNFI, in the lumbar spinal cords of RTL4101 -treated mice was lower than those in vehicle-treated cuthanized on Day 20 (peak) and 60. Notably, many fewer immune cells were identified in the spinal cords of RTL-treated mice (Figs. 46 and 48), suggesting that RTL401 might reduce CNS damage through blocking the infiltration of immune cells.

In summary, it was shown that RTL401, when administered after the peak of relapsing EAE, a time-point corresponding to the established stage of MS dramatically reduced inflammation, demyelination and axonal loss and injury in the CNS. Moreover, treatment with RTL401 increased remyclination or regeneration of the myelin sheath and prevented further damage by infiltrating cells. Thus, 5 i.v. and 3 s.c. injection of RTL401 administered after the peak of disease ameliorated the severity of EAE and assoicated neuroaxonal damage. These results provide the necessary foundation for the clinical application of RTLs in MS patients to prevent or treat myelin and axonal injuries.

It is to be understood that the invention described herein is not limited to the particular formulations, methods, and materials disclosed herein as such formulations, methods, and materials may vary somewhat. It is also to be understood that the terminology employed herein is used for the purpose of describing particular exemplary embodiments only and is not intended to be limiting since the scope of the present invention will be limited only by the appended claims and equivalents thereof.

All publications and patents cited herein arc incorporated herein by reference for the purpose of describing and disclosing, for example, the materials and methodologies that are described in the publications, which might be used in connection with the presently described invention. The publications discussed above and throughout the text are provided solely for their disclosure prior to the filing date of the present application. Nothing herein is to be construed as an admission that the inventors are not entitled to antedate such disclosure by virtue of prior invention.

### References

Abbas, A.K., Murphy, K.M. Functional diversity of helper T lymphocytes. Nature 383:787(1996).
Alderuccio, Frank and Toh, Ban Hock. Spontaneous Autoimmune Gastritis in C3H/He Mice: A New Mouse Model for Gastric Autoimmunity. AJP 153(4): 1311-1317 (1998*)*.
Altman, J.D. et al. Phenotypic analysis of antigen-specific T lymphocytes. Science 274, 94-96(1996).
Arimilli, S., Cardoso, C., Mukku, P., Baichwal, V. & Nag, B. Refolding and reconstitution of functionally active complexes of human leukocyte antigen DR2 and myelin basic protein peptide from recombinant alpha and beta polypeptide chains. Journal of Biological Chemistry 270(2), 971-977 (1995).
Auffray et al. Isotypic and allotypic variation of human class 11 histocompatibility antigen alpha-chain genes. Nature 308 (5957), 327-333 (1984).
Ausubel et al, In Current Protocols in Molecular Biology, Greene Publishing Associates and Wiley-Intersciences (1987).
Bebo, B. F. Jr., Jeanette C. Schuster, Arthur A. Vandenbark, Halina Offner. 1999. Androgens alter the cytokine profile and reduce encephalitogenicity of myelin reactive T-cells. J. Immunol. 162:35.
Bebo, B. F., Jr, A. Fyfe-Johnson, K. Adlard, A. G. Beam, A. A. Vandenbark, H. Offner. Low-dose estrogen therapy ameliorates experimental autoimmune encephalomyelitis in two different inbred mouse strains. J. Immunol. 166:2080 (2001).
Benoist et al. The murine Ia alpha chains, E alpha and A alpha, show a surprising degree of sequence homology. Proc..Natl.Acad. Sci. U.S.A. 80 (2), 534-538 (1983).
Bolstad, Anne Isine Bolstad and Jonsson, Roland. Genetic aspects of Sjögren's syndrome. Arthritis Res, 4:353-359 (2002).
Boniface, J.J. & Davis, M.M. T-cell recognition of antigen. A process controlled by transient intermolecular interactions. Annals New York Acad. Sciences. 766, 62-69 (1995).
Bourdette, D.N. et al. Myelin basic protein specific T-cells in the CNS and lymph nodes of rats with EAE are different. J. Neurosci. Res. 30, 308-315 (1991).
Brogdon, J., Eckels, D.D., Davies, C., White, S. and Doyle, C. A site for CD4 binding in the beta-1 domain of the MHC class II protein HLA-DR1. J. Immunol. 161:5472 (1988).
Brown, J.H., Jardetzky, T.S., Gorga, J.C., Stern, L.J., Urban, R.G. & Strominger, J. L Three dimensional structure of the human class II histocompatibility antigen HLA-DR1. Nature 364, 33-39 (1993).
Browning et al., The HLA-A, B,C genotype of the class I negative cell line Daudi reveals novel HLA-A and -B alleles. Tissue Antigens 45 (3), 177-187 (1995).
Burley, S.K., and Petsko, G.A. Science 229,23-28 (1985).
Burrows, G.G. et al, Variation in H-2Kk peptide motif revealed by sequencing naturally processed peptides from T-cell hybridoma class I molecules. J. Neurosci. Res. 45, 803-811 (1996).
Burrows, G.G. et al. Multiple Class I Motifs Revealed by Sequencing Naturally Processed Peptides Eluted from Rat T-cell MHC Molecules. Rapid Communication. J. Neurosci. Res. 49, 107-116 (1997).
Burrows, G. G. Bebo, B. F. Jr., Adlard, K. L., Vandenbark, A. A., and Offner, H. J. Immunol. 161, 5987-5996 (1998).
Burrows, G. G., Chang, J.W., Bachinger, H-P., Bourdette, D.N., Wegmann, K.W., Offner, H.and Vandenbark A. A. Protein Engineering 12, 771-778 (1999).
Burrows, G. G., Adlard, K. L., Bebo, 13. F. Jr., Chang, J. W., Tenditnyy, K..,Vandenbark, A. A, and Offner, H. J. Immunol. 164, 6366-6371 (2000).
Burrows, G. G., Y. K. Chou, C. Wang, J. W. Chang, T. P. Finn, N. E. Culbertson, J. Kim, D. N. Bourdette, D. A. Lewinsohn, D. M. Lewinsohn. Rudimentary TCR signaling triggers default IL-10 secretion by human Th1 cells. J. Immunol. 167:4386, (2001).
Cammarota, G. et al. Identification of a CD4 binding site on the b2 domain of HLA-DR molecules. Nature 356, 799-801 (1992).
Caspi, R.R. et al. A new model of autoimmune disease: Experimental autoimmune uveorentinitis induced in mice with two different retinal antigens. J. Immunol. 140, 1490-1495 (1988).
Chang, J. W. et al., Design, engineering, and production of human recombinant T-cell receptor ligands derived from human leukocyte antigen DR2. J. Biol, Chem. 276:24170, (2001).
Chaurhary et al. Nature 339: 394-397 (1989).
Chen, G. C. and Yang, J. T. Anal. Letters 10,1195-1207 (1977).
Cobbold, S.P., Nash, J.A., Prospera, T.D. & Waldham, H. Therapy with monoclonal antibodies by elimination of T-cell subsets in vivo. Nature 312, 548-551 (1988).
Cobbold, S.P., Nash, J.A., Prospero, T.D. & Waldham, H. Therapy with monoclonal antibodies by elimination of T-cell subsets in vivo. Nature 31.2, 548-551 (1988).
Collins et al. Nature 371 (6498): 626-629 (1994).
Compton, L. A., and Johnson, W. C. Jr. Analytical Biochemistry 155, 155-67 (1986).
Connolly, M. L. Science 221, 709-13 (1983).
Connolly, M.L. Biopolymers 25, 1229-47 (1986).
Corr, M. et al. T-cell receptor-MHC class I peptide interactions: affinity, kinetics, and specificity. Science 265, 946-949 (1995).
Cua, D. J., H. Groux, D. R. Hinton, S. A. Stahlman, R. L. Coffman. Transgenic interleukin 10 prevents induction of experimental autoimmune encephalomyelitis. J. Exp. Med. 189:1005 (1999).
Cush, J.J. & Lipsky, P.E. Phenoytpie analysis of synovial tissue and peripheral blood lymphocytes isolated from patients with rheumatoid arthritis. Arthritis Rheum. 31, 1230-1238 (1988).
Das et al. Structure and nucleotide sequence of the heavy chain gene of HLA-DR. Proc. Natl. Acad. Sci. U.S.A. 80 (12), 3543-3547 (1983).
Davis, M.M., Boniface, J.J., Reich, Z., Lyons, D., Hampl, J., Arden, B., Chien, Y. Ligand recognition by alpha beta T-cell receptors. Annu. Rev. Immunol. 16:523 (1998).
Derman, A.I., Prinz, W.A., Belin, D. & Beckwith, J. Mutations that allow disulfide bond formation in the cytoplasm of Escherichia coli. Science 262, 1744-1747 (1993).
Desbarats, J., Freed, J.H., Campbell, P.A., & Newell, M.K. Fas (CD95) expression and death-mediating function are induced by CD4 cross-linking on CD4+ T-cells. PNAS 93, 11014-11018 (1996).
deVries, J. E. The role of IL-13 and its receptor in allergy and inflammatory responses. J Allergy Clin Immunol 102:165 (1998).
Eck, M. J., Atwell, S. K., Shoelson, S. E., and Harrison, S. C. Structure of the regulatory domains of the Sre-family tyrosine kinase Lck. Nature 368:764 (1994).
Edwards et al. Science 276 (5320): 1868-1871 (1997).
Elder, M. E., Lin, D., Clever, J., Chan, A. C., Hope, T. J., Weiss, A., and Parslow, T. G. Human severe combined immunodeficiency due to a defect in ZAP-70, a T-cell tyrosine kinase. Science 264:1596 (1994).
et al, Sequence analysis and structure-function correlations of murine q, k, u, s, and f haplotype I-A beta cDNA clones. Proc. Natl. Acad. Sci. U.S.A. 83 (11), 3594-3598 (1986),
Fearon, D.T., Locksley, R.M. The instructive role of innate immunity in the acquired immune response. Science 272:50 (1996).
Ferrin, T.E., Huang, C.C., Jarvis, L.E. & Langridge, R. The MIDAS display system. J. Mol. Graphics 6, 13-27 (1988).
Fleury et al., CELL 66, 1037-1049 (1991).
Fremont, et al. Structures of an MHC class II molecule with covalently bound single peptides. Science 272, 1001-1004 (1996).
Fremont, D. H., D. Monnaie, C. A. Nelson, W. A. Hendrickson, E. R. Unanue. Crystal structure of I-Ak in complex with a dominant epitope of lysozyme. Immunity 8:305 (1998).
Fujii, Y. et al. Experimiental autoimmune adrenalitis: a murine model for Addison's disease. Autoimmunity 12(1):47-52 (1992).
Germain, R.N. Major histocompatibility complex-dependent antigen processing and peptide presentation: providing ligands for the clonal activation of T lymphocytes. Cell 76:287 (1994).
Gold, D. P., H. Offner, D. Sun, S. Wiley, A. A. Vandenbark and D. B. Wilson. Analysis of T-cell receptor chains in Lewis rats with experimental autoimmune encephalomyelitis: Conserved complementarity determining region 3. J. Exp. Med. 174:1467 (1991).
Golding, H., J. McCluskey, T. I. Munitz, R. N. Germain, D. H. Margulies and A. Singer. T-cell recognition of a chimaeric class II/class I MHC molecule and the role of L3T4. Nature, 317:425(1985).
Gordon, E. J., K. J. Myers, J. P. Dougherty, H. Rosen, Y. Ron. Both anti-GD11a (LFA-1) and anti-CD11b (MAC-1) therapy delay the onset and diminish the severity of experimental autoimmune encephalomyelitis. J. Neuroimmunol. 62:153 (1995).
Govaerts, A. et al. HLA and multiple sclerosis: population and family studies. Tissue Antigens 25, 187-199 (1985).
Harlow and Lane (1988). Antibodies, A Laboratory Manual, Cold Spring Harbor Laboratory, New York.
Hashim, G.A., Day, E.D., Fredane, L., Intintola, P., and Carvalho, E. Biological activity of region 65 to 102 of the myelin basic protein. J. Neurosci. Res. 16, 467-478 (1986).
He, X., C. Radu, J. Sidney, A. Sete, E. S. Ward, K. C. Garcia. Structural snapshot of aberrant antigen presentation linked to autoimmunity: the immunodominant epitope of MBP complexed with I-Au. Immunity 17:83 (2002).
Heiden, W., Moeckel, G., and Brickmann, J. J. Comp.-Aided Mol. Design 7,503-514. (1993)
Hemmer, B., Fleckenstein, B. T., Vergelli, M., Jung, G., McFarland, H., Martin, R. and Wiesmüller, K. H. J. Exp. Med. 185, 1651-1660 (1997).
Hershey, G. K. K, 2003. IL-13 receptors and signaling pathways: An evolving web. J Allergy Clin Immunol 111:677 (2003).
Housset, D., Habersetzer-Rochat, C., Astier, J.P. & Fontecilla-Camps, J.C. Crystal structure of toxin 11 from the scorpion Androctonus Australis Hector refined at 1.3 angstroms resolution. J. Mol. Biol. 238, 88 (1994).
Howell, M.D. et al. Vaccination against experimental allergic encephalomyelitis with T-cell receptor peptides. Science 246,668-670 (1989).
Huan, J., S. Subramanian, R. Jones, C. Rich, J. Link, J. Mooney, D. N. Bourdette, A. A. Vandenbark, G. G. Burrows, and H. Offner. Monomeric recombinant TCR ligand reduces relapse rate and severity of experimental autoimmune encephalomyelitis in SJL/J mice through cytokine switch. J Immunol 172:4556 (2004).
Huang, B., Yachou, A., Fleury, S., Hendrickson, W. & Sekaly, R. Analysis of the contact sites on the CD4 molecule with class II MHC molecule. J. Immunol. 158,216-225 (1997).
Ide T., et al. An experimental animal model of primary biliary cirrhosis induced by lipopolysaccharide and pyruvate dehydrogenase. Kurume Med. J. 43(3):185-8 (1996).
Innis et al. PCR Protocols, A Guide to Methods and Applications; Innis et al. (eds.), Academic Press, Inc., San Diego, California (1990).
Ito, A., A. Matejuk, C. Hopke, H. Drought, J. Dwyer, A. Zamora, S. Subramanian, A. A. Vandenbark, H. Offner. Transfer of severe experimental autoimmune encephalomyelitis by IL-12- and IL-18-potentiated T-cells is estrogen sensitive. J. Immunol. 170:4802 (2003).
Jameson, B.A., McDonnel, J.M., Marini, J.C. & Korngold, R. A rationally designed CD4 analogue inhibits experimental allergic encephalomyelitis. Nature 368, 744-746 (1994).
Janes, P. W., Ley, S.C., Magee, A.I. Aggregation of lipid rafts accompanies signaling via the T-cell antigen receptor. J Cell Biol. 14: 447 (1999).
Janeway, C.A. Jr. and Bottomly, K. Cell 76, 275-285 (1994).
Janoway & Travers. Immunobiology: the immune system in health and disease, Current Biology Ltd./Garland Publishing, Inc. New York (1997).
Janeway, C.A., Jr. Presidential Address to The American Association of Immunologists. The road less traveled by: the role of innate immunity in the adaptive immune response. J. Immunol. 161:539(1998).
Kanellis, John et al., Modulation of Inflammation by Slit Protein In vivo in Experimental Crescentic Glomerulonephritis American Journal of Pathology, 165,(1): 341-352 ( 2004).
Karnitz, L., Sutor, S. L., Torigoe, T., Reed, J. C., Bell, M. P., McKean, D. J., Leibson, P. J., and Abraham, R. T. Effects of p561ck deficiency on the growth and cytolytic effector function of an interleukin-2-dependent cytotoxic T-cell line, Mol. Cell. Biol. 12:4521. (1992)
Kato et al.,. Molecular analysis of HLA-B39 subtypes. Immunogenetics 37 (3), 212-216 (1993).
Kelly & Trowsciale. Complete nucleotide sequence of a functional HLA-DP beta gene and the region between the DP beta 1 and DP alpha 1 genes: comparison of the 5' ends of HLA class II genes Nucleic Acids Res. 13 (5), 1607-1621 (1985).
Kersh, E. N., Kersh, G. J., Allen, P. M. Partially phosphorylated T-cell receptor zeta molecules can inhibit T-cell activation. J. Exp. Med. 190:1627 (1999).
King, J. and Leimmli, U.K.. Bacteriophage T4 tail assembly: structural proteins and their genetic identification. J. Mol. Biol, 75, 315-337 (1973).
König, R., Shen, X. & Germain, R.N. Involvement of both major histocompadbility complex class II and ß chains in CD4 function indicates a role for ordered oligomerization in T-cell activation, J. Exp. Med. 182, 779-787 (1995).
König, R., Huang, L.Y. & Germain, R. MHC class II interaction with CD4 mediated by a region analogous to the MHC class I binding site for CD8. Nature 356, 796-798 (1992).
Kozono, H., White, J., Clements, J., Marrack, P. & Kappler, J. Production of soluble MHC class II proteins with covalently bound single peptides. Nature 369, 151-154 (1994).
Kress et al., Alternative RNA splicing in expression of the H-2K gene. Nature 306 (5943), 602-604 (1983).
Krishnan, VV., et al. Effect ofmysoin B on guinea pig muscles-light microscopic and immunologic aspects. Indian J. Exp. Biol. 32(6):405-8 (1994).
Kyle, V., Coughlan, R.J., Tighe, H., Waldmann, H., and Hazleman, B.L. Beneficial effect of monoclonal antibody to interleukin 2 receptor on activated T-cells in rheumatoid arthritis. Ann. Rheum. Dis. 48:428(1989).
Larhammar et al. Exon-intron organization and complete nucleotide sequence of a human major histocompatibility antigen DC beta gene. Proc. Natl. Acad. Sci. U.S.A. 80 (23), 7313-7317 (1983).
Lawrence et al. The genomic organisation and nucleotide sequence of the HLA-SB(DP) alpha gene Nucleic Acids Res. 13 (20), 7515-7528 (1985).
Lehmann, P. V., T. Forsthuber, A. Miller., E. E. Sercarz. Spreading of T-cell autoimmunity to cryptic determinants of an autoantigen. Nature 358:155 (1992).
Li, W., Whaley, C.D., Mondino, A. and Mueller, D.L. Blocked Signal Transduction to the ERK and JNK Protein Kinases in Anergic CD4+ T-cells Science 271:1272 (1996).
Li, Y., Li, H., Martin, R., and Manuzza, R. A. J. Mol. Biol. 304, 177-188 (2000).
MacDonald, H.R., Casanova, J.L., Maryanski, J.L., Corottini, J.C. Oligoolonal expansion of major histocompatibility complex class I-restricted cytolytic T lymphocytes during a primary immune response in vivo: direct monitoring by flow cytometry and polymerase chain reaction. J. Exp.Med. 177, 1487-1492 (1993).
Madden, D.R., Gorga, Strominger, J.L. & Wiley, D.C. The structure of HLA-B27 reveals nonamer self-peptides bound in an extended conformation. Nature 353, 321-325 (1991).
Martenson, R.E. Myelin basic protein speciation in Experimental Allergic Encephalomyelitis: A useful Model for Multiple Sclerosis. Alan R. Liss, Inc., 150 Fifth Avenue, New York, NY 10011. pp. 511-521 (1984).
Martin R, Utz U, Coligan JE, Richert: JR, Flerlage M, Robinson E, Stone R, Biddison WE, MeFarlin DE, McFarland HF. Diversity in fine specificity and receptor usage of the human CD4+ cytotoxic T-cell response specific for the immunodominant myelin basic protein peptide 87-106. J Immunol. 148:1359 (1992).
Matejuk, A., A. A. Vandenbark, G. G. Burrows, B. F. Bebo, Jr, H. Offner. Reduced chemokine and chemokine receptor expression in spinal cords of TCR BV8S2 transgenic mice protected against experimental autoimmune encephalomyelitis with BV8S2 protein. J. Imnunol. 164:3924 (2000).
Matejuk, A., K. Adlard, A. Zamora, M. Silverman, A. A. Vandenbark, H. Offner. 17b-estradiol inhibits cytokine, chemokine, and chemokine receptor mRNA expression in the central nervous system of female mice with experimental encephalomyelitis. J. Neurosci. Res. 65:529 (2001).
Matejuk, A., J. Dwyer, C. Hopke, A. A. Vandenbark, and H. Offner, Differential expression of TGF-ß3 is associated with protection against experimental autoimmune encephalomyelitis. Cytokine 25:45 (2003).
Matejuk, A., J. Dwyer, C. Hopke, A. A. Vandenbark, H. Offner. Differential expression of TCiF-β3 is associated with protection against experimental autoimmune encephalomyelitis. Cytokine 25:45 (2004).
Matsubara, S., et al. Experimental allergic myositis in SJL/J mouse. Reappraisal of immune reaction based on changes after single immunization. J. Neuroimmunol. 119(2):223-30 (2001).
Matsui, K., Boniface, J.J., Steffner, P., Reay, P.A., Davis, M.M. Kinetics of T-cell receptor binding to peptide/I-EK complexes: correlation of the dissociation rate with T-cell responsiveness. Proc. Natl. Acad. Sci. U.S.A. 91, 12862-12866 (1994).
Matsui, K. et al. Low affinity interaction of peptide-MHC complexes with T-cell receptor. Science 254, 1788-1791 (1991).
McHeyzer, M.G., Davis, M.M. Antigen specific development of primary and memory T-cells in vivo. Science 268, 106-111 (1995).
Mege, D., Di Bartolo, V., Germain, B., Tuosto, L., Michel, F., and Acuto, O. Mutation of Tyrosines 492/493 in the Kinase Domain of ZAP-70 Affects Multiple T-cell Receptor Signaling Pathways J. Biol. Chem. 271:32644 (1996).
Miltenyi et al. Cytometry 11: 231-238 (1990).
Mitragotri et al. Pharmaceutical Research 13 (3): 411-20 (1996).
Moebius, U., Pallai, P., Harrison, S.C. & Reinherz, E.L. Delineation of an extended surface contact area on human CD4 involved in class II MHC binding. PNAS 90, 8259-8263 (1993).
Moon, Changjong and Shin, Taekyun, Increased expression of osteopontin in the spinal cords of Lewis rats with experimental autoimmune neuritis. J. Vet. Sci. 5(4), 289-293 (2004)
Moore et al. DNA sequence of a gene encoding a BALF/c mouse Ld transplantation antigen. Science 215 (4533), 679-682 (1982).
Mosmann, T.R., Cherwinski, H., bound, M.W., Giedlin, M.A., Coffman, R.L. Two types of murine helper T-cell clones. I. Definition according to profiles of lymphokine activities and secreted proteins. J. Immunol. 136:2348 (1986).
Mowatt, McI. Prostaglandins and the Induction of Food Sensitivity Enteropathy. Gut 46:154-155(2000).
Mukai, T., M. Iwawake, P. Gao, M. Tomura, Y. Yashiro-Ohtani, S. Ono, M. Murai, K. Matsushima, M. Durimoto, M. Kogo. IL-12 plays a pivotal role in LFA-1-mcdiated T-cell adhesiveness by up-regulation of CCR5 expression. J. Leukocyte Biol. 70:422 (2000).
Murthy, V. L., and Stern, L. J. Structure 5, 1385-1396 (1997).
Nag, B., Deshpande, S.V., Sharma, S.D., & Clark, B.R. Cloned T-cells internalize peptide from bound complexes of peptide and purified class II major histocompatibility complex antigen. J. Biol. Chem. 268, 14360-14366 (1993).
Nag, B., Kendrick, T., Arimilli, S., Yu, S.C., & Sriram, S. Soluble MHC II-peptide complexes induce antigen-specific apoptosis in T-cells. Cell. Immunol. 170, 25-33 (1996).
Nag, B., Passmore, D., Kendrick, T., Bhayani, H., & Sharma, S.D. N-linked oligosaccharides of murine major histocompatibility complex class II molecule. Role in antigenic peptide binding, T-cell recognition, and clonal nonresponsiveness. J. Biol. Chem. 267,22624-22629 (1992).
Nag B., Arimilli S., Mukku P.V. &. Astafieva, I. Functionally active recombinant alpha and beta chain-peptide complexes of human major histocompatibility class II molecules. Journal of Biological Chemistry 271(17), 10413 -10418 (1996).
Nag B. et al. Stimulation ofT-cells by antigenic peptide complexed with isolated chains of major histocompatibility complex class II molecules. Proceedings of the National Academy of Sciences of the United States of America 90(4), 1604-1608 (1993).
Negulescu, P.A., T.B. Krasieva, A. Khan, H.H. Kerschbaum, and M.D. Cahalan. Polarity of T-cell shape, motility, and sensitivity to antigen. Immunity 4:421 (1996).
Nicolle, M.W. et al. Specific tolerance to an acetylcholine receptor epitope induced in vitro in myasthenia gravis CD4+ lymphocytes by soluble major histocompatibility complex class II-peptide complexes. J. Clin Invest. 93, 1361-1369 (1994).
Ng, H.P. et al. Development of a Murine Model of Autoimmune Thyroiditis Induced with Homologous Mouse Thyroid Peroxidase. Endocrinology 145(2):809-816 (2004).
Ohman, L. et al. Acellular Bordetella pertussis vaccine enhances mucosal interleukin-10 production, induces apoptosis of activated Th1 cells and attenuates colitis in Galphai-2 deficient mice. Clin. Exp. Immunol. 141(1):37-46 (2005).
Oksenberg, J.R. et al. Selection of T-cell receptor V-D-J gene rearrangements with specificity for a MBP peptide in brain lesions of MS. Nature 362, 68-70 (1993).
Oldenborg, Per-Arne et al. Lethal autoimmune hemolytic anemia in CD47-deficient nonobese diabetic (NOD) mice. Blood 99(10):3500-3504. (2002).
Olerup, O., and Zetterquist, H. HLA-DR typing by PCR amplification with sequence specific primers (PCR-SSP) in two hours: An alternative to serological DR typing in clinical practice including donor-recipient matching in cadaveric transplantation. Tissue Antigens 39:225 (1992).
Ota, K. et al. T-cell recognition of an immunodominant myelin basic protein epitope in multiple sclerosis. Nature 346,183-187(1990).
Paterson, P. Y. J. Chron. Dis. 26,119-26 (1973).
Paterson, P.Y. Multiple sclerosis: An immunologic reassessment, J. Chron. Dis. 26, 119-125 (1981).
Pitt, D., P. Werner, and C. S. Raine. Glutamate excitotoxicity in a model of multiple sclerosis. Nature Med. 6:67 (2000).
Ploegh, H. and Benaroch, P. Nature 364, 16-17 (1993).
Ploix, C., D. Lo, M. J. Carson. A ligand for the chemokine receptor CCR7 can influence the homeostatic proliferation of CD4 T-cells and progression of autoimmunity. J. Immunol. 167:6724 (2001).
Provencher, S.W. and Glockner, J. Biochemistry 20, 33-37 (1981).
Qu, WM, et al. A novel autoimmune pancreatitis model in MRL mice treated with polyinosinic:polycytidylic acid. Clin. Exp. Immunol. 129(1):27-34 (2002).
Quill, H. & Schwartz, R., H. Stimulation of normal inducer T-cell clones with antigen presented by purified Ia molecules in planer lipid membranes: specific induction of a long-lived state of proliferative nonresponsiveness. J. Immunol. 138, 3704-3712 (1987).
Redpath, S., Alam, S.M. Lin, C.M., O'Rourke, A.M., and Gascoigne, N.R. Cutting edge: Trimolecular interaction of TCR with MHC class II and bacterial superantigen shows a similar affinity to MHC:peptide ligands. J. Immunol. 163:6 (1999).
Reiner, S.L., Wang, Z.E., Hatam, F., Scott, P., Locksley, R.M. TH1 and TH2 cell antigen receptors in experimental leishmaniasis. Science 259, 1457-1460 (1993).
Ren, R., Mayer, B. J., Cicchetti, P., and Baltimore, D. Identification of a ten-amino acid proline-rich SH3 binding site. Science 259:1157 (1993).
Rhode, P.R. et al, Single-chain MHC class II molecules induce T-cell activation and apoptosis. J. Immunol. 157, 4885-4891 (1996).
Riehl, T. et al. TNFR1 mediates the radioprotective effects of Lipopolysaccharides in the mouse intestine. Am J Physiol Gastrointest Liver Physiol 286: G166-G17 (2004).
Robertson, Morag et al., Neutralizing Tumor Necrosis Factor-α Activity Suppresses Activation of Infiltrating Macrophages in Experimental Autoimmune Uveoretinitis. IOVS, 44(7):3034-3041 (2003).
Romagnani, P. CCR8 with I-309/CCL1, and CRTH2 with prostaglandin D2 play a critical role in the allergen-induced recruitment of Th2 cells in the target tissues of allergic inflammation. Mol. Immunol. 38:881 (2002).
Sallusto, F., D. Lenig, C.R. Mackay, A. Lanzavecchia. Flexible programs of chemokine receptor expression on human polarized T helper 1 and 2 lymphocytes. J. Exp. Med. 187:875 (1998).
Sambrook et al. In Molecular Cloning: A Laboratory Manual, Cold Spring Harbor, New York. (1989).
Samoilova, E. B., J. L. Horton, B. Milliard, T.-S. T. Liu, and Y. Chen. IL-6-deficient mice are resistant to experimental autoimmune encephalomyelitis: Roles of IL-6 in the activation and differentiation of autoreactive T-cells. J Immunol 161:6480 (1998). Scliaefiei', H.J. and Weber, M.J. Mitogen-activated protein kinases: specific messages from ubiquitous messengers. Mol. Cell. Biol. 19:2435 (1999).
Schafer, P. H., Pierce, S. K. and Jardetzky T. S. Seminars in Immunology 7,389-98 (1995).
Schepart et al. The nucleotide sequence and comparative analysis of the H-2Dp class I H-2 gene. J. Immunol. 136 (9), 3489-3495 (1986).
Schutyser, E., S. Struyf, J. VanDamme. The CC chemokine CCL20 and its receptor CCR6. Cytokine Growth Factor Rev. 14:409 (2003).
Schwartz, R. H. Models ofT-cell anergy: is there a common molecular mechanism? J. Exp. Med. 184, 1-8 (1996).
Scott, C. A., P. A. Peterson, L. Teyton, 1. A. Wilson. Crystal structures of two I-Ad-peptide complexes reveal that high affinity can be achieved without large anchor residues. Immunity 8:319 (1998).
Seay, A.R. et al. Experimenal viral polymositis:age dependency and immune responses to Ross River virus infection in mice. Neurology 31(6):656-60 (1981).
Service et al. Science 277(5330): 1199-1200 (1997).
Sharma, S.D. et al. Antigen-specific therapy of experimental allergic encephalomyelitis by soluble class II major histocompatibility complex-peptide complexes. PNAS 88:11465-11469 (1991).
Shan-Lancaster, J., Shaw, A. S., Rothbard, J. B., Allen, P. M. Partial T-cell signaling: Altered phospho-zeta and lack of Zap70 recruitment in APL-indiiced T-cell anergy. Cell 79:913 (1994).
Smith, K. J., Pyrdol, J., Gauthier, L., Wiley, D. C., and Wucherpfennig, K. W. J. Exp. Med. 188, 1511-1520 (1998).
Spack, E.G. et al. Induction of tolerance in experimental autoimmune myasthenia gravis with solubilized MHC class II: acetylcholine receptor complexes. J. Autoimmun. 8, 787-807(1995).
Srinivas et al., Pharmacokinetics and pharmacodynamics of CTLA4Ig (BMS-188667), a novel immunosuppressive agent, in monkeys following multiple doses. J. Pharm. Sci. 85(1):1-4, (1996)).
Steinle et al., Isolation and characterization of a genomic HLA-Cw6 clone. Tissue Antigens 39(3), 134-137 (1992).
Steinman, L. Autoimmune disease. Sci. Am, 269, 106-114 (1993).
Studier, F. W., A. H. Rosenberg, J. J. Dunn, and J. W. Dubendorff. Use of T7 RNA polymerase to direct expression of cloned genes. Methods Enzymol. 185:60 (1990).
Swanborg, R.H. Autoimmune effector cells. V. A monoclonal antibody specific for rat helper T lymphocytes inhibits adoptive transfer of auto-immune encephalomyelitis. J. Immunol. 130, 1503-1505(1983).
Syha, J., Henkes, W. & Reske, K. Complete cDNA sequence coding for the MHC class II RTI.B -chain of the Lewis rat. Nuc. Acids. Res. 17(10), 3985 (1989).
Syha-Jedelhauser, J., Wendling, U. & Reske, K. Complete coding nucleotide sequence of cDNA for the Class II RTLB ß-chain of the Lewis rat. Biochim. Biophys. Acta 1089, 414-416 (1991).
Sykulev, Y. et al. Kinetics and affinity of reactions between an antigen-specific T-cell receptor and peptide-MHC complexes. Immunity 1, 15-22 (1994).
ten Bosch, G.J., Toornvliet, A.C., Friede, T., Melief, C.J., and Leeksma, O.C. Recognition of peptides corresponding to the joining region of p210BCR-ABL protein by human T-cells Leukemia 9:1344 (1995).
Theien, B. E., C. L. Vanderlugt, T. N. Eagar, G. Nickerson-Nutter, R. Nazareno, V. K. Kuchroo, S. D. Miller. Discordant effects of anti-VLA-4 treatment before and after onset of relapsing experimental autoimmune encephalomyelitis. J Clin. Invest. 107:995 (2001).
Thompson, D. and Larson, G. Western blots using stained protein gels. Biotechniques 12, 656-658 (1992).
Tonnell et al. Do beta: a new beta chain gene in HLA-D with a distinct regulation of expression. EMBO J. 4 (11), 2839-2847 (1985).
Vandenbark, A.A., Vainiene, M., Celnik, B., Hashim, G.A., Buenafe, A.C. & Offner, H. Definition of encephalitogenic and immunodominant epitopes of guinea pig myelin basic protein (Gp-BP) in Lewis rats tolerized neonatally with Gp-BP peptides. J. Immunol. 153, 852-861 (1994).
Vandenbark, A.A., Hashim, G. & Offner, H. Immunization, with a synthetic T-cell receptor V-region peptide protects against experimental autoimmune encephalomyelitis. Nature 341, 541-544 (1989).
Vandenbark, A.A., Gill, T. & Offner, H. A myelin basic protein specific T lymphocyte line which mediates EAE. J. Imnumol. 135, 223-228 (1985).
Vandenbark, A. A., C. Rich, J. Mooney, A. Zamora, C. Wang, J. Huan, L. Fugger, H. Offner, R. Jones, G. G. Burrows. Recombinant TCR ligand induces tolerance to MOG 35-55 peptide and reverses clinical and histological signs of chronic experimental autoimmune encephalomyelitis in HLA-DR2 transgenic mice. J. Immunol. 171:127 (2003).
Vanderlugt, C. L., S. D. Miller. Epitope spreading in immune-mediated diseases: implications for immunotherapy. Nat. Rev. Immunol. 2:85 (2003).
Van Oers, N., Killeen, N., and Weiss, A. Lck regulates the tyrosine phosphorylation of the T-cell receptor subunits and ZAP-70 in murine thymocytes J. Exp. Med. 183:1053 (1996).
Veillette, A., Bookman, M.A., Horak, E.M. & Bolen, J.B. The CD4 and CDS T-cell surface antigens are associated with the internal membrane tyrosine-protein kinase p56lck. Cell 55, 301-308 (1988).
Vilanova, M., Tavares, D., Ferreira, P., Oliveira, L., Nobrega, A., Appelberg, R., and Arala-Chaves M. Role of monocytes in the up-regulation of the early activation marker CD69 on B and T murine lymphocytes induced by microbial mitogenis. Scand J. Immunol. 43:155(1996).
Walker, P.R., Ohteki, T., Lopez, J.A., MacDonald, H.R., Maryanski, J.L. Distinct phenotypcs of antigen-selected CD8 T-cells emerge at different stages of an in vivo immune response. J. Immunol. 155, 3443-3452 (1995).
Walter et al., Sequence, expression, and mapping of rat Mhc class Ib gene. Immunogenetics 39 (5), 351-354 (1994).
Water et al., Genomic organization and sequence of the rat major histocompatibility complex class Ia gene RT1.Au Immunogenetics 41 (5), 332 (1995).
Wang, C., J. L., Mooney, R. Meza-Romero, Y. K. Chou, J. Huan, A. A. Vandenbark, H. Offner, G. G. Burrows. Recombinant TCR ligand induces early TCR signaling and a unique pattern of downstream activation, J. Immunol. 171:1934. (2003)
Wegmann KW, Zhao W., Griffin AC., and Hickey WF. Identification of myocarditogenic peptides derived from cardiac myosin capable of inducing experimental allergic myocarditis in the Lewis rat. The utility of a class II binding motif in selecting self-reactive peptides. J. Immunol. 153(2), 892-900 (1994).
Weinberg, A. D. et al. TGF-ß enhances the in vivo effector function and memory phenotype of Ag-specific T helper cells in EAE. J. Immunol. 148, 2109-2117 (1992).
Weinberg, A. D, et al. Target organ specific upregulation of the MRC OX-40 marker and selective production of Th1 lymphokine mRNA by encephalitogenic T helper cells isolated from the spinal cord of rats with experimental autoimmune encephalomyelitis. J. Immunol. 152,4712-5721 (1994).
Weinberg, A.D., Bourdette, D.N., Sullivan, T.J., Lemon, M., Wallin, J.J., Maziarz, R., Davey, M., Palida, F., Godfrey, W., Engleman, E., Fulton, R.J., Offner, H., and Vandenbark, A.A. Selective depletion of myelin-reactive T-cells with the anti-OX-40 antibody ameliorates autoimmune encephalomyelitis. Nature Medicine 2:183 (1996).
Weiner, H.L. et al. Double-blind pilot trial of oral tolerization with myelin antigens in MS. Science 259, 1321-1324 (1993).
White, J., M. Blackman, J. Bill, J. Kappler, P. Barrack, D. P. Gold and W. Born. Two better cell lines for making hybridomas expressing specific T-cell receptors. J. Immunol. 143:1822 (1989).
Wülfing, C., Rabinowitz, J.D., Beeson, C., Sjaastad, M.D., McConnell, H.M. and Davis, M.M., Kinetics and Extent of T-cell Activation as Measured with the Calcium Signal. J. Exp. Med. 185:1815(1997).
Yednock, T.A. et al. Prevention of experimental autoimmune encephalomyelitis by antibodies against 4/ß1 integrin. Nature 356, 63 (1992).
Young, D. A., L. D. Lowe, S. S. Booth, M. J. Whitters, L. Nicholson, V. K. Kuchroo, and M. Collins. IL-4, IL-10, IL-13, and TGF-B from an altered peptide ligand-specific Th2 cell clone down-regulate adoptive transfer of experimental autoimmune encephalomyciitis. J Immunol 164:3563 (2000).
Zhao, B., Carson, M., Ealick, S. E. & Bugg, C.E. Structure of scorpion toxin variant-3 at 1.2 angstroms resolution. J. Mol. Biol. 227, 239-252 (1992).
Zinn-Justin, S., Guenneugues, M., Drakopoulou, Gilquin, B., Vita, C. & Menez, A. Transfer of a beta-hairpin from the functional site of snake curaremimetic toxins to the alpha/beta scaffold of scorpion toxins: Three-dimensional solution structure of the chimeric protein. Biochemistry 35(26): 8535-43 (1996).
Zurawski, G., and J. E. deVries. Interleukin 13, an interleukin 4-like cytokine that acts on monocytes and B cells, but not on T-cells. Immunol Today 15:19(1994).

The application provides, amongst other things, the subject-matter of the following clauses.

### CLAUSES

1. A composition for modulating a T-cell-mediated immune response directed against an antigenic determinant in a mammalian subject, comprising: an immune-modulatory effective amount of a purified MHC Class II polypeptide comprising covalently linked first and second domains, wherein the first domain is a human MHC class II β1 domain and the second domain is a mammalian MHC class II α1 domain, wherein the amino terminus of the second domain is covalently linked to the carboxy terminus of the first domain, and wherein the MHC class II molecule does not include an α2 or a β2 domain; and an antigenic determinant, said composition effective to modulate one or more immune response(s) or immune regulatory activity(ies) of a T-cell in said subject.
2. The composition of clause 1, wherein said subject is a mammalian cell, tissue, organ, or individual.
3. The composition of clause 1, wherein covalent linkage between the β1 and α1 domains of said MHC Class II polypeptide is provided by a peptide linker sequence.
4. The composition of clause 1, wherein said antigenic determinant is a peptide antigen.
5. The composition of clause 1, wherein said antigenic determinant is covalently linked to an amino terminus of the first domain of said MHC Class II polypeptide.
6. The composition of clause 1, wherein said antigenic determinant is associated with said MHC Class II polypeptide by non-covalent interaction.
7. The composition of clause 1, wherein said MHC Class II polypeptide further comprises a covalently linked detectable marker or toxic moiety.
8. The composition of clause 1, wherein said MHC Class II polypeptide comprises α1 and β1 domains of an HLA-DR protein, or portions thereof comprising an Ag-binding pocket/T-cell receptor (TCR) interface.
9. The composition of clause 1, wherein said MHC Class II polypeptide comprises α1 and β1 domains of an HLA-I7Q protein, or portions thereof comprising an Ag-binding pocket/T-cell receptor (TCR) interface.
10. The composition of clause 1, wherein said MI-IC Class II polypeptide comprises a1 and β1 domains of an HLA-DP protein, or portions thereof comprising an Ag-binding pocl<ctlT-ccll receptor (TCR) interface.
11. The composition of clause 1, herein the MHC class II MHC component excludes a CD4 interactive domain of the corresponding, native MHC class II molecule.
12. The composition of clause 1, wherein the MHC Class II polypeptide is modified by one or more amino acid substitution(s), addition(s), deletion(s), or rearrangemcnt(s) at a target site corresponding to a self-associating interface identified in a native MHC polypeptide or RTL comprising the native MHC polypeptide, whereby the modified RTL exhibits reduced aggregation in solution compared to aggregation exhibited by an unmodified, control RTL having the MHC component structure set forth in a) or b) but incorporating the native MHC polypeptide having an intact self-associating interface.
13. The composition of clause I , wherein the MHC Class II polypeptide is modified by one or more amino acid substitution(s) or deletion(s) at one or more target site(s) characterized by the presence of a hydrophobic residue within a β-sheet platform of a native MHC polypeptide or RTL comprising the native MHC polypeptide.
14. The composition of clause 13, wherein said one or more target sites define a self-binding motif within β-sheet platform central core of the native MHC polypeptide or RTL comprising the native MHC polypeptide.
15. The composition of clause 13, wherein said one or more target sites comprise(s) one or any combination of residues of the central core portion of the β-sheet platform selected from V1 02, 1104, A1 06, F1 08, and L1 10.
16. The composition of clause 15, wherein said one or combination of residues is/are modified by substitution with a non-hydrophobic amino acid.
17. The composition of clause 15, wherein said one or combination of residues is/are modified by substitution with a poplar or charged amino acid.
18. The composition of clause 15, wherein said one or combination of residues is/are modified by substitution with a serine or aspartate residue.
19. The composition of clause 15, wherein said one or combination of residues is/are modified by substitution with a serine or aspartate residue.
20. The composition of clause 15, wherein each of the residues V 102, 1104, A 106, F1 08, and L1 10 of the central core portion of the β-sheet are modified by substitution with a non-hydrophobic amino acid.
21. The composition of clause 13, wherein said one or more target sites comprise(s) one or any combination of residues of the β-sheet platform selected from L9, F19, L28, F32, V45, V51, A133, V138, and L141.
22. The composition of clause 1, wherein said composition is effective to modulate T-cell activity in a T-cell receptor (TCR)-mediated, Ag-specific manner.
23. The composition of clause 1, wherein said composition effective to inhibit T-cell proliferation or inflammatory cytokine production *in vitro or in vivo.*
24. The composition of clause 1, wherein said composition is effective to reduce a pathogenic activity or pathogenic potential of a T-cell associated with an autoimmune disease in a mammalian cell or subject.
25. The composition of clause 15 wherein said composition is effective to reduce or prevent proliferation of a T-cell, a macrophage, a B cell, a dendritic cell, or an NK cell.
26. The composition of clause 1, wherein said composition is effective to induce a T suppressor phenotype, whereby a T-cell exposed to said composition suppresses an immune activity of another cell selected from a T-cell, a macrophage, a B cell, a dendritic cell, or an NK cell.
27. The composition of clause 15 wherein said composition is effective to modulate expression of one or more cytokine(s) by a T-cell, a macrophage, a B cell, a dendritic cell, or an NK cell.
29. The composition of clause 27, wherein the cytokine is IFN-γ.
30. The composition of clause 27, wherein the cytokine is TNF-α.
31. The composition of clause 27, wherein the cytokine is IL-2.
32. The composition of clause 27, wherein the cytokine is IL-4.
33. The composition of clause 27, wherein the cytokine is IL-6.
34. The composition of clause 27, wherein the cytokine is IL-10.
35. The composition of clause 27, wherein the cytokine is IL-13.
36. The composition of clause 27, wherein the cytokine is MCP-1.
37. The composition of clause 27, wherein the cytokine is TGFβ 1.
38. The composition of clause 27, wherein the cytokine is TGFβ3.
39. The composition of clause 27, wherein said composition is effective to modulate expression of said cytokine(s) by said T-cell, macrophage, B cell, dendritic cell, or NK cell in a peripheral blood, spleen, lymph node, or central nervous system (CNS) compartment of said subject.
40. The composition of clause 27, wherein modulation of expression of said one or more cytokine(s) is effected by modulation of mRNA transcription, mRNA stability, protein synthesis, or protein secretion by said T-cell, macrophage, B cell, dendritic cell, or NK cell.
41. The composition of clause 1, wherein said composition is effective to modulate expression of one or more adhesion/homing marker(s) by a T-cell, a macrophage, a B cell, a dendritic cell, or an NK cell.
42. The composition of clause 41, wherein said adhesion/homing marker is VLA-4.
43. The composition of clause 41, wherein said adhesion/homing marker is LFA-1.
44. The composition of clause 41, wherein said composition is effective to modulate expression of said adhesion/homing marker(s) by said T-cell, macrophage, B cell, dendritic cell, or NK cell in a peripheral blood, spleen, lymph node, or central nervous system (CNS) compartment of said subject.
45. The composition of clause 41, wherein modulation of expression of said one or more adhesion/homing marker(s) is effected by modulation of mRNA transcription, mRNA stability, protein synthesis, or protein secretion by said T-cell, macrophage, B cell, dendritic cell, or NK cell.
46. The composition of clause 1, wherein said composition is effective to modulate expression of one or more chemokine(s) by a T-cell, a macrophage, a B cell, a dendritic cell, or an NK cell.
47. The composition of clause 46, wherein said chemokine is RANTES.
48. The composition of clause 46, wherein said chemokine is MIP-2.
49. The composition of clause 46, wherein said chemokine is IP-10.
50. The composition of clause 46, wherein said composition is effective to modulate expression of said chemokine(s) by said T-cell, macrophage, B cell, dendritic cell, or NK cell in a peripheral blood, spleens, lymph node, or central nervous system (CNS) compartment of said subject.
51. The composition of clause 46, wherein modulation of expression of said one or more chemokine(s) is effected by modulation of mRNA transcription, mRNA stability, protein synthesis, or protein secretion by said T-cell, macrophage, B cell, dendritic cell, or NK cell.
52. The composition of clause 1, wherein said composition is effective to modulate expression of one or more chemokine receptor(s) by a T-cell, a macrophage, a B cell, a dendritic cell, or an NK cell.
53. The composition of clause 52, wherein said chemokine receptor is CCR1.
54. The composition of clause 52, wherein said chemokine receptor is CCR2.
55. The composition of clause 52, wherein said chemokine receptor is CCR3.
56. The composition of clause 52, wherein said chemokine receptor is CCR5.
57. The composition of clause 52, wherein said chemokine receptor is CCR6.
58. The composition of clause 52, wherein said chemokine receptor is CCR7.
59. The composition of clause 52, wherein said chemokine receptor is CCR8.
60. The composition of clause 52, wherein said composition is effective to modulate expression of said chemokine receptor(s) by a T-cell, a macrophage, a B cell, a dendritic cell, or an NK cell in a peripheral blood, spleen, lymph node, or central nervous system (CNS) compartment of said subject.
61. The composition of clause 52, wherein modulation of expression of said one or more chemokine receptor(s) is effected by modulation of mRNA transcription, mRNA stability, protein synthesis, or protein secretion by said T-cell, macrophage, B cell, dendritic cell, or NK cell.
62. The composition of clause 1, wherein said composition is effective to modulate expression of multiple Th1 cytokines by cells selected from T-cells, macrophages, B cells, dendritic cells, and NK cells.
63. The composition of clause 1, wherein said composition is effective to modulate expression of multiple Th2 cytokines by cells selected from T-cells, macrophages, B cells, dendritic cells, and NK cells.
64. The composition of clause 1, wherein said composition is effective to modulate expression of one or more T-cell regulatory marker(s) by a T-cell.
65. The composition of clause 64, wherein said T-cell regulatory markers is Foxp3.
66. The composition of clause 64, wherein said T-cell regulatory markers is TGFβ1.
67. The composition of clause 64, wherein said composition is effective to modulate expression of said T-cell regulatory marker(s) by said T-cell in a peripheral blood, spleens, lymph node, or central nervous system (CNS) compartment of said subject.
68. The composition of clause 64, wherein modulation of expression of said one or more T-cell regulatory marker(s) is effected by modulation of mRNA transcription, mRNA stability, protein synthesis, or protein secretion by said T-cell.
69. The composition of clause 1, wherein said composition is effective to induce a change in location, migration, chemotaxis, and/or infiltration by a T-cell, a macrophage, a B cell, a dendritic cell, or an NK cell in a peripheral blood, spleen, lymph node, or central nervous system (CNS) compartment of said subject.
70. The composition of clause 69, wherein said composition is effective to mediate a decrease in numbers of inflammatory mononuclear cells in said CNS compartment.
71. The composition of clause 70, wherein said composition is effective to mediate a decrease in numbers of inflammatory mononuclear cells in a spinal cord tissue of said subject.
72. The composition of clause 69, wherein said composition is effective to mediate a decrease in numbers of CD4+ T-cells in said CNS compartment.
73. The composition of clause 72, wherein said composition is effective to mediate a decrease in numbers of CD4+ T-cells in a spinal cord tissue of said subject.
74. A method for modulating a T-cell-mediated immune response directed against an antigenic determinant in a mammalian subject, comprising administering to said subject an immune-modutatory effective amount of a composition comprising a purified MHC Class II polypeptide comprising covalently linked first and second domains, wherein the first domain is a human MHC class II β1 domain and the second domain is a mammalian MHC class II α1 domain, wherein the amino terminus of the second domain is covalently linked to the carboxy terminus of the first domain, and wherein the MHC class II molecule does not include an α2 or a β2 domain, and an antigenic determinant, sufficient to modulate one or more immune response(s) or immune regulatory activity(ies) of a T-cell in said subject.
75. The method of clause 74, wherein said subject is a mammalian cell, tissue, organ, or individual.
76. The method of clause 74, wherein said antigenic determinant is a peptide antigen.
77. The method of clause 74, wherein said antigenic determinant is covalently linked to an amino terminus of the first domain of said MHC Class II polypeptide.
78. The method of clause 74, wherein said antigenic determinant is associated with said MHC Class II polypeptide by non-covalent interaction.
79. The method of clause 74, wherein said MHC Class II polypeptides further comprises a covalently linked detectable marker or toxic moiety.
80. The method of clause 74, wherein said MHC Class II polypeptide-comprises α1 and β1 domains of an HI,A-DR protein, or portions thereof comprising an Ag-binding pocket/T-cell receptor (TCR) interface.
81. The method of clause 74, wherein said MHC Class II polypeptide comprises α1 and β1 domains of an HLA-DQ protein, or portions thereof comprising an Ag-binding pocket/T-cell receptor (TCR) interface.
82. The method of clause 74, wherein said MHC Class II polypeptide comprises α1 and β1 domains of an HLA-DP protein, or portions thereof comprising an Ag-binding pocket/T-cell receptor (TCR) interface.
83. The method of clause 74, wherein the MHC class II MHC component excludes a CD4 interactive domain of the corresponding, native MHC class II molecule.
84. The method of clause 74, wherein the MHC Class II polypeptide is modified by one or more amino acid substitution(s), addition(s), deletion(s), or rearrangement(s) at a target site corresponding to a self-associating interface identified in a native MHC polypeptide or RTL comprising the native MHC polypeptide, whereby the modified RTL exhibits reduced aggregation in solution compared to aggregation exhibited by an unmodified, controls RTL having the MHC component structure set forth in a) or b) but incorporating the native MHC polypeptide having an intact self-associating interface.
85. The method of clause 74, wherein the MHC Class II polypeptide is modified by one or more amino acid substitution(s) or deletion(s) at one or more target site(s) characterized by the presence of a hydrophobic residue within a β-sheet platform of a native MHC polypeptide or RTL comprising the native MHC polypeptide.
86. The method of clause 85, wherein said one or more target sites define a self-binding motif within β-sheet platform central core of the native MHC polypeptide or RTL comprising the native MHC polypeptide.
87. The method of clause 85, wherein said one or more target sites comprise(s) one or any combination of residues of the central core portion of the β-sheet platform selected from V102, 1104, A106, F108, and L1 10.
88. The method of clause 87, wherein said one or combination of residues is/are modified by substitution with a non-hydrophobic amino acid.
89. The method of clause 87, wherein said one or combination of residues is/are modified by substitution with a polar or charged amino acid.
90. The method of clause 87, wherein said one or combination of residues is/are modified by substitution with a serine or aspartate residue.
91. The method of clause 87, wherein said one or combination of residues is/are modified by substitution with a serine or aspartate residue.
92. The method of clause 87, wherein each of the residues V102, 1104, A106, F108, and L1 10 of the central core portion of the β-sheet are modified by substitution with a non-hydrophobic amino acid.
93. The method of clause 85, wherein said one or more target sites comprise(s) one or any combination of residues of the β-sheet platform selected from L9, F 19, L28, F32, V45, V51, A133, V138, and L141.
94. The method of clause 74, wherein said composition is effective to modulate T-cell activity in said subject a T-cell receptor (TCR)-mediated, Ag-specific manner.
95. The method of clause 74, wherein said composition effective to inhibit T- cell proliferation or inflammatory cytokine production in said subject.
96. The method of clause 74, wherein said composition is effective to reduce a pathogenic activity or pathogenic potential of a T-cell associated with an autoimmune disease in said subject.
97. The method of clause 74, wherein said composition is effective to reduce or prevent proliferation of a T-cell, a macrophage, a B cell, a dendritic cell, or an NK cell in said subject.
98. The method of clause 74, wherein said composition is effective to induce a T suppressor phenotype, whereby a T-cell exposed to said composition suppresses an immune activity of another cell selected from a T-cell, a macrophage, a B cell, a dendritic cell, or an NK cell in said subject.
99. The method of clause 74, wherein said composition is effective to modulate expression of one or more cytokine(s) by a T-cell, a macrophage, a B cell, a dendritic cell, or an NK cell in said subject.
100. The method of clause 99, wherein the cytokine is IFN-γ.
101. The method of clause 99, wherein the cytokine is TNF-α.
102. The method of clause 99, wherein the cytokine is IL-2.
103. The method of clause 99, wherein the cytokine is IL-4.
104. The method of clause 99, wherein the cytokine is IL-6.
105. The method of clause 99, wherein the cytokine is IL-10.
106. The method of clause 99, herein the cytokine is IL-13.
107. The method of clause 99, wherein the cytokine is MCP-1.
108. The method of clause 99, wherein the cytokine is TGFβ1
109. The method of clause 99, wherein the cytokine is TGFβ3.
110. The method of clause 99, wherein said composition is effective to modulate expression of said cytokine(s) by said T-cell, macrophage, B cell, dendritic cell, or NK cell in a peripheral blood, spleen, lymph node, or central nervous system (CNS) compartment of said subject.
111. The method of clause 99, wherein modulation of expression of said one or more cytokine(s) is effected by modulation of mRNA transcription, mRNA stability, protein synthesis, or protein secretion by said T-cell, macrophage, B cell, dendritic cell, or NK cell in said subject.
112. The method of clause 74, wherein said composition is effective to modulate expression of one or more adhesion/homing marker(s) by a T-cell, a macrophage, a B cell, a dendritic cell, or an NK cell in said subject.
113. The method of clause 112, wherein said adhesion/homing marker is Via-4.
114. The method of clause 112, wherein said adhesion/homing marker is LFA-1.
115. The method of clause 112, wherein said composition is effective to modulate expression of said adhesion/homing marker(s) by said T-cell, macrophage, B cell, dendritic cell, or NK cell in a peripheral blood, spleen, lymph node, or central nervous system (CNS) compartment of said subject.
116. The method of clause 112, wherein modulation of expression of said one or more adhesion/homing marker(s) is effected by modulation of mRNA transcription, mRNA stability, protein synthesis, or protein secretion by said T-cell, macrophage, B cell, dendritic cell, or NK cell in said subject.
117. The method of clause 74, wherein said composition is effective to modulate expression of one or more chemokine(s) by a T-cell, a macrophage, a B cell, a dendritic cell, or an NK cell in said subject.
118. The method of clause 117, wherein said chemokine is RANTES. 1 19. The method of clause 117, wherein said chemokine is MIP-2.
120. The method of clause 117, wherein said chemokine is IP-10.
121. The method of clause 117, wherein said composition is effective to modulate expression of said chemokine(s) by said T-cell, macrophage, B cell, dendritic cell, or NK cell in a peripheral blood, spleen, lymph node, or central nervous system (CNS) compartment of said subject.
122. The method of clause 117, wherein modulation of expression of said one or more chemokine(s) is effected by modulation of mRNA transcription, mRNA stability, protein synthesis, or protein secretion by said T-cell, macrophage, B cell, dendritic cell, or NK cell in said subject.
123. The method of clause 74, wherein said composition is effective to modulate expression of one or more chemokine receptor(s) by a T-cell, a macrophage, a B cell, a dendritic cell, or an NK cell in said subject.
124. The method of clause 123, wherein said chemokine receptor is CCR1.
125. The method of clause 123, wherein said chemokine receptor is CCR2.
126. The method of clause 123, wherein said chemokine receptor is CCR3.
127. The method of clause 123, wherein said chemokine receptor is CCR5.
128. The method of clause 123, wherein said chemokine receptor is CCR6.
129. The method of clause 123, wherein said chemokine receptor is CCR7.
130. The method of clause 123, wherein said chemokine receptor is CCR8.
131. The method of clause 123 , wherein said composition is effective to modulate expression of said chemokine receptor(s) by a T-cell, a macrophage, a B cell, a dendritic cell, or an NK cell in a peripheral blood, spleen, lymph node, or central nervous system (CNS) compartment of said subject.
132. The method of clause 123, wherein modulation of expression of said one or more chemokine receptor(s) is effected by modulation of mRNA transcription, mRNA stability, protein synthesis, or protein secretion by said T-cell, macrophage, B cell, dendritic cell, or NK cell.
133. The method of clause 74, wherein said composition is effective to modulate expression of multiple Th1 cytokines by cells selected from T-cells, macrophages, B cells, dendritic cells, and NK cells in said subject.
134. The method of clause 74, wherein said composition is effective to modulate expression of multiple Th2 cytokines by cells selected from T-cells, macrophages, B cells, dendritic cells, and NK cells in said subject.
135. The method of clause 74, wherein said composition is effective to modulate expression of one or more T-cell regulatory marker(s) by a T-cell in said subject.
136. The method of clause 135, wherein said T-cell regulatory markers is Foxp3.
137. The method of clause 135, wherein said T-cell regulatory markers is TGFβ1.
138. The method of clause 135, wherein said composition is effective to modulate expression of said T-cell regulatory marker(s) by said T-cell in a peripheral blood, spleen, lymph node, or central nervous system (CNS) compartment of said subject.
139. The method of clause 135, wherein modulation of expression of said one or more T-cell regulatory marker(s) is effected by modulation of mRNA transcription, mRNA stability, protein synthesis, or protein secretion by said T-cell.
140. The method of clause 74, wherein said composition is effective to induce a change in location, migration, chemotaxis, and/or infiltration by a T-cell, a macrophage, a B cell, a dendritic cell, or an NK cell in a peripheral blood, spleen, lymph node, or central nervous system (CNS) compartment of said subject.
141. The method of clause 140, wherein said composition is effective to mediate a decrease in numbers of inflammatory mononuclear cells in said CNS compartment.
142. The method of clause 141, wherein said composition is effective to mediate a decrease in numbers of inflammatory mononuclear cells in a spinal cord tissue of said subject.
143. The method of clause 140, wherein said composition is effective to mediate a decrease in numbers of CD4+ T-cells in said CNS compartment.
144. The method of clause 143, wherein said composition is effective to mediate a decrease in numbers of CD4+ T-cells in a spinal cord tissue of said subject.
145. A method for modulating a T-cell-mediated immune response directed against an antigenic determinant in a mammalian cell, tissue or subject, comprising: contacting the cell or tissue with, or administering to said subject, an immune- modulatory effective amount of a purified MHC Class II polypeptide comprising covalently linked first and second domains, wherein the first domain is a human MHC class II β1 domain and the second domain is a mammalian MHC class II α1 domain, wherein the amino terminus of the second domain is covalently linked to the carboxy terminus of the first domain, wherein the MHC class II molecule does not include an α2 or a β2 domain, and wherein the polypeptide further comprises said antigenic determinant covalently linked to an amino terminus of the first domain.
146. A method for inducing an immunoregulatory cell against an antigenic determinant, comprising: administering an immunomodulatory effective amount of the composition of clause 1 to the immunoregulatory cell; and subsequently presenting the antigenic determinant to the immunoregulatory cell, whereby presentation of the antigenic determinant result in induction of the immunoregulatory cell to yield an immunomodulatory activity by said immunoregulatory cell.
147. The method of clause 146, wherein the immunoregulatory cell reduces inflammation and cellular recruitment when antigen is subsequently encountered with an immunogenic stimulus.
148. The method of clause 146, wherein the antigenic determinant is a tissue specific antigenic determinant.
149. The method of clause 146, wherein the immunoregulatory cell is induced as compared to a control.
150. The method of clause 146, wherein the immunoregulatory cell is *in vivo.*
151. The method of clause 146, wherein the immunoregulatory cell is *in vitro.*
152. A method for modulating expression of a cytokine in a mammalian T-cell, comprising contacting the T-cell with an effective amount of the composition of clause 1, thereby modulating expression of the cytokine by the T-cell.
153. The method of clause 153, herein the cell is *in vivo.*
154. The method of clause 153, wherein the cell is *in vitro.*
156. A immune modulatory composition for eliciting an immune response in a mammalian subject comprising a nucleic acid molecule encoding the purified MHC Class II polypeptide of clause 1.
157. The immune modulatory composition of clause 156, wherein the nucleic acid is operably linked to a promoter.
158. A method for reducing an immune response against an antigenic determinant in a subject, comprising: administering a therapeutically effective amount of the composition of clause 1, or of a nucleic acid of clause 156; and subsequently presenting the antigenic determinant to the subject, wherein administration of the polypeptide or the nucleic acid reduces the immune response when the antigenic determinant is presented in the subject.
159. The method of clause 158, wherein the reduced immune response is a decrease in an influx or proliferation of a T-cell, a macrophage, a B cell, or an NK cell.
160. The method of clause 158, wherein the reduced immune response is a reduction in the expression of a cytokine.
161. The method of clause 158, wherein the reduced immune response is an induction of a T suppressor cell response.
162. A method for inducing an immunoregulatory cell against an antigenic determinant, comprising: administering a therapeutically effective amount of the composition of clause 1 to the immunoregulatory cell; and subsequently presenting the antigenic determinant to the immunoregulatory cell; wherein the presentation of the antigenic determinant results in an induction of the immunoregulatory cell.
163. The method of clause 162, wherein the immunoregulatory cell reduces inflammation and cellular recruitment when the antigen is subsequently encountered with an immunogenic stimulus.
164. The method of clause 162, wherein the antigenic determinant is a tissue specific antigenic determinant.
165. The method of clause 162, wherein the immunoregulatory cell is induced as compared to a control.
166. The method of clause 162, wherein the immunoregulatory cell is *in vivo.*
167. The method of clause 162, wherein the immunoregulatory cell is *in vitro.*
168. A method for modulating expression of a cytokine in a mammalian T-cell, comprising contacting the T-cell with an effective amount of the composition of clause 1, sufficient to modulate expression of the cytokine by the T-cell.
169. The method of clause 168, wherein the cell is *in vivo.*
170. The method of clause 168, wherein the cell is *in vitro.*
171. A method of treating or preventing an immune-mediated disorder in a subject, comprising: administering to the subject a therapeutically effective amount of the composition of clause 1 or of a nucleic acid of clause 156; wherein subsequent presentation of the antigenic determinant to an immune cell of the subject results in treatment or prevention of the immune- mediated disorder.
172. The method of clause 171, wherein the immune-mediated disorder is graft rejection, graft versus host disease, unwanted delayed-type hypersensitivity reaction, T-cell mediated pulmonary disease, insulin dependent diabetes mellitus, systemic lupus erythematosus, rheumatoid arthritis, coeliac disease, multiple sclerosis, neuritis, polymyositis, psoriasis, vitiligo, Sjogren's syndrome, rheumatoid arthritis, autoimmune pancreatitis, inflammatory bowel diseases, Crohn's disease, ulcerative colitis, active chronic hepatitis, glomerulonephritis, scleroderma, sarcoidosis, autoimmune thyroid diseases, Hashimoto's thyroiditis, Graves disease, myasthenia gravis, asthma, Addison's disease, autoimmune uveoretinitis, pemphigus vulgaris, primary biliary cirrhosis, pernicious anemia, sympathetic opthalmia, uveitus, autoimmune hemolytic anemia, pulmonary fibrosis, allergies or idiopathic pulmonary fibrosis.
173. A pharmaceutical composition comprising the composition of clause 1 including a pharmaceutically acceptable carrier.
174. A method of treating a disease caused by antigen-specific T-cells in a mammalian subject, comprising administering to said subject a composition according to clause 1, thereby treating the disease.
175. A method for modulating immune activity of a T-cell in a mammalian subject, comprising administering to said subject an immunomodutatory effective amount of the composition of clause 1.
176. The method of clause 175, further comprising administering a second therapeutic agent to said subject.
177. The method of clause 176, wherein the second therapeutic agent is administered to said subject in a combined formulation with the composition of clause 1.
178. The method of clause 176, wherein said second therapeutic agent is administered to said subject in a coordinate administration protocol, simultaneously with, prior to, or after administration of said composition of clause 1 to said subject.
179. The method of clause 176, wherein said second therapeutic agent is selected from immunoglobulins; copolymer 1, copolymer 1 -related peptides, and T-cells treated with copolymer 1 or copolymer 1 -related peptides; blocking monoclonal antibodies, transforming growth factor-β, anti-TNF α antibodies; steroidal agents; antiinflammatory agents; immunosuppresive agents; alkylating agents; anti-metabolites; antibiotics; corticosteroids; glatiramer acetate; recombinant β interferons; proteosome inhibitors; and diketopiperazines.
180. The composition of clause 1, wherein said composition is effective to ameliorate axonal loss.
181. The composition of clause 1, wherein said composition is effective to prevent axonal loss.
182. A method for ameliorating axonal loss from a T-cell-mediated immune response directed against an antigenic determinant in a mammalian cell, tissue or subject, comprising: contacting the cell or tissue with, or administering to said subject, an immune- mediatory effective amount of a purified MHC Class II polypeptide comprising covalently linked first and second domains, wherein the first domain is a human MHC class II β1 domain and the second domain is a mammalian MHC class II α1 domain, wherein the amino terminus of the second domain is covalently linked to the carboxy terminus of the first domain, wherein the MHC class II molecule does not include an α2 or a β2 domain, and wherein the polypeptide further comprises said antigenic determinant covalently linked to an amino terminus of the first domain.
183. A composition for modulating a T-cell-mediated immune response directed against an antigenic determinant in a mammalian subject, comprising: an immune-modulatory effective amount of a first immune modulatory agent comprising a purified MHC Class II polypeptides comprising covalently linked first and second domains, wherein the first domain is a human MHC class II β1 domain and the second domain is a mammalian MHC class II α1 domain, wherein the amino terminus of the second domain is covalently linked to the carboxy terminus of the first domain, and wherein the MHC class II molecule does not include an α2 or a β2 domain; an antigenic determinant; and a second immune mediatory agent selected from immunoglobulins; copolymer 1; copolymer 1 -related peptides; and T-cells treated with copolymer 1 or copolymer 1-related peptides; blocking monoclonal antibodies, transforming growth factors-β, anti-TNF α antibodies; steroidal agents; anti-inflammatory agents; immunosuppresive agents; alkylating agents; anti-metabolites; antibiotics; corticosteroids; proteosome inhibitors; glatiramer acetate; recombinant β interferons; and diketopiperazines. said composition effective to modulate one or more immune response(s) or immune regulatory activity(ies) of a T-cell in said subject.
184. A method of treating or preventing a neurodegenerative disorder in a subject comprising: administering to said subject a therapeutically effective amount of the composition of clause 1 or of a nucleic acid of clause 156; wherein subsequent presentation of the antigenic determinant to an immune cell of the subject results in treatment or prevention of the neurodegenerative disorder.
185. The method of clause 184, wherein the neurodegenerative disorder is multiple sclerosis, Parkinson's disease, Alzheimer's disease, progressive multifocal leukoencephalopathy, disseminated necrotizing leulcoencephalopathy, acute disseminated encephalomyelitis, Schilder disease, central pontine myelinolysis, radiation necrosis, Binswanger disease, adrenoleukodystrophy, adrenomyeloneuropathy, Leber's hereditary optic atrophy, and HTLV-associated myelopathy.
186. The composition of clause 1, wherein said composition is effective to ameliorate demyelination.
187. The composition of clause 1, wherein said composition is effective to prevent demyelination.
188. The composition of clause 1, wherein said composition is effective in treating demyelination.
189. The composition of clause 1, wherein said composition is effective to preserve nerofilaments.
190. The composition of clause 1, wherein said composition is effective to stimulate remyelination.
191. A method for ameliorating demyelination from a T-cell-mediated immune response directed against an antigenic determinant in a mammalian cell, tissue or subject, comprising: contacting the cell or tissue with, or administering to said subject, an immune-modutatory effective amount of a purified MHC Class II polypeptide comprising covalently linked first and second domains, wherein the first domain is a human MHC class II β1 domain and the second domain is a mammalian MHC class II α1 domain, wherein the amino terminus of the second domain is covalently linked to the carboxy terminus of the first domain, wherein the MHC class II molecule does not include an α2 or a β2 domain, and wherein the polypeptide further comprises said antigenic determinant covalently linked to an amino terminus of the first domain.
192. The composition of clause 7, wherein the detectable marker is radionuclides, paramagnetic isotopes, fluorescent markers, enzymes, cofactors, chemiluminescent compounds or bioluminescent compounds.
193. The composition of clause 7, wherein the toxic moiety is protein toxins, chemotherapeutic agents, antibodies to a cytotoxic T-cell surface molecule, lipases, or radioisotopes.
194. The composition of clause 1, wherein said composition is effective to reduce pathogenic activity or pathogenic potential of a T-cell associated with a neurodegenerative disease in a mammalian cell or subject.
195. The composition of clause 1, wherein said composition is effective to reduce pathogenic activity or pathogenic potential of a T-cell associated with a demyelinating disease in a mammalian cell or subject.
196. The composition of clause 1, wherein said composition is effective to prevent or inhibit infiltration of activated inflammatory cells in a central nervous system of a mammalian subject.
197. The composition of clause 1, wherein said composition is effective to prevent relapses of an autoimmune disease in a mammalian cell or subject.
198. The composition of clause 1, wherein said composition is effective to prevent relapses of a neurodegenerative disease in a mammalian cell or subject.
199. The composition of clause 1, wherein said composition is effective to increase secretion of antiinflammatory factors in a mammalian cell or subject.
200. The method of clause 74, wherein said composition is effective to reduce pathogenic activity or pathogenic potential of a T-cell associated with a neurodegenerative disease in a mammalian cell or subject.
201. The method of clause 74, wherein said composition is effective to reduce pathogenic activity or pathogenic potential of a T-cell associated with a demyelinating disease in a mammalian cell or subject.
202. The method of clause 74, wherein said composition is effective to prevent or inhibit infiltration of activated inflammatory cells in a central nervous system of a mammalian subject.
203. The method of clause 74, wherein said composition is effective to prevent relapses of an autoimmune disease in a mammalian cell or subject.
204. The method of clause 74, wherein said composition is effective to prevent relapses of a neurodegenerative disease in a mammalian cell or subject.
205. The method of clause 74, wherein said composition is effective to increase secretion of antiinflammatory factors in a mammalian cell or subject.

## Claims

1. A composition comprising a purified major histocompatibility complex (MHC) class II polypeptide comprising covalently linked first and second domains, wherein the first domain is a mammalian MHC class II β1 domain and the second domain is a mammalian MHC class II α1 domain, wherein the amino terminus of the second domain is covalently linked to the carboxy terminus of the first domain, wherein the MHC class II molecule does not include an α2 or a β2 domain, and wherein the polypeptide further comprises an antigenic determinant covalently linked to the amino terminus of the first domain, for use in a method for reducing demyelination, reducing axon loss, or reducing neuroaxonal injury in a subject with an autoimmune disease, the method comprising administering to said subject a therapeutically effective amount of the composition.

2. The composition of claim 1, wherein the MHC class II polypeptide is modified by substitution of one or more hydrophobic residues within a β-sheet platform of a native MHC class II polypeptide with a non-hydrophobic residue, whereby the modified MHC class II polypeptide exhibits reduced aggregation in solution compared to an unmodified control MHC class II polypeptide having the MHC class II polypeptide, structure set forth in claim 1, but incorporating an unmodified β-sheet platform of the native MHC class II polypeptide, preferably wherein the one or more hydrophobic residues within the β-sheet platform are selected from V102, I104, A106, F108, and L110.

3. The composition of claim 2, wherein the one or more hydrophobic residues are substituted by a polar or charged residue, preferably a serine or aspartate residue.

4. The composition of any one of claims 1 to 3, wherein the MHC class II polypeptide comprises α1 and β1 domains of an HLA-DR protein, or an HLA-DQ protein, or an HLA-DP protein.

5. The composition of any one of claims 1 to 4, wherein the MHC class II polypeptide excludes a CD4 interactive domain of the corresponding MHC class II molecule.

6. The composition of any one of claims 1 to 5, wherein treating demyelination, reducing neuroaxonal injury, or reducing neuron loss comprises reducing infiltration by a T-cell, a macrophage, a B-cell, a dendritic cell, or an NK cell in a central nervous system compartment of the subject.

7. The composition of claim 6, wherein reducing infiltration comprises a decrease in number of inflammatory mononuclear cells in the central nervous system compartment.

8. The composition of claim 6, wherein the T-cell comprises a CD4+ T-cell.

9. The composition of any one of claims 6 to 8, wherein the central nervous system compartment comprises spinal cord tissue of the subject.

10. The composition of any one of claims I to 9, wherein reducing demyelination comprises increasing remyelination of axons.

11. The composition of any one of claims I to 10, wherein the antigenic determinant comprises a myelin oligodendrocyte glycoprotein (MOG) peptide, preferably MOG 35-55.

12. The composition of any one of claims 1 to 10, wherein the antigenic determinant comprises a myelin basic protein (MBP) peptide, preferably, MBP 85-99.

13. The composition of any one of claims 1 to 10, wherein the antigenic determinant comprises a proteolipid protein (PLP) peptide, preferably PLP 139-151.

14. The composition of any one of claim 1 to 13, wherein the composition is effective to treat or prevent relapses of the autoimmune disease.

15. The composition of any one of claims 1 to 14, wherein the autoimmune disease is multiple sclerosis.


SEQUENCE LISTING

<110> OREGON HEALTH & SCIENCE UNIVERSITY

<120> RECOMBINANT MHC MOLECULES USEFUL FOR MANIPULATION
      OF ANTIGEN-SPECIFIC T CELLS

<130> JA40858P.EPPD1

<140> EP
<141> 2007-10-05

<150> EP 06748442.8
<151> 2007-10-05

<150> PCT/US06/09884
<151> 2006-03-16

<150> 11/373,047
<151> 2006-03-10

<150> 60/713,230
<151> 2005-08-31

<150> 60/663,048
<151> 2005-03-18

<160> 95

<170> PatentIn version 3.5

<210> 1
<211> 566
<212> DNA
<213> Artificial Sequence

<220>
<221> source
<223> /note="Description of Artificial Sequence: Synthetic
      polynucleotide"


<220>
<221> CDS
<222> (3)..(557)

<400> 1
cc atg ggc aga gac tcc cca agg gat ttc gtg tac cag ttc aag ggc          47
   Met Gly Arg Asp Ser Pro Arg Asp Phe Val Tyr Gln Phe Lys Gly
   1               5                   10                  15

ctg tgc tac tac acc aac ggg acg cag cgc ata cgg gat gtg atc aga          95
Leu Cys Tyr Tyr Thr Asn Gly Thr Gln Arg Ile Arg Asp Val Ile Arg
                20                  25                  30

tac atc tac aac cag gag gag tac ctg cgc tac gac agc gac gtg ggc         143
Tyr Ile Tyr Asn Gln Glu Glu Tyr Leu Arg Tyr Asp Ser Asp Val Gly
            35                  40                  45

gag tac cgc gcg ctg acc gag ctg ggg cgg ccc tca gcc gag tac ttt         191
Glu Tyr Arg Ala Leu Thr Glu Leu Gly Arg Pro Ser Ala Glu Tyr Phe
        50                  55                  60

aac aag cag tac ctg gag cag acg cgg gcc gag ctg gac acg gtc tgc         239
Asn Lys Gln Tyr Leu Glu Gln Thr Arg Ala Glu Leu Asp Thr Val Cys
    65                  70                  75

aga cac aac tac gag ggg tcg gag gtc cgc acc tcc ctg cgg cgg ctt         287
Arg His Asn Tyr Glu Gly Ser Glu Val Arg Thr Ser Leu Arg Arg Leu

80                  85                  90                  95

gga ggt caa gac gac att gag gcc gac cac gta gcc gcc tat ggt ata          335
Gly Gly Gln Asp Asp Ile Glu Ala Asp His Val Ala Ala Tyr Gly Ile
                100                 105                 110

aat atg tat cag tat tat gaa tcc aga ggc cag ttc aca cat gaa ttt          383
Asn Met Tyr Gln Tyr Tyr Glu Ser Arg Gly Gln Phe Thr His Glu Phe
            115                 120                 125

gat ggt gac gag gaa ttc tat gtg gac ttg gat aag aag gag acc atc          431
Asp Gly Asp Glu Glu Phe Tyr Val Asp Leu Asp Lys Lys Glu Thr Ile
        130                 135                 140

tgg agg atc ccc gag ttt gga cag ctg aca agc ttt gac ccc caa ggt          479
Trp Arg Ile Pro Glu Phe Gly Gln Leu Thr Ser Phe Asp Pro Gln Gly
    145                 150                 155

gga ctt caa aat ata gct ata ata aaa cac aat ttg gaa atc ttg atg          527
Gly Leu Gln Asn Ile Ala Ile Ile Lys His Asn Leu Glu Ile Leu Met
160                 165                 170                 175

aag agg tca aat tca acc caa gct gtc aac taactcgag                        566
Lys Arg Ser Asn Ser Thr Gln Ala Val Asn
                180                 185


<210> 2
<211> 185
<212> PRT
<213> Artificial Sequence

<220>
<221> source
<223> /note="Description of Artificial Sequence: Synthetic
      polypeptide"

<400> 2
Met Gly Arg Asp Ser Pro Arg Asp Phe Val Tyr Gln Phe Lys Gly Leu
1               5                   10                  15


Cys Tyr Tyr Thr Asn Gly Thr Gln Arg Ile Arg Asp Val Ile Arg Tyr
            20                  25                  30


Ile Tyr Asn Gln Glu Glu Tyr Leu Arg Tyr Asp Ser Asp Val Gly Glu
        35                  40                  45


Tyr Arg Ala Leu Thr Glu Leu Gly Arg Pro Ser Ala Glu Tyr Phe Asn
    50                  55                  60


Lys Gln Tyr Leu Glu Gln Thr Arg Ala Glu Leu Asp Thr Val Cys Arg
65                  70                  75                  80


His Asn Tyr Glu Gly Ser Glu Val Arg Thr Ser Leu Arg Arg Leu Gly
                85                  90                  95


Gly Gln Asp Asp Ile Glu Ala Asp His Val Ala Ala Tyr Gly Ile Asn
            100                 105                 110


Met Tyr Gln Tyr Tyr Glu Ser Arg Gly Gln Phe Thr His Glu Phe Asp
        115                 120                 125

Gly Asp Glu Glu Phe Tyr Val Asp Leu Asp Lys Lys Glu Thr Ile Trp
    130                 135                 140

Arg Ile Pro Glu Phe Gly Gln Leu Thr Ser Phe Asp Pro Gln Gly Gly
145                 150                 155                 160

Leu Gln Asn Ile Ala Ile Ile Lys His Asn Leu Glu Ile Leu Met Lys
                165                 170                 175

Arg Ser Asn Ser Thr Gln Ala Val Asn
            180                 185

<210> 3
<211> 113
<212> DNA
<213> Artificial Sequence

<220>
<221> source
<223> /note="Description of Artificial Sequence: Synthetic
      polynucleotide"

<220>
<221> CDS
<222> (3)..(113)

<400> 3
cc atg ggc aga gac tcc cca cag aag agc cag agg act cag gat gag      47
   Met Gly Arg Asp Ser Pro Gln Lys Ser Gln Arg Thr Gln Asp Glu
   1               5                   10                  15

aac cca gtg gtg cac ttc gga ggt gga ggc tca cta gtg ccc cga ggc      95
Asn Pro Val Val His Phe Gly Gly Gly Gly Ser Leu Val Pro Arg Gly
                20                  25                  30

tct gga ggt gga ggc tcc                                              113
Ser Gly Gly Gly Gly Ser
            35

<210> 4
<211> 37
<212> PRT
<213> Artificial Sequence

<220>
<221> source
<223> /note="Description of Artificial Sequence: Synthetic
      polypeptide"

<400> 4
Met Gly Arg Asp Ser Pro Gln Lys Ser Gln Arg Thr Gln Asp Glu Asn
1               5                   10                  15

Pro Val Val His Phe Gly Gly Gly Gly Ser Leu Val Pro Arg Gly Ser
            20                  25                  30

Gly Gly Gly Gly Ser
        35

<210> 5
<211> 83
<212> DNA
<213> Artificial Sequence

<220>
<221> source
<223> /note="Description of Artificial Sequence: Synthetic
      primer"


<220>
<221> CDS
<222> (3)..(83)

<400> 5
cc atg ggc aga gac tcc tcc ggc aag gat tcg cat cat gcg gcg cgg          47
   Met Gly Arg Asp Ser Ser Gly Lys Asp Ser His His Ala Ala Arg
   1               5                   10                  15

acg acc cac tac ggt gga ggt gga ggc tca cta gtg                         83
Thr Thr His Tyr Gly Gly Gly Gly Gly Ser Leu Val
                20                  25


<210> 6
<211> 27
<212> PRT
<213> Artificial Sequence

<220>
<221> source
<223> /note="Description of Artificial Sequence: Synthetic
      peptide"

<400> 6
Met Gly Arg Asp Ser Ser Gly Lys Asp Ser His His Ala Ala Arg Thr
1               5                   10                  15


Thr His Tyr Gly Gly Gly Gly Gly Ser Leu Val
            20                  25


<210> 7
<211> 89
<212> DNA
<213> Artificial Sequence

<220>
<221> source
<223> /note="Description of Artificial Sequence: Synthetic
      primer"


<220>
<221> CDS
<222> (3)..(89)

<400> 7
cc atg ggc aga gac tcc aaa ctg gaa ctg cag tcc gct ctg gaa gaa          47
   Met Gly Arg Asp Ser Lys Leu Glu Leu Gln Ser Ala Leu Glu Glu
   1               5                   10                  15

gct gaa gct tcc ctg gaa cac gga ggt gga ggc tca cta gtg                 89
Ala Glu Ala Ser Leu Glu His Gly Gly Gly Gly Ser Leu Val
                20                  25

<210> 8
<211> 29
<212> PRT
<213> Artificial Sequence

<220>
<221> source
<223> /note="Description of Artificial Sequence: Synthetic
      peptide"

<400> 8
Met Gly Arg Asp Ser Lys Leu Glu Leu Gln Ser Ala Leu Glu Glu Ala
1               5                   10                  15

Glu Ala Ser Leu Glu His Gly Gly Gly Gly Ser Leu Val
            20                  25

<210> 9
<211> 28
<212> DNA
<213> Artificial Sequence

<220>
<221> source
<223> /note="Description of Artificial Sequence: Synthetic
      primer"

<400> 9
aattcctcga gatggctctg cagacccc                                        28

<210> 10
<211> 30
<212> DNA
<213> Artificial Sequence

<220>
<221> source
<223> /note="Description of Artificial Sequence: Synthetic
      primer"

<400> 10
tcttgacctc caagccgccg cagggaggtg                                      30

<210> 11
<211> 31
<212> DNA
<213> Artificial Sequence

<220>
<221> source
<223> /note="Description of Artificial Sequence: Synthetic
      primer"

<400> 11
cggcggcttg gaggtcaaga cgacattgag g                                    31

<210> 12
<211> 37
<212> DNA
<213> Artificial Sequence

<220>
<221> source
<223> /note="Description of Artificial Sequence: Synthetic

primer"

<400> 12
gcctcggtac cttagttgac agcttgggtt gaatttg                                37


<210> 13
<211> 26
<212> DNA
<213> Artificial Sequence

<220>
<221> source
<223> /note="Description of Artificial Sequence: Synthetic
      primer"

<400> 13
cagggaccat gggcagagac tcccca                                            26


<210> 14
<211> 30
<212> DNA
<213> Artificial Sequence

<220>
<221> source
<223> /note="Description of Artificial Sequence: Synthetic
      primer"

<400> 14
gcctcctcga gttagttgac agcttgggtt                                        30


<210> 15
<211> 128
<212> DNA
<213> Artificial Sequence

<220>
<221> source
<223> /note="Description of Artificial Sequence: Synthetic
      primer"

<400> 15
gaaatcccgc ggggagcctc cacctccaga gcctcggggc actagtgagc ctccacctcc       60

gaagtgcacc actgggttct catcctgagt cctctggctc ttctgtgggg agtctctgcc      120

ctcagtcc                                                               128


<210> 16
<211> 31
<212> DNA
<213> Artificial Sequence

<220>
<221> source
<223> /note="Description of Artificial Sequence: Synthetic
      primer"

<400> 16
gctccccgcg ggatttcgtg taccagttca a                                      31


<210> 17
<211> 92
<212> DNA

<213> Artificial Sequence

<220>
<221> source
<223> /note="Description of Artificial Sequence: Synthetic
      primer"

<400> 17
tattaccatg ggcagagact cctccggcaa ggattcgcat catgcggcgc ggacgaccca      60

ctacggtgga ggtggaggct cactagtgcc cc                                    92


<210> 18
<211> 92
<212> DNA
<213> Artificial Sequence

<220>
<221> source
<223> /note="Description of Artificial Sequence: Synthetic
      primer"

<400> 18
ggggcactag tgagcctcca cctccaccgt agtgggtcgt ccgcgccgca tgatgcgaat      60

ccttgccgga ggagtctctg cccatggtaa ta                                    92


<210> 19
<211> 98
<212> DNA
<213> Artificial Sequence

<220>
<221> source
<223> /note="Description of Artificial Sequence: Synthetic
      primer"

<400> 19
tattaccatg ggcagagact ccaaactgga actgcagtcc gctctggaag aagctgaagc      60

ttccctggaa cacggaggtg gaggctcact agtgcccc                              98


<210> 20
<211> 98
<212> DNA
<213> Artificial Sequence

<220>
<221> source
<223> /note="Description of Artificial Sequence: Synthetic
      primer"

<400> 20
ggggcactag tgagcctcca cctccgtgtt ccagggaagc ttcagcttct tccagagcgg      60

actgcagttc cagtttggag tctctgccca tggtaata                              98


<210> 21
<211> 29
<212> DNA
<213> Artificial Sequence

<220>
<221> source
<223> /note="Description of Artificial Sequence: Synthetic

primer"

<400> 21
attaccatgg gggacacccg accacgttt                                          29


<210> 22
<211> 45
<212> DNA
<213> Artificial Sequence

<220>
<221> source
<223> /note="Description of Artificial Sequence: Synthetic
      primer"

<400> 22
ggatgatcac atgttcttct ttgatgactc gccgctgcac tgtga                        45


<210> 23
<211> 45
<212> DNA
<213> Artificial Sequence

<220>
<221> source
<223> /note="Description of Artificial Sequence: Synthetic
      primer"

<400> 23
tcacagtgca gcggcgagtc atcaaagaag aacatgtgat catcc                        45


<210> 24
<211> 92
<212> PRT
<213> Homo sapiens

<400> 24
Arg Pro Arg Phe Leu Trp Gln Leu Lys Phe Glu Cys His Phe Phe Asn
1               5                   10                  15


Gly Thr Glu Arg Val Arg Leu Leu Glu Arg Cys Ile Tyr Asn Gln Glu
            20                  25                  30


Glu Ser Val Arg Phe Asp Ser Asp Val Gly Glu Tyr Arg Ala Val Thr
        35                  40                  45


Glu Leu Gly Arg Pro Asp Ala Glu Tyr Trp Asn Ser Gln Lys Asp Leu
    50                  55                  60


Leu Glu Gln Arg Arg Ala Ala Val Asp Thr Tyr Cys Arg His Asn Tyr
65                  70                  75                  80


Gly Val Gly Glu Ser Phe Thr Val Gln Arg Arg Val
                85                  90


<210> 25
<211> 19
<212> PRT
<213> Artificial Sequence

<220>
<221> source
<223> /note="Description of Artificial Sequence: Synthetic
      peptide"

<400> 25
Gly Ser Leu Pro Gln Lys Ser Gln Arg Ser Gln Asp Glu Asn Pro Val
1               5                   10                  15

Val His Phe

<210> 26
<211> 15
<212> PRT
<213> Artificial Sequence

<220>
<221> source
<223> /note="Description of Artificial Sequence: Synthetic
      peptide"

<400> 26
Ser Gly Lys Asp Ser His His Ala Ala Arg Thr Thr His Tyr Gly
1               5                   10                  15

<210> 27
<211> 17
<212> PRT
<213> Artificial Sequence

<220>
<221> source
<223> /note="Description of Artificial Sequence: Synthetic
      peptide"

<400> 27
Lys Leu Glu Leu Gln Ser Ala Leu Glu Glu Ala Glu Ala Ser Leu Glu
1               5                   10                  15

His

<210> 28
<211> 95
<212> DNA
<213> Artificial Sequence

<220>
<221> source
<223> /note="Description of Artificial Sequence: Synthetic
      primer"

<400> 28
tattaccatg ggcagagact ccccacagaa gagccagagg tctcaggatg agaacccagt      60

ggtgcacttc ggaggtggag gctcactagt gcccc                                 95

<210> 29
<211> 94
<212> DNA
<213> Artificial Sequence

<220>
<221> source
<223> /note="Description of Artificial Sequence: Synthetic
      primer"

<400> 29
ggggcactag tgagcctcca cctccgaagt gcaccactgg gttctcatcc tgagacctct      60

ggctcttctg tggggagtct ctgcccatgg taat                                  94


<210> 30
<211> 19
<212> PRT
<213> Artificial Sequence

<220>
<221> source
<223> /note="Description of Artificial Sequence: Synthetic
      peptide"

<400> 30
Gly Ser Leu Pro Gln Lys Ser Gln Arg Thr Gln Asp Glu Asn Pro Val
1               5                   10                  15


Val His Phe



<210> 31
<211> 37
<212> DNA
<213> Artificial Sequence

<220>
<221> source
<223> /note="Description of Artificial Sequence: Synthetic
      primer"

<400> 31
tggtgctcga gttaattggt gatcggagta tagttgg                               37


<210> 32
<211> 20
<212> DNA
<213> Artificial Sequence

<220>
<221> source
<223> /note="Description of Artificial Sequence: Synthetic
      primer"

<400> 32
taatacgact cactataggg                                                  20


<210> 33
<211> 19
<212> DNA
<213> Artificial Sequence

<220>
<221> source
<223> /note="Description of Artificial Sequence: Synthetic
      primer"

<400> 33

gctagttatt gctcagcgg                                                    19


<210> 34
<211> 132
<212> DNA
<213> Artificial Sequence

<220>
<221> source
<223> /note="Description of Artificial Sequence: Synthetic
      primer"

<400> 34
aggctgccac aggaaacgtg ggcctccacc tccagagcct cggggcacta gtgagcctcc      60

acctccacgc ggggtaacga tgtttttgaa gaagtgaaca accgggtttt ctcgggtgtc     120

ccccatggta at                                                         132


<210> 35
<211> 20
<212> DNA
<213> Artificial Sequence

<220>
<221> source
<223> /note="Description of Artificial Sequence: Synthetic
      primer"

<400> 35
ccacgtttcc tgtggcagcc                                                  20


<210> 36
<211> 22
<212> DNA
<213> Artificial Sequence

<220>
<221> source
<223> /note="Description of Artificial Sequence: Synthetic
      primer"

<400> 36
tcaaagtcaa acataaactc gc                                               22


<210> 37
<211> 22
<212> DNA
<213> Artificial Sequence

<220>
<221> source
<223> /note="Description of Artificial Sequence: Synthetic
      primer"

<400> 37
gcgagtttat gtttgacttt ga                                               22


<210> 38
<211> 15
<212> PRT
<213> Artificial Sequence

<220>

<221> source
<223> /note="Description of Artificial Sequence: Synthetic
      peptide"

<400> 38
Glu Asn Pro Val Val His Phe Phe Lys Asn Ile Val Thr Pro Arg
1               5                   10                  15


<210> 39
<211> 17
<212> PRT
<213> Artificial Sequence

<220>
<221> source
<223> /note="Description of Artificial Sequence: Synthetic
      peptide"

<400> 39
Ala Thr Gly Phe Lys Gln Ser Ser Lys Ala Leu Gln Arg Pro Val Ala
1               5                   10                  15


Ser



<210> 40
<211> 13
<212> PRT
<213> Artificial Sequence

<220>
<221> source
<223> /note="Description of Artificial Sequence: Synthetic
      peptide"

<400> 40
His Ser Leu Gly Lys Trp Leu Gly His Pro Asp Lys Phe
1               5                   10


<210> 41
<211> 14
<212> PRT
<213> Artificial Sequence

<220>
<221> source
<223> /note="Description of Artificial Sequence: Synthetic
      peptide"

<400> 41
Asn Thr Trp Thr Thr Cys Gln Ser Ile Ala Phe Pro Ser Lys
1               5                   10


<210> 42
<211> 21
<212> PRT
<213> Artificial Sequence

<220>
<221> source
<223> /note="Description of Artificial Sequence: Synthetic
      peptide"

<400> 42

Met Glu Val Gly Trp Tyr Arg Ser Pro Phe Ser Arg Val Val His Leu
1               5                   10                  15

Tyr Arg Asn Gly Lys
            20

<210> 43
<211> 21
<212> PRT
<213> Artificial Sequence

<220>
<221> source
<223> /note="Description of Artificial Sequence: Synthetic
      peptide"

<400> 43
Val His Phe Phe Lys Asn Ile Val Thr Pro Arg Thr Pro Pro Pro Ser
1               5                   10                  15

Gln Gly Lys Gly Arg
            20

<210> 44
<211> 5
<212> PRT
<213> Artificial Sequence

<220>
<221> source
<223> /note="Description of Artificial Sequence: Synthetic
      peptide"

<400> 44
Gly Gly Gln Asp Asp
1               5

<210> 45
<211> 15
<212> PRT
<213> Artificial Sequence

<220>
<221> source
<223> /note="Description of Artificial Sequence: Synthetic
      peptide"

<400> 45
Gly Gly Gly Gly Ser Leu Val Pro Arg Gly Ser Gly Gly Gly Gly
1               5                   10                  15

<210> 46
<211> 20
<212> DNA
<213> Artificial Sequence

<220>
<221> source
<223> /note="Description of Artificial Sequence: Synthetic
      primer"

<400> 46
ggaaacccag aggcattgac                                                20

<210> 47
<211> 20
<212> DNA
<213> Artificial Sequence

<220>
<221> source
<223> /note="Description of Artificial Sequence: Synthetic
      primer"

<400> 47
tcaggatctg gcccttgaac                                                   20


<210> 48
<211> 21
<212> DNA
<213> Artificial Sequence

<220>
<221> source
<223> /note="Description of Artificial Sequence: Synthetic
      primer"

<400> 48
tgctgatggg aggagatgtc t                                                 21


<210> 49
<211> 20
<212> DNA
<213> Artificial Sequence

<220>
<221> source
<223> /note="Description of Artificial Sequence: Synthetic
      primer"

<400> 49
tgctgtctgg cctgctgtta                                                   20


<210> 50
<211> 20
<212> DNA
<213> Artificial Sequence

<220>
<221> source
<223> /note="Description of Artificial Sequence: Synthetic
      primer"

<400> 50
cagccgatgg gttgtacctt                                                   20


<210> 51
<211> 18
<212> DNA
<213> Artificial Sequence

<220>
<221> source
<223> /note="Description of Artificial Sequence: Synthetic
      primer"

<400> 51

ggcagccttg tcccttga                                                    18


<210> 52
<211> 18
<212> DNA
<213> Artificial Sequence

<220>
<221> source
<223> /note="Description of Artificial Sequence: Synthetic
      primer"

<400> 52
gatgccccag gcagagaa                                                    18


<210> 53
<211> 20
<212> DNA
<213> Artificial Sequence

<220>
<221> source
<223> /note="Description of Artificial Sequence: Synthetic
      primer"

<400> 53
cacccaggga attcaaatgc                                                  20


<210> 54
<211> 19
<212> DNA
<213> Artificial Sequence

<220>
<221> source
<223> /note="Description of Artificial Sequence: Synthetic
      primer"

<400> 54
ccacggcctt ccctacttc                                                   19


<210> 55
<211> 22
<212> DNA
<213> Artificial Sequence

<220>
<221> source
<223> /note="Description of Artificial Sequence: Synthetic
      primer"

<400> 55
tgggagtggt atcctctgtg aa                                               22


<210> 56
<211> 20
<212> DNA
<213> Artificial Sequence

<220>
<221> source
<223> /note="Description of Artificial Sequence: Synthetic
      primer"

<400> 56
gggacagatc ttgagcaagc                                                    20


<210> 57
<211> 19
<212> DNA
<213> Artificial Sequence

<220>
<221> source
<223> /note="Description of Artificial Sequence: Synthetic
      primer"

<400> 57
tgcagccttc ctccctctc                                                     19


<210> 58
<211> 22
<212> DNA
<213> Artificial Sequence

<220>
<221> source
<223> /note="Description of Artificial Sequence: Synthetic
      primer"

<400> 58
cctcaccatc atcctcactg ca                                                 22


<210> 59
<211> 22
<212> DNA
<213> Artificial Sequence

<220>
<221> source
<223> /note="Description of Artificial Sequence: Synthetic
      primer"

<400> 59
tcttctctgg gttggcacac ac                                                 22


<210> 60
<211> 18
<212> DNA
<213> Artificial Sequence

<220>
<221> source
<223> /note="Description of Artificial Sequence: Synthetic
      primer"

<400> 60
tgggctgctg tccctcaa                                                      18


<210> 61
<211> 19
<212> DNA
<213> Artificial Sequence

<220>
<221> source
<223> /note="Description of Artificial Sequence: Synthetic
      primer"

<400> 61
cccgggtgct gtttgtttt                                                    19


<210> 62
<211> 17
<212> DNA
<213> Artificial Sequence

<220>
<221> source
<223> /note="Description of Artificial Sequence: Synthetic
      primer"

<400> 62
cgatgacggg ccagtga                                                      17


<210> 63
<211> 20
<212> DNA
<213> Artificial Sequence

<220>
<221> source
<223> /note="Description of Artificial Sequence: Synthetic
      primer"

<400> 63
cgcagggatg atttcaagct                                                   20


<210> 64
<211> 20
<212> DNA
<213> Artificial Sequence

<220>
<221> source
<223> /note="Description of Artificial Sequence: Synthetic
      primer"

<400> 64
gggccctagc catcttagct                                                   20


<210> 65
<211> 20
<212> DNA
<213> Artificial Sequence

<220>
<221> source
<223> /note="Description of Artificial Sequence: Synthetic
      primer"

<400> 65
tcccactggg ccttaaaaaa                                                   20


<210> 66
<211> 25
<212> DNA
<213> Artificial Sequence

<220>
<221> source
<223> /note="Description of Artificial Sequence: Synthetic

primer"

<400> 66
gtgtacatag caacaagcct caaag                                          25


<210> 67
<211> 20
<212> DNA
<213> Artificial Sequence

<220>
<221> source
<223> /note="Description of Artificial Sequence: Synthetic
      primer"

<400> 67
cccccacata gggatcatga                                                20


<210> 68
<211> 18
<212> DNA
<213> Artificial Sequence

<220>
<221> source
<223> /note="Description of Artificial Sequence: Synthetic
      primer"

<400> 68
gggcaccacc ctgtgaaa                                                  18


<210> 69
<211> 18
<212> DNA
<213> Artificial Sequence

<220>
<221> source
<223> /note="Description of Artificial Sequence: Synthetic
      primer"

<400> 69
tggaggcagg agccatga                                                  18


<210> 70
<211> 23
<212> DNA
<213> Artificial Sequence

<220>
<221> source
<223> /note="Description of Artificial Sequence: Synthetic
      primer"

<400> 70
caattttcca gcaagacaat cct                                            23


<210> 71
<211> 23
<212> DNA
<213> Artificial Sequence

<220>
<221> source

<223> /note="Description of Artificial Sequence: Synthetic
      primer"

<400> 71
tctcctgtgg atcgggtata gac                                              23


<210> 72
<211> 21
<212> DNA
<213> Artificial Sequence

<220>
<221> source
<223> /note="Description of Artificial Sequence: Synthetic
      primer"

<400> 72
aagatgcctg gcttcctctg t                                                21


<210> 73
<211> 22
<212> DNA
<213> Artificial Sequence

<220>
<221> source
<223> /note="Description of Artificial Sequence: Synthetic
      primer"

<400> 73
ggtctgcctg gagatgtagc tt                                               22


<210> 74
<211> 19
<212> DNA
<213> Artificial Sequence

<220>
<221> source
<223> /note="Description of Artificial Sequence: Synthetic
      primer"

<400> 74
ccaggcacgc aactttgag                                                   19


<210> 75
<211> 21
<212> DNA
<213> Artificial Sequence

<220>
<221> source
<223> /note="Description of Artificial Sequence: Synthetic
      primer"

<400> 75
actaccacca cggcaatgat c                                                21


<210> 76
<211> 21
<212> DNA
<213> Artificial Sequence

<220>

<221> source
<223> /note="Description of Artificial Sequence: Synthetic
      primer"

<400> 76
ccagcgatct tcccattctt c                                                21


<210> 77
<211> 19
<212> DNA
<213> Artificial Sequence

<220>
<221> source
<223> /note="Description of Artificial Sequence: Synthetic
      primer"

<400> 77
gccctgcaca ctcccctta                                                   19


<210> 78
<211> 18
<212> DNA
<213> Artificial Sequence

<220>
<221> source
<223> /note="Description of Artificial Sequence: Synthetic
      primer"

<400> 78
ccgcttctgc tcccactc                                                    18


<210> 79
<211> 18
<212> DNA
<213> Artificial Sequence

<220>
<221> source
<223> /note="Description of Artificial Sequence: Synthetic
      primer"

<400> 79
ggtacctccc cctggctt                                                    18


<210> 80
<211> 24
<212> DNA
<213> Artificial Sequence

<220>
<221> source
<223> /note="Description of Artificial Sequence: Synthetic
      primer"

<400> 80
actgctcagc tacacaaagc aact                                             24


<210> 81
<211> 19
<212> DNA
<213> Artificial Sequence

<220>
<221> source
<223> /note="Description of Artificial Sequence: Synthetic
      primer"

<400> 81
tgagatgccc agggatggt                                                    19


<210> 82
<211> 20
<212> DNA
<213> Artificial Sequence

<220>
<221> source
<223> /note="Description of Artificial Sequence: Synthetic
      primer"

<400> 82
ggagatggat gtgccaaacg                                                   20


<210> 83
<211> 22
<212> DNA
<213> Artificial Sequence

<220>
<221> source
<223> /note="Description of Artificial Sequence: Synthetic
      primer"

<400> 83
cgagctcact ctctgtggtg tt                                                22


<210> 84
<211> 19
<212> DNA
<213> Artificial Sequence

<220>
<221> source
<223> /note="Description of Artificial Sequence: Synthetic
      primer"

<400> 84
ggcccttctc caggacaga                                                    19


<210> 85
<211> 20
<212> DNA
<213> Artificial Sequence

<220>
<221> source
<223> /note="Description of Artificial Sequence: Synthetic
      primer"

<400> 85
gctgatcatg gctgggttgt                                                   20


<210> 86
<211> 82
<212> PRT
<213> Homo sapiens

<400> 86
Glu Glu His Val Ile Ile Gln Ala Glu Phe Tyr Leu Asn Pro Asp Gln
1               5                   10                  15

Ser Gly Glu Phe Met Phe Asp Phe Asp Gly Asp Glu Ile Phe His Val
            20                  25                  30

Asp Met Ala Lys Lys Glu Thr Val Trp Arg Leu Glu Glu Phe Gly Arg
        35                  40                  45

Phe Ala Ser Phe Glu Ala Gln Gly Ala Leu Ala Asn Ile Ala Val Asp
    50                  55                  60

Lys Ala Asn Leu Glu Ile Met Thr Lys Arg Ser Asn Tyr Thr Pro Ile
65                  70                  75                  80

Thr Asn

<210> 87
<211> 92
<212> PRT
<213> Mus musculus

<400> 87
Arg Pro Trp Phe Leu Glu Tyr Cys Lys Ser Glu Cys His Phe Tyr Asn
1               5                   10                  15

Gly Thr Gln Arg Val Arg Leu Leu Val Arg Tyr Phe Tyr Asn Leu Glu
            20                  25                  30

Glu Asn Leu Arg Phe Asp Ser Asp Val Gly Glu Phe Arg Ala Val Thr
        35                  40                  45

Glu Leu Gly Arg Pro Asp Ala Glu Asn Trp Asn Ser Gln Pro Glu Phe
    50                  55                  60

Leu Glu Gln Lys Arg Ala Glu Val Asp Thr Val Cys Arg His Asn Tyr
65                  70                  75                  80

Glu Ile Phe Asp Asn Phe Leu Val Pro Arg Arg Val
                85                  90

<210> 88
<211> 82
<212> PRT
<213> Mus musculus

<400> 88
Glu Glu His Thr Ile Ile Gln Ala Glu Phe Tyr Leu Leu Pro Asp Lys
1               5                   10                  15

Arg Gly Glu Phe Met Phe Asp Phe Asp Gly Asp Glu Ile Phe His Val
            20                  25                  30

seqlisting

Asp Ile Glu Lys Ser Glu Thr Ile Trp Arg Leu Glu Glu Phe Ala Lys
        35                  40                  45

Phe Ala Ser Phe Glu Ala Gln Gly Ala Leu Ala Asn Ile Ala Val Asp
    50                  55                  60

Lys Ala Asn Leu Asp Val Met Lys Glu Arg Ser Asn Asn Thr Pro Asp
65                  70                  75                  80

Ala Asn

<210> 89
<211> 97
<212> PRT
<213> Rattus rattus

<400> 89
Met Gly Arg Asp Ser Pro Arg Asp Phe Val Tyr Gln Phe Lys Gly Leu
1               5                   10                  15

Cys Tyr Tyr Thr Asn Gly Thr Gln Arg Ile Arg Asp Val Ile Arg Tyr
            20                  25                  30

Ile Tyr Asn Gln Glu Glu Tyr Leu Arg Tyr Asp Ser Asp Val Gly Glu
        35                  40                  45

Tyr Arg Ala Leu Thr Glu Leu Gly Arg Pro Ser Ala Glu Tyr Trp Asn
    50                  55                  60

Ser Gln Lys Gln Tyr Leu Glu Gln Thr Arg Ala Glu Leu Asp Thr Val
65                  70                  75                  80

Cys Arg His Asn Tyr Glu Gly Ser Glu Val Arg Thr Ser Leu Arg Arg
                85                  90                  95

Leu

<210> 90
<211> 83
<212> PRT
<213> Rattus rattus

<400> 90
Ala Asp His Val Ala Ala Tyr Gly Ile Asn Met Tyr Gln Tyr Tyr Glu
1               5                   10                  15

Ser Arg Gly Gln Phe Thr His Glu Phe Asp Gly Asp Glu Glu Phe Tyr
            20                  25                  30

Val Asp Leu Asp Lys Lys Glu Thr Ile Trp Arg Ile Pro Glu Phe Gly
        35                  40                  45

Gln Leu Thr Ser Phe Asp Pro Gln Gly Gly Leu Gln Asn Ile Ala Ile
    50                  55                  60

Ile Lys His Asn Leu Glu Ile Leu Met Lys Arg Ser Asn Ser Thr Gln
65                  70                  75                  80

Ala Val Asn

<210> 91
<211> 84
<212> PRT
<213> Homo sapiens

<400> 91
Gly Ser His Ser Met Arg Tyr Phe Tyr Thr Ala Met Ser Arg Pro Gly
1               5                   10                  15

Arg Gly Glu Pro Arg Phe Ile Ala Val Gly Tyr Val Asp Asp Thr Gln
            20                  25                  30

Phe Val Arg Phe Asp Ser Asp Ala Ala Ser Pro Arg Thr Glu Pro Arg
        35                  40                  45

Pro Pro Trp Ile Glu Gln Glu Gly Pro Glu Tyr Trp Asp Arg Asn Thr
    50                  55                  60

Gln Ile Phe Lys Thr Asn Thr Gln Thr Tyr Arg Glu Asn Leu Arg Ile
65                  70                  75                  80

Ala Leu Arg Tyr

<210> 92
<211> 100
<212> PRT
<213> Homo sapiens

<400> 92
Tyr Asn Gln Ser Glu Ala Gly Ser His Ile Ile Gln Arg Met Tyr Gly
1               5                   10                  15

Cys Asp Leu Gly Pro Asp Gly Arg Leu Leu Arg Gly His Asp Gln Ser
            20                  25                  30

Ala Tyr Asp Gly Lys Asp Tyr Ile Ala Leu Asn Glu Asp Leu Ser Ser
        35                  40                  45

Trp Thr Ala Ala Asp Thr Ala Ala Gln Ile Thr Gln Arg Lys Trp Glu
    50                  55                  60

Ala Ala Arg Val Ala Glu Gln Leu Arg Ala Tyr Leu Glu Gly Leu Cys
65                  70                  75                  80

Val Glu Trp Leu Arg Arg Tyr Leu Glu Asn Gly Lys Glu Thr Leu Gln

85                  90                  95

Arg Ala Asp Pro
            100

<210> 93
<211> 641
<212> DNA
<213> Homo sapiens

<220>
<221> CDS
<222> (3)..(632)

<400> 93
cc atg ggg gac acc cga gaa aac ccg gtt gtt cac ttc ttc aaa aac      47
   Met Gly Asp Thr Arg Glu Asn Pro Val Val His Phe Phe Lys Asn
   1               5                   10                  15

atc gtt acc ccg cgt gga ggt gga ggc tca cta gtg ccc cga ggc tct      95
Ile Val Thr Pro Arg Gly Gly Gly Gly Ser Leu Val Pro Arg Gly Ser
                20                  25                  30

gga ggt gga ggc cca cgt ttc ctg tgg cag cct aag agg gag tgt cat      143
Gly Gly Gly Gly Pro Arg Phe Leu Trp Gln Pro Lys Arg Glu Cys His
            35                  40                  45

ttc ttc aat ggg acg gag cgg gtg cgg ttc ctg gac aga tac ttc tat      191
Phe Phe Asn Gly Thr Glu Arg Val Arg Phe Leu Asp Arg Tyr Phe Tyr
        50                  55                  60

aac cag gag gag tcc gtg cgc ttc gac agc gac gtg ggg gag ttc cgg      239
Asn Gln Glu Glu Ser Val Arg Phe Asp Ser Asp Val Gly Glu Phe Arg
    65                  70                  75

gcg gtg acg gag ctg ggg cgg cct gac gct gag tac tgg aac agc cag      287
Ala Val Thr Glu Leu Gly Arg Pro Asp Ala Glu Tyr Trp Asn Ser Gln
80                  85                  90                  95

aag gac atc ctg gag cag gcg cgg gcc gcg gtg gac acc tac tgc aga      335
Lys Asp Ile Leu Glu Gln Ala Arg Ala Ala Val Asp Thr Tyr Cys Arg
                100                 105                 110

cac aac tac ggg gtt gtg gag agc ttc aca gtg cag cgg cga gtc atc      383
His Asn Tyr Gly Val Val Glu Ser Phe Thr Val Gln Arg Arg Val Ile
            115                 120                 125

aaa gaa gaa cat gtg atc atc cag gcc gag ttc tat ctg aat cct gac      431
Lys Glu Glu His Val Ile Ile Gln Ala Glu Phe Tyr Leu Asn Pro Asp
        130                 135                 140

caa tca ggc gag ttt atg ttt gac ttt gat ggt gat gag att ttc cat      479
Gln Ser Gly Glu Phe Met Phe Asp Phe Asp Gly Asp Glu Ile Phe His
    145                 150                 155

gtg gat atg gca aag aag gag acg gtc tgg cgg ctt gaa gaa ttt gga      527
Val Asp Met Ala Lys Lys Glu Thr Val Trp Arg Leu Glu Glu Phe Gly
160                 165                 170                 175

cga ttt gcc agc ttt gag gct caa ggt gca ttg gcc aac ata gct gtg      575
Arg Phe Ala Ser Phe Glu Ala Gln Gly Ala Leu Ala Asn Ile Ala Val
                180                 185                 190

gac aaa gcc aac ttg gaa atc atg aca aag cgc tcc aac tat act ccg      623
Asp Lys Ala Asn Leu Glu Ile Met Thr Lys Arg Ser Asn Tyr Thr Pro
            195                 200                 205

atc acc aat taactcgag                                                    641
Ile Thr Asn
        210


<210> 94
<211> 210
<212> PRT
<213> Homo sapiens

<400> 94
Met Gly Asp Thr Arg Glu Asn Pro Val Val His Phe Phe Lys Asn Ile
1               5                   10                  15

Val Thr Pro Arg Gly Gly Gly Gly Ser Leu Val Pro Arg Gly Ser Gly
            20                  25                  30

Gly Gly Gly Pro Arg Phe Leu Trp Gln Pro Lys Arg Glu Cys His Phe
        35                  40                  45

Phe Asn Gly Thr Glu Arg Val Arg Phe Leu Asp Arg Tyr Phe Tyr Asn
    50                  55                  60

Gln Glu Glu Ser Val Arg Phe Asp Ser Asp Val Gly Glu Phe Arg Ala
65                  70                  75                  80

Val Thr Glu Leu Gly Arg Pro Asp Ala Glu Tyr Trp Asn Ser Gln Lys
                85                  90                  95

Asp Ile Leu Glu Gln Ala Arg Ala Ala Val Asp Thr Tyr Cys Arg His
            100                 105                 110

Asn Tyr Gly Val Val Glu Ser Phe Thr Val Gln Arg Arg Val Ile Lys
        115                 120                 125

Glu Glu His Val Ile Ile Gln Ala Glu Phe Tyr Leu Asn Pro Asp Gln
    130                 135                 140

Ser Gly Glu Phe Met Phe Asp Phe Asp Gly Asp Glu Ile Phe His Val
145                 150                 155                 160

Asp Met Ala Lys Lys Glu Thr Val Trp Arg Leu Glu Glu Phe Gly Arg
                165                 170                 175

Phe Ala Ser Phe Glu Ala Gln Gly Ala Leu Ala Asn Ile Ala Val Asp
            180                 185                 190

Lys Ala Asn Leu Glu Ile Met Thr Lys Arg Ser Asn Tyr Thr Pro Ile
        195                 200                 205

Thr Asn
    210


<210> 95

<211> 5
<212> PRT
<213> Artificial Sequence

<220>
<221> source
<223> /note="Description of Artificial Sequence: Synthetic
      peptide"

<400> 95
Gly Gly Gly Gly Ser
1               5